# EUROPEAN PATENT SPECIFICATION

(11) **EP 1 708 741 B1**
(45) Date of publication and mention of the grant of the patent: **30.03.2016**
(21) Application number: 04821230.2
(22) Date of filing: 23.12.2004
(51) Int. Cl.: A61K 39/00

(54) **RECOMBINANT NUCLEIC ACID MOLECULES ENCODING FUSION PROTEINS COMPRISING ANTIGENS AND BACTERIAL SECRETORY SIGNAL POLYPEPTIDES, EXPRESSION CASSETTES, AND BACTERIA, AND METHODS OF USE THEREOF**
FUSIONSPROTEINE KODIERENDE REKOMBINANTE NUCLEINSÄURE-MOLEKÜLE MIT ANTIGENEN UND BAKTERIELLEN SEKRETORISCHEN SIGNAL-POLYPEPTIDEN, EXPRESSIONSKASSETTEN UND BAKTERIEN UND ANWENDUNGSVERFAHREN DAFÜR
MOLECULES D'ACIDES NUCLEIQUES CODANT POUR DES PROTEINES DE FUSION COMPRENANT DES ANTIGENES ET DES POLYPEPTIDES DE SIGNAL BACTERIENS, CASSETTES D'EXPRESSION, ET BACTERIES, ET LEUR METHODES D'UTILISATION

(30) Priority: 24.12.2003 US 532598 P; 02.02.2004 US 541515 P; 06.02.2004 US 773792; 06.02.2004 US 773618; 26.03.2004 US 556744 P; 30.06.2004 US 883599; 23.07.2004 WO PCT/US2004/023881; 05.08.2004 US 599377 P; 01.10.2004 US 615287 P; 06.10.2004 US 616750 P
(43) Date of publication of application: 11.10.2006
(73) Proprietor: Aduro Biotech, Berkeley, California 94710-2225 (US)
(72) Inventor: DUBENSKY, Thomas, W., Jr., Piedmont, CA 94611 (US); PORTNOY, Daniel, A., Albany, CA 94706 (US); LUCKETT, William, S., Jr., Richmond, CA 94805 (US); COOK, David, N., Lafayette, CA 94549 (US)
(74) Representative: Brasnett, Adrian Hugh
(86) International application number: PCT/US2004/044080
(87) International publication number: WO 2005/071088

(56) References cited:
- WO-A-01/27295
- WO-A-02/33109
- WO-A-03/083056
- WO-A-2004/084636
- WO-A1-01/76642
- FR-A- 2 686 896
- US-A- 5 830 702
- GUZMÁN C A ET AL: "Attenuated Listeria monocytogenes carrier strains can deliver an HIV-1 gp120 T helper epitope to MHC class II-restricted guman CD4+ T cells" EUROPEAN JOURNAL OF IMMUNOLOGY, WEINHEIM, DE, vol. 28, June 1998 (1998-06), pages 1807-1814, XP002104786 ISSN: 0014-2980
- SHEN HAO ET AL: "Recombinant Listeria monocytogenes as a live vaccine vehicle for the induction of protective anti-viral cell-mediated immunity" PROCEEDINGS OF THE NATIONAL ACADEMY OF SCIENCES OF THE UNITED STATES OF AMERICA, vol. 92, no. 9, 1995, pages 3987-3991, XP002338965 ISSN: 0027-8424
- LENZ LAUREL L ET AL: "SecA2-dependent secretion of autolytic enzymes promotes Listeria monocytogenes pathogenesis." PROCEEDINGS OF THE NATIONAL ACADEMY OF SCIENCES OF THE UNITED STATES OF AMERICA, vol. 100, no. 21, 14 October 2003 (2003-10-14), pages 12432-12437, XP002338966 ISSN: 0027-8424
- BROCKSTEDT D G ET AL: "Listeria-based cancer vaccines that segregate immunogenicity from toxicity" PROCEEDINGS OF THE NATIONAL ACADEMY OF SCIENCES OF USA, NATIONAL ACADEMY OF SCIENCE. WASHINGTON, US, vol. 101, no. 38, 21 September 2004 (2004-09-21), pages 13832-13837, XP002306827 ISSN: 0027-8424
- Shubhra Gupta ET AL: "Project Report Codon Optimization", , 5 March 2003 (2003-03-05), XP055169157, Retrieved from the Internet: URL:http://www.guptalab.org/shubhg/pdf/shu bhra_codon.pdf [retrieved on 2015-02-11]
- PARK S ET AL: "Optimized Production and Purification of Bacillus anthracis Lethal Factor", PROTEIN EXPRESSION AND PURIFICATION, ACADEMIC PRESS, SAN DIEGO, CA, vol. 18, no. 3, 1 April 2000 (2000-04-01), pages 293-302, XP004435542, ISSN: 1046-5928, DOI: 10.1006/PREP.2000.1208
- STEIDLER L ET AL: "Biological containment of genetically modified Lactococcus lactis for intestinal delivery of human interleukin 10", NATURE BIOTECHNOLOGY, NATURE PUBLISHING GROUP, NEW YORK, NY, US, vol. 21, no. 7, 1 July 2003 (2003-07-01), pages 785-789, XP002276104, ISSN: 1087-0156, DOI: 10.1038/NBT840
- ADAM EYRE-WALKER AND MICHAEL BULMER: "Reduced synonymous substitution rate at the start of enterobacterial genes", NUCLEIC ACIDS RESEARCH, INFORMATION RETRIEVAL LTD, vol. 21, no. 19, 25 September 1993 (1993-09-25), pages 4599-4603, XP009182547, ISSN: 0305-1048, DOI: 10.1093/NAR/21.19.4599
- POP O ET AL: "The twin-arginine sognal peptide of PhoD and the TatAd/Cd proteins of Bacilus subtilis from an autonomous Tat translocation system", JOURNAL OF BIOLOGICAL CHEMISTRY, AMERICAN SOCIETY FOR BIOCHEMISTRY AND MOLECULAR BIOLOGY, US, vol. 277, no. 5, 1 February 2002 (2002-02-01), pages 3268-3273, XP002989191, ISSN: 0021-9258, DOI: 10.1074/JBC.M110829200
- GUZMÁN C A ET AL: "Attenuated Listeria monocytogenes carrier strains can deliver an HIV-1 gp120 T helper epitope to MHC class II-restricted guman CD4+ T cells", EUROPEAN JOURNAL OF IMMUNOLOGY, WILEY - V C H VERLAG GMBH & CO. KGAA, DE, vol. 28, 1 June 1998 (1998-06-01), pages 1807-1814, XP002104786, ISSN: 0014-2980, DOI: 10.1002/(SICI)1521-4141(199806)28:06<1807: :AID-IMMU1807>3.0.CO;2-W
- Dennis M. Burns ET AL: "Rare codons in E. coli and S. typhimurium signal sequences", FEBS Letters, vol. 189, no. 2, 1 September 1985 (1985-09-01), pages 318-324, XP055169170, ISSN: 0014-5793, DOI: 10.1016/0014-5793(85)81048-6
- WELKOS S L ET AL: "Sequence and analysis of the DNA encoding protective antigen of Bacillus anthracis", GENE, ELSEVIER, AMSTERDAM, NL, vol. 69, no. 2, 30 September 1988 (1988-09-30), pages 287-300, XP023543885, ISSN: 0378-1119, DOI: 10.1016/0378-1119(88)90439-8 [retrieved on 1988-09-30]
- JACONO-CONNORS L C ET AL: "PROTECTION AGAINST ANTHRAX WITH RECOMBINANT VIRUS-EXPRESSED PROTECTIVE ANTIGEN IN EXPERIMENTAL ANIMALS", INFECTION AND IMMUNITY, AMERICAN SOCIETY FOR MICROBIOLOGY, US, vol. 59, no. 6, 1 January 1991 (1991-01-01), pages 1961-1965, XP000916157, ISSN: 0019-9567

## Description

### FIELD OF THE INVENTION

The field of this invention relates generally to novel polynucleotides and expression cassettes useful for expression of polypeptides, including heterologous polypeptides, in recombinant bacteria. In particular, this invention relates to recombinant bacteria comprising the novel expression cassettes and/or nucleic acid molecules which are useful in vaccine compositions.

### BACKGROUND OF THE INVENTION

Microbes have begun to be developed for use as vaccines that deliver heterologous antigens. Heterologous antigen delivery is provided by microbes that have been modified to contain nucleic acid sequences encoding a protein or antigen originating from a different species. Heterologous antigen delivery is especially advantageous for treating or preventing diseases or conditions that result from especially virulent or lethal sources, such as cancer and pathogenic agents (for example, HIV or Hepatitis B), wherein injection of a native infectious agent or cancer cell is potentially deleterious to the recipient organism, and administration of attenuated or killed agent or cell has proven unsuccessful in eliciting an effective immune response, or where sufficient attenuation of the infectious agent or cancer cell cannot be assured with acceptable certainty. Recently, certain bacterial strains have been developed as recombinant vaccines. For instance, an oral vaccine of attenuated *Salmonella* modified to express *Plasmodium berghei* circumsporozite antigen has been shown to protect mice against malaria (Aggarwal et al. 1990. J. Exp. Med. 172:1083).

*Listeria monocytogenes* (*Listeria*) is a Gram-positive facultative intracellular bacterium that is being developed for use in antigen-specific vaccines due to its ability to prime a potent CD4+/CD8+ T-cell mediated response via both MHC class I and class II antigen presentation pathways. See, for instance, U.S. Patent Nos. 6,051,237, 6,565,852, and 5,830,702.

*Listeria* has been studied for a number of years as a model for stimulating both innate and adaptive T cell-dependent antibacterial immunity. The ability of *Listeria* to effectively stimulate cellular immunity is based on its intracellular lifecycle. Upon infecting the host, the bacterium is rapidly taken up by phagocytes including macrophages and dendritic cells (DC) into a phagolysosomal compartment. The majority of the bacteria are subsequently degraded. Peptides resulting from proteolytic degradation of pathogens within phagosomes of infected APCs are loaded directly onto MHC class II molecules, and the processed antigens are expressed on the surface of the antigen presenting cell via the class II endosomal pathway, and these MHC II-peptide complexes activate CD4+ "helper" T cells that stimulate the production of antibodies. Within the acidic compartment, certain bacterial genes are activated including the cholesterol-dependent cytolysin, LLO, which can degrade the phagolysosome, releasing the bacterium into the cytosolic compartment of the host cell, where the surviving *Listeria* propagate. Efficient presentation of heterologous antigens via the MHC class I pathway requires de novo endogenous protein expression by *Listeria.* Within the cytoplasm of antigen presenting cells (APC), proteins synthesized and secreted by *Listeria* are sampled and degraded by the proteosome. The resulting peptides are shuttled into the endoplasmic reticulum by TAP proteins and loaded onto MHC class I molecules. The MHC I-peptide complex is delivered to the cell surface, which in combination with sufficient co-stimulation (signal2) activates and stimulates cytotoxic T lymphocytes (CTLs) having the cognate T cell receptor to expand and subsequently recognize the MHC I-peptide complex displayed on, for example tumor cells. In the appropriate microenvironment, the activated T cell targets and kills the cancerous cell.

Given the mechanisms by which *Listeria* programs the presentation of heterologous antigens via the MHC class I pathway, the efficiency of both expression of heterologous genes and secretion of the newly synthesized protein from the bacterium into the cytoplasm of the infected (antigen presenting) cell is directly related to the potency of CD8+ T cell priming and/or activation. Since the level of Ag-specific T cell priming is directly related to vaccine efficacy, the efficiency of heterologous protein expression and secretion is linked directly to vaccine potency.

Thus, novel methods are needed in the art to optimize the efficiency of heterologous protein expression and secretion to maximize the potency of *Listeria-based* vaccines and other bacteria-based vaccines. It would also be beneficial to optimize the efficiency of heterologous protein expression and secretion in bacterial host expression systems where expression and secretion of large quantities of heterologous protein is desired.

Guzman et al (European Journal Of Immunology, vol. 28, June 1998, pages 1807-1814 describe attenuated Listeria monocytogenes carrier strains which can deliver an HIV-1 gp120 T helper epitope to MHC class II-restricted guman CD4+ T cells. Shen et al Proceedings Of The National Academy Of Sciences Of The United States Of America, vol. 92, no. 9, 1995, pages 3987-3991, describe recombinant Listeria monocytogenes as a live vaccine vehicle for the induction of protective anti-viral cell-mediated immunity. US 5,830,702 describes vaccines comprising an attenuated strain of Listeria spp. expressing a selected foreign antigen and methods of eliciting protective immunity by administering an effective amount of the vaccine. WO 01/27295 describes *Listeria* expression vectors for expressing the human tumor antigens tyrosinase, Trp-1, MelanA/MART-1 and Trp-2 or antigen epitopes derived therefrom, and to attenuated listeria bacteria containing these expression vectors.

### BRIEF SUMMARY OF THE INVENTION

The present disclosure generally relates to novel polynucleotides including novel recombinant nucleic acid molecules, expression cassettes, and vectors for use in expressing and/or secreting polypeptides (e.g. heterologous polypeptides) in bacteria, especially *Listeria.* In some embodiments, these polynucleotides provide enhanced expression and/or secretion of polypeptides in bacteria. The present disclosure also generally relates to bacteria comprising the recombinant nucleic acid molecules, expression cassettes, or vectors, as well as pharmaceutical, immunogenic, and vaccine compositions comprising the bacteria. These bacteria and compositions are useful in the induction of immune responses and in the treatment and/or prevention of a wide array of diseases or other conditions, including cancer, infections and autoimmunity.

The present invention provides a recombinant *Listeria* bacterium comprising a recombinant nucleic acid molecule, wherein the recombinant nucleic acid molecule comprises: (a) a first polynucleotide encoding a secretory signal peptide that is a signal peptide native to *Listeria*; wherein the first polynucleotide is codon-optimized for expression in the *Listeria* bacterium such that the codons encoding said signal peptide sequence are the codons of Table 3:

| Amino Acid | Optimal *Listeria* Codon |
|---|---|
| Alanine | GCA |
| Arginine | CGT |
| Asparagine | AAT |
| Aspartate | GAT |
| Cysteine | TGT |
| Glutamine | CAA |
| Glutamate | GAA |
| Glycine | GGT |
| Histidine | CAT |
| Isoleucine | ATT |
| Leucine | TTA |
| Lysine | AAA |
| Methionine | ATG |
| Phenylalanine | TTT |
| Proline | CCA |
| Serine | AGT |
| Threonine | ACA |
| Tryptophan | TGG |
| Tyrosine | TAT |
| Valine | GTT |

and (b) a second polynucleotide sequence encoding a polypeptide heterologous to the signal peptide, wherein the second polynucleotide sequence is in the same translational reading frame as the first polynucleotide sequence, wherein the recombinant nucleic acid molecule encodes a fusion protein comprising the signal peptide and the polypeptide.

The invention further provides method of making a recombinant *Listeria* bacterium comprising a recombinant nucleic acid molecule, wherein the recombinant nucleic acid molecule comprises: (a) a first polynucleotide encoding a secretory signal peptide that is a signal peptide native to *Listeria*; and (b) a second polynucleotide sequence encoding a polypeptide heterologous to the signal peptide, wherein the second polynucleotide sequence is in the same translational reading frame as the first polynucleotide sequence, wherein the recombinant nucleic acid molecule encodes a fusion protein comprising the signal peptide and the polypeptide; wherein said method comprises (i) providing said recombinant nucleic acid molecule and codon-optimizing the first polynucleotide to replace at least one codon of the native coding sequence of the polynucleotide with a codon that is more frequently used by Listeria, and (ii) expressing said recombinant nucleic acid molecule in the Listeria bacterium.

Further aspects of the invention are defined in the accompanying claims.

In one aspect, described herein is a recombinant nucleic acid molecule, comprising a first polynucleotide encoding a signal peptide, wherein the first polynucleotide is codon-optimized for expression in a bacterium, and a second polynucleotide encoding a polypeptide (e.g., an antigen), wherein the second polynucleotide is in the same translational reading frame as the first polynucleotide, and wherein the recombinant nucleic acid molecule encodes a fusion protein comprising the signal peptide and the polypeptide. In some embodiments, the second polynucleotide is also codon-optimized for expression in bacteria, such as *Listeria.* This disclosure also provides expression cassettes comprising this recombinant nucleic acid molecule and further comprising a promoter operably linked to the recombinant nucleic acid molecule. Vectors and bacteria comprising the recombinant nucleic acid molecules and/or expression cassette are also provided, as are pharmaceutical compositions, immunogenic compositions, and vaccines comprising the bacteria. Methods of using the bacteria or compositions comprising the bacteria to induce immune responses and/or to prevent or treat a condition such as a disease in a host are also provided.

In another aspect, described herein is a recombinant nucleic acid molecule, comprising (a) a first polynucleotide encoding a signal peptide native to a bacterium, wherein the first polynucleotide is codon-optimized for expression in the bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the polypeptide encoded by the second polynucleotide is heterologous to the signal peptide. In some embodiments, the polypeptide encoded by the second polynucleotide is foreign to the bacterium. This disclosure also provides expression cassettes comprising this recombinant nucleic acid molecule and further comprising a promoter operably linked to the recombinant nucleic acid molecule. Vectors and bacteria comprising the recombinant nucleic acid molecule and/or expression cassette are also provided, as are pharmaceutical compositions, immunogenic compositions, and vaccines comprising the bacteria. Methods of using the bacteria or compositions comprising the bacteria to induce an immune response and/or to prevent or treat a condition (e.g., a disease) in a host are also provided.

In another aspect, described herein is a recombinant *Listeria* bacterium comprising a recombinant nucleic acid molecule, wherein the recombinant nucleic acid molecule comprises (a) a first polynucleotide encoding a signal peptide, wherein the first polynucleotide is codon-optimized for expression in *Listeria,* and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the polypeptide encoded by the second polynucleotide is heterologous to the signal peptide. In some embodiments, the polypeptide is foreign to the *Listeria* bacterium. In some embodiments, the signal peptide is native to *Listeria.* Pharmaceutical compositions, immunogenic compositions, and vaccines, comprising the *Listeria* are also provided. Methods of using the *Listeria* (or compositions comprising the *Listeria*) to induce an immune response and/or to prevent or treat a condition (e.g., a disease) in a host are also provided.

In another aspect, described herein is a recombinant nucleic acid molecule, comprising a first polynucleotide encoding a non-secA1 bacterial signal peptide, and a second polynucleotide encoding a polypeptide (such as an antigen), wherein the second polynucleotide is in the same translational reading frame as the first polynucleotide, and wherein the recombinant nucleic acid molecule encodes a fusion protein comprising the signal peptide and the polypeptide. In some embodiments, the polypeptide is heterologous to the signal peptide. In some embodiments, the first and/or second polynucleotides are codon-optimized for expression in bacteria, such as *Listeria.* This disclosure also provides expression cassettes comprising this recombinant nucleic acid molecule and further comprising a promoter operably linked to the recombinant nucleic acid molecule. Vectors and bacteria comprising the recombinant nucleic acid molecule and/or expression cassette are also provided, as are pharmaceutical compositions, immunogenic compositions, and vaccines comprising the bacteria. Methods of using the bacteria or compositions comprising the bacteria to induce immune responses and/or to treat a condition such as a disease in a host are also provided.

In still another aspect, described herein is a recombinant *Listeria* bacterium comprising a recombinant nucleic acid molecule, wherein the recombinant nucleic acid molecule comprises (a) a first polynucleotide encoding a non-secA1 bacterial signal peptide, and (b) a second polynucleotide encoding a polypeptide either heterologous to the signal peptide or foreign to the bacterium, wherein the second polynucleotide is in the same translational reading frame as the first polynucleotide, wherein the recombinant nucleic acid molecule encodes a fusion protein comprising the signal peptide and the polypeptide. In some embodiments, the polypeptide encoded by the second polynucleotide is heterologous to the signal peptide or foreign to the bacterium (i.e., heterologous to the bacterium), or both. Pharmaceutical compositions, immunogenic compositions, and vaccines comprising the *Listeria* are also provided. Methods of using the *Listeria* (or compositions comprising the *Listeria*) to induce an immune response and/or to prevent or treat a condition (e.g., a disease) in a host are also provided.

In another aspect, described herein is a recombinant nucleic acid molecule, wherein the recombinant nucleic acid molecule comprises a polynucleotide encoding a polypeptide foreign to *Listeria* (e.g., a cancer or non-Listerial infectious disease antigen), wherein the polynucleotide encoding the foreign polypeptide is codon-optimized for expression in *Listeria.* In some embodiments, the recombinant nucleic acid molecule further comprises a polynucleotide that encodes a signal peptide in the same translational reading frame as the polynucleotide encoding the polypeptide foreign to *Listeria.* In some embodiments, the signal peptide is native to the *Listeria* bacterium. In other embodiments, the signal peptide is foreign to the *Listeria* bacterium. In some embodiments, the polynucleotide encoding the signal peptide is also codon-optimized for expression in *Listeria. Listeria* comprising the recombinant nucleic acid molecule are also provided. Pharmaceutical compositions, immunogenic compositions, and vaccines comprising the *Listeria* are also provided. In addition, described herein are methods of using the recombinant *Listeria* bacteria to induce immune responses and/or to prevent or treat a condition (such as, but not limited to, a disease) in a host.

In another aspect, described herein is a recombinant *Listeria* bacterium comprising an expression cassette, wherein the expression cassette comprises a polynucleotide encoding a polypeptide foreign to *Listeria* (e.g., a cancer or non-Listerial infectious disease antigen), wherein the polynucleotide encoding the foreign polypeptide is codon-optimized for expression in *Listeria,* and a promoter, operably linked to the polynucleotide encoding the foreign polypeptide. In some embodiments, the expression cassette further comprises a polynucleotide that encodes a signal peptide (a signal peptide either native or foreign to the *Listeria* bacterium) in the same translational reading frame as the polynucleotide encoding the polypeptide foreign to *Listeria* and operably linked to the promoter so that the expression cassette expresses a fusion protein comprising the signal peptide and the foreign polypeptide. In some embodiments, the polynucleotide encoding the signal peptide is also codon-optimized for expression in *Listeria.* Pharmaceutical compositions, immunogenic compositions, and vaccines comprising the *Listeria* are also provided. In addition, described herein are methods of using the recombinant *Listeria* bacteria to induce immune responses and/or to prevent or treat a condition (e.g., a disease) in a host.

In another aspect, described herein is a recombinant nucleic acid molecule, wherein the recombinant nucleic acid molecule comprises (a) a first polynucleotide encoding a non-Listerial signal peptide, and (b) a second polynucleotide encoding a polypeptide that is in the same translational reading frame as the first polynucleotide, wherein the recombinant nucleic acid molecule encodes a fusion protein comprising both the non-Listerial signal peptide and the polypeptide. This disclosure also provides an expression cassette comprising the recombinant nucleic acid molecule, wherein the expression cassette further comprises a promoter operably linked to the first and second polynucleotides of the recombinant nucleic acid molecule. Vectors comprising the recombinant nucleic acid molecule and/or the expression cassette are also provided. In addition, a *Listeria* bacterium comprising the recombinant nucleic acid molecule and/or the expression cassette is also provided. Pharmaceutical compositions, immunogenic compositions, and vaccines, comprising the *Listeria* bacterium are also provided. Methods of using the *Listeria* bacterium (or compositions comprising the *Listeria* bacterium) to induce an immune response and/or to prevent or treat a condition (e.g., a disease) in a host are also provided.

In a further aspect, described herein is a recombinant *Listeria* bacterium comprising a recombinant nucleic acid molecule, wherein the recombinant nucleic acid molecule comprises (a) a first polynucleotide encoding a non-Listerial signal peptide, and (b) a second polynucleotide encoding a polypeptide that is in the same translational reading frame as the first polynucleotide, wherein the recombinant nucleic acid molecule encodes a fusion protein comprising both the non-Listerial signal peptide and the polypeptide. Pharmaceutical compositions, immunogenic compositions, and vaccines, comprising the *Listeria* are also provided. Methods of using the *Listeria* (or compositions comprising the *Listeria*) to induce an immune response and/or to prevent or treat a condition (e.g., a disease) in a host are also provided.

In still another aspect, described herein is a *Listeria* bacterium (for instance, from the species *Listeria monocytogenes*) comprising an expression cassette which comprises a first polynucleotide encoding a non*-Listerial* signal peptide, a second polynucleotide encoding a polypeptide (e.g., an antigen) that is in the same translational reading frame as the first polynucleotide, and a promoter operably linked to both the first and second polynucleotides. The expression cassette encodes a fusion protein comprising both the non*-Listerial* signal peptide and the polypeptide. In some embodiments, the first and/or second polynucleotides are codon-optimized for expression in *Listeria.* Pharmaceutical compositions, immunogenic compositions, and vaccines comprising the *Listeria* are also provided. In addition, described herein are methods of using the recombinant *Listeria* bacteria to induce immune responses and/or to prevent or treat a condition (e.g., a disease) in a host.

This disclosure also provides a recombinant nucleic acid molecule, comprising (a) a first polynucleotide encoding a bacterial autolysin, or a catalytically active fragment or catalytically active variant thereof, and (b) a second polynucleotide encoding a polypeptide, wherein the second polynucleotide is in the same translational reading flame as the first polynucleotide, wherein the recombinant nucleic acid molecule encodes a protein chimera comprising the polypeptide encoded by the second polynucleotide and the autolysin, or catalytically active fragment or catalytically active variant thereof, wherein, in the protein chimera, the polypeptide is fused to or is positioned within the autolysin, or catalytically active fragment or catalytically active variant thereof. Vectors and bacteria comprising the recombinant nucleic acid molecule and/or expression cassette are also provided, as are pharmaceutical compositions, immunogenic compositions, and vaccines, comprising the bacteria. Methods of using the bacteria or compositions comprising the bacteria to induce immune responses and/or to treat a condition such as a disease in a host are also provided.

In another aspect, described herein is a recombinant nucleic acid molecule, wherein the nucleic acid molecule encodes at least two discrete non-Listerial polypeptides. This disclosure further provides an expression cassette comprising the recombinant nucleic acid molecules and further comprising a promoter, wherein the promoter is operably linked to the recombinant nucleic acid molecule. Vectors comprising the recombinant nucleic acid molecule and/or expression cassette are further provides. In addition a recombinant *Listeria* bacterium comprising the recombinant nucleic acid molecule (and/or the expression cassette) is also provided. Pharmaceutical compositions, immunogenic compositions, and vaccines, comprising the *Listeria* are also provided. Methods of using the *Listeria* (or compositions comprising the *Listeria*) to induce an immune response and/or to prevent or treat a condition (e.g., a disease) in a host are also provided.

In an additional aspect, described herein is a recombinant *Listeria* bacterium comprising a polycistronic expression cassette, wherein the polycistronic expression cassette encodes at least two discrete non-Listerial polypeptides. Pharmaceutical compositions, immunogenic compositions, and vaccines, comprising the *Listeria* are also provided. Methods of using the *Listeria* (or compositions comprising the *Listeria*) to induce an immune response and/or to prevent or treat a condition (e.g., a disease) in a host are also provided.

In other aspects, described herein is a recombinant nucleic acid molecule, comprising (a) a first polynucleotide encoding a signal peptide, (b) a second polynucleotide encoding a secreted protein, or a fragment thereof, wherein the second polynucleotide is in the same translational reading frame as the first polynucleotide, and (c) a third polynucleotide encoding a polypeptide heterologous to the secreted protein, or fragment thereof, wherein the third polynucleotide is in the same translational reading frame as the first and second polynucleotides, wherein the recombinant nucleic acid molecule encodes a protein chimera comprising the signal peptide, the polypeptide encoded by the third polynucleotide, and the secreted protein, or fragment thereof, and wherein the polypeptide encoded by the third polynucleotide is fused to the secreted protein, or fragment thereof, or is positioned within the secreted protein, or fragment thereof, in the protein chimera. An expression cassette comprising the recombinant nucleic acid molecule and further comprising a promoter operably linked to the first, second, and third polynucleotides of the recombinant nucleic acid molecule is also provided. Vectors and bacteria comprising the recombinant nucleic acid molecule and/or expression cassette are also provided, as are pharmaceutical compositions, immunogenic compositions, and vaccines, comprising the bacteria. Methods of using the bacteria or compositions comprising the bacteria to induce an immune response and/or to prevent or treat a condition in a host are also provided.

In some embodiments, the methods of inducing an immune response in a host to an antigen comprise administering to the host an effective amount of a composition comprising a recombinant bacterium described herein (e.g., in any of the aspects above, or in the Detailed Description of the Invention or Examples, below) to the host, wherein a polypeptide encoded by the recombinant nucleic acid molecule, expression cassette, and/or vector in the bacterium comprises the antigen. In some embodiments, the methods of preventing or treating a condition, such as a disease, in a host comprise administering to the host an effective amount of a composition comprising a recombinant bacterium described herein to the host.

This disclosure further provides the use of a recombinant bacterium described herein (e.g., in any of the aspects above, or in the Detailed Description of the Invention or Examples, below) in the manufacture of a medicament for inducing an immune response in a host to an antigen, wherein a polypeptide encoded by the recombinant nucleic acid molecule, expression cassette, and/or vector in the bacterium comprises the antigen. In some embodiments, the antigen is a heterologous antigen. This disclosure also provides the use of a recombinant bacterium described herein in the manufacture of a medicament for preventing or treating a condition in a host (e.g., a disease such as cancer or an infectious disease). This disclosure further provides the recombinant bacteria described herein for use in inducing an immune response in a host to an antigen, wherein a polypeptide encoded by the recombinant nucleic acid molecule, expression cassette, and/or vector in the bacterium comprises the antigen. This disclosure further provides the recombinant bacteria described herein for use in the prevention or treatment of a condition (such as a disease) in a host.

In further aspects, described herein are improved methods of expressing and secreting heterologous proteins in host bacteria.

Methods of making bacteria comprising each of the recombinant nucleic acid molecules and expression cassettes described above are also provided. Methods of using the bacteria to produce vaccines are also provided.

This disclosure further provides a variety of polynucleotides encoding signal peptides and/or antigens, including the polynucleotides which have been codon-optimized for expression in *Listeria monocytogenes.*

### DRAWINGS

Figure 1 shows the *hly* promoter alignment for the *Listerial monocytogenes* DP-L4056 (SEQ ID NO:1) (bottom sequence) and EGD strains (SEQ ID NO:2) (top sequence).
Figure 2 shows the sequence (SEQ ID NO:3) of a polynucleotide encoding a fusion protein comprising the LLO signal peptide, LLO PEST sequence, and the full-length human EphA2 antigen.
Figure 3 shows the sequence (SEQ ID NO:4) of the fusion protein encoded by the polynucleotide shown in Figure 2.
Figure 4 shows the native nucleotide sequence (SEQ ID NO:5) that encodes the human EphA2 extracellular domain (EX2).
Figure 5 shows a nucleotide sequence (SEQ ID NO:6) encoding the human EphA2 extracellular domain that has been codon-optimized for expression in *Listeria monocytogenes.*
Figure 6 shows the amino acid sequence (SEQ ID NO:7) of the human EphA2 extracellular domain (EX2).
Figure 7 shows a non-codon optimized polynucleotide sequence (SEQ ID NO:8) encoding a fusion protein comprising an LLO signal peptide, LLO PEST sequence and the extracellular domain of human EphA2.
Figure 8 shows the sequence (SEQ ID NO:9) of the fusion protein encoded by the coding sequence shown in Figure 7.
Figure 9 shows an expression cassette (SEQ ID NO:10) comprising the *hly* promoter and encoding a fusion protein comprising an LLO signal peptide, LLO PEST sequence and the extracellular domain of human EphA2. In this sequence, the sequence encoding the human EphA2 extracellular domain is codon-optimized for expression in *Listeria monocytogenes.*
Figure 10 shows the amino acid sequence (SEQ ID NO:11) encoded by the expression cassette of Figure 9.
Figure 11 shows an expression cassette (SEQ ID NO:12) comprising the *hly* promoter and encoding a fusion protein comprising an LLO signal peptide, LLO PEST sequence and the extracellular domain of human EphA2. In this sequence, the sequences encoding the LLO signal peptide , LLO PEST, and human EphA2 extracellular domain have all been codon-optimized for expression in *Listeria monocytogenes.*
Figure 12 shows the amino acid sequence (SEQ ID NO:13) encoded by the expression cassette of Figure 11.
Figure 13 shows an expression cassette (SEQ ID NO:14) comprising the *hly* promoter and encoding a fusion protein comprising the phoD Tat signal peptide and the extracellular domain of human EphA2. In this sequence, the sequences encoding the phoD Tat signal peptide and human EphA2 extracellular domain have both been codon-optimized for expression in *Listeria monocytogenes.*
Figure 14 shows the amino acid sequence (SEQ ID NO:15) encoded by the expression cassette of Figure 13.
Figure 15 shows the native nucleotide sequence (SEQ ID NO:16) that encodes the human EphA2 intracellular domain (CO).
Figure 16 shows a nucleotide sequence (SEQ ID NO:17) encoding the human EphA2 intracellular domain that has been codon-optimized for expression in *Listeria monocytogenes.*
Figure 17 shows the amino acid sequence (SEQ ID NO:18) of the human EphA2 intracellular domain (EX2).
Figure 18 shows a non-codon optimized polynucleotide sequence (SEQ ID NO:19) encoding a fusion protein comprising an LLO signal peptide, LLO PEST sequence and the intracellular domain of human EphA2.
Figure 19 shows the sequence (SEQ ID NO:20) of the fusion protein encoded by the coding sequence shown in Figure 18.
Figure 20 shows an expression cassette (SEQ ID NO:21) comprising the *hly* promoter and encoding a fusion protein comprising an LLO signal peptide, LLO PEST sequence and the intracellular domain of human EphA2. In this sequence, the sequence encoding the human EphA2 intracellular domain is codon-optimized for expression in *Listeria monocytogenes.*
Figure 21 shows the amino acid sequence (SEQ ID NO:22) encoded by the expression cassette of Figure 20.
Figure 22 shows an expression cassette (SEQ ID NO:23) comprising the *hly* promoter and encoding a fusion protein comprising an LLO signal peptide, LLO PEST sequence and the intracellular domain of human EphA2. In this sequence, the sequences encoding the LLO signal peptide, LLO PEST, and human EphA2 intracellular domain have all been codon-optimized for expression in *Listeria monocytogenes.*
Figure 23 shows the amino acid sequence encoded (SEQ ID NO:24) by the expression cassette of Figure 22.
Figure 24 shows an expression cassette (SEQ ID NO:25) comprising the *hly* promoter and encoding a fusion protein comprising a phoD Tat signal peptide and the intracellular domain of human EphA2. In this sequence, the sequences encoding both the phoD Tat signal peptide and human EphA2 intracellular domain have been codon-optimized for expression in *Listeria monocytogenes.*
Figure 25 shows the amino acid sequence (SEQ ID NO:26) encoded by the expression cassette of Figure 24.
Figure 26 shows a codon-optimized expression cassette (SEQ ID NO:27) comprising the *hly* promoter and encoding a fusion protein comprising an LLO signal peptide and the NY-ESO-1 antigen. Both the sequences encoding the signal peptide and the antigen are codon-optimized for expression in *Listeria monocytogenes.*
Figure 27 shows the amino acid sequence (SEQ ID NO:28) encoded by the expression cassette of Figure 26.
Figure 28 shows a polynucleotide (SEQ ID NO:29) comprising the *hly* promoter operably linked to a codon-optimized sequence encoding a Usp45 signal peptide.
Figure 29 shows a polynucleotide (SEQ ID NO:30) comprising the *hly* promoter operably linked to a native sequence encoding a p60 signal peptide.
Figure 30 shows a polynucleotide (SEQ ID NO:31) comprising the *hly* promoter operably linked to a codon-optimized sequence encoding a p60 signal peptide.

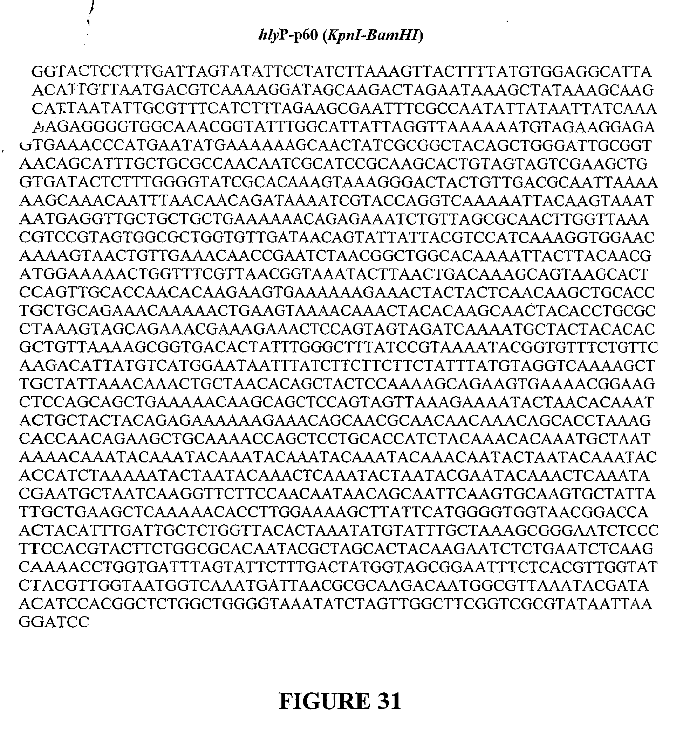

Figure 31 shows the sequence (SEQ ID NO:32) of an hlyP-p60 gene fragment.
Figure 32 (includes Figure 32A, 32B, and 32C) shows the sequence (SEQ ID NO:33) of pAM401-MCS, the pAM401 plasmid containing a multiple cloning site (MCS) from pPL2 vector.
Figure 33 shows the coding sequence (SEQ ID NO:34) for human mesothelin which has been codon-optimized for expression in *Listeria monocytogenes.*
Figure 34 shows the amino acid sequence of human mesothelin (SEQ ID NO:35).
Figure 35 shows the coding sequence (SEQ ID NO:36) for murine mesothelin which has been codon-optimized for expression in *Listeria monocytogenes.*
Figure 36 shows the amino acid sequence (SEQ ID NO:37) of murine mesothelin.
Figure 37 shows a Western blot analysis of secreted protein from recombinant *Listeria* encoding a native EphA2 CO domain sequence.
Figure 38 shows a Western blot analysis of secreted protein from recombinant *Listeria* encoding native or codon-optimized LLO secA1 signal peptide fused with codon-optimized EphA2 EX2 domain sequence.
Figure 39 shows a Western blot analysis of secreted protein from recombinant *Listeria* encoding native or codon-optimized LLO secA1 signal peptide or codon-optimized Tat signal peptide fused with codon-optimized EphA2 CO domain sequence.
Figure 40 shows a Western blot analysis of lysate from 293 cells 48 hr following transfection with pCDNA4 plasmid DNA encoding full-length native EphA2 sequence.
Figure 41 is a graph showing that immunization of Balb/C mice bearing CT26.24 (huEphA2+) lung tumors with recombinant *Listeria* encoding OVA.AH1 or OVA.AH1-A5 confers long-term survival.
Figure 42 is a graph showing the increased survival of Balb/C mice bearing CT26.24 (huEphA2+) lung tumors when immunized with recombinant *Listeria* encoding codon-optimized secA1 signal peptide fused with codon-optimized EphA2 EX2 domain sequence.
Figure 43 is a graph showing that immunization of Balb/C mice bearing CT26.24 (huEphA2+) lung tumors with recombinant *Listeria* encoding EphA2 CO domain confers long-term survival.
Figure 44 is a graph showing that immunization of Balb/C mice bearing CT26.24 (huEphA2+) lung tumors with recombinant *Listeria* encoding EphA2 CO domain but not with plasmid DNA encoding full-length EphA2 confers long-term survival.
Figure 45 is a graph showing that *Listeria* expressing hEphA2 elicits an EphA2 specific CD8+ T cell response.
Figure 46 is a graph showing that both CD4+ and CD8+ T cell responses contribute to the hEphA2-directed anti-tumor efficacy of *Listeria* expressing hEphA2.
Figure 47 shows the sequence (SEQ ID NO:38) of the *Listeria monocytogenes* strain 104035 *hly* promoter operably linked to Protective Antigen signal peptide from *B. anthracis*, codon-optimized for secretion in *Listeria monocytogenes.* Six additional nucleotides (5'-GGATCC-3') corresponding to the *Bam HI* restriction enzyme recognition site were included at the carboxy terminus of the signal peptide sequence, facilitating operable in-frame linkage to any selected coding sequence. The 5' end of the hly promoter contains a unique *Kpn I* restriction enzyme recognition site.

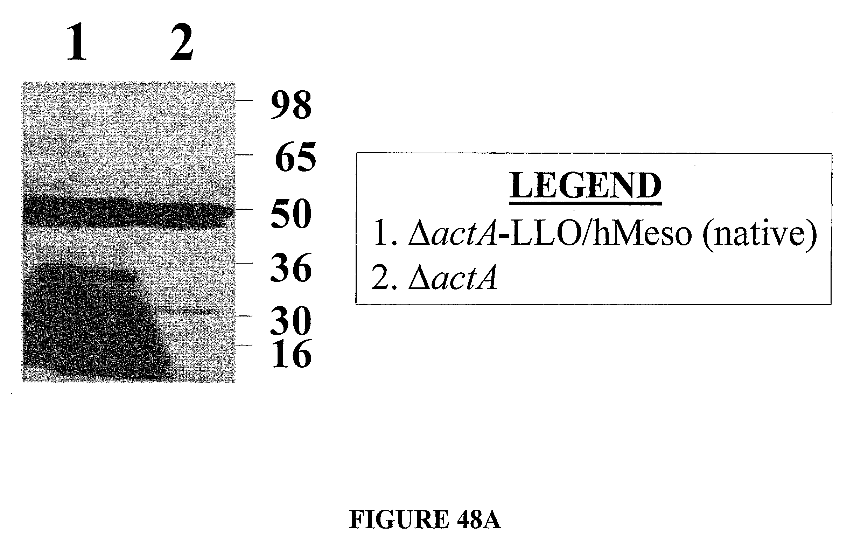

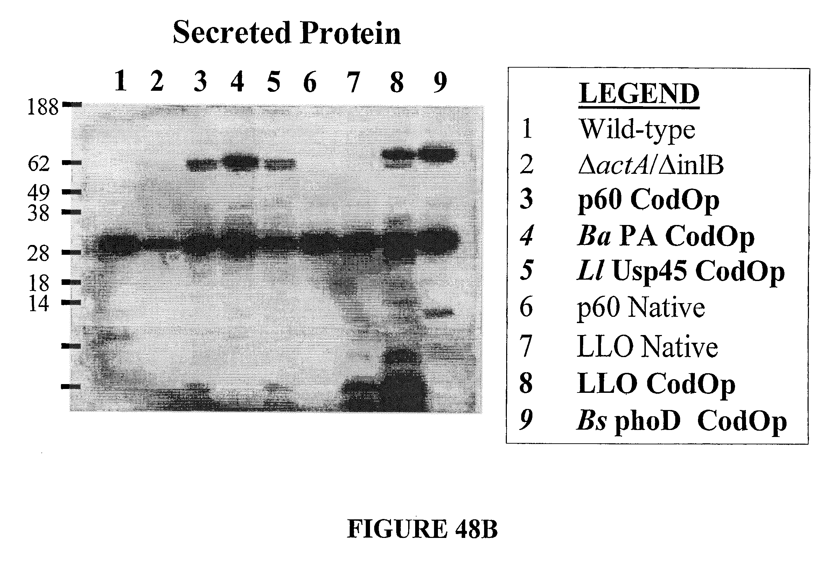

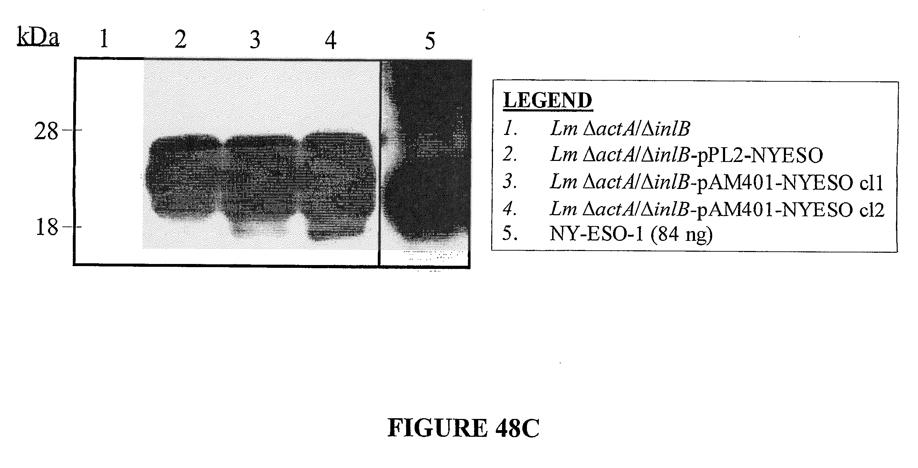

Figure 48 shows the efficient expression and secretion of full-length human tumor antigens from recombinant *Listeria.* Figure 48A shows mesothelin expression/secretion with constructs consisting of LLO signal peptide fused with human mesothelin, using native codons. Figure 48B shows mesothelin expression/secretion with constructs comprising various signal peptides fused with human mesothelin codon-optimized for expression in *Listeria.* Figure 48C shows the expression/secretion of NY-ESO-1 with constructs comprising codon-optimized LLO signal peptide fused with human mesothelin codon-optimized NY-ESO-1.
Figure 49 shows the coding sequences of phEphA2KD (SEQ ID NO:39).
Figure 50 shows the *Mlu* I subfragment (SEQ ID NO:40) of codon-optimized human EphA2 containing the actA-plcB intergenic region.
Figure 51 shows the sequence (SEQ ID NO:41) of the *hly* promoter-70 N-terminal p60 amino acids.
Figure 52 shows the *KpnI-BamHI* sub-fragment (SEQ ID NO:42) of plasmid pPL2-hlyP-Np60 CodOp(1-77).
Figure 53 shows the *KpnI-BamHI* sub-fragment (SEQ ID NO:43) of plasmid pPL2-hlyP-Np60 CodOp(1-77)-Mesothelin.
Figure 54 shows the *KpnI-BamHI* sub-fragment (SEQ ID NO:44) of plasmid pPL2-hlyP-Np60 CodOp(1-77)-Mesothelin ΔSP/ΔGPI.
Figure 55 shows the Western blot analysis of the expression and secretion of antigens from recombinant *Listeria* comprising antigen-bacterial protein chimeras.
Figure 56 shows the Western blot analysis of the expression of the intracellular domain (ICD) of EphA2 from a bicistronic message.
Figure 57 shows the Western blot analysis of the plasmid based expression and secretion of murine mesothelin as a function of N-terminal fusion with various codon-optimized signal peptides as evidenced in different bacterial fractions: secreted protein (Figure 57A); cell wall (Figure 57B); and cell lysate (Figure 57C).
Figure 58 shows the Western blot analysis of chromosomal-based expression and secretion of human mesothelin in *Listeria monocytogenes.* Western blot analysis of mesothelin expression in various bacterial cell fractions, with results from control *Listeria* (not encoding mesothelin) and *Listeria* encoding mesothelin expressed from the indicated signal sequences, is shown.
Figure 59A and 59B are graphs showing the delivery of a heterologous antigen (AH1-A5) to MHC Class I pathway by a *Listeria* vaccine. The *Listeria* vaccine comprised *Listeria* expressing a p60-AH1-A5 protein chimera (AH1-A5 embedded in p60) (Fig. 59A) or *Listeria* expressing a fusion protein comprising an LLO signal peptide and AH1-A5 (Fig. 59B).
Figure 60A and 60B are graphs showing the *Listeria* vaccine mediated delivery of bacteria-specific antigens to MHC Class I pathway, where the vaccine comprised *Listeria* expressing a p60-AH1-A5 protein chimera (AH1-A5 embedded in p60) (Fig. 60A) or *Listeria* expressing a fusion protein comprising an LLO signal peptide and AH1-A5 (Fig. 60B), and where the test peptides added to the cell based assay were no test peptide (unstimulated) (Fig. 60A), LLO₉₁₋₉₉ (Fig.60A), no test peptide (Fig. 60B), or p60₂₁₇₋₂₂₅ (Fig. 60B).
Figure 61 is a graph showing the therapeutic efficacy of *Listeria* expressing human mesothelin in vaccinated tumor-bearing animals, where tumor cells were engineered to express human mesothelin.
Figure 62 is a graph showing the reduction in lung tumor nodule level in tumor-bearing mice vaccinated with *Listeria* expressing human mesothelin, where the tumor cells were engineered to express human mesothelin.
Figure 63 is a graph showing a control study using CT.26 parental target cells, i.e., cells not engineered to express human mesothelin, that demonstrates the anti-tumor efficacy of Lm-Meso vaccination is mesothelin specific.
Figure 64 is a graph showing that vaccination with *Listeria* expressing codon optimized human mesothelin reduces tumor volume.
Figure 65 shows the results of ELISPOT experiments which show the immunogenicity of a *Listeria* Δ*actA*/Δ*inlB*-hMesothelin strain where the nucleic acid encoding hMesothelin has been integrated into the *Listeria* genome.

### DETAILED DESCRIPTION OF THE INVENTION

### I. Introduction

The present disclosure provides a variety of polynucleotides including recombinant nucleic acid molecules, expression cassettes, and expression vectors useful for expression and/or secretion of polypeptides, including heterologous polypeptides (e.g. antigens and/or mammalian proteins), in bacteria, such as *Listeria.* In some embodiments, these polynucleotides can be used for enhanced expression and/or secretion of polypeptides in bacteria. Some of the expression cassettes comprise codon-optimized coding sequences for the polypeptide and/or for the signal peptide. In addition, some of the expression cassettes for use in bacteria contain signal peptide sequences derived from other bacterial sources and/or from a variety of different secretory pathways. Bacteria comprising the expression cassettes are also provided, as are compositions, such as vaccines, containing the bacteria. Methods of using the polynucleotides, bacteria, and compositions to induce an immune response and/or to prevent or treat a condition, such as a disease (e.g. cancer), in a host are also provided.

This disclosure is based, in part, on the discovery that codon-optimization of the signal peptide sequence in an expression cassette enhances the expression and/or secretion of a heterologous polypeptide (such as an antigen) from recombinant bacteria (particularly in combination with codon-optimization of the heterologous polypeptide), even when the signal peptide sequence is native to the bacteria (see, e.g., Examples 19 and 27, below). Additionally, it has been discovered that signal peptide sequences from non-sccA1 secretory pathways and/or signal peptide sequences from non-Listerial bacterial sources can also be used to effect efficient expression and/or secretion of heterologous polypeptides from *Listeria* (see, e.g., Examples 19, 27, and 30 below). This disclosure is also based, in part, on the additional discovery that codon-optimization of the coding sequences of heterologous polypeptides enhances expression and/or secretion of the heterologous polypeptides in *Listeria* (see e.g., Example 19, below). Enhanced expression and/or secretion of the heterologous protein obtained through optimization of the expression cassette has also been shown to lead to enhanced immunogenicity of the bacteria comprising the optimized expression cassettes (see, e.g., Example 20, below). In addition, expression cassettes encoding protein chimeras comprising a heterologous antigen embedded within an autolysin have been shown to useful in effecting efficient expression and secretion of a heterologous antigen in *Listeria* (see, e.g., Example 29, below). The autolysin protein chimeras have also been shown to be immunogenic (see, e.g., Example 31A, below). In addition, *Listeria,* comprising codon-optimized expression cassettes and/or expression cassettes comprising non-Listerial signal peptides have also been shown to be immunogenic, reduce tumor volume, and increase survival in a mouse model (see, e.g., Examples 31B-E, below).

Accordingly, in one aspect, described herein is a recombinant nucleic acid molecule, comprising a first polynucleotide encoding a signal peptide, wherein the first polynucleotide is codon-optimized for expression in a bacterium, and a second polynucleotide encoding a polypeptide (e.g., an antigen), wherein the second polynucleotide is in the same translational reading frame as the first polynucleotide, and wherein the recombinant nucleic acid molecule encodes a fusion protein comprising the signal peptide and the polypeptide. In some embodiments, the second polynucleotide is codon-optimized as well (typically for expression in the same type of bacteria as the first polynucleotide). In some embodiments, the first polynucleotide or the first and second polynucleotides are codon-optimized for expression in *Listeria*, (*Bacillus*, *Yersinia pestis*, *Salmonella*, *Shigella*, *Brucella*, mycobacteria or *E. coli.* In some embodiments, the polynucleotide(s) is codon-optimized for expression in *Listeria,* such as *Listeria monocytagenes.* In some embodiments, the polypeptide encoded by the second polynucleotide is (or comprises) an antigen, which, in some instances, may be a non-bacterial antigen. For instance, the antigen is, in some embodiments a tumor-associated antigen or is derived from such a tumor-associated antigen. For instance, in some embodiments, the antigen is K-Ras, H-Ras, N-Ras,12-K-Ras, mesothelin, PSCA, NY-ESO-1, WT-1, survivin, gp100, PAP, proteinase 3, SPAS-1, SP-17, PAGE-4, TARP, or CEA, or is derived from K-Ras, H-Ras, N-Ras, 12-K-Ras, mesothelin, PSCA, NY-ESO-1,WT-1, survivin, gp100, PAP, proteinase 3, SPAS-1, SP-17, PAGE-4, TARP, or CEA. For instance, in some embodiments, the antigen is mesothelin, or an antigenic fragment or antigenic variant of mesothelin. In some other embodiments, the antigen is NY-ESO-1, or an antigenic fragment or antigenic variant of NY-ESO-1. In some embodiments, the antigen is an infectious disease antigen or is derived from an infectious disease antigen. In some embodiments, the signal peptide is bacterial (Listerial or non-Listerial). In some embodiments, the signal peptide encoded by the codon-optimized first polynucleotide is native to the bacterium. In other embodiments, the signal peptide encoded by the codon-optimized first polynucleotide is foreign to the bacterium. In some embodiments, the signal peptide is a secA1 signal peptide, such as an LLO signal peptide from *Listeria monocytogenes,* a Usp45 signal peptide from *Lactococcus lactis,* or a Protective Antigen signal peptide from *Bacillus anthracis.* In some embodiments, the signal peptide is a secA2 signal peptide. For instance, the signal peptide may be the p60 signal peptide from *Listeria monocytogenes.* In addition, the recombinant nucleic acid molecule optionally comprises a third polynucleotide sequence encoding p60, or a fragment thereof, in the same translational reading frame as the first and second polynucleotides, wherein the second polynucleotide is positioned within the third polynucleotide or between the first and third polynucleotides. In still further embodiments, the signal peptide is a Tat signal peptide, such as a *B*. *subtilis* Tat signal peptide (e.g., PhoD). This disclosure also provides expression cassettes comprising the recombinant nucleic acid molecule and further comprising a promoter operably linked to the recombinant nucleic acid molecule (e.g., to the first and second polynucleotides (and third polynucleotide, if present)). Expression vectors and recombinant bacteria (e.g. *Listeria)* comprising the expression cassette are also provided, as are pharmaceutical compositions, immunogenic compositions, and vaccines, comprising the bacteria. Methods of using the bacteria or compositions comprising the bacteria to induce an immune response and/or prevent or treat a condition, such as a disease, are also provided. The use of the bacterium in the manufacture of a medicament for inducing an immune response in a host to an antigen, wherein the polypeptide encoded by the second polynucleotide comprises the antigen is also provided.

In a second aspect, described herein is a recombinant nucleic acid molecule, comprising (a) a first polynucleotide encoding a signal peptide native to a bacterium, wherein the first polynucleotide is codon-optimized for expression in the bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the polypeptide encoded by the second polynucleotide is heterologous to the signal peptide. In some embodiments, the second polynucleotide is heterologous to the first polynucleotide. In some embodiments, the polypeptide is foreign to the bacterium to which the signal peptide is native. In some embodiments, the polypeptide encoded by the second polynucleotide is heterologous to the signal peptide, foreign to the bacterium, or both. In some embodiments, the bacterium from which the signal peptide is derived is an intracellular bacterium. In some embodiments, the bacterium is selected from the group consisting of *Listeria, Bacillus, Yersinia pestis, Salmonella*, *Shigella*, *Brascella,* mycobacteria and *E*. *coli.* In some embodiments the bacterium is a *Listeria* bacterium (e.g., *Listeria monocytogenes).* In some embodiments, second polynucleotide is codon-optimized for expression in the bacterium. In some embodiments, the codon-optimization of the first and/or second polynucleotide enhances expression in and/or secretion from the bacterium of the encoded fusion protein (relative to the non-codon-optimized sequence). In some embodiments, the polypeptide encoded by the second polynucleotide comprises an antigen. The polypeptide encoded by the second polynucleotide is an antigen. In some embodiments, the antigen is a non-bacterial antigen. In some embodiments, the antigen is a tumor-associated antigen or comprises an antigen derived from a tumor-associated antigen. In some embodiments, the antigen is selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or is derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA. For instance, in some embodiments, the antigen is mesothelin, or an antigenic fragment or variant thereof, or is NY-ESO-1, or an antigenic fragment or variant thereof. In some alternative embodiments, the antigen is an infectious disease antigen or is derived from an infectious disease antigen. In some embodiments, the signal peptide is a secA1 signal peptide (e.g., LLO signal peptide from *Listeria, monocytogenes*). In some embodiments, the signal peptide is a secA2 signal peptide (e.g., p60 signal peptide from *Listeria monocytogenes*). An expression cassette comprising the recombinant nucleic acid molecule and further comprising a promoter operably linked to the first and second polynucleotides of the recombinant nucleic acid molecule is also provided. An expression vector comprising the expression cassette is also provided. A recombinant bacterium comprising the recombinant nucleic acid molecule, wherein the first polynucleotide is codon-optimized for expression in the recombinant bacterium is also provided. In some embodiments, the recombinant bacterium is an intracellular bacterium. In some embodiments, the recombinant bacterium is selected from the group consisting of *Listeria, Bacillus, Yersinia pestis*, *Salmonella, Shigella, Brucella,* mycobacteria and *E. coli.* In some embodiments, the bacterium is a recombinant *Listeria* bacterium (e.g., a recombinant *Listeria monocytogenes* bacterium). An immunogenic composition comprising the recombinant bacterium, wherein the polypeptide encoded by the second polynucleotide is an antigen is further provided. Methods of inducing an immune response in a host to an antigen comprising administering to the host an effective amount of a composition comprising the recombinant bacterium, wherein the polypeptide encoded by the second polynucleotide is (or comprises) the antigen, are also provided. The use of the bacterium in the manufacture of a medicament for inducing an immune response in a host to an antigen, wherein the polypeptide encoded by the second polynucleotide comprises the antigen is also provided.

In a third aspect, described herein is a recombinant *Listeria* bacterium (e.g., *Listeria monocytogenes)* comprising a recombinant nucleic acid molecule, wherein the recombinant nucleic acid molecule comprises (a) a first polynucleotide encoding a signal peptide, wherein the first polynucleotide is codon-optimized for expression in the *Listeria* bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the polypeptide encoded by the second polynucleotide is heterologous to the signal peptide. In some embodiments, the recombinant nucleic acid molecule is part of an expression cassette that further comprises a promoter operably linked to both the first and second polynucleotides. In other words, in some embodiments the recombinant *Listeria* bacterium comprises an expression cassette which comprises the recombinant nucleic acid molecule, wherein the expression cassette further comprises a promoter operably linked to both the first and second polynucleotides of the recombinant nucleic acid molecule. In some embodiments, the expression cassette is a polycistronic expression cassette. In some embodiments, the second polynucleotide is codon-optimized for expression in the *Listeria* bacterium. In some embodiments, the codon-optimization of the first and/or second polynucleotide enhances expression in and/or secretion from the *Listeria* bacterium of the encoded fusion protein (relative to the non-codon-optimized sequence). In some embodiments, the polypeptide encoded by the second polynucleotide is foreign to the *Listeria* bacterium (i.e., heterologous to the *Listeria* bacterium). In some embodiments, the polypeptide encoded by the second polynucleotide comprises an antigen (e.g., a non-Listerial or non-bacterial antigen). In some embodiments, the polypeptide encoded by the second polynucleotide is an antigen. In some embodiments, the antigen is a tumor-associated antigen or is derived from a tumor-associated antigen. In some embodiments, the antigen is selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or is derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA. For instance, in some embodiments, the antigen is mesothelin, or an antigenic fragment or antigenic variant thereof In some embodiments, the antigen is human mesothelin. In some embodiments, the antigen is human mesothelin deleted of its signal peptide and GPI linker domain. In some alternative embodiments, the antigen is NY-ESO-1, or an antigenic fragment or antigenic variant thereof. In some alternative embodiments, the antigen is an infectious disease antigen or is an antigen derived from an infectious disease antigen. In some embodiments, the signal peptide is non-Listerial. In some embodiments, the signal peptide is bacterial. In some embodiments, the signal peptide is foreign to the *Listeria* bacterium. In other embodiments, the signal peptide is native to the *Listeria* bacterium. In some embodiments, the signal peptide is a secA1 signal peptide (e.g., LLO signal peptide from *Listeria monocytogenes,* Usp45 signal peptide from *Lactococcus lactis,* and Protective Antigen signal peptide from *Bacillus anthracis*). In some embodiments, the signal peptide is a secA2 signal peptide (e.g., p60 signal peptide from *Listeria monocytogenes).* In some embodiments the signal peptide is a Tat signal peptide (e.g., PhoD signal peptide from *B. subtilis*). In some embodiments, the *Listeria* bacterium is attenuated. For instance, the *Listeria* may be attenuated for cell-to-cell spread, entry into non-phagocytic cells, or proliferation. In some embodiments, the recombinant *Listeria* bacterium is deficient with respect to ActA, Internalin B, or both Act A and Internalin B (e.g., an Δ*actA*Δ*inlB* double deletion mutant). In some embodiments, the recombinant *Listeria* bacterium is deleted in functional ActA, Internalin B, or both Act A and Internalin B. In some embodiments, the nucleic acid of the recombinant bacterium has been modified by reaction with a nucleic acid targeting compound (e.g., a psoralen compound). This disclosure also provides a pharmaceutical composition comprising the recombinant *Listeria* bacterium and a pharmaceutically acceptable carrier, as well an immunogenic composition comprising the recombinant *Listeria* bacterium, wherein the polypeptide encoded by the second polynucleotide is an antigen. This disclosure also provides a vaccine comprising the recombinant *Listeria* bacterium. Methods of inducing an immune response in a host to an antigen comprising administering to the host an effective amount of a composition comprising the recombinant bacterium, wherein the polypeptide encoded by the second polynucleotide is (or comprises) an antigen are also provided. Also provided are methods of preventing or treating a condition (e.g., a disease such as cancer or an infectious disease) in a host comprising administering to the host an effective amount of a composition comprising the recombinant *Listeria* bacterium The use of the bacterium in the manufacture of a medicament for inducing an immune response in a host to an antigen, wherein the polypeptide encoded by the second polynucleotide comprises the antigen is also provided.

In a fourth aspect, described herein is a recombinant nucleic acid molecule, comprising a first polynucleotide encoding a non-secA1 bacterial signal peptide, and a second polynucleotide encoding a polypeptide (e.g., an antigen), wherein the second polynucleotide is in the same translational reading frame as the first polynucleotide, and wherein the recombinant nucleic acid molecule encodes a fusion protein comprising the signal peptide and the polypeptide. In some embodiments, the first polynucleotide and/or the second polynucleotide is codon-optimized for expression in a particular type of bacterium. In some embodiments, the codon-optimization of the first and/or second polynucleotide enhances expression in and/or secretion from the bacterium of the fusion protein (relative to the non-codon-optimized sequence). In some embodiments, the first polynucleotide and/or the second polynucleotide is codon-optimized for expression in *Listeria*, *Bacillus*, *Yersiniα pestis*, *Salmonella*, *Shigella*, *Brucella*, mycobacterial or *E. coli.* In some embodiments, the polynucleotide(s) is codon-optimized for expression in *Listeria*, such as *Listeria, monocytogenes.* In some embodiments, the signal peptide encoded by the codon-optimized first polynucleotide is native to the bacterium for which it is codon-optimized. In some embodiments, the first polynucleotide encoding the signal peptide is heterologous to the second polynucleotide. In some embodiments, the polypeptide encoded by the second polynucleotide is heterologous to the signal peptide. In some embodiments, the polypeptide encoded by the second polynucleotide comprises an antigen. In some embodiments, the polypeptide encoded by the second polynucleotide is an antigen, which, in some instances, may be a non-bacterial antigen. In some embodiments, the antigen is a tumor-associated antigen or is derived from such a tumor-associated antigen. For instance, in some embodiments, the antigen is K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA, or is derived from K-Ras, H-Ras, N-Ras, 12-K-Ras, mesothelin, PSCA, NY-ESO-1, WT-1, surviving, gp100, PAP, proteinase 3, SPAS-1, SP-17, PAGE-4, TARP, or CEA. For instance, in some embodiments, the antigen is mesothelin, or is an antigenic fragment or antigenic variant of mesothelin. In some other embodiments, the antigen is NY-ESO-1, or an antigenic fragment or antigenic variant of NY-ESO-1. In some embodiments, the antigen is an infectious disease antigen or is derived from an infectious disease antigen. In some embodiments, the signal peptide encoded by the first polynucleotide of the recombinant nucleic acid molecule is Listerial. In other embodiments, the signal peptide is non-Listerial. In some embodiments, the signal peptide is derived from a gram positive bacterium. In some embodiments, the signal peptide is derived from a bacterium belonging to the genus *Bacillus, Staphylococcus*, or *Lactococcus.* In some embodiments, the signal peptide is a secA2 signal peptide. For instance, the signal peptide may be the p60 signal peptide from *Listeria monacytogenes.* In addition, the recombinant nucleic acid molecule optionally comprises a third polynucleotide sequence encoding p60, or a fragment thereof, in the same translational reading frame as the first and second polynucleotides, wherein the second polynucleotide is positioned within the third polynucleotide or between the first and third polynucleotides. In still further embodiments, the signal peptide is a Tat signal peptide, such as a *B. subtilis* Tat signal peptide (e.g., a *B. subtilis* PhoD signal peptide). This disclosure also provides expression cassettes comprising the recombinant nucleic acid molecule and further comprising a promoter operably linked to the first and second polynucleotides of the recombinant nucleic acid molecule. Expression vectors and bacteria comprising the expression cassette and/or recombinant nucleic acid molecule are also provided, as are pharmaceutical compositions, immunogenic compositions, and vaccines, comprising the bacteria. In some embodiments, the recombinant bacterium comprising the expression cassette or recombinant nucleic acid molecule is an intracellular bacterium. In some embodiments, the bacterium is a bacterium selected from the group consisting of *Listeria, Bacillus*, *Yersinia pestis*, *Salmonella*, *Shigella*, *Brucella*, mycobacteria or *E. coli.* In some embodiments, the bacterium is a *Listeria* bacterium (e.g., a member of the species *Listeria monocytogenes*). In some embodiments, the polypeptide encoded by the second polynucleotide is foreign to the bacterium (i.e., heterologous to the bacterium). Methods of using the bacteria or compositions comprising the bacteria to induce an immune response and/or to prevent or treat a condition (e.g., a disease) in a host are also provided. In some embodiment, the condition is cancer. In other embodiments, the condition is an infectious disease. The use of the bacterium in the manufacture of a medicament for inducing an immune response in a host to an antigen, wherein the polypeptide encoded by the second polynucleotide comprises the antigen is also provided.

In another aspect, described herein is a recombinant *Listeria* bacterium comprising a recombinant nucleic acid molecule, wherein the recombinant nucleic acid molecule comprises (a) a first polynucleotide encoding a non-secA1 bacterial signal peptide, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the polypeptide encoded by the second polynucleotide is heterologous to the signal peptide or is foreign to the bacterium, or both. In some embodiments, the *Listeria* bacterium belongs to the species *Listeria monocytogenes.* In some embodiments, the recombinant nucleic acid molecule is part of an expression cassette that further comprises a promoter operably linked to both the first and second polynucleotides. In other words, in some embodiments, the recombinant *Listeria* bacterium comprises an expression cassette which comprises the recombinant nucleic acid molecule, wherein the expression cassette further comprises a promoter operably linked to both the first and second polynucleotides of the recombinant nucleic acid molecule. In some embodiments, the expression cassette is a polycistronic expression cassette. In some embodiments, the first polynucleotide, the second polynucleotide, or both the first and second polynucleotide are codon-optimized for expression in the *Listeria* bacterium (e.g., *Listeria monocytogenes).* In some embodiments, the codon-optimization of the first and/or second polynucleotide enhances expression in and/or secretion from the bacterium of the fusion protein (relative to the non-codon-optimized sequence). In some embodiments, the first and second polynucleotides are heterologous to each other. In some embodiments, the polypeptide encoded by the second polynucleotide and the signal peptide are heterologous to each other. In some embodiments, the polypeptide encoded by the second polynucleotide is foreign to the *Listeria* bacterium (i.e., heterologous to the *Listeria* bacterium). In some embodiments, the polypeptide encoded by the second polynucleotide comprises an antigen. In some embodiments, the polypeptide encoded by the second polynucleotide is an antigen (e.g., a non-Listerial or non-bacterial antigen). In some embodiments, the antigen is a tumor-associated antigen or is derived from a tumor-associated antigen. In some embodiments, the antigen is selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or is derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA. For instance, in some embodiments, the antigen is mesothelin, or an antigenic fragment or antigenic variant thereof. In some embodiments, the antigen is human mesothelin. In some embodiments, the antigen is human mesothelin deleted of its signal peptide and GPI linker domain. In some alternative embodiments, the antigen is an infectious disease antigen or is derived from an infectious disease antigen. In some embodiments, the signal peptide is non-Listerial. In some embodiments, the non-secA1 signal peptide is a Listerial signal peptide. In other embodiments, the non-sccA1 signal peptide is a non-Listerial signal peptide. In some embodiments, the signal peptide is a secA2 signal peptide (e.g., p60 signal peptide from *Listeria monocytogenes).* In some embodiments, the recombinant nucleic acid molecule comprising a secA2 signal peptide, further comprises a third polynucleotide encoding a secA2 autolysin (e.g., p60 or N-acetylmuramidase), or a fragment thereof (e.g., a catalytically active fragment), in the same translational reading frame as the first and second polynucleotides, wherein the second polynucleotide is positioned within the third polynucleotide or between the first and third polynucleotides of the recombinant nucleic acid molecule. In some embodiments, the second polynucleotide is positioned within the third polynucleotide. In some embodiments the signal peptide is a Tat signal peptide. In some embodiments, the signal peptide is a Tat signal peptide derived *B. subtilis.* (e.g., PhoD signal peptide from *B. subtilis*). In some embodiments, the *Listeria* bacterium is attenuated. For instance, the *Listeria* may be attenuated for cell-to-cell spread, entry into non-phagocytic cells, or proliferation. In some embodiments, the recombinant *Listeria* bacterium is deficient with respect to ActA, Internalin B, or both Act A and Internalin B (e.g., an Δ*actA*Δ*inlB* double deletion mutant). In some embodiments, the recombinant *Listeria* bacterium is deleted in functional ActA, Internalin B, or both Act A and Internalin B. In some embodiments, the nucleic acid of the recombinant bacterium has been modified by reaction with a nucleic acid targeting compound (e.g., a psoralen compound). This disclosure also provides a pharmaceutical composition comprising the recombinant *Listeria* bacterium and a pharmaceutically acceptable carrier. This disclosure also provides an immunogenic composition comprising the recombinant bacterium, wherein the polypeptide encoded by the second polynucleotide is an antigen. This disclosure also provides a vaccine comprising the recombinant *Listeria* bacterium. Methods of inducing an immune response in a host to an antigen comprising administering to the host an effective amount of a composition comprising the recombinant bacterium, wherein the polypeptide encoded by the second polynucleotide is (or comprises) an antigen are also provided. Also provided are methods of preventing or treating a condition (e.g., a disease such as cancer or an infectious disease) in a host comprising administering to the host an effective amount of a composition comprising the recombinant *Listeria* bacterium. The use of the bacterium in the manufacture of a medicament for inducing an immune response in a host to an antigen, wherein the polypeptide encoded by the second polynucleotide comprises the antigen is also provided.

In another aspect, described herein is a recombinant nucleic acid molecule comprising a polynucleotide encoding a polypeptide foreign to a *Listeria* bacterium (such as an antigen like a cancer antigen or a non-Listerial bacterial antigen), wherein the polynucleotide is codon-optimized for expression in *Listeria.* In some embodiments, the codon-optimization of the polynucleotide enhances expression in and/or secretion from a *Listeria* bacterium of the polypeptide (relative to the non-codon-optimized sequence). In some embodiments, the foreign polypeptide comprises an antigen. In some embodiments, the foreign polypeptide is an antigen. In some embodiments, the antigen is a non-bacterial antigen. For instance, the antigen is, in some embodiments a tumor-associated antigen or is derived from such a tumor-associated antigen. For instance, in some embodiments, the polypeptide is K-Ras, H-Ras, N-Ras,12-K-Ras, mesothelin, PSCA, NY-ESO-1, WT-1, survivin, gp100, PAP, proteinase 3, SPAS-1, SP-17, PAGE-4, TARP, or CEA, or is derived from K-Ras, H-Ras, N-Ras,12-K-Ras, mesothelin, PSCA, NY-ESO-1, WT-1, survivin, gp100, PAP, proteinase 3, SPAS-1, SP-17, PAGE-4, TARP, or CEA. In some embodiments, the antigen is mesothelin, or is an antigenic fragment or antigenic variant of mesothelin. In some other embodiments, the antigen is NY-ESO-1, or is an antigenic fragment or variant of NY-ESO-1. In some other embodiments, the antigen is an infectious disease antigen or is derived from an infectious disease antigen. In some embodiments, the recombinant nucleic acid molecule further comprises a polynucleotide encoding a signal peptide in the same translational frame as the foreign polypeptide so that the recombinant nucleic acid molecule encodes a fusion protein comprising the signal peptide and the foreign polypeptide. In some embodiments, the polynucleotide encoding the signal peptide (which may or may not be native to *Listeria*) is codon-optimized for expression in *Listeria monocytogenes.* This disclosure further provides an expression cassette comprising the recombinant nucleic acid molecule and further comprising a promoter operably linked to the first and second polynucleotides of the recombinant nucleic acid molecule. A vector (e.g., an expression vector) comprising the recombinant nucleic acid molecule and/or expression cassette is also provided. This disclosure also provides a recombinant *Listeria* bacterium comprising the recombinant nucleic acid molecule and/or expression cassette. In some embodiments, the *Listeria* bacterium belongs to the species *Listeria monocytogenes.* Pharmaceutical compositions, immunogenic compositions, and vaccines comprising the recombinant *Listeria* bacteria are also provided. This disclosure further provides a method of inducing an immune response in host to an antigen comprising administering to the host an effective amount of a composition comprising the recombinant *Listeria* bacterium, wherein the polypeptide is (or comprises) the antigen. In addition, described herein are methods of using the recombinant *Listeria* bacteria to induce an immune response and/or prevent or treat a condition (e.g., a disease). The use of the bacterium in the manufacture of a medicament for inducing an immune response in a host to an antigen, wherein the foreign polypeptide comprises the antigen is also provided.

In another aspect, described herein is a recombinant *Listeria* bacterium comprising an expression cassette, wherein the expression cassette comprises a polynucleotide encoding a polypeptide foreign to the *Listeria* bacterium (such as an antigen like a cancer antigen or a non-Listerial bacterial antigen), wherein the polynucleotide is codon-optimized for expression in *Listeria,* and a promoter, operably linked to the polynucleotide encoding the foreign polypeptide. In some embodiments, the *Listeria* bacterium belongs to the species *Listeria monocytogenes.* In some embodiments, the codon-optimization of the polynucleotide enhances expression in and/or of the polypeptide from a *Listeria* bacterium of the polypeptide (relative to the non-codon-optimized sequence). In some embodiments, the foreign polypeptide comprises an antigen. In some embodiments, the foreign polypeptide is an antigen, which, in some instances, may be a non-bacterial antigen. For instance, the antigen is, in some embodiments a tumor-associated antigen or is derived from such a tumor-associated antigen. For instance, in some embodiments, the polypeptide is K-Ras, H-Ras, N-Ras, 12-K-Ras, mesothelin, PSCA, NY-ESO-1, WT-1, survivin, ge100, PAP, proteinase 3, SPAS-1, SP-17, PAGE-4, TARP, or CEA, or is derived from K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA. In some embodiments, the antigen is mesothelin, or is an antigenic fragment or antigenic variant of mesothelin. In some other embodiments, the antigen is NY-ESO-1, or is an antigenic fragment or antigenic variant of NY-ESO-1. In some other embodiments, the antigen is an infectious disease antigen or is derived from an infectious disease antigen. In some embodiments, the expression cassette Further comprises a polynucleotide encoding a signal peptide which is operably linked to the promoter and in the same translational frame as the foreign polypeptide so that the expression cassette encodes a fusion protein comprising the signal peptide and the foreign polypeptide. In some embodiments, the polynucleotide encoding the signal peptide (which may or may not be native to *Listeria)* is codon-optimized for expression in *Listeria manacytogenes.* Pharmaceutical compositions, immunogenic compositions, and vaccines comprising the recombinant *Listeria* bacteria are also provided. This disclosure further provides a method of inducing an immune response in host to an antigen comprising administering to the host an effective amount of a composition comprising the recombinant *Listeria* bacterium. In addition, described herein are methods of using the recombinant *Listeria* bacteria to induce an immune response and/or prevent or treat a condition (e.g., a disease). The use of the bacterium in the manufacture of a medicament for inducing an immune response in a host to an antigen, wherein the foreign polypeptide comprises the antigen is also provided.

In a further aspect, described herein is a recombinant *Listeria* bacterium. (e.g., *Listeria monocytogenes)* comprising a recombinant nucleic acid molecule, wherein the recombinant nucleic acid molecule comprises (a) a first polynucleotide encoding a non-Listerial signal peptide; and (b) a second polynucleotide encoding a polypeptide that is in the same translational reading frame as the first polynucleotide, wherein the recombinant nucleic acid molecule encodes a fusion protein comprising both the non-Listerial signal peptide and the polypeptide. In some embodiments, the recombinant nucleic acid molecule is positioned in an expression cassette that further comprises a promoter operably linked to both the first and second polynucleotides. Thus, in some embodiments the recombinant *Listeria* bacterium comprises an expression cassette which comprises the recombinant nucleic acid molecule, wherein the expression cassette further comprises a promoter operably linked to both the first and second polynucleotides of the recombinant nucleic acid molecule. In some embodiments, the expression cassette is a polycistronic expression cassette (e.g., a bicistronic expression cassette). In some embodiments, the first polynucleotide, the second polynucleotide, or both the first and second polynucleotide are codon-optimized for expression in *Listeria* (e.g., *Listeria monocytogenes).* In some embodiments, the codon-optimization of the first and/or second polynucleotide enhances expression of the fusion protein in and/or secretion of the fusion protein from the bacterium (relative to the non-codon-optimized sequence). In some embodiments, the first and second polynucleotides are heterologous to each other. In some embodiments, the polypeptide encoded by the second polynucleotide and the signal peptide are heterologous to each other. In some embodiments, the polypeptide encoded by the second polynucleotide is foreign to the *Listeria* bacterium (i.e., heterologous to the *Listeria* bacterium). In some embodiments, the polypeptide encoded by the second polynucleotide comprises an antigen (e.g., a non-Listerial antigen). The polypeptide encoded by the second polynucleotide is, in some embodiments, an antigen. In some embodiments, the antigen is a tumor-associated antigen or is derived from a tumor-associated antigen. In some embodiments, the antigen is selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or is derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA. For instance, in some embodiments, the antigen is mesothelin, or an antigenic fragment or antigenic variant thereof. In some embodiments, the antigen is human mesothelin. In some embodiments, the antigen is human mesothelin deleted of its signal peptide and GPI linker domain. In some alternative embodiments, the antigen is an infectious disease antigen or is derived from an infectious disease antigen. In some embodiments, the signal peptide is bacterial. In some embodiments, the signal peptide is derived from an intracellular bacterium. In some embodiments, the signal peptide is derived from a gram positive bacterium. In some embodiments, the signal peptide is from abacterium belonging to the genus *Bacillus, Staphylocoecus,* or *Lacotococcus* (e.g., *Bacillus anthracis, Bacillus subtilis*, *Staphylococcus aureus*, or *Lactococcus lactis*). In some embodiments, the signal peptide is a secA1 signal peptide (e.g., Usp45 signal peptide from *Laetococcus lactis* or Protective Antigen signal peptide from *Bacillus anthracis*). In some embodiments, the signal peptide is a secA2 signal peptide. In some embodiments the signal peptide is a Tat signal peptide (e.g., PhoD signal peptide from *B. subtilis*). In some embodiments, the *Listeria* bacterium is attenuated. For instance, in some embodiments, the *Listeria* are attenuated for cell-to-cell spread, entry into non-phagocytic cells, or proliferation. In some embodiments, the recombinant *Listeria* bacterium is deficient with respect to ActA, Internalin B, or both Act A and Internalin B (e.g., an Δ*actA*Δ*inlB* double deletion mutant). In some embodiments, the recombinant *Listeria* bacterium is deleted in functional ActA, Internalin B, or both Act A and Internalin B. In some embodiments, the nucleic acid of the recombinant bacterium has been modified by reaction with a nucleic acid targeting compound (e.g., a psoralen compound). This disclosure also provides a pharmaceutical composition comprising the recombinant *Listeria* bacterium and a pharmaceutically acceptable carrier. This disclosure further provides an immunogenic composition comprising the recombinant bacterium, wherein the polypeptide encoded by the second polynucleotide is an antigen. This disclosure also provides a vaccine comprising the recombinant *Listeria* bacterium. Methods of inducing an immune response in a host to an antigen comprising administering to the host an effective amount of a composition comprising the recombinant *Listeria* bacterium, wherein the polypeptide encoded by the second polynucleotide is (or comprises) an antigen are also provided. Also provided are methods of preventing or treating a condition (e.g., a disease such as cancer or an infectious disease) in a host comprising administering to the host an effective amount of a composition comprising the recombinant *Listeria* bacterium. The use of the bacterium in the manufacture of a medicament for inducing an immune response in a host to an antigen, wherein the polypeptide encoded by the second polynucleotide comprises the antigen is also provided.

In still another aspect, described herein is a recombinant *Listeria* bacterium (for instance, from the species *Listeria monocytogenes*) comprising an expression cassette which comprises a first polynucleotide encoding a non*-Listerial* signal peptide, a second polynucleotide encoding a polypeptide that is in the same translational reading frame as the first polynucleotide, and a promoter operably linked to both the first and second polynucleotides. The expression cassette encodes a fusion protein comprising both the non-*Listerial* signal peptide and the polypeptide. In some embodiments, the *Listeria* bacterium is attenuated for cell-to-cell spread, entry into non-phagocytic cells, or proliferation. In some embodiments, the first polynucleotide, the second polynucleotide, or both the first and second polynucleotides are codon-optimized for expression in *Listeria.* In some embodiments, the codon-optimization of the first and/or second polynucleotide enhances expression in and/or secretion from the bacterium of the encoded fusion protein (relative to the non-codon-optimized sequence). In some embodiments, the first polynucleotide and/or second polynucleotide is codon-optimized for expression in *Listeria monocytogenes.* In some embodiments, the polypeptide encoded by the second polynucleotide comprises an antigen. In some embodiments, the polypeptide encoded by the second polynucleotide is an antigen, which, in some instances, may be a non-bacterial antigen. For instance, the antigen is, in some embodiments a tumor-associated antigen or is derived from such a tumor-associated antigen. For instance, in some embodiments, the antigen is K-Ras, H-Ras, N-Ras,12-K-Ras, mesothelin, PSCA, NY-ESO-1, WT-1, survivin, gp100, PAP, proteinase 3, SPAS-1, SP-17, PAGE-4, TARP, or CEA, or is derived from K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA. For instance, in some embodiments, the antigen is mesothelin, or is a antigenic fragment or antigenic variant of mesothelin. In some other embodiments, the antigen is NY-ESO-1, or an antigenic fragment or antigenic variant of NY-ESO-1. In some embodiments, the antigen is an infectious disease antigen or is derived from an infectious disease antigen. In preferred embodiments, the signal peptide is bacterial. In some embodiments, the signal peptide is from a bacterium belonging to the genus *Bacillus, Staphylococcus,* or *Lactococcus*. For instance, in some embodiments, the signal peptide is from *Bacillus anthracis, Bacillus subtilis*, *Staphylococcus aureus*, or *Lactococcus lactis.* In some embodiments, the signal peptide is a secA1 signal peptide, such as a Usp45 signal peptide from *Lactococcus lactis* or a Protective Antigen signal peptide from *Bacillus anthracis*. In some embodiments, the signal peptide is a secA2 signal peptide. In still further embodiments, the signal peptide is a Tat signal peptide, such as a *B*. *subtilis* Tat signal peptide (e.g., PhoD). Pharmaceutical compositions, immunogenic compositions, and vaccines comprising the recombinant *Listeria* bacteria described herein are also provided. In addition, described herein are methods of using the recombinant *Listeria* bacteria to induce an immune response and/or to prevent or treat a condition such as a disease. The use of the bacterium in the manufacture of a medicament for inducing an immune response in a host to an antigen, wherein the polypeptide encoded by the second polynucleotide comprises the antigen is also provided.

This disclosure further provides a recombinant nucleic acid molecule, comprising (a) a first polynucleotide encoding a bacterial autolysin, or a catalytically active fragment or catalytically active variant thereof; and (b) a second polynucleotide encoding a polypeptide, wherein the second polynucleotide is in the same translational reading flame as the first polynucleotide, wherein the recombinant nucleic acid molecule encodes a protein chimera comprising the polypeptide encoded by the second polynucleotide and the autolysin, or catalytically active fragment or catalytically active variant thereof, wherein, in the protein chimera, the polypeptide is fused to the autolysin, or catalytically active fragment or catalytically active variant thereof, or is positioned within the autolysin, or catalytically active fragment or catalytically active variant thereof. In some embodiments, the first polynucleotide encodes a bacterial autolysin. In some embodiments, the protein chimera is catalytically active as an autolysin. In some embodiments, the bacterial autolysin is from an intracellular bacterium (e.g., *Listeria*). In some embodiments, the bacterial autolysin is a Listerial autolysin. In some embodiments, the second polynucleotide encoding the polypeptide is positioned within the first polynucleotide encoding the autolysin, or catalytically active fragment or catalytically active variant thereof, and the recombinant nucleic acid molecule encodes a protein chimera in which the polypeptide is positioned within the autolysin, or catalytically active fragment or catalytically active variant thereof (i.e., the polypeptide is embedded within the autolysin or catalytically active fragment or variant). In some alternative embodiments, the second polynucleotide is positioned outside of the first polynucleotide encoding the autolysin, or catalytically active fragment or catalytically active variant thereof, and the recombinant nucleic acid molecule encodes a protein chimera in which the polypeptide is fused to the autolysin, or catalytically active fragment or catalytically active variant thereof. In some embodiments, the polypeptide is heterologous to the autolysin. In some embodiments, the first polynucleotide and the second polynucleotide are heterologous to each other. In some embodiments, the recombinant nucleic acid molecule further comprises (c) a third polynucleotide encoding a signal peptide in the same translational reading frame as the first and second polynucleotides, wherein the recombinant nucleic acid molecule encodes a protein chimera comprising the signal peptide, the polypeptide encoded by the second polynucleotide, and the autolysin, or catalytically active fragment or catalytically active variant thereof. In some embodiments, the signal peptide is a secA2 signal peptide (such as p60). In some embodiments, the signal peptide is the signal peptide associated with the autolysin in nature (e.g., the signal peptide is p60 and the autolysin is p60). In some embodiments, the autolysin is a secA2-dependent autolysin. In some embodiments, the autolysin is a peptidoglycan hydrolase (e.g., N-acetylmuramidase or p60). In some embodiments, the polypeptide encoded by the second polynucleotide comprises an antigen. In some embodiments, the polypeptide is an antigen (e.g., a tumor-associated antigen, an antigen derived from a tumor-associated antigen, an infectious disease antigen, or an antigen derived from an infectious disease antigen. In some embodiments, the antigen is selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE4, TARP, and CEA, or is derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA. For instance, in some embodiments, the antigen is mesothelin, or an antigenic fragment or antigenic variant thereof. In some embodiments, the antigen is human mesothelin. In some embodiments, the antigen is human mesothelin deleted of its signal peptide and GPI anchor. This disclosure also provides an expression cassette comprising the recombinant nucleic acid molecule, further comprising a promoter operably linked to the first and second polynucleotides of the recombinant nucleic acid molecule, as well as an expression vector comprising the expression cassette. This disclosure further provides a recombinant bacterium comprising the recombinant nucleic acid molecule. In some embodiments, the recombinant bacterium is an intracellular bacterium, such as a *Listeria* bacterium (e.g., *Listeria monocytogenes*). In some embodiments, the polypeptide encoded by the second polynucleotide is foreign to the recombinant bacterium. A pharmaceutical composition comprising (a) the recombinant bacterium, and (b) a pharmaceutically acceptable carrier is also provided. In addition, an immunogenic composition comprising the recombinant bacterium, wherein the polypeptide encoded by the second polynucleotide is an antigen, is also provided. Also provided is a vaccine comprising the recombinant bacterium, wherein the polypeptide encoded by the second polynucleotide is an antigen. This disclosure also provides a method of inducing an immune response in a host to an antigen comprising administering to the host an effective amount of a composition comprising the recombinant bacterium, wherein the polypeptide encoded by the second polynucleotide is (or comprises) the antigen. A method of preventing or treating a condition in a host comprising administering to the host an effective amount of a composition comprising the recombinant bacterium is also provided. The use of the bacterium in the manufacture of a medicament for inducing an immune response in a host to an antigen, wherein the polypeptide encoded by the second polynucleotide comprises the antigen is also provided.

In yet another aspect, described herein is a recombinant *Listeria* bacterium comprising apolycistronic expression cassette, wherein the polycistronic expression cassette encodes at least two discrete non-Listerial polypeptides. For instance, in some embodiments, the expression cassette comprises a first polynucleotide encoding the first non-Listerial polypeptide, a second polynucleotide encoding the second non-Listerial polypeptide, and a promoter operably linked to the first and second polynucleotides. In some embodiments, the expression cassette further comprises an intergenic sequence between the first and second polynucleotides. In some embodiments, the polycistronic expression cassette is a bicistronic expression cassette which encodes two discrete non-Listerial polypeptides. In some embodiments, the recombinant *Listeria* bacterium belongs to the species *Listeria monocytogenes.* In some embodiments, at least one of the non-Listerial polypeptides encoded by the polycistronic expression cassette comprises an antigen. In some embodiments, at least two of the non-Listerial polypeptides each comprise fragments of the same antigen. In some embodiments, the antigen is a tumor-associated antigen or is derived from a tumor-associated antigen. For instance, in some embodiments, the antigen is an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or is derived from an antigen selected from the group consisting ofK-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA. In some embodiments, the antigen is mesothelin, or an antigenic fragment or antigenic variant thereof. In some embodiments, the antigen is human mesothelin. In some embodiments, the antigen is human mesothelin deleted of its signal peptide and GPI anchor. In some embodiments, the antigen is an infectious disease antigen or is derived from an infectious disease antigen. In some embodiments, at least one of the non-Listerial polypeptides encoded by the polycistronic expression cassette comprises a signal peptide (either a Listerial signal peptide or a non-Listerial signal peptide). In some embodiments, the signal peptide is a secA1 signal peptide. In some embodiments, the signal peptide is a secA2 signal peptide. In other embodiments, the signal peptide is a Tat signal peptide. In some embodiments, the expression cassette comprises a polynucleotide encoding the signal peptide, wherein the polynucleotide encoding the signal peptide is codon-optimized for expression in *Listeria.* This disclosure also provides a pharmaceutical composition comprising: (a) the recombinant *Listeria* bacterium, and (b) a pharmaceutically acceptable carrier. Also provided is an immunogenic composition comprising the recombinant *Listeria* bacterium. Also provided is a vaccine comprising the recombinant *Listeria* bacterium. A method of inducing an immune response in a host to an antigen comprising administering to the host an effective amount of a composition comprising the recombinant *Listeria* bacterium is also provided wherein at least one of the non-Listerial polypeptides comprises an antigen. A method of preventing or treating a condition in a host comprising administering to the host an effective amount of a composition comprising the recombinant *Listeria* bacterium is also provided. The use of the bacterium in the manufacture of a medicament for inducing an immune response in a host to an antigen, wherein at least one of the non-Listerial polypeptides encoded by the polycistronic expression cassette comprises the antigen is also provided.

In other aspects, described herein is a recombinant nucleic acid molecule, comprising (a) a first polynucleotide encoding a signal peptide, (b) a second polynucleotide encoding a secreted protein, or a fragment thereof, wherein the second polynucleotide is in the same translational reading frame as the first polynucleotide, and (c) a third polynucleotide encoding a polypeptide heterologous to the secreted protein, or fragment thereof, wherein the third polynucleotide is in the same translational reading frame as the first and second polynucleotides, wherein the recombinant nucleic acid molecule encodes a protein chimera comprising the signal peptide, the polypeptide encoded by the third polynucleotide, and the secreted protein, or fragment thereof, and wherein the polypeptide encoded by the third polynucleotide is fused to the secreted protein, or fragment thereof, or is positioned within the secreted protein, or fragment thereof, in the protein chimera. In some embodiments, the secreted protein is a naturally secreted protein (i.e., a protein that is secreted from its native cell). In some embodiments, the third polynucleotide is positioned within the second polynucleotide in the recombinant nucleic acid molecule, and the polypeptide encoded by the third polynucleotide is positioned with the secreted protein, or fragment thereof, in the protein chimera encoded by the recombinant nucleic acid molecule. In some embodiments, the third polynucleotide is positioned outside of the second polynucleotide in the recombinant nucleic acid molecule and the polypeptide encoded by the third polynucleotide is fused to the secreted protein or fragment thereof, in the protein chimera. An expression cassette comprising the recombinant nucleic acid molecule and further comprising a promoter operably linked to the first, second, and third polynucleotides of the recombinant nucleic acid molecule is also provided. In some embodiments, the polypeptide encoded by the second polynucleotide comprises an antigen. In some embodiments, the polypeptide encoded by the second polynucleotide is an antigen. For instance, in some embodiments, the antigen is a tumor-associated antigen or is derived from a tumor-associated antigen. (e.g., an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or is derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA). In some embodiments, the antigen is mesothelin, or an antigenic fragment or antigenic variant thereof. For instance, in some embodiments, the antigen is human mesothelin or is human mesothelin deleted of its signal peptide and GPI anchor. In alternative embodiments, the antigen is an infectious disease antigen or is derived from an infectious disease antigen. An expression vector comprising the expression cassette is also provided. Recombinant bacteria comprising the recombinant nucleic acid molecules are also provided. A recombinant *Listeria* bacterium (e.g., *Listeria monocytogenes*) is also provided and in some embodiments, the polypeptide encoded by the third nucleotide is foreign to the *Listeria* bacterium. This disclosure also provides an immunogenic composition comprising the recombinant bacterium, wherein the polypeptide encoded by the third polynucleotide is an antigen. Also provided is a method of inducing an immune response in a host to an antigen comprising administering to the host an effective amount of a composition comprising the recombinant bacterium, wherein the polypeptide encoded by the third polynucleotide is (or comprises) an antigen. Pharmaceutical compositions and vaccines, comprising the bacteria are also provided, as are methods of using the recombinant bacteria or compositions comprising the bacteria to prevent or treat a condition in a host. The use of the bacterium in the manufacture of a medicament for inducing an immune response in a host to an antigen, wherein the polypeptide encoded by the third polynucleotide comprises the antigen is also provided.

In further aspects, described herein are improved methods of expressing and secreting heterologous proteins in host bacteria. This disclosure also provides methods of improving expression and secretion of heterologous proteins in bacteria. This disclosure further provides methods of making the recombinant nucleic acid molecule, expression cassettes, expression vectors, and recombinant bacteria described herein.

This disclosure also provides a variety of polynucleotides useful in optimizing expression of heterologous polynucleotides in bacteria such as *Listeria.*

It will be understood that embodiments set forth in a Markush group, Markush claim, or by way of "or language," encompass each separate embodiment, any combination of each of separate embodiments, as well as an invention consisting of or comprising all of each of the separate embodiments, unless dictated otherwise explicitly or by the context.

Further descriptions of the aspects and embodiments described above as well as additional embodiments and aspects of this disclosure are provided below.

### II. Recombinant nucleic acid molecules

Described herein arc a variety of polynucleotides useful for expression of polynucleotides, such as heterologous polynucleotides, in bacteria such as *Listeria.* For instance, recombinant nucleic acid molecules comprising novel combinations of sequences encoding signal peptides (or polypeptides comprising signal peptides) with coding sequences of polypeptides such as heterologous antigens are provided. Recombinant nucleic acid molecules comprising codon-optimized polynucleotide sequences are provided. In some embodiments, these recombinant nucleic acid molecules are heterologous in that they comprise polynucleotides (i.e., polynucleotide sequences) which are not naturally found in combination with each other as part of the same nucleic acid molecule. In some embodiments, the recombinant nucleic acid molecules are isolated. In some embodiments, the recombinant nucleic acid molecules are positioned within the sequences of expression cassettes, expression vectors, plasmid DNA within bacteria, and/or even the genomic DNA of bacteria (following insertion). In some embodiments, the recombinant nucleic acid molecules provide enhanced expression and/or secretion of the polypeptide (e.g., a heterologous polypeptide) within a bacterium.

In some embodiments, the recombinant nucleic acid molecule is DNA. In some embodiments, the recombinant nucleic acid molecule is RNA. In some embodiments, the recombinant nucleic acid is single-stranded. In other embodiments, the recombinant nucleic acid is double-stranded.

In some embodiments, the recombinant nucleic acid molecules described herein encode a fusion protein such as fusion protein comprising a signal peptide and another polypeptide, such as a polypeptide heterologous to the signal peptide. In some embodiments, the signal peptide is a bacterial signal peptide. It is understood that the recited polypeptide components of a fusion protein may, but need not necessarily be, directly fused to each other. The polypeptide components of a fusion protein, may in some embodiments be separated on the polypeptide sequence by one or more intervening amino acid sequences. In some embodiments the other polypeptide is non-bacterial, for instance, mammalian or viral.

For instance, in one aspect, described herein is a recombinant nucleic acid molecule, comprising: (a) a first polynucleotide encoding a signal peptide, wherein the first polynucleotide is codon-optimized for expression in a bacterium; and (b) a second polynucleotide encoding a polypeptide (e.g., an antigen), wherein the second polynucleotide is in the same translational reading frame as the first polynucleotide, wherein the recombinant nucleic acid molecule encodes a fusion protein comprising the signal peptide and the polypeptide. In additional embodiments, the second polynucleotide (the polynucleotide encoding the polypeptide, such as an antigen) is also codon-optimized for expression in a bacterium. The bacterium for which the first and/or second polynucleotide is codon-optimized should be the bacterium of a type in which the recombinant nucleic acid molecule is intended to be placed.

In another aspect, described herein is a recombinant nucleic acid molecule, comprising (a) a first polynucleotide encoding a signal peptide native to a bacterium, wherein the first polynucleotide is codon-optimized for expression in the bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the polypeptide encoded by the second polynucleotide is heterologous to the signal peptide. In some embodiments, the second polynucleotide is heterologous to the first polynucleotide. In some embodiments, the polypeptide is heterologous to the bacterium to which the signal peptide is native (i.e., foreign to the bacterium). In some embodiments, the polypeptide encoded by the second polynucleotide is heterologous to the signal peptide, foreign to the bacterium, or both. In some embodiments, the bacterium from which the signal peptide is derived is an intracellular bacterium. In some embodiments, the bacterium is selected from the group consisting of *Listeria, Bacillus, Yersinia pestis, Salmonella, Shigella, Brucella,* mycobacteria and *E*. *coli.* In some embodiments, the signal peptide is native to a *Listeria* bacterium. In some embodiments, the signal peptide is native to a *Listeria* bacterium belonging to the species *Listeria monocytogenes.* In some embodiments, the second polynucleotide is codon-optimized for expression in the bacterium.

In another aspect, described herein is a recombinant nucleic acid molecule, wherein the recombinant nucleic acid molecule comprises (a) a first polynucleotide encoding a signal peptide, wherein the first polynucleotide is codon-optimized for expression in a *Listeria* bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the signal peptide is native to the *Listeria* bacterium. In some other embodiments, the signal peptide is foreign to the *Listeria* bacterium. In some embodiments, the signal peptide is heterologous to the polypeptide encoded by the second polynucleotide. In some embodiments, the polypeptide encoded by the second polynucleotide is heterologous to the *Listeria* bacterium. In some embodiments, the *Listeria* bacterium belongs to the species *Listeria motocytogenes*.

This disclosure also provides a recombinant nucleic acid molecule comprising a polynucleotide encoding a polypeptide foreign to a *Listeria* bacterium (e.g., a cancer or non-Listerial infectious disease antigen), wherein the polynucleotide encoding the foreign polypeptide is codon-optimized for expression in the *Listeria* bacterium.

In another aspect, described herein is a recombinant **nucleic** acid molecule, comprising: (a) a first polynucleotide encoding a non-secA1 bacterial signal peptide, and (b) a second polynucleotide encoding a polypeptide, such as an antigen, wherein the second polynucleotide is in the same translational reading frame as the first polynucleotide, wherein the recombinant nucleic acid molecule encodes a fusion protein comprising the signal peptide and the polypeptide. In some embodiments, the non-secA1 bacterial signal peptide is a secA2 signal peptide or a Tat signal peptide. In some embodiments, the first polynucleotide encoding the non-secA1 signal peptide is codon-optimized for expression in the bacteria in which the recombinant nucleic acid molecule is intended to be placed (e.g., *Listeria*). In some embodiments, the second polynucleotide encoding a polypeptide, such as an antigen, is codon-optimized for expression in the bacteria in which the recombinant nucleic acid molecule is intended to be placed. In some embodiments, the polypeptide encoded by the second polynucleotide is heterologous to the signal peptide. In some embodiments, the polypeptide encoded by the second polynucleotide is foreign to the bacterium in which the recombinant nucleic acid molecule is to be incorporated or has been incorporated. In some embodiments, the polypeptide encoded by the second polynucleotide is foreign to the bacterium in which the recombinant nucleic acid molecule is to be incorporated or has been incorporated and the polypeptide encoded by the second polynucleotide is also heterologous to the signal peptide.

This disclosure further provides a recombinant nucleic acid molecule, comprising a first polynucleotide encoding a non-secA1 bacterial signal peptide, a second polynucleotide encoding a polypeptide (e.g., heterologous protein and/or antigen), and a third polynucleotide encoding a SecA2 autolysin, or fragment thereof, in the same translational reading frame as the first and second polynucleotides, wherein the second polynucleotide is positioned within the third polynucleotide or between the first and third polynucleotides. In some embodiments, the recombinant nucleic acid molecule encodes a fusion protein comprising the signal peptide, the polypeptide, and the autolysin. In some embodiments, the fragment of the autolysin is catalytically active as an autolysin. In some embodiments, the autolysin is from an intracellular bacterium. In some embodiments, the autolysin is a peptidoglycan hydrolase. In some embodiments, the bacterial autolysin is a Listerial autolysin. In some embodiments, the autolysin is p60. In some embodiments, the autolysin is N-acetylmuramidase.

This disclosure also provides a recombinant nucleic acid molecule, wherein the recombinant nucleic acid molecule comprises (a) a first polynucleotide encoding a non-Listerial signal peptide; and (b) a second polynucleotide encoding a polypeptide that is in the same translational reading frame as the first polynucleotide, wherein the recombinant nucleic acid molecule encodes a fusion protein comprising both the non-Listerial signal peptide and the polypeptide. In some embodiments, the non-Listerial signal peptide is heterologous to the polypeptide encoded by the second polynucleotide. In some embodiments, the first polynucleotide, the second polynucleotide, or both the first and second polynucleotides are codon-optimized for expression in a *Listeria* bacterium.

This disclosure also provides a recombinant nucleic acid molecule, comprising (a) a first polynucleotide encoding a bacterial autolysin, or a catalytically active fragment or catalytically active variant thereof, and (b) a second polynucleotide encoding a polypeptide, wherein the second polynucleotide is in the same translational reading frame as the first polynucleotide, wherein the recombinant nucleic acid molecule encodes a protein chimera comprising the polypeptide encoded by the second polynucleotide and the autolysin, or catalytically active fragment or catalytically active variant thereof, wherein, in the protein chimera, the polypeptide is fused to the autolysin, or catalytically active fragment or catalytically active variant thereof, or is positioned within the autolysin, or catalytically active fragment or catalytically active variant thereof. In some embodiments, the second polynucleotide is positioned within the first polynucleotide, and the recombinant nucleic acid molecule encodes a protein chimera in which the polypeptide encoded by the second polynucleotide is positioned within the autolysin, or catalytically active fragment or catalytically active variant thereof. In some embodiments, the second polynucleotide is positioned outside the second polynucleotide, and the recombinant nucleic acid molecule encodes a protein chimera in which the polypeptide encoded by the second polynucleotide is fused to the autolysin, or catalytically active fragment or catalytically active variant thereof. In some embodiments, the first polynucleotide encodes an autolysin. In some embodiments, the recombinant nucleic acid molecule further comprises (c) a third polynucleotide encoding a signal peptide in the same translational reading frame as the first and second polynucleotides, wherein the recombinant nucleic acid molecule encodes a protein chimera comprising the signal peptide, the polypeptide encoded by the second polynucleotide, and the autolysin, or catalytically active fragment or catalytically active variant thereof. In some embodiments, the polypeptide encoded by the second polynucleotide is heterologous to the autolysin. In some embodiments, the fragments of the autolysin are at least about 30, at least about 40, at least about 50, or at least about 100 amino acids in length. In some embodiments, the autolysin is from an intracellular bacterium. In some embodiments, the bacterial autolysin is a Listerial autolysin. Catalytically active variants of an autolysin include variants that differ from the original autolysin in one or more substitutions, deletions, additions, and/or insertions. In some embodiments, the autolysin is a peptidoglycan hydrolase. In some embodiments, the autolysin is p60. In some embodiments, the autolysin is N-acetylmuramidase.

Additional autolysins can be identified and characterized by zymography, a technique known to those skilled in the art (see, e.g., Lenz, et al. (2003) Proc. Natl. Acad. Sci. USA 100:12432-12437). Zymography can also be used determine whether a given fragment and/or variant of an autolysin is catalytically active as an autolysin. The technique can also be used to assess whether or not a particular protein chimera is catalytically active as an autolysin.

In some embodiments, the catalytically active fragments and/or variants of the autolysin are at least about 10%, at least about 30%, at least about 50%, at least about 75%, at least about 90%, or at least about 95% as catalytically active as an autolysin as the native autolysin.

In some embodiments, the protein chimera is catalytically active as an autolysin. In some embodiments, the protein chimera is at least about 10%, at least about 30%, at least about 50%, at least about 75%, at least about 90%, or at least about 95% as catalytically active as an autolysin as the native autolysin.

Another option for heterologous protein expression is to utilize a protein "scaffold" into which a heterologous protein is functionally inserted "in-frame." In this composition, whole genes or components of the gene corresponding to, for example, MHC class I or MHC class II epitopes are inserted within and through a scaffold protein. The scaffold protein can be a highly expressed bacterial proteins (such as a *Listeria* protein, like LLO or p60), but in another embodiment can be a heterologous protein that is selected for its high expression, stability, secretion, and or (lack of) immunogenicity. Representative examples of scaffold proteins are chicken ovalbumin, or other human proteins, such as β-globin or albumin.

This disclosure also provides a recombinant nucleic acid molecule, comprising (a) a first polynucleotide encoding a signal peptide, (b) a second polynucleotide encoding a secreted protein, or a fragment thereof, wherein the second polynucleotide is in the same translational reading frame as the first polynucleotide, and (c) a third polynucleotide encoding a polypeptide heterologous to the secreted protein, or fragment thereof, wherein the third polynucleotide is in the same translational reading frame as the first and second polynucleotides, wherein the recombinant nucleic acid molecule encodes a protein chimera comprising the signal peptide, the polypeptide encoded by the second polynucleotide, and the secreted protein, or fragment thereof, and wherein the polypeptide is fused to the secreted protein, or fragment thereof, or is positioned within the secreted protein, or fragment thereof, in the protein chimera. In some embodiments, the second polynucleotide encodes a secreted protein. In some embodiments, the secreted protein is a protein that is secreted from its native cell. In some embodiments, the third polynucleotide is positioned within the second polynucleotide in the recombinant nucleic acid molecule, and the polypeptide encoded by the third polynucleotide is positioned with the secreted protein, or fragment thereof, in the protein chimera encoded by the recombinant nucleic acid molecule. In some embodiments, the third polynucleotide is positioned outside of the second polynucleotide in the nucleic acid molecule and the polypeptide encoded by the third polynucleotide is fused to the secreted protein or fragment thereof, in the protein chimera. In some embodiments, the secreted protein is ovalbumin. In some embodiments, a truncated form of ovalbumin, is used. In some embodiments, the secreted protein is p60. In some embodiments, the secreted protein is N-acetylmuramidase. In some embodiments, the signal peptide is the signal peptide normally associated with the secreted protein. In some embodiments, the signal peptide is heterologous to the secreted protein. In some embodiments, the fragments of the secreted protein are at least about 30, at least about 40, at least about 50, or at least about 100 amino acids in length.

In some embodiments, the recombinant nucleic acid molecule, expression cassette, or expression vector comprises a coding sequence for a polypeptide that is foreign to the bacteria, embedded within part or a whole coding sequence of a protein that is highly expressed within the bacteria. In some embodiments, the highly expressed sequence is native to the bacteria in which the sequence is to be expressed. In other embodiments, the highly expressed sequence is not native to the bacteria in which it is to be expressed, but provides sufficient expression, nonetheless.

In another aspect, described herein is a recombinant nucleic acid molecule, wherein the nucleic acid molecule encodes at least two discrete non-Listerial polypeptides. In some embodiments, the polynucleotides encoding the non-Listerial polypeptides are codon-optimized for expression in a *Listeria* bacterium.

Methods of preparing recombinant nucleic acid molecules, including those described above, are well known to those of ordinary skill in the art. For instance, recombinant nucleic acid molecules can be prepared by synthesizing long oligonucleotides on a DNA synthesizer which overlap with each other and then performing extension reaction and/or PCR to generate the desired quantity of double-stranded DNA. The double-stranded DNA can be cut with restriction enzymes and inserted into the desired expression or cloning vectors. Sequencing may be performed to verify that the correct sequence has been obtained. Also by way of non-limiting example, alternatively, one or more portions of the recombinant nucleic acid molecules may be obtained from plasmids containing the portions. PCR of the relevant portions of the plasmid and/or restriction enzyme excision of the relevant portions of the plasmid can be performed, followed by ligation and/or PCR to combine the relevant polynucleotides to generate the desired recombinant nucleic acid molecules. Such techniques are standard in the art. Standard cloning techniques may also be used to insert the recombinant nucleic acid sequence into a plasmid and replicate the recombinant nucleic acid within a host cell, such as bacteria. The recombinant **nucleic** acid can then be isolated from the host cell.

This disclosure also provides a method of using any of the recombinant nucleic acid molecules described herein to produce a recombinant bacterium (e.g. a recombinant *Listeria* bacterium). In some embodiments, the method of using a recombinant nucleic acid molecule described herein to make a recombinant bacterium comprises introducing the recombinant nucleic acid molecule into a bacterium. In some embodiments, the recombinant nucleic acid molecule is integrated into the genome of the bacterium. In some other embodiments, the recombinant nucleic acid molecule is on a plasmid which is incorporated within the bacterium. In some embodiments, incorporation of the recombinant nucleic acid molecule into the bacterium occurs by conjugation. The introduction into the bacterium can be effected by any of the standard techniques known in the art. For instance, incorporation of the recombinant nucleic acid molecule into the bacterium can occur by conjugation, transduction (transfection), or transformation.

### III. Signal peptides

In some embodiments, the recombinant **nucleic** acid molecules, expression cassettes, and/or vectors of this disclosure encode fusion proteins or protein chimeras which comprise signal peptides and are suitable for expression in and secretion from host cells such as bacteria. Thus, in some embodiments, the recombinant nucleic acid molecules, expression cassettes and/or vectors of this disclosure comprise polynucleotides encoding signal peptides.

The terms "signal peptide" and "signal sequence," are used interchangeably herein. In some embodiments, the signal peptide helps facilitate transportation of a polypeptide fused to the signal peptide across the cell membrane of a cell (e.g., a bacterial cell) so that the polypeptide is secreted from the cell. Accordingly, in some embodiments, the signal peptide is a "secretory signal peptide" or "secretory sequence". In some embodiments, the signal peptide is positioned at the N-terminal end of the polypeptide to be secreted.

In some embodiments, the sequence encoding the signal peptide in the recombinant nucleic acid molecule or expression cassette is positioned within the recombinant nucleic acid molecule or expression cassette such that the encoded signal peptide will effect secretion of the polypeptide to which it is fused from the desired host cell (e.g., a bacterium). In some embodiments, in a recombinant nucleic acid molecule or an expression cassette, the polynucleotide encoding the signal peptide is positioned in frame (either directly or separated by intervening polynucleotides) at the 5' end of the polynucleotide that encodes the polypeptide to be secreted (e.g., a polypeptide comprising an antigen).

In some embodiments, the signal peptides that are a part of the fusion proteins and/or protein chimeras encoded by the recombinant nucleic acid molecules, expression cassettes and/or expression vectors, are heterologous to at least one other polypeptide sequence in the fusion protein and/or protein chimera. In some embodiments, the signal peptide encoded by the recombinant nucleic acid molecule, expression cassette and/or expression vector is heterologous (i.e., foreign) to the bacterium into which the recombinant nucleic acid molecule, expression cassette and/or expression vector is to be incorporated or has been incorporated. In some embodiments, the signal peptide is native to the bacterium in which the recombinant nucleic acid molecule, expression cassette and/or expression vector is to be incorporated.

In some embodiments, the polynucleotide encoding the signal peptide is codon-optimized for expression in a bacterium (e.g., *Listeria* such as *Listeria monocytogenes*). In some embodiments, the polynucleotide that is codon-optimized for a particular bacterium is foreign to the bacterium. In other embodiments, the polynucleotide that is codon-optimized for a particular bacterium is native to that bacterium.

A large variety of signal peptides are known in the art. In addition, a variety of algorithms and software programs, such as the "SignalP" algorithms, which can be used to predict signal peptide sequences are available in the art. For instance, see: Antelmann et al., Genome Res., 11:1484-502 (2001); Menne et al., Bioinformatics, 16:741-2 (2000); Nielsen et al., Protein Eng., 10:1-6 (1997); Zhang et al., Protein Sci., 13:2819-24 (2004); Bendtsen et al., J. Mol. Biol., 340:783-95 (2004) (regarding SignalP 3.0); Hiller et al., Nucleic Acids Res., 32:W375-9 (2004); Schneider et al., Proteomics 4:1571-80 (2004); Chou, Curr. Protein Pept. Sci., 3:615-22 (2002); Shah et al., Bioinformatics, 19:1985-96 (2003); and Yuan et al., Biochem. Biophys. Res. Commun. 312:1278-83 (2003).

In some embodiments the signal peptide is prokaryotic. In some alternative embodiments, the signal peptide is eukaryotic. The use of eukaryotic signal peptides for expression of proteins in *Escherichia coli* for example, is described in Humphreys et al., Protein Expression and Purification, 20:252-264 (2000).

In some embodiments, the signal peptide is a bacterial signal peptide. In some embodiments, the signal peptide is a non-Listerial signal peptide. In some embodiments, the signal peptide is a Listerial signal peptide. In some embodiments the signal peptide is derived from a gram-positive bacterium. In some embodiments, the signal peptide is derived from an intracellular bacterium.

In some embodiments, the signal peptide (e.g., a non-secA1 bacterial signal peptide) used in a recombinant nucleic acid molecule, expression cassette, or expression vector is derived from *Listeria*. In some embodiments, this signal peptide is derived from *Listeria monocytogenes.* In some embodiments, the signal peptide is a signal peptide from *Listeria monocytogenes.* In some embodiments, the signal peptide is not derived from *Listeria,* but is instead derived from a bacterium other than a bacterium belonging to the genus *Listeria.* In some embodiments, the bacterial signal peptide is derived from a *Bacillus* bacterium. In some embodiments, the bacterial signal peptide is derived from *Bacillus subtilis.* In some embodiments, the bacterial signal peptide is derived from a bacterium belonging to the genus *Staphylococcus.* In some embodiments, the bacterial signal peptide is derived from a *Lactococcus* bacterium. In some embodiments, the bacterial signal peptide is derived from a *Bacillus, Staphylococcus,* or *Lactococcus* bacterium. In some embodiments, the bacterial signal peptide is a signal peptide from a *Bacillus, Staphylococcus*, or *Lactococcus* bacterium. In some embodiments, the bacterial signal peptide is a signal peptide derived from *Bacillus anthracis*, *Bacillus subtilis*, *Staphylococcus aureus*, or *Lactococcus lactis.* In some embodiments, the bacterial signal peptide is a signal peptide from *Bacillus anthracis*. In some embodiments, the bacterial signal peptide is a signal peptide from *Bacillus subtilis.* In some embodiments, the bacterial signal peptide is a signal peptide from *Lactococcus lactis.* In some embodiments, the bacterial signal peptide is a signal peptide from *Staphylococcus aureus*.

In some embodiments of the polynucleotides described herein, the signal peptide that is derived from an organism, such as a bacterium, is identical to a naturally occurring signal peptide sequence obtained from the organism. In other embodiments, the signal peptide sequence encoded by the recombinant nucleic acid molecule, expression cassette, and/or expression vector is derived from a naturally occurring signal peptide sequence, i.e., a fragment and/or variant of a naturally occurring signal peptide sequence, wherein the fragment or variant still functions as a signal peptide. A variant includes polypeptides that differ from the original sequence by one or more substitutions, deletions, additions, and/or insertions. For instance, in some embodiments the signal peptide that is encoded by the polynucleotides contains one or more conservative mutations. Possible conservative amino acid changes are well known to those of ordinary skill in the art. See, e.g., Section IV of the Detailed Description, below, for additional information regarding conservative amino acid changes.

A signal peptide derived from another signal peptide (i.e., a fragment and/or variant of the other signal peptide) is preferably substantially equivalent to the original signal peptide. For instance, the ability of a signal peptide derived from another signal peptide to function as a signal peptide should be substantially unaffected by the variations (deletions, mutations, etc.) made to the original signal peptide sequence. In some embodiments, the derived signal peptide is at least about 70%, at least about 80%, at least about 90%, or at least about 95% able to function as a signal peptide as the native signal peptide sequence. In some embodiments, the signal peptide has at least about 70%, at least about 80%, at least about 90%, or at least about 95% identity in amino acid sequence to the original signal peptide. In some embodiments, the only alterations made in the sequence of the signal peptide are conservative amino acid substitutions. Fragments of signal peptides are preferably at least about 80% or at least about 90% of the length of the original signal peptides.

In some embodiments, the signal peptide encoded by a polynucleotide in the recombinant nucleic acid molecules, expression cassettes, or expression vectors is a secA1 signal peptide, a secA2 signal peptide, or a Twin-arginine translocation (Tat) signal peptide. In some embodiments, the signal peptide is a secA1 signal peptide signal peptide. In some embodiments, the signal peptide is a non-secA1 signal peptide. In some embodiments, the signal peptide is a secA2 signal peptide. In some embodiments, the signal peptide is a twin-arginine translocation (Tat) signal peptide. In some embodiments, these secA1, secA2, or Tat signal peptides are derived from *Listeria.* In some embodiments, these secA1, secA2, or Tat signal peptides are non-Listerial. For instance, in some embodiments, the secA1, secA2, and Tat signal peptides are derived from bacteria belonging to one of the following genera: *Bacillus, Staphylococcus,* or *Lactococcus*.

Bacteria utilize diverse pathways for protein secretion, including secA1, secA2, and Twin-Arg Translocation (Tat). Which pathway is utilized is largely determined by the type of signal sequence located at the N-terminal end of the pre-protein. The majority of secreted proteins utilize the Sec pathway, in which the protein translocates through the bacterial membrane-embedded proteinaceous Sec pore in an unfolded confirmation. In contrast, the proteins utilizing the Tat pathway are secreted in a folded conformation. Nucleotide sequence encoding signal peptides corresponding to any of these protein secretion pathways can be fused genetically in-frame to a desired heterologous protein coding sequence. The signal peptides optimally contain a signal peptidase cleavage site at their carboxyl terminus for release of the authentic desired protein into the extra-cellular environment (Sharkov and Cai. 2002 J. Biol. Chem. 277:5796-5803; Nielsen et. al. 1997 Protein Engineering 10:1-6; and, www.cbs.dtu.dk/services/SignalP/).

The signal peptides used in the polynucleotides of this disclosure can be derived not only from diverse secretion pathways, but also from diverse bacterial genera. Signal peptides generally have a common structural organization, having a charged N-terminus (N-domain), a hydrophobic core region (H-domain) and a more polar C-terminal region (C-domain), however, they do not show sequence conservation. In some embodiments, the C-domain of the signal peptide carries a type I signal peptidase (SPase I) cleavage site, having the consensus sequence A-X-A, at positions -1 and -3 relative to the cleavage site. Proteins secreted via the sec pathway have signal peptides that average 28 residues. The secA2 protein secretion pathway was first discovered in *Listeria monocytogenes;* mutants in the secA2 paralogue are characterized by a rough colony phenotype on agar media, and an attenuated virulence phenotype in mice (Lenz and Portnoy, 2002 Mol. Microbiol. 45:1043-1056; and, Lenz et. al 2003 PNAS 100:12432-12437). Signal peptides related to proteins secreted by the Tat pathway have a tripartite organization similar to Sec signal peptides, but are characterized by having an RR-motif (R-R-X-#-#, where # is a hydrophobic residue), located at the N-domain / H-domain boundary. Bacterial Tat signal peptides average 14 amino acids longer than sec signal peptides. The *Bacillus subtilis* secretome may contain as many as 69 putative proteins that utilize the Tat secretion pathway, 14 of which contain a SPase I cleavage site (Jongbloed et. al. 2002 J. Biol. Chem. 277:44068-44078; Thalsma et. al., 2000 Microbiol. Mol. Biol. Rev. 64:515-547).

Shown in Table 1 below are non-limiting examples of signal peptides that can be used in fusion compositions (including protein chimera compositions) with a selected other polypeptide such as a heterologous polypeptide, resulting in secretion from the bacterium of the encoded protein.

**Table 1. Some exemplary signal peptides**

| Secretion Pathway | Signal Peptide Amino Acid Sequence (NH₂-CO₂) | Signal peptidase Site (cleavage site represented by) | Gene | Genus/species |
|---|---|---|---|---|
| **secA1** | MKKIML VFITLILVSLPIAQQ TEAKD (SEQ ID NO:45) | TEA'KD (SEQ ID NO:54) | *hly*(LLO) | *Listeria monocytogenes* |
| | MKKIIS AILMSTVILSAAAP LSGVYADT (SEQ ID NO:46) | VYA'DT (SEQ ID NO:55) | Usp45 | *Lactococcus lactis* |
| | MKKRKVLIPLMALSTILVSS TGNLEVIQAEV (SEQ ID NO:47) | IQA'EV (SEQ ID NO:56) | *pag* (Protective Antigen) | *Bacillus anthracis* |
| | | | | |
| **secA2** | MNMKKATIAATAGIAVTAF AAPTIASAST (SEQ ID NO:48) | ASA'ST (SEQ ID NO:57) | *iap* invasion-associated protein p60 | *Listeria monocylogenes* |
| | MQKTRKERILEALQEEKKN KKSKKFKTGATIAGVTAIAT SITVPGIEVIVSADE (SEQ ID NO:49) | VSA'DE (SEQ ID NO:58) | NamA Imo2691 (autolysin) | *Listeria monocytogenes* |
| | MKKLKMASCALVAGLMFS GLTPNAFAED (SEQ ID NO:50) | AFA'ED (SEQ ID NO:59) | * BA_0281 (NLP/P60 Family) | *Bacillus anthracis* |
| | MAKKFNYKLPSMVALTLVG SAVTAHQVQAAE (SEQ ID NO:51) | VQA'AE (SEQ ID NO:60) | ** atl* (autolysin) | *Staphylococcus aureus* |
| | | | | |
| **Tat** | MTDKKSENQTEKTETKENK GMTRREMLKLSAVAGTGIA VGATGLGTILNVVDQVDKA LT (SEQ ID NO:52) | DKA'LT (SEQ ID NO:61) | lmo0367 | *Listeria monocytogenes* |
| | MAYDSRFDEWVQKLKEESF QNNTFDRRKFIQGAGKIAGL SLGLTIAQSVGAFG (SEQ ID NO:53) | VGA'FG (SEQ ID NO:62) | PhoD (alkaline phosphatase) | *Bacillus subtilis* |

| | | | | |
|---|---|---|---|---|
| * Bacterial autolysins secreted by sec pathway (not determined whether secA1 or secA2). | | | | |

Accordingly, in some embodiments, the sequence that encodes the signal peptide encodes a secA1 signal peptide. An example of a secA1 signal peptide is the Listeriolysin O (LLO) signal peptide from *Listeria monocytogenes.* In some embodiments, the recombinant nucleic acid molecule or expression cassette comprising a polynucleotide encoding an LLO signal peptide further comprises a polynucleotide sequence encoding the LLO PEST sequence. Other examples of secA1 signal peptides suitable for use in the present invention include the signal peptides from the Usp45 gene in *Lactococcus lactis* (see Table 1, above, and Example 12 below) and Pag (Protective Antigen) gene from *Bacillus anthracis*. Thus, in some embodiments, the signal peptide is a protective antigen signal peptide from *Bacillus anthracis*. In some other embodiments, the signal peptide is a secA1 signal peptide other than the protective antigen signal peptide from *Bacillus anthracis*. Another example of a secA1 signal peptide is the SpsB signal peptide from *Staphylococcus aureus* (Sharkov et al., J. of Biological Chemistry, 277: 5796-5803 (2002)).

In some alternative embodiments, the heterologous coding sequences are genetically fused with signal peptides that are recognized by the secA2 pathway protein secretion complex. An auxiliary SecA paralog (SecA2) has been identified in nine Gram-positive bacteria that cause severe or lethal infections of humans. SecA2 is required for secretion of a subset of the exported proteomes (secretomes) of *Listeria,* Mycobacteria, and Streptococci (Braunstein et al., Mol. Microbiol. 48:453-64 (2003); Bensing et al., Mol. Microbiol., 44:1081-94 (2002); Lenz et al., Mol. Microbiol., 45:1043-1056 (2002); and Braunstein et al., J. Bacteriology, 183:6979-6990 (2001)). The *Listeria monocytogenes* SecA2 was identified through its association with bacterial smooth-rough variation, and mutations in *secA2* reduced virulence of *L. monocytogenes* and *Mycobacterium tuberculosis.*

For example, the *Listeria* protein p60 is a peptidoglycan autolysin that is secreted by the secA2 pathway. As an example, the secA2 signal peptide and signal peptidase cleavage site from p60 can be linked genetically with the amino terminus of a desired protein (e.g. antigen)-encoding gene. In one embodiment, the pre-protein comprised of the secA2 signal peptide and signal peptidase-antigen fusion is translated from an expression cassette within a bacterium, transported through the Gram-positive cell wall, in which the authentic heterologous protein is released into the extracellular milieu.

Alternatively, a heterologous sequence can be incorporated "in-frame" within p60, such that the heterologous protein is secreted in the form of a chimeric p60-heterologous protein. Insertion of the heterologous protein coding sequence in-frame into p60 can occur, for example, at the junction between the signal peptidase cleavage site and the mature p60 protein. In this embodiment, the chimeric protein retains the appropriate secA2 secretion signals, and also its autolysin activity, meaning that the heterologous protein is secreted as a gratuitous passenger of p60. In-frame incorporation of the heterologous antigen into p60 can be engineered at any point within p60 that retains both the secretion and autolysin activities of the p60 protein. An example of a partial expression cassette suitable for insertion of the desired antigen or other heterologous polypeptide coding sequence is described in Example 13, below.

In some embodiments, the fusion protein encoded by the recombinant nucleic acid molecule is a chimera comprising a bacterial protein having a particular desirable property (in addition to the desired heterologous protein such as an antigen). In some embodiments the chimera comprises a hydrolase. In some embodiments, the recombinant nucleic acid molecule encodes a p60 chimera comprising the endopeptidase p60, a peptidoglycan hydrolase that degrades the bacterial cell wall. In some embodiments, the fusion protein encoded by the recombinant nucleic acid molecule comprises a *L. monocytogenes* hydrolase, for example, p60 (see, e.g., Genbank accession no. NP_464110) or *N-*acetylmuramidase (NamA) (Genbank accession no. NP_466213), both of which are secA2 dependent secreted proteins that degrade the cell wall. Such particular protein chimera compositions take advantage of not only molecular chaperones required for secretion of bacterial proteins, but also of the activity of the bacterial protein that can facilitate its secretion. Particular protein chimeras comprised of precise placement of a heterologous protein encoding sequence with a *L. monocytogenes* hydrolase result in the efficient expression and secretion of the heterologous protein. (See, e.g., the specific example, Example 29, below.) Accordingly, in some embodiments, the signal peptide encoded by the recombinant nucleic acid molecule as part of a fusion protein is p60 signal peptide. In some embodiments, the signal peptide encoded by the recombinant nucleic acid molecule as part of a fusion protein is a NamA signal peptide.

In some embodiments, the recombinant nucleic acid molecule comprises a third polynucleotide sequence encoding p60 protein, or a fragment thereof, in the same translational reading frame as both the first polynucleotide encoding the p60 signal peptide and the second polynucleotide encoding the other polypeptide (e.g., antigen). The recombinant nucleic acid molecule then encodes a fusion protein comprising the signal peptide, the polypeptide encoded by the second polynucleotide (e.g., an antigen), and the p60 protein, or a fragment thereof. In such embodiments, the second polynucleotide is preferably positioned either within the third polynucleotide or between the first and third polynucleotides.

In some embodiments, the secA2 signal peptide is a secA2 signal peptide derived from *Listeria.* For instance, in some embodiments, the signal peptide is a secA2 signal peptide such as the p60 signal peptide or the N-acetylmuramidase (NamA) signal peptide from *L. monocytogenes.* In addition, other *L. monocytogenes* proteins have been identified as not being secreted in the absence of secA2 (Lenz et al., Mol. Microbiology 45:1043-1056 (2002)) and polynucleotides encoding the signal peptides from these proteins can be used in some embodiments. Additionally, secA2 signal peptides from bacteria other than *Listeria* can be utilized for expression and secretion of heterologous proteins from recombinant *Listeria* or other bacteria. For instance, as an illustrative but non-limiting example, secA2 signal peptides from *B. anthracis* can be used in the recombinant nucleic acid molecules and/or expression cassettes. In other embodiments, a secA2 signal peptide from S. *aureus* is used. See Table 1. Proteins secreted via the SecA2 pathway in other bacteria have also been identified (see, e.g., Braunstein et al., Mol. Microbiol., 48:453-64 (2003) and Bensing et al., Mol. Microbiol. 44:1081-94 (2002)).

Additional proteins secreted via the secA2 pathway can be identified. SecA2 homologues have been identified in a number of bacterial species (see, e.g., Lenz et al., Mol. Microbiology 45:1043-1056 (2002) and Braunstein et al., J. Bacteriology, 183:6979-6990 (2001)). Additional secA2 homologues can be identified by further sequence comparison using techniques known to those skilled in the art. Once a homologue is identified, the homologue can be deleted from the bacterial organism to generate a *ΔsecA2* mutant. The supernatant proteins of the wild-type and mutant bacterial cultures can be TCA-precipitated and analyzed by any of the proteomics techniques known in the art to determine which proteins are secreted by the wild-type bacteria, but not the *ΔsecA2* mutant. For instance, the secreted proteins can be analyzed via SDS-PAGE and silver staining. The resulting bands can be compared to identify those proteins for which secretion did not occur in the absence of the SecA2. (See, e.g., Lenz et al., Mol. Microbiology 45:1043-1056 (2002)). TheN-terminal sequences of these proteins can then be analyzed (e.g., with an algorithm to predict the signal peptide cleavage site) to determine the secA2 signal peptide sequence used by that protein. N-terminal sequencing by automated Edman degradation can also be performed to identify the sequence of the signal peptide.

In alternative embodiments, the polynucleotides encode polypeptides (e.g., heterologous polypeptide sequences) that are genetically fused with signal peptides that are recognized by the Tat pathway protein secretion complex. The Tat secretion pathway is utilized by bacteria, including *Listeria spp.,* for secretion of proteins that are folded within the bacterium. For example, the *Listeria innocua* protein YwbN has a putative Tat motif at its amino terminus and thus uses the Tat pathway for secretion (Genbank Accession No. NP_469731 [gi|16799463|ref|NP_469731.1| conserved hypothetical protein similar to *B*. *subtilis* YwbN protein (Listeria innocua)]). Another protein containing a Tat signal peptide is the YwbN protein from *Listeria monocytogenes* strain EGD(e) (Genbank Accession No. NP_463897 [gi|16802412|ref|NP_463897.1| conserved hypothetical protein similar to *B. subtilis* YwbN protein (Listeria monocytogenes EGD (e)]). As an example, the YwbN signal peptide and signal peptidase cleavage site from YwbN can be linked genetically with the amino terminus of a desired protein (e.g. antigen)-encoding gene. In this composition, the pre-protein comprised of the Tat signal peptide and signal peptidase-antigen fusion will be translated from an expression cassette within the bacterium, transported through the Gram-positive cell wall, in which the authentic heterologous protein is released into the extracellular milieu. Another protein predicted to be secreted from *Listeria innocua* via the Tat pathway is 3-oxoxacyl-acyl carrier protein synthase (Genbank Accession No. NP_471636 [gi|16801368|ref|NP_471636.1| similar to 3 (oxoacyl (acyl (carrier protein synthase (Listeria innocua)]). Polynucleotides encoding signal sequences from any of these proteins predicted to be secreted from *Listeria* via the Tat secretory pathway may be used in the polynucleotides, expression cassettes, and/or expression vectors described herein.

Tat signal sequences from other bacteria can also be used as signal peptides, including, but not restricted to, phoD from *B*. *subtilis*. Examples of Tat signal peptides from *Bacillus subtilis*, such as phoD, are described in Jongbloed et al., J. of Biological Chemistry, 277:44068-44078 (2002); Jongbloed et al., J. of Biological Chemistry, 275:41350-41357 (2000), Pop et al., J. of Biological Chemistry, 277:3268-3273 (2002); van Dijl et al., J. of Biotechnology, 98:243-254 (2002); and Tjalsma et al., Microbiology and Molecular Biology Reviews, 64: 515-547 (2000). Other proteins identified in *B*. *subtilis* that have been predicted to be secreted by the Tat pathway include those sequences having the following Genbank/Embl Accession Nos.: CAB15017 [gi|2635523|emb|CAB15017.1| similar to two (component sensor histidine kinase (YtsA) (Bacillus subtilis)]; CAB12056 [gi|2632548|emb|CAB12056.1| phosphodiesterase/alkaline phosphatase D (Bacillus subtilis)]; CAB12081 [gi|2632573|emb|CAB12081.1| similar to hypothetical proteins (Bacillus subtilis)]; CAB13278 [gi|2633776|emb|CAB13278.1| similar to hypothetical proteins (Bacillus subtilis)]; CAB14172 [gi|2634674|emb|CAB14172.1| menaquinol:cytochrome c oxidoreductase (iron (sulfur subunit) (Bacillus subtilis)]; CAB15089 [gi|2635595|emb|CAB15089.1| yubF (Bacillus subtilis)]; and CAB15852 [gi|2636361|emb|CAB15852.1| alternate gene name: ipa (29d~similar to hypothetical proteins (Bacillus subtilis). Thus, in some embodiments, the signal peptide encoded by the polynucleotide in the recombinant nucleic acid molecule and/or the expression cassettes is a Tat signal peptide derived from *B. subtilis*. Information on Tat signal peptides from *Pseudomonas aeruginosa* is provided in Ochsner et al., PNAS, 99: 8312-8317 (2002). Also, Tat signal peptides from a wide variety of other bacteria are described in Dilks et al., J. of Batcteriology, 185: 1478-1483 (2003) and Berks et al., Molecular Microbiology, 35:260-274 (2004).

Additional Tat signal peptide may be identified and distinguished from Sec-type signal peptides by their "twin-arginine" consensus motif. As noted above, signal peptides related to proteins secreted by the Tat pathway have a tripartite organization similar to Sec signal peptides, but are characterized by having an RR-motif (R-R-X-#-#, where # is a hydrophobic residue) located at the N-domain / H-domain boundary. Tat signal peptides are also generally longer and less hydrophobic than the Sec-type signal peptides. See, e.g., Berks et al., Adv. Microb. Physiol., 47:187-254 (2003) and Berks et al., Mol. Microbiol. 35:260-74 (2000).

In addition, techniques analogous to those described above for the identifying new proteins secreted by the SecA2 pathway and their corresponding SecA2 signal peptides can also be used to identify new proteins secreted via the Tat pathway and their signal peptides. The reference Jongbloed et al., J. Biological Chem., 277:44068-44078 (2002) provides examples of techniques which can be used to identify a protein expressed by a type of bacteria as a protein secreted via the twin-arginine translocation pathway.

### IV. Polypeptides

The recombinant nucleic acid molecules described herein, as well as the expression cassettes or expression vectors described herein, can be used to encode any desired polypeptide. In particular, the recombinant nucleic acid molecules, expression cassettes, and expression vectors are useful for expressing heterologous polypeptides in a bacterium.

In some embodiments (depending on the recombinant nucleic acid molecule, expression cassette or expression vector used), the polypeptide encoded by a polynucleotide of the recombinant nucleic acid molecule, expression cassette, and/or expression vector is encoded as part of a fusion protein with a signal peptide. In other embodiments, the encoded polypeptide is encoded as a discrete polypeptide by the recombinant nucleic acid molecule, expression cassette, or expression vector. In still other embodiments, the polypeptide encoded by a polynucleotide of the recombinant nucleic acid molecule, expression cassette, or expression vector is encoded as part of a fusion protein that does not include a signal peptide. In still other embodiments, the polypeptide encoded by a polynucleotide of the recombinant nucleic acid molecule, expression cassette, or expression vector of this disclosure is encoded as part of a fusion protein (also referred to herein as a protein chimera) in which the polypeptide is embedded within another polypeptide sequence.

Thus, it is understood that each of the polypeptides listed herein (below and elsewhere) which are encoded by polynucleotides of the recombinant nucleic acid molecules, expression cassettes, or expression vectors of this disclosure may be expressed as either fusion proteins (fused to signal peptides and/or to or in other polypeptides) or as discrete polypeptides by the recombinant nucleic acid molecule, expression cassette, or expression vector, depending on the particular recombinant nucleic acid molecule, expression cassette or expression vector used. For instance, in some embodiments, a recombinant nucleic acid molecule comprising a polynucleotide encoding the antigen CEA will encode CEA as a fusion protein with a signal peptide.

In some embodiments, the polypeptide is part of a fusion protein encoded by the recombinant nucleic acid molecule, expression cassette, or expression vector and is heterologous to the signal peptide of the fusion protein. In some embodiments, the polypeptide is positioned within another polypeptide sequence (e.g., a secreted protein or an autolysin, or fragments or variants thereof) to which it is heterologous.

In some embodiments, the polypeptide is bacterial (either Listerial or non-Listerial). In some embodiments, the polypeptide is not bacterial. In some embodiments, the polypeptide encoded by the polynucleotide is a mammalian polypeptide. For instance, the polypeptide may correspond to a polypeptide sequence found in humans (i.e., a human polypeptide). In some embodiments, the polypeptide is Listerial. In some embodiments, the polypeptide is non-Listerial. In some embodiments, the polypeptide is not native (i.e., is foreign) to the bacterium in which the recombinant nucleic acid molecule, expression cassette, and/or expression vector is to be incorporated or is incorporated.

In some embodiments, the polynucleotide encoding the polypeptide is codon-optimized for expression in a bacterium. In some embodiments, the polynucleotide encoding the polypeptide is fully codon-optimized for expression in a bacterium. In some embodiments, the polypeptide which is encoded by the codon-optimized polynucleotide is foreign to the bacterium (i.e., is heterologous to the bacterium).

The term "polypeptide" is used interchangeably herein with "peptide" and "protein" and no limitation with respect to the length or size of the amino acid sequence contained therein is intended. Typically, however, the polypeptide will comprise at least about 6 amino acids. In some embodiments, the polypeptide will comprise, at least about 9, at least about 12, at least about 20, at least about 30, or at least about 50 amino acids. In some embodiments, the polypeptide comprises at least about 100 amino acids. In some embodiments, the polypeptide is one particular domain of a protein (e.g., an extracellular domain, an intracellular domain, a catalytic domain, or a binding domain). In some embodiments, the polypeptide comprises an entire (i.e., full-length) protein.

In some embodiments, the polypeptide that is encoded by a polynucleotide of a recombinant nucleic acid molecule, expression cassette, and/or expression vector comprises an antigen or a protein that provides a palliative treatment for a disease. In some embodiments, the polypeptide that is encoded by a polynucleotide of a recombinant nucleic acid molecule, expression cassette, and/or expression vector is an antigen or a protein that provides a palliative treatment for a disease. In some embodiments, the polypeptide that is encoded is a therapeutic protein (or comprises a therapeutic protein).

In some embodiments, the polypeptide that is encoded by a polynucleotide of a recombinant nucleic acid molecule, expression cassette, and/or vector comprises an antigen (e.g., any of the antigens described herein). In some embodiments, the polypeptide that is encoded by a polynucleotide of a recombinant nucleic acid molecule, expression cassette, and/or vector is an antigen. In some embodiments, the antigen is a bacterial antigen. In some embodiments, the antigen is a non-Listerial bacterial antigen. In some embodiments, however, the antigen is a non-Listerial antigen. In other embodiments, the antigen is a non-bacterial antigen. In some embodiments, the antigen is a mammalian antigen. In some embodiments, the antigen is a human antigen. In some embodiments, the polypeptide is (or comprises) an antigen comprising one or more immunogenic epitopes. In some embodiments, the antigen comprises one or more MHC class I epitopes. In other embodiments, the antigen comprises one or more MHC class II epitope. In some embodiments, the epitope is a CD4+ T-cell epitope. In other embodiments, the epitope is a CD8+ T-cell epitope.

The polynucleotide encoding an antigen is not limited to any exact nucleic acid sequence (e.g., that encoding a naturally occurring, full-length antigen) but can be of any sequence that encodes a polypeptide that is sufficient to elicit the desired immune response when administered to an individual within the bacteria or compositions of this disclosure The term "antigen," as used herein, is also understood to include fragments of larger antigen proteins so long as the fragments are antigenic (i.e., immunogenic). In addition, in some embodiments, the antigen encoded by a polynucleotide of the recombinant nucleic acid, expression cassette, or expression vector may be a variant of a naturally occurring antigen sequence. (Similarly for polynucleotides encoding other, non-antigen proteins, the sequences of the polynucleotides encoding a given protein may vary so long as the desired protein that is expressed provides the desired effect (e.g. a palliative effect) when administered to an individual.)

An antigen that is derived from another antigen includes an antigen that is an antigenic (i.e., immunogenic) fragment of the other antigen, an antigenic variant of the other antigen, or an antigenic variant of a fragment of the other antigen. A variant of an antigen includes antigens that differ from the original antigen in one or more substitutions, deletions, additions, and/or insertions.

The antigenic fragment may be of any length, but is most typically at least about 6 amino acids, at least about 9 amino acids, at least about 12 amino acids, at least about 20 amino acids, at least about 30 amino acids, at least about 50 amino acids, or at least about 100 amino acids. An antigenic fragment of an antigen comprises at least one epitope from the antigen. In some embodiments, the epitope is a MHC class I epitope. In other embodiments, the epitope is a MHC class II epitope. In some embodiments, the epitope is a CD4+ T-cell epitope. In other embodiments, the epitope is a CD8+ T-cell epitope.

A variety of algorithms and software packages useful for predicting antigenic regions (including epitopes) within proteins are available to those skilled in the art. For instance, algorthims that can be used to select epitopes that bind to MHC class I and class II molecules are publicly available. For instance, the publicly available "SYFPEITHI" algorithm can be used to predict MHC-binding peptides (Rammensee et al. (1999) Immunogenetics 50:213-9). For other examples of publicly available algorithms, see the following references: Parker et al. (1994) J. Immunol 152:163-75; Singh and Raghava (2001) Bioinformatics 17:1236-1237; Singh and Raghava (2003) Bioinformatics 19:1009-1014; Mallios (2001) Bioinformatics 17:942-8; Nielsen et al. (2004) Bioinformatics 20:1388-97; Donnes et al. (2002) BMC Bioinformatics 3:25; Bhasin, et al. (2004) Vaccine 22:3195-204; Guan et al. (2003) Nucleic Acids Res 31:3621-4; Reche et al. (2002) Hum. Immunol. 63:701-9; Schirle et al. (2001) J. Immunol Methods 257:1-16; Nussbaum et al. (2001) Immunogenetics (2001) 53:87-94; Lu et al. (2000) Cancer Res. 60:5223-7. See also, e.g., Vector NTI® Suite (Informax, Inc, Bethesda, MD), GCG Wisconsin Package (Accelrys, Inc., San Diego, CA), Welling, et al. (1985) FEBS Lett. 188:215-218, Parker, et al. (1986) Biochemistry 25:5425-5432, Van Regenmortel and Pellequer (1994) Pept. Res. 7:224-228, Hopp and Woods (1981) PNAS 78:3824-3828, and Hopp (1993) Pept. Res. 6:183-190. Some of the algorthims or software packages discussed in the references listed above in this paragraph are directed to the prediction of MHC class I and/or class II binding peptides or epitopes, others to identification of proteasomal cleavage sites, and still others to prediction of antigenicity based on hydrophilicity.

Once a candidate antigenic fragment believed to contain at least one epitope of the desired nature has been identified, the polynucleotide sequence encoding that sequence can be incorporated into an expression cassette and introduced into a *Listeria* vaccine vector or other bacterial vaccine vector. The immunogenicity of the antigenic fragment can then be confirmed by assessing the immune response generated by the *Listeria* or other bacteria expressing the fragments. Standard immunological assays such as ELISPOT assays, Intracellular Cytokine Staining (ICS) assay, cytotoxic T-cell activity assays, or the like, can be used to verify that the fragment of the antigen chosen maintains the desired imunogenicity. Examples of these types of assays are provided in the Examples below (see, e.g., Example 21). In addition, the anti-tumor efficacy of the *Listeria* and/or bacterial vaccines can also be assessed using the methods described below in the Examples (e.g., implantation of CT26 murine colon cells expressing the antigen fragment in mice, followed by vaccination of the mice with the candidate vaccine and observation of effect on tumor size, metastasis, survival, etc. relative to controls and/or the full-length antigen).

In addition, large databases containing epitope and/or MHC ligand information using for identifying antigenic fragments are publicly available. See, e.g., Brusic et al. (1998) Nucleic Acids Res. 26:368-371; Schonbach et al. (2002) Nucleic Acids Research 30:226-9; and Bhasin et al. (2003) Bioinformatics 19:665-666; and Rammensee et al. (1999) Immunogenetics 50:213-9.

The amino acid sequence of an antigenic variant has at least about 60%, at least about 70%, at least about 80%, at least about 90%, at least about 95%, or at least about 98% identity to the original antigen.

In some embodiments, the antigenic variant is a conservative variant that has at least about 80% identity to the original antigen and the substitutions between the sequence of the antigenic variant and the original antigen are conservative amino acid substitutions. The following substitutions are considered conservative amino acid substitutions: valine, isoleucine, or leucine are substituted for alanine; lysine, glutamine, or asparagine are substituted for arginine; glutamine, histidine, lysine, or arginine are substituted for asparagine; glutamic acid is substituted for aspartic acid; serine is substituted for cysteine; asparagine is substituted for glutamine; aspartic acid is substituted for glutamic acid; proline or alanine is substituted for glycine; asparagine, glutamine, lysine or arginine is substituted for histidine; leucine, valine, methionine, alanine, phenylalanine, or norleucine is substituted for isoleucine; norleucine, isoleucine, valine, methionine, alanine, or phenylalanine is substituted for leucine; arginine, glutamine, or asparagine is substituted for lysine; leucine, phenylalanine, or isoleucine is substituted for methionine; leucine, valine, isoleucine, alanine, or tyrosine is substituted for phenylalanine; alanine is substituted for proline; threonine is substituted for serine; serine is substituted for threonine; tyrosine or phenylalanine is substituted for tryptophan; tryptophan, phenylalanine, threonine, or serine is substituted for tyrosine; tryptophan, phenylalanine, threonine, or serine is substituted for tyrosine; isoleucine, leucine, methionine, phenylalanine, alanine, or norleucine is substituted for valine. In some embodiments, the antigenic variant is a convervative variant that has at least about 90% identity to the original antigen.

In some embodiments, an antigen derived from another antigen is substantially equivalent to the other antigen. An antigen derived from another antigen is substantially equivalent to the original antigen from which it is derived if the antigen if the derived antigen has at least about 70% identity in amino acid sequence to the original antigen and maintains at least about 70% of the immunogenicity of the original antigen. In some embodiments, the substantially equivalent antigen has at least about 80%, at least about 90%, at least about 95%, or at least about 98% identity in amino acid sequence to the original antigen. In some embodiments, the substantially equivalent antigen comprises only conservative substitutions relative to the original antigen. In some embodiments, the substantially equivalent antigen maintains at least about 80%, at least about 90%, or at least about 95% of the immunogenicity of the original antigen. To determine the immunogenicity of a particular derived antigen and compare to that of the original antigen to determine whether the derived antigen is substantially equivalent to the original antigen, one can test both the derived and original antigen in any of a number of immunogenicity assays known to those skilled in the art. For instance, *Listeria* expressing either the original antigen or the derived antigen can be prepared as described herein. The ability of those *Listeria* expressing the different antigens to produce an immune response can be measured by vaccinating mice with the *Listeria* and then assessing the immunogenic response using the standard techniques of ELISPOT assays, Intracellular Cytokine Staining (ICS) assay, cytotoxic T-cell activity assays, or the like. Examples of these types of assays are provided in the examples below (see, e.g., Example 21).

In some embodiments, the polypeptide encoded by a polynucleotide of the recombinant nucleic acid molecule, expression cassette, and/or vector comprises an antigen. In some embodiments, the antigen is selected from the group consisting of a tumor-associated antigen, a polypeptide derived from a tumor-associated antigen, an infectious disease antigen, and a polypeptide derived from an infectious disease antigen.

In some embodiments, the polypeptide encoded by a polynucleotide of the recombinant nucleic acid molecule, expression cassette, and/or vector comprises a tumor-associated antigen or comprises an antigen derived from a tumor-associated antigen. In some embodiments, the polypeptide comprises a tumor-associated antigen. In some embodiments, the encoded polypeptide comprises more than one antigen that is a tumor-associated antigen or an antigen derived from a tumor-associated antigen. For instance, in some embodiments, the encoded polypeptide comprises both mesothelin (or an antigenic fragment or antigenic variant thereof) and K-Ras, 12-K-Ras, or PSCA (or an antigenic fragment or antigenic variant of K-Ras, 12-K-Ras, or PSCA).

In some embodiments, the antigen encoded by a polynucleotide of the recombinant nucleic acid molecule, expression cassette, and/or expression vector is a tumor-associated antigen or is an antigen that is derived from a tumor-associated antigen. In some embodiments, the antigen is a tumor-associated antigen.

In some embodiments, a polynucleotide in a recombinant nucleic acid molecule, expression cassette, and/or expression vector encodes an antigen (or encodes a polypeptide comprising an antigen) that is not identical to a tumor-associated antigen, but rather is an antigen derived from a tumor-associated antigen. For instance, in some embodiments, the antigen encoded by a polynucleotide of a recombinant nucleic acid molecule, expression cassette, and/or expression vector may comprise a fragment of a tumor-associated antigen, a variant of a tumor-associated antigen, or a variant of a fragment of a tumor-associated antigen. In some cases, an antigen, such as a tumor antigen, is capable of inducing a more significant immune response in a vaccine when the amino acid sequence differs slightly from that endogenous to a host. In other cases, the derived antigen induces a less significant immune response than the original antigen, but is, for instance, more convenient for heterologous expression in a Listerial vaccine vector due to a smaller size. In some embodiments, the amino acid sequence of a variant of a tumor-associated antigen, or a variant of a fragment of a tumor-associated antigen, differs from that of the tumor-associated antigen, or its corresponding fragment, by one or more amino acids. The antigen derived from a tumor-associated antigen will comprise at least one epitope sequence capable of inducing the desired immune response upon expression of the polynucleotide encoding the antigen within a host.

Accordingly, in some embodiments, a polynucleotide in the recombinant nucleic acid molecule, expression cassette, or vector encodes a polypeptide that comprises an antigen derived from a tumor-associated antigen, wherein the antigen comprises at least one antigenic fragment of a tumor-associated antigen. In some embodiments, a polynucleotide in the recombinant nucleic acid molecule, expression cassette, or vector encodes an antigen that is derived from a tumor-associated antigen, wherein the antigen comprises at least one antigenic fragment of a tumor-associated antigen. The antigenic fragment comprises at least one epitope of the tumor-associated antigen. In some embodiments, the antigen that is derived from another antigen is an antigenic (i.e., immunogenic) fragment or an antigenic variant of the other antigen. In some embodiments, the antigen is an antigenic fragment of the other antigen. In some embodiments, the antigen is an antigenic variant of the other antigen.

A large number of tumor-associated antigens that are recognized by T cells have been identified (Renkvist et al., Cancer Immunol Innumother 50:3-15 (2001)). These tumor-associated antigens may be differentiation antigens (e.g., PSMA, Tyrosinase, gp100), tissue-specific antigens (e.g. PAP, PSA), developmental antigens, tumor-associated viral antigens (e.g. HPV 16 E7), cancer-testis antigens (e.g. MAGE, BAGE, NY-ESO-1), embryonic antigens (e.g. CEA, alpha-fetoprotein), oncoprotein antigens (e.g. Ras, p53), over-expressed protein antigens (e.g. ErbB2 (Her2/Neu), MUC1), or mutated protein antigens. The tumor-associated antigens that may be encoded by the heterologous nucleic acid sequence include, but are not limited to, 707-AP, Annexin II, AFP, ART-4, BAGE, β-catenin/m, BCL-2, bcr-abl, bcr-abl p190, bcr-abl p210, BRCA-1, BRCA-2, CAMEL, CAP-1, CASP-8, CDC27/m, CDK-4/m, CEA (Huang et al., Exper Rev. Vaccines (2002)1:49-63), CT9, CT10, Cyp-B, Dek-cain, DAM-6 (MAGE-B2), DAM-10 (MAGE-B1), EphA2 (Zantek et al., Cell Growth Differ. (1999) 10:629-38; Carles-Kinch et al., Cancer Res. (2002) 62:2840-7), ELF2M, EphA2 (Zantek et al., Cell Growth Duffer. (1999) 10:629-38; Carles-Kinch et al., Cancer Res. (2002) 62:2840-7), ETV6-AML1, G250, GAGE-1, GAGE-2, GAGE-3, GAGE-4, GAGE-5, GAGE-6, GAGE-7B, GAGE-8, GnT-V, gp100, HAGE, HER2/neu, HLA-A*0201-R170I, HPV-E7, H-Ras, HSP70-2M, HST-2, hTERT, hTRT, iCE, inhibitors of apoptosis (e.g. survivin), KIAA0205, K-Ras, 12-K-Ras (K-Ras with codon 12 mutation), LAGE, LAGE-1, LDLR/FUT, MAGE-1, MAGE-2, MAGE-3, MAGE-6, MAGE-A1, MAGE-A2, MAGE-A3, MAGE-A4, MAGE-A6, MAGE-A10, MAGE-A12, MAGE-B5, MAGE-B6, MAGE-C2, MAGE-C3, MAGE-D, MART-1, MART-1/Melan-A, MC1R, MDM-2, mesothelin, Myosin/m, MUC1, MUC2, MUM-1, MUM-2, MUM-3, neo-polyA polymerase, NA88-A, N-Ras, NY-ESO-1, NY-ESO-1 a (CAG-3), PAGE-4, PAP, Proteinase 3 (PR3) (Molldrem et al., Blood (1996) 88:2450-7; Molldrem et al., Blood (1997) 90:2529-34), P15, p190, Pm1/RARα, PRAME, PSA, PSM, PSMA, RAGE, RAS, RCAS1, RU1, RU2, SAGE, SART-1, SART-2, SART-3, SP17, SPAS-1, TEL/AML1, TPI/m, Tyrosinase, TARP, TRP-1 (gp75), TRP-2, TRP-2/INT2, WT-1, and alternatively translated NY-ESO-ORF2 and CAMEL proteins, derived from the NY-ESO-1 and LAGE-1 genes.

In some embodiments, the antigen encoded by the polynucleotide in the recombinant nucleic acid molecule, expression cassette, and/or vector may encompass any tumor-associated antigen that can elicit a tumor-specific immune response, including antigens yet to be identified. In some embodiments, the polynucleotide in the recombinant nucleic acid molecule, expression cassette, and/or vector encodes more than one tumor-associated antigen.

In some embodiments, the antigen is mesothelin (Argani et al., Clin Cancer Res. 7(12):3862-8 (2001)), Sp17 (Lim et al., Blood 97(5):1508-10 (2001)), gp100 (Kawakami et al., Proc. Natl. Acad. Sci. USA 91:6458 (1994)), PAGE-4 (Brinkmann et al., Cancer Res. 59(7):1445-8 (1999)), TARP (Wolfgang et al., Proc. Natl. Acad. Sci. USA 97(17):9437-42 (2000)), EphA2 (Tatsumi et al., Cancer Res. 63(15):4481-9 (2003)), PR3 (Muller-Berat et al., Clin. Immunol. Immunopath. 70(1):51-9 (1994)), prostate stem cell antigen (PSCA) (Reiter et al., Proc. Natl. Acad. Sci., 95:1735-40 (1998); Kiessling et al., Int. J. Cancer, 102:390-7 (2002)), or SPAS-1 (U.S. Patent Application Publication No. 2002/0150588).

In some embodiments of this disclosure the antigen encoded by the recombinant nucleic acid molecule or expression cassette is CEA. In other embodiments, the antigen is an antigenic fragment and/or antigenic variant of CEA. CEA is a 180-kDA membrane intercellular adhesion glycoprotein that is over-expressed in a significant proportion of human tumors, including 90% of colorectal, gastric, and pancreatic, 70% of non-small cell lung cancer, and 50% of breast cancer (Hammarstrom, Semin. Cancer Biol., 9:67-81). A variety of immunotherapeutics such as anti-idiotype monoclonal antibody mimicking CEA (Foon et al., Clin. Cancer Res., 87:982-90 (1995), or vaccination using a recombinant vaccinia virus expressing CEA (Tsang et al., J. Natl. Cancer Inst., 87:982-90 (1995)) have been investigated, unfortunately, however, with limited success. Nonetheless, investigators have identified a HLA*0201-restricted epitope, CAP-1(CEA605-613), that is recognized by human T cell lines that were generated from vaccinated patients. Vaccination of patients with DC pulsed with this epitope failed to induce clinical responses (Morse et al., Clin. Cancer Res., 5:1331-8 (1999)). Recently, a CEA605-613 peptide agonist was identified with a heteroclitic aspartate to asparagine substitution at position 610 (CAP1-6D). Although this amino acid substitution did not alter MHC binding affinity of this peptide, the use of the altered peptide ligand (APL) resulted in improved generation of CEA-specific cytotoxic T lymphocytes (CTL) *in vitro.* CAP1-6D-specific CTL maintained their ability to recognize and lyse tumor cells expressing native CEA (Zaremba et al., Cancer Res., 57:4570-7 (1997); Salazar et al., Int. J. Cancer, 85:829-38 (2000)). Fong et al. demonstrated induction of CEA-specific immunity in patients with colon cancer vaccinated with Flt3-ligand expanded DC incubated with this APL. Encouragingly, 2 of 12 patients after vaccination experienced dramatic tumor regressions that correlated with the induction of peptide-MHC tetramer⁺ T cells (Fong et al., Proc. Natl. Acad. Sci. U.S.A., 98:8809-14 (2001)).

In another embodiment, the antigen is proteinase-3 or is derived from proteinase-3. For instance, in one embodiment, the antigen comprises the HLA-A2.1-restricted peptide PR1 (aa 169-177; VLQELNVTV (SEQ ID NO:63)). Information on proteinase-3 and/or the PR1 epitope is available in the following references: US Patent No. 5,180,819, Molldrem, et al., Blood, 90:2529-2534 (1997); Molldrem et al., Cancer Research, 59:2675-2681 (1999); Molldrem, et al., Nature Medicine, 6:1018-1023 (2000); and Molldrem et al., Oncogene, 21: 8668-8673 (2002).

In some embodiments, the polypeptide encoded by a polynucleotide in the recombinant nucleic acid molecule, expression cassette, and/or vector comprises an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or comprises an antigen derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA.

In some embodiments, the polypeptide encoded by a polynucleotide in the recombinant nucleic acid molecule, expression cassette, and/or vector comprises an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA. In some embodiments, the polypeptide comprises K-Ras. In some embodiments, the polypeptide comprises H-Ras. In some embodiments, the polypeptide comprises N-Ras. In some embodiments, the polypeptide comprises K-Ras. In some embodiments, the polypeptide comprises mesothelin (e.g., human mesothelin). In some embodiments, the polypeptide comprises PSCA. In some embodiments, the polypeptide comprises NY-ESO-1. In some embodiments, the polypeptide comprises WT-1. In some embodiments, the polypeptide comprises survivin. In some embodiments, the polypeptide comprises gp100. In some embodiments, the polypeptide comprises PAP. In some embodiments, the polypeptide comprises proteinase 3. In some embodiments, the polypeptide comprises SPAS-1. In some embodiments, the polypeptide comprises SP-17. In some embodiments, the polypeptide comprises PAGE-4. In some embodiments, the polypeptide comprises TARP. In some embodiments, the polypeptide comprises CEA.

In some embodiments, the antigen encoded by a polynucleotide in the recombinant nucleic acid molecule, expression cassette, and/or vector is an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA. In some embodiments, the antigen is K-Ras. In some embodiments, the antigen is H-Ras. In some embodiments, the antigen is N-Ras. In some embodiments, the antigen is K-Ras. In some embodiments, the antigen is mesothelin. In some embodiments, the antigen is PSCA. In some embodiments, the antigen is NY-ESO-1. In some embodiments, the antigen is WT-1. In some embodiments, the antigen is survivin. In some embodiments, the antigen is gp100. In some embodiments, the antigen is PAP. In some embodiments, the antigen is proteinase 3. In some embodiments, the antigen is SPAS-1. In some embodiments, the antigen is SP-17. In some embodiments, the antigen is PAGE-4. In some embodiments, the antigen is TARP. In some embodiments, the antigen is CEA. In some embodiments, the antigen is human mesothelin.

In some embodiments, the antigen is mesothelin, SPAS-1, proteinase-3, EphA2, SP-17, gp100, PAGE-4, TARP, or CEA, or an antigen derived from one of those proteins. In some embodiments the antigen is mesothelin or is derived from mesothelin. In other embodiments, the antigen is EphA2 or is an antigen derived from EphA2. In some embodiments, the antigen encoded by a polynucleotide in a recombinant nucleic acid molecule, expression cassette, or expression vector described herein is not Epha2 (or an antigen derived from Epha2). In some embodiments, the antigen is a tumor-associated antigen other than Epha2. In some embodiments, the antigen is derived from a tumor-associated antigen other than Epha2. In some embodiments, the polypeptide encoded by a polynucleotide in the recombinant nucleic acid molecule, expression cassette, and/or expression vector comprises an antigen other than Epha2. In some embodiments, the polypeptide encoded by a polynucleotide in the recombinant nucleic acid molecule, expression cassette, and/or expression vector comprises an antigen other than Epha2 or an antigen derived from Epha2.

In some embodiments, a polynucleotide in the recombinant nucleic acid molecule, expression cassette, and/or expression vector encodes a polypeptide comprising an antigen derived from K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA. In some embodiments, the polypeptide comprises an antigen derived from K-Ras. In some embodiments, the polypeptide comprises an antigen derived from H-Ras. In some embodiments, the polypeptide comprises an antigen derived from N-Ras. In some embodiments, the polypeptide comprises an antigen derived from 12-K-Ras. In some embodiments, the polypeptide comprises an antigen derived from mesothelin. In some embodiments, the polypeptide comprises an antigen derived from PSCA. In some embodiments, the polypeptide comprises an antigen derived from NY-ESO-1. In some embodiments, the polypeptide comprises an antigen derived from WT-1. In some embodiments, the polypeptide comprises an antigen derived from survivin. In some embodiments, the polypeptide comprises an antigen derived from gp100. In some embodiments, the polypeptide comprises an antigen derived from PAP. In some embodiments, the polypeptide comprises an antigen derived from proteinase 3. In some embodiments, the polypeptide comprises an antigen derived from SPAS-1. In some embodiments, the polypeptide comprises an antigen derived from SP-17. In some embodiments, the polypeptide comprises an antigen derived from PAGE-4. In some embodiments, the polypeptide comprises an antigen derived from TARP. In some embodiments, the polypeptide comprises an antigen derived from CEA.

In some embodiments, a polynucleotide in the recombinant nucleic acid molecule, expression cassette, and/or expression vector encodes an antigen derived from K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA. In some embodiments, the antigen is derived from K-Ras. In some embodiments, the antigen is derived from H-Ras. In some embodiments, the antigen is derived from N-Ras. In some embodiments, the antigen is derived from 12-K-Ras. In some embodiments, the antigen is an antigen derived from mesothelin. In some embodiments, the antigen is an antigen derived from PSCA. In some embodiments, the antigen is an antigen derived from NY-ESO-1. In some embodiments, the antigen is an antigen derived from WT-1. In some embodiments, the antigen is an antigen derived from survivin. In some embodiments, the antigen is an antigen that is derived from gp100. In some embodiments, the antigen is an antigen that is derived from PAP. In some embodiments, the antigen is an antigen that is derived from proteinase 3. In some embodiments, the antigen is an antigen derived from SPAS-1. In some embodiments, the antigen is an antigen derived from SP-17. In some embodiments, the antigen is an antigen derived from PAGE-4. In some embodiments, the antigen is an antigen derived from TARP. In some embodiments, the antigen is an antigen derived from CEA.

In some embodiments, the antigen is mesothelin, or an antigenic fragment or antigenic variant thereof. Thus, in some embodiments, the polypeptide encoded by a polynucleotide in the recombinant nucleic acid molecule, expression cassette and/or vector comprises mesothelin, or an antigenic fragment or antigenic variant thereof. In some embodiments, the polypeptide encoded by the polynucleotide is mesothelin, or an antigenic fragment or antigenic variant thereof.

In some embodiments, the antigen is mesothelin (e.g., human mesothelin) in which the mesothelin signal peptide and/or GPI (glycosylphosphatidylinositol) anchor has been deleted. Accordingly, in some embodiments, the polypeptide encoded by the polynucleotide comprises mesothelin in which the mesothelin signal peptide and/or GPI anchor has been deleted. In some embodiments, the polypeptide encoded by the polynucleotide is mesothelin in which the mesothelin signal peptide and/or GPI anchor has been deleted. In some embodiments, the polypeptide encoded by the polynucleotide is human mesothelin in which the mesothelin signal peptide and/or GPI anchor has been deleted. In some embodiments, the polypeptide encoded by the polynucleotide is human mesothelin in which both the mesothelin signal peptide and GPI anchor have been deleted.

In some embodiments, the antigen is NY-ESO-1, or an antigenic fragment or antigenic variant thereof. Thus, in some embodiments, the polypeptide encoded by a polynucleotide in the recombinant nucleic acid molecule, expression cassette, or vector comprises an antigen which is NY-ESO-1, or an antigenic fragment or antigenic variant thereof. In some embodiments, the polypeptide is an antigen which is NY-ESO-1, or an antigenic fragment or antigenic variant thereof.

In some embodiments, a polypeptide encoded by polynucleotide in a recombinant nucleic acid molecule, expression cassette, or vector comprises at least one antigenic fragment of a tumor-associated antigen, e.g., human prostate stem cell antigen (PSCA; GenBank Acc. No.AF043498), human testes antigen (NY-ESO-1; GenBank Acc. No. NM_001327), human carcinoembryonic antigen (CEA; GenBank Acc. No. M29540), human Mesothelin (GenBank Acc. No. U40434), human survivin (GenBank Acc. No. U75285), human Proteinase 3 (GenBank No. X55668), human K-Ras (GenBank Acc. Nos. M54969 & P01116), human H-Ras (GenBank Acc. No. P01112), human N-Ras (GenBank Acc. No. P01111), and human 12-K-Ras (K-Ras comprising a Gly12Asp mutation) (see, e.g., GenBank Acc. No. K00654). In some embodiments, a polypeptide encoded by polynucleotide in a recombinant nucleic acid molecule, expression cassette, or expression vector comprises an antigenic fragment of a tumor-associated antigen with at least one conservatively substituted amino acid. In some embodiments, a polypeptide encoded by polynucleotide in a recombinant nucleic acid molecule, expression cassette, or expression vector comprises an antigenic fragment with at least one deleted amino acid residue. In some embodiments, a polypeptide encoded by polynucleotide in a recombinant nucleic acid molecule, expression cassette, or expression vector comprises combinations of antigenic sequences derived from more than one type of tumor-associated antigen, e.g., a combination of antigenic fragments derived from both mesothelin and Ras.

Exemplary regions of tumor antigens predicted to be antigenic include the following: amino acids 25-35; 70-80; and 90-118 of the PSCA amino acid sequence in (GenBank Acc. No. AF043498; amino acids 40-55, 75-85, 100-115, and 128-146 of the NY-ESO-1 of GenBank Acc. No. NM_001327; amino acids 70-75,150-155,205-225,330-340, and 510-520 of the CEA amino acid sequence of GenBank Acc. No. M29540; amino acids 90-110, 140-150, 205-225, 280-310, 390-410, 420-425, and 550-575; of the mesothelin polypeptide sequence of GenBank Acc. No. U40434; amino acids 12-20, 30-40, 45-55, 65-82, 90-95, 102-115, and 115-130 of the surviving polypeptide sequence of GenBank Acc. No. U75285; amino acids 10-20, 30-35, 65-75, 110-120, and 160-170, of the amino acid sequence of proteinase-3 found in GenBank Acc. No. X55668; amino acids 10-20,30-50,55-75, 85-110,115-135,145-155, and 160-185 of GenBank Acc. Nos. P01117 or M54968 (human K-Ras); amino acids 10-20, 25-30, 35-45, 50-70, 90-110, 115-135, and 145-175 of GenBank Acc. No. P01112 (human H-Ras); amino acids 10-20, 25-45, 50-75, 85-110, 115-135, 140-155, and 160-180 of GenBank Acc. No. P01111 (human N-Ras); and the first 25-amino acids of 12-K-Ras (sequence disclosed in GenBank Acc. No. K00654). These antigenic regions were predicted by Hopp-Woods and Welling antigenicity plots.

In some embodiments, the polypeptides encoded by the polynucleotides of this disclosure either as discrete polypeptides, as fusion proteins with the chosen signal peptide, or as a protein chimera in which the polypeptide has been inserted in another polypeptide, are polypeptides comprising one or more of the following peptides of human mesothelin: SLLFLLFSL (amino acids 20-28; (SEQ ID NO:64)); VLPLTVAEV (amino acids 530-538; (SEQ ID NO:65)); ELAVALAQK (amino acids 83-92; (SEQ ID NO:66)); ALQGGGPPY (amino acids 225-234; (SEQ ID NO:67)); FYPGYLCSL (amino acids 435-444; (SEQ ID NO:68)); and LYPKARLAF (amino acids 475-484; (SEQ ID NO:69)). For instance, in some embodiments, the antigen encoded by a polynucleotide of this disclosure is an (antigenic) fragment of human mesothelin comprising one or more of these peptides. Additional information regarding these mesothelin peptide sequences and their correlation with medically relevant immune responses can be found in the PCT Publication WO 2004/006837.

Alternatively, polynucleotides in the recombinant nucleic acid molecule, expression cassette, or expression vector can encode an autoimmune disease-specific antigen (or a polypeptide comprising an autoimmune disease-specific antigen). In a T cell mediated autoimmune disease, a T cell response to self antigens results in the autoimmune disease. The type of antigen for use in treating an autoimmune disease with the vaccines of the present invention might target the specific T cells responsible for the autoimmune response. For example, the antigen may be part of a T cell receptor, the idiotype, specific to those T cells causing an autoimmune response, wherein the antigen incorporated into a vaccine of this disclosure would elicit an immune response specific to those T cells causing the autoimmune response. Eliminating those T cells would be the therapeutic mechanism to alleviating the autoimmune disease. Another possibility would be to incorporate into the recombinant nucleic acid molecule a polynucleotide encoding an antigen that will result in an immune response targeting the antibodies that are generated to self antigens in an autoimmune disease or targeting the specific B cell clones that secrete the antibodies. For example, a polynucleotide encoding an idiotype antigen may be incorporated into the recombinant nucleic acid molecule that will result in an anti-idiotype immune response to such B cells and/or the antibodies reacting with self antigens in an autoimmune disease. Autoimmune diseases treatable with vaccines comprising bacteria comprising the expression cassettes and recombinant nucleic acid molecules of the present invention include, but are not limited to, rheumatoid arthritis, multiple sclerosis, Crohn's disease, lupus, myasthenia gravis, vitiligo, scleroderma, psoriasis, pemphigus vulgaris, fibromyalgia, colitis and diabetes. A similar approach may be taken for treating allergic responses, where the antigens incorporated into the vaccine bacterium target either T cells, B cells or antibodies that arc effective in modulating the allergic reaction. In some autoimmune diseases, such as psoriasis, the disease results in hyperproliferative cell growth with expression of antigens that may be targeted as well. Such an antigen that will result in an immune response to the hyperproliferative cells is considered.

In some embodiments, the antigen is an antigen that targets unique disease associated protein structures. One example of this is the targeting of antibodies, B cells or T cells using idiotype antigens as discussed above. Another possibility is to target unique protein structures resulting from a particular disease. An example of this would be to incorporate an antigen that will generate an immune response to proteins that cause the amyloid plaques observed in diseases such as Alzheimer's disease, Creutzfeldt-Jakob disease (CJD) and Bovine Spangiform Encephalopathy (BSE). While this approach may only provide for a reduction in plaque formation, it may be possible to provide a curative vaccine in the case of diseases like CJD. This disease is caused by an infectious form of a prion protein. In some embodiments, the polynucleotides of this disclosure encode an antigen to the infectious form of the prion protein such that the immune response generated by the vaccine may eliminate, reduce, or control the infectious proteins that cause CJD.

In some embodiments, the polypeptide encoded by a polynucleotide of the recombinant nucleic acid molecule, expression cassette, and/or expression vector comprises an infectious disease antigen or an antigen derived from an infectious disease antigen. In some embodiments, the polypeptide comprises an infectious disease antigen. In some other embodiments, the polypeptide comprises an antigen derived from an infectious disease antigen. In some embodiments, the polypeptide encoded by a polynucleotide of the recombinant nucleic acid molecule, expression cassette, and/or expression vector is an infectious disease antigen or is an antigen derived from an infectious disease antigen. In some embodiments, the polypeptide encoded by the recombinant nucleic acid molecule, expression cassette, and/or expression vector is an infectious disease antigen. In some embodiments, the polypeptide encoded by the recombinant nucleic acid molecule, expression cassette, and/or expression vector is derived from an infectious disease antigen.

In other embodiments of this disclosure the antigen is derived from a human or animal pathogen. The pathogen is optionally a virus, bacterium, fungus, or a protozoan. For instance, the antigen may be a viral or fungal or bacterial antigen. In one embodiment, the antigen encoded by the recombinant nucleic acid molecule, expression cassette, and/or expression vector that is derived from the pathogen is a protein produced by the pathogen, or is derived from a protein produced by the pathogen. For instance, in some embodiments, the polypeptide encoded by the recombinant nucleic acid molecules, expression cassette and/or expression vector is a fragment and/or variant of a protein produced by the pathogen.

For instance, in some embodiments, the antigen is derived from Human Immunodeficiency virus (such as gp 120, gp 160, gp41, gag antigens such as p24gag and p55gag, as well as proteins derived from the pol, env, tat, vif, rev, nef, vpr, vpu and LTR regions of HIV), Feline Immunodeficiency virus, or human or animal herpes viruses. For example, in some embodiments, the antigen is gp 120. In one embodiment, the antigen is derived from herpes simplex virus (HSV) types 1 and 2 (such as gD, gB, gH, Immediate Early protein such as ICP27), from cytomegalovirus (such as gB and gH), from metapneumovirus, from Epstein-Barr virus or from Varicella Zoster Virus (such as gpI, II or III). (See, e. g., Chee et al. (1990) Gytomegaloviruses (J. K. McDougall, ed., Springer Verlag, pp. 125-169; McGeoch et al. (1988) J. Gen. Virol. 69: 1531-1574; U.S. Pat. No. 5,171,568; Baer et al. (1984) Nature 310: 207-211; and Davison et al. (1986) J. Gen. Virol 67: 1759-1816.)

In another embodiment, the antigen is derived from a hepatitis virus such as hepatitis B virus (for example, Hepatitis B Surface antigen), hepatitis A virus, hepatitis C virus, delta hepatitis virus, hepatitis E virus, or hepatitis G virus. See, e. g., WO 89/04669; WO 90/11089; and WO 90/14436. The hepatitis antigen can be a surface, core, or other associated antigen. The HCV genome encodes several viral proteins, including E1 and E2. See, e. g., Houghton et al., Hepatology 14: 381-388 (1991).

An antigen that is a viral antigen is optionally derived from a virus from any one of the families Picornaviridae (e. g., polioviruses, rhinoviruses, etc.); Caliciviridae; Togaviridae (e. g., rubella virus, dengue virus, etc.); Flaviviridae; Coronaviridae; Reoviridae (e. g., rotavirus, etc.); Birnaviridae; Rhabodoviridae (e. g., rabies virus, etc.); Orthomyxoviridae (e. g., influenza virus types A, B and C, etc.); Filoviridae; Paramyxoviridae (e. g., mumps virus, measles virus, respiratory syncytial virus, parainfluenza virus, etc.); Bunyaviridae; Arenaviridae; Retroviradae (e. g., HTLV-I; HTLV-11; HIV-1 (also known as HTLV-111, LAV, ARV, hTLR, etc.)), including but not limited to antigens from the isolates HIVI11b, HIVSF2, HTVLAV, HIVLAI, HIVMN); HIV-1CM235, HIV-1; HIV-2, among others; simian immunodeficiency virus (SIV)); Papillomavirus, the tick-borne encephalitis viruses; and the like. See, e. g. Virology, 3rd Edition (W. K. Joklik ed. 1988); Fundamental Virology, 3rd Edition (B. N. Fields, D. M. Knipe, and P.M. Howley, Eds. 1996), for a description of these and other viruses. In one embodiment, the antigen is Flu-HA (Morgan et al., J. Immunol. 160:643 (1998)).

In some alternative embodiments, the antigen is derived from bacterial pathogens such *as Mycobacterium, Bacillus, Yersinia, Salmonella, Neisseria, Borrelia* (for example, OspA or OspB or derivatives thereof), Chlamydia, or Bordetella (for example, P.69, PT and FHA), or derived from parasites such as plasmodium or Toxoplasma. In one embodiment, the antigen is derived from *Mycobacterium tuberculoses* (e.g. ESAT-6, 85A, 85B, 85C, 72F), *Bacillus anthracis* (e.g.PA), or *Yersinia pestis* (e.g. F1, V). In addition, antigens suitable for use in the present invention can be obtained or derived from known causative agents responsible for diseases including, but not limited to, Diptheria, Pertussis, Tetanus, Tuberculosis, Bacterial or Fungal Pneumonia, Otitis Media, Gonorrhea, Cholera, Typhoid, Meningitis, Mononucleosis, Plague, Shigellosis or Salmonellosis, Legionaire's Disease, Lyme Disease, Leprosy, Malaria, Hookworm, Onchocerciasis, Schistosomiasis, Trypanosomiasis, Leishmaniasis, Giardia, Amoebiasis, Filariasis, Borelia, and Trichinosis. Still further antigens can be obtained or derived from unconventional pathogens such as the causative agents of kuru, Creutzfeldt-Jakob disease (CJD), scrapie, transmissible mink encephalopathy, and chronic wasting diseases, or from proteinaceous infectious particles such as prions that are associated with mad cow disease.

In still other embodiments, the antigen is obtained or derived from a biological agent involved in the onset or progression of neurodegenerative diseases (such as Alzheimer's disease), metabolic diseases (such as Type I diabetes), and drug addictions (such as nicotine addiction). Alternatively, the antigen encoded by the recombinant nucleic acid molecule is used for pain management and the antigen is a pain receptor or other agent involved in the transmission of pain signals.

In some embodiments, the antigen is a human protein or is derived from a human protein. In other embodiments, the antigen is a non-human protein or is derived from a non-human protein (a fragment and/or variant thereof). In some embodiments, the antigen portion of the fusion protein encoded by the expression cassette is a protein from a non-human animal or is a protein derived from a non-human animal. For instance, even if the antigen is to be expressed in a *Listeria*-based vaccine that is to be used in humans, in some embodiments, the antigen can be murine mesothelin or derived from murine mesothelin.

### V. Codon-optimization

In some embodiments, one or more of the polynucleotides (i.e., polynucleotide sequences) within the recombinant nucleic acid molecule, expression cassette and/or expression vector are codon-optimized (relative to the native coding sequence). In some embodiments, a polynucleotide in the recombinant nucleic acid molecules (and/or in the expression cassette and/or expression vector) described herein that encodes a signal peptide is codon-optimized for expression in a bacterium. In some embodiments, a polynucleotide encoding a polypeptide other than a signal peptide, such as an antigen or other therapeutic protein, is codon-optimized for expression in a bacterium. In some embodiments, both a polynucleotide encoding a signal peptide and a polynucleotide encoding another polypeptide fused to the signal peptide are codon-optimized for expression in a bacterium. In some embodiments, a polynucleotide encoding a secreted protein (or fragment thereof) used as a scaffold or a polynucleotide encoding an autolysin (or fragment or variant thereof) is codon-optimized.

A polynucleotide comprising a coding sequence is "codon-optimized" if at least one codon of the native coding sequence of the polynucleotide has been replaced with a codon that is more frequently used by the organism in which the coding sequence is to be expressed (the "target organism") than the original codon of the native coding sequence. For instance, a polynucleotide encoding a non-bacterial antigen that is to be expressed in a particular species of bacteria is codon-optimized if at least one of the codons from the native bacterial polynucleotide sequence is replaced with a codon that is preferentially expressed in that particular species of bacteria in which the non-bacterial antigen is to be expressed. As another example, a polynucleotide encoding a human cancer antigen that is to be part of an expression cassette in recombinant *Listeria monocytogenes* is codon-optimized if at least one codon in the polynucleotide sequence is replaced with a codon that is more frequently used by *Listeria monocytogenes* for that amino acid than the codon in the original human sequence would be. Likewise, a polynucleotide encoding a signal peptide native to *Listeria monocytogenes* (such as the LLO signal peptide from *L. monocytogenes*) that is to be part of an expression cassette to encode a fusion protein comprising a human cancer antigen in recombinant *Listeria monocytogenes* is codon-optimized if at least one codon in the polynucleotide sequence encoding the signal peptide is replaced with a codon that is more frequently used by *Listeria monocytogenes* for that amino acid than the codon in the original (native) sequence is. In some embodiments, at least one codon that is replaced in the codon-optimized sequence is replaced with the codon most frequently used by the target organism to code for the same amino acid.

In some embodiments, at least two codons of the native coding sequence of the polynucleotide have been replaced with a codon that is more frequently used by the organism in which the coding sequence is to be expressed than the original codon of the native coding sequence. In some embodiments, at least about five codons, at least about 10 codons, or at least about 20 codons of the native coding sequence of the polynucleotide have been replaced with a codon that is more frequently used by the organism in which the coding sequence is to be expressed than the original codon of the native coding sequence.

In some embodiments, at least about 10% of the codons in the codon-optimized polynucleotide have been replaced with codons more frequently (or most frequently) used by the target organism (than the original codons of the native sequence). In other embodiments, at least about 25% of the codons in the codon-optimized polynucleotide have been replaced with codons more frequently used (or most frequently) used by the target organism. In other embodiments, at least about 50% of the codons in the codon-optimized polynucleotide have been replaced with codons more frequently used (or most frequently) used by the target organism. In still other embodiments, at least about 75% of the codons in the codon-optimized polynucleotide have been replaced with codons more frequently used (or most frequently used) by the target organism.

The codon preferences of different organisms have been widely studied by those skilled in the art. For instance, see Sharp et al., Nucleic Acids Res., 15:1281-95 (1987) and Uchijima et al., The Journal of Immunoloy, 161:5594-9 (1998). As a result, codon usage tables are publicly available for a wide variety of organisms. For instance, codon usage tables can be found on the internet at www.kazusa.or.jp/codon/ for a wide variety of organisms as well as on other publicly available sites. (See, e.g., Nakamura et al. (2000) Nucleic Acids Research 28:292.) An exemplary codon usage table from \vww.kazusa.or.jp/codon/, the codon usage table for *Listeria monocytogenes* (http://www.kazusa.or.jp/codon/cgi-bin/showcodon.cgi?species=*Listeria*+monocytogenes+[gbbct]), is reproduced for convenience below in Table 2A. Exemplary codon usage tables for (*Bacillus anthracis, Mycobacterium tuberculosis, Salmonella typhimuriun, Mycobacterium bovis BCG,* and *Shigella flexneri* are also provided in Tables 2B, 2C, 2D, 2E, and 2F, respectively, below.

**Table 2A: Codon Usage Table for Listeria Monocytogenes (from www.kazusa.or.jp/codon/).**

| *Listeria monocytogenes:* 3262 CDS's (1029006 codons) | | | | | | | | | | | |
|---|---|---|---|---|---|---|---|---|---|---|---|
| fields: [triplet] [frequency: per thousand] ([number]) | | | | | | | | | | | |
| UUU | 29.4 | (30274) | UCU | 13.2 | (13586) | UAU | 22.9 | (23604) | UGU | 3.8 | (3960) |
| UUC | 14.1 | (14486) | UCC | 6.5 | (6714) | UAC | 10.7 | (11055) | UGC | 1.9 | (1972) |
| UUA | 36.8 | (37821) | UCA | 10.4 | (10751) | UAA | 2.2 | (2307) | UGA | 0.6 | (583) |
| UUG | 12.3 | (12704) | UCG | 6.1 | (6278) | UAG | 0.4 | (372) | UGG | 9.3 | (9580) |
| | | | | | | | | | | | |
| CUU | 21.0 | (21567) | CCU | 8.4 | (8622) | CAU | 12.0 | (12332) | CGU | 12.6 | (12930) |
| CUC | 5.4 | (5598) | CCC | 1.7 | (1780) | CAC | 5.2 | (5336) | CGC | 7.0 | (7215) |
| CUA | 12.9 | (13279) | CCA | 18.5 | (18996) | CAA | 29.9 | (30719) | CGA | 5.6 | (5732) |
| CUG | 5.0 | (5120) | CCG | 7.0 | (7219) | CAG | 5.1 | (5234) | CGG | 2.8 | (2884) |
| | | | | | | | | | | | |
| AUU | 49.3 | (50692) | ACU | 17.1 | (17614) | AAU | 33.0 | (33908) | AGU | 14.1 | (14534) |
| AUC | 18.4 | (18894) | ACC | 6.9 | (7089) | AAC | 15.3 | (15790) | AGC | 8.8 | (9031) |
| AUA | 9.4 | (9642) | ACA | 26.5 | (27318) | AAA | 61.6 | (63379) | AGA | 6.9 | (7111) |
| AUG | 25.9 | (26651) | ACG | 12.9 | (13285) | AAG | 10.4 | (10734) | AGG | 1.2 | (1254) |
| | | | | | | | | | | | |
| GUU | 26.4 | (27202) | GCU | 24.3 | (24978) | GAU | 39.8 | (40953) | GGU | 24.2 | (24871) |
| GUC | 8.7 | (8990) | GCC | 8.4 | (8612) | GAC | 14.3 | (14751) | GGC | 14.2 | (14581) |
| GUA | 21.6 | (22247) | GCA | 28.6 | (29401) | GAA | 60.4 | (62167) | GGA | 19.1 | (19612) |
| GUG | 13.1 | (13518) | GCG | 16.6 | (17077) | GAG | 13.1 | (13507) | GGG | 8.7 | (9003) |

**Table 2B: Codon Usage Table for Bacillus anthracis (from www.kazusa.or.jp/codon/). Bacillus anthracis [gbbct]: 312 CDS's (90023 codons)**

| fields: [triplet] [frequency: per thousand] ([number]) | | | | | | | | | | | |
|---|---|---|---|---|---|---|---|---|---|---|---|
| UUU | 32.4 | (2916) | UCU | 17.2 | (1547) | UAU | 31.9 | (2876) | UGU | 5.1 | (455) |
| UUC | 10.4 | (934) | UCC | 5.0 | (453) | UAC | 9.5 | (853) | UGC | 1.8 | (164) |
| UUA | 43.7 | (3931) | UCA | 14.8 | (1330) | UAA | 2.2 | (199) | UGA | 0.5 | (47) |
| UUG | 11.4 | (1024) | UCG | 4.2 | (375) | UAG | 0.7 | (66) | UGG | 9.3 | (835) |
| | | | | | | | | | | | |
| CUU | 14.4 | (1300) | CCU | 10.7 | (967) | CAU | 15.5 | (1392) | CGU | 9.8 | (883) |
| CUC | 3.7 | (335) | CCC | 2.7 | (242) | CAC | 4.2 | (379) | CGC | 2.5 | (223) |
| CUA | 12.4 | (1117) | CCA | 17.8 | (1599) | CAA | 32.3 | (2912) | CGA | 6.3 | (569) |
| CUG | 4.4 | (392) | CCG | 5.9 | (534) | CAG | 9.5 | (859) | CGG | 2.0 | (179) |
| | | | | | | | | | | | |
| AUU | 44.5 | (4009) | ACU | 21.0 | (1890) | AAU | 44.0 | (3959) | AGU | 17.4 | (1565) |
| AUC | 11.9 | (1072) | ACC | 5.0 | (453) | AAC | 14.1 | (1268) | AGC | 5.2 | (467) |
| AUA | 22.7 | (2042) | ACA | 26.8 | (2414) | AAA | 64.3 | (5786) | AGA | 13.7 | (1236) |
| AUG | 23.3 | (2098) | ACG | 9.4 | (844) | AAG | 22.7 | (2047) | AGG | 4.1 | (368) |
| | | | | | | | | | | | |
| GUU | 20.3 | (1824) | GCU | 17.8 | (1598) | GAU | 39.3 | (3536) | GGU | 17.9 | (1611) |
| GUC | 4.6 | (414) | GCC | 4.1 | (372) | GAC | 9.0 | (811) | GGC | 5.8 | (524) |
| GUA | 26.4 | (2374) | GCA | 23.5 | (2117) | GAA | 53.9 | (4855) | GGA | 24.5 | (2203) |
| GUG | 10.8 | (973) | GCG | 7.9 | (709) | GAG | 17.9 | (1614) | GGG | 12.0 | (1083) |

| | | | | | | | | | | | |
|---|---|---|---|---|---|---|---|---|---|---|---|
| Coding GC 34.55% 1st letter GC 44.99% 2nd letter GC 33.16% 3rd letter GC 25.51 % | | | | | | | | | | | |

**Table 2C: Codon Usage Table for Mycobacterium tuberculosis(from www.kazusa.or.jp/codon/). Mycobacterium tuberculosis [gbbct]: 363 CDS's (131426 codons)**

| fields: [triplet] [frequency: per thousand] ([number]) | | | | | | | | | | | |
|---|---|---|---|---|---|---|---|---|---|---|---|
| UUU | 5.4 | (709) | UCU | 2.0 | (265) | UAU | 6.0 | (788) | UGU | 2.5 | (326) |
| UUC | 25.6 | (3359) | UCC | 11.4 | (1499) | UAC | 17.6 | (2307) | UGC | 5.6 | (738) |
| UUA | 1.8 | (231) | UCA | 4.3 | (571) | UAA | 0.4 | (52) | UGA | 1.5 | (201) |
| UUG | 14.8 | (1945) | UCG | 19.2 | (2522) | UAG | 0.8 | (103) | UGG | 17.9 | (2352) |
| | | | | | | | | | | | |
| CUU | 5.9 | (778) | CCU | 3.9 | (511) | CAU | 5.4 | (7711) | CGU | 8.0 | (1048) |
| CUC | 17.7 | (2329) | CCC | 18.3 | (2411) | CAC | 14.7 | (1928) | CGC | 26.7 | (3508) |
| CUA | 4.0 | (521) | CCA | 6.4 | (843) | CAA | 7.8 | (1030) | CGA | 5.8 | (764) |
| CUG | 45.9 | (6032) | CCG | 33.2 | (4359) | CAG | 24.2 | (3176) | CGG | 21.1 | (2772) |
| | | | | | | | | | | | |
| AUU | 7.6 | (993) | ACU | 4.1 | (545) | AAU | 4.8 | (637) | AGU | 4.0 | (531) |
| AUC | 32.7 | (4300) | ACC | 36.0 | (4735) | AAC | 26.3 | (3451) | AGC | 15.0 | (1976) |
| AUA | 2.1 | (282) | ACA | 4.7 | (616) | AAA | 5.8 | (761) | AGA | 1.5 | (192) |
| AUG | 19.7 | (2591) | ACG | 16.4 | (2158) | AAG | 26.5 | (3485) | AGG | 3.3 | (429) |
| | | | | | | | | | | | |
| GUU | 8.3 | (1095) | GCU | 11.2 | (1473) | GAU | 15.6 | (2046) | GGU | 18.7 | (2455) |
| GUC | 32.3 | (4249) | GCC | 51.5 | (6769) | GAC | 44.6 | (5858) | GGC | 48.6 | (6383) |
| GUA | 4.7 | (622) | GCA | 12.4 | (1625) | GAA | 16.8 | (2211) | GGA | 9.0 | (1183) |
| GUG | 35.7 | (4687) | GCG | 41.7 | (5482) | GAG | 35.8 | (4702) | GGG | 16.9 | (2215) |

| | | | | | | | | | | | |
|---|---|---|---|---|---|---|---|---|---|---|---|
| Coding GC 64.43% 1st letter GC 65.27% 2nd letter GC 48.28% 3rd letter GC 79.75% | | | | | | | | | | | |

**Table 2D: Codon Usage Table for Salmonella typhimurium (from www.kazusa.or.jp/codon/). Salmonella typhimurium [gbbct]: 1322 CDS's (416065 codons)**

| fields: [triplet] [frequency: per thousand] ([number]) | | | | | | | | | | | |
|---|---|---|---|---|---|---|---|---|---|---|---|
| UUU | 21.7 | (9041) | UCU | 8.5 | (3518) | UAU | 16.5 | (6853) | UGU | 4.6 | (1920) |
| UUC | 15.1 | (6265) | UCC | 10.6 | (4430) | UAC | 11.6 | (4826) | UGC | 6.1 | (2524) |
| UUA | 13.6 | (5650) | UCA | 7.9 | (3286) | UAA | 1.8 | (731) | UGA | 1.1 | (465) |
| UUG | 12.1 | (5025) | UCG | 9.4 | (3924) | UAG | 0.3 | (121) | UGG | 19.1 | (5851) |
| | | | | | | | | | | | |
| CUU | 12.1 | (5038) | CCU | 7.9 | (3290) | CAU | 12.1 | (5047) | CGU | 18.1 | (7542) |
| CUC | 10.6 | (4396) | CCC | 7.0 | (2921) | CAC | 9.2 | (3818) | CGC | 20.8 | (8659) |
| CUA | 4.7 | (1958) | CCA | 6.5 | (2712) | CAA | 12.8 | (5315) | CGA | 4.1 | (1695) |
| CUG | 49.3 | (20508) | CCG | 22.7 | (9463) | CAG | 30.8 | (12803) | CGG | 7.2 | (3004) |
| | | | | | | | | | | | |
| AUU | 28.1 | (11700) | ACU | 8.2 | (3401) | AAU | 19.5 | (8107) | AGU | 8.6 | (3569) |
| AUC | 23.9 | (9941) | ACC | 24.0 | (9980) | AAC | 21.4 | (8920) | AGC | 18.0 | (7485) |
| AUA | 6.7 | (2771) | ACA | 8.0 | (3316) | AAA | 33.0 | (13740) | AGA | 3.2 | (1348) |
| AUG | 26.1 | (10842) | ACG | 18.6 | (7743) | AAG | 12.4 | (5151) | AGG | 2.3 | (959) |
| | | | | | | | | | | | |
| GUU | 16.4 | (6831) | GCU | 14.4 | (5985) | GAU | 32.9 | (13700) | GGU | 18.1 | (7541) |
| GUC | 17.7 | (7367) | GCC | 27.5 | (11462) | GAC | 21.5 | (8949) | GGC | 33.0 | (13730) |
| GUA | 11.9 | (4935) | GCA | 14.8 | (6156) | GAA | 36.1 | (15021) | GGA | 9.1 | (3788) |
| GUG | 24.3 | (10092) | GCG | 37.0 | (15387) | GAG | 20.9 | (8715) | GGG | 11.6 | (4834) |

| | | | | | | | | | | | |
|---|---|---|---|---|---|---|---|---|---|---|---|
| Coding GC 52.45% 1^{st} letter GC 58.32% 2nd letter GC 41.31% 3rd letter GC 57.71% | | | | | | | | | | | |

**Table 2E: Codon Usage Table for Mycobacterium bovis BCG (from www.kazusa.or.jp/codon). Mycobacterium bovis BCG [gbbct]: 51 CDS's (16528 codons)**

| fields: [triplet] [frequency: per thousand] ([number]) | | | | | | | | | | | |
|---|---|---|---|---|---|---|---|---|---|---|---|
| UUU | 4.7 | (77) | UCU | 1.9 | (31) | UAU | 6.6 | (109) | UGU | 2.0 | (33) |
| UUC | 27.4 | (453) | UCC | 11.9 | (189) | UAC | 17.0 | (281) | UGC | 6.7 | (110) |
| UUA | 1.6 | (26) | UCA | 4.5 | (74) | UAA | 0.9 | (15) | UGA | 1.3 | (22) |
| UUG | 14.7 | (243) | UCG | 20.8 | (343) | UAG | 0.8 | (14) | UGG | 14.3 | (237) |
| | | | | | | | | | | | |
| CUU | 5.6 | (92) | CCU | 2.9 | (48) | CAU | 4.9 | (81) | CGU | 9.4 | (155) |
| CUC | 14.8 | (244) | CCC | 16.3 | (270) | CAC | 17.2 | (285) | CGC | 33.8 | (559) |
| CUA | 5.1 | (85) | CCA | 5.1 | (84) | CAA | 7.3 | (120) | CGA | 7.1 | (118) |
| CUG | 51.5 | (852) | CCG | 31.0 | (512) | CAG | 25.5 | (421) | CGG | 26.7 | (441) |
| | | | | | | | | | | | |
| AUU | 6.1 | (100) | ACU | 3.1 | (51) | AAU | 4.8 | (80) | AGU | 2.8 | (46) |
| AUC | 39.6 | (654) | ACC | 36.8 | (609) | AAC | 22.3 | (369) | AGC | 14.5 | (240) |
| AUA | 2.2 | (37) | ACA | 4.4 | (73) | AAA | 6.2 | (102) | AGA | 1.1 | (19) |
| AUG | 20.2 | (334) | ACG | 17.4 | (288) | AAG | 24.5 | (405) | AGG | 3.8 | (62) |
| | | | | | | | | | | | |
| GUU | 7.8 | (129) | GCU | 9.6 | (158) | GAU | 13.4 | (222) | GGU | 16.9 | (280) |
| GUC | 30.1 | (497) | GCC | 54.3 | (898) | GAC | 45.6 | (754) | GGC | 42.6 | (704) |
| GUA | 4.1 | (67) | GCA | 12.5 | (206) | GAA | 16.5 | (273) | GGA | 7.3 | (120) |
| GUG | 37.6 | (621) | GCG | 41.7 | (689) | GAG | 32.7 | (541) | GGG | 16.7 | (276) |

| | | | | | | | | | | | |
|---|---|---|---|---|---|---|---|---|---|---|---|
| Coding GC 64.82% 1st letter GC 65.36% 2nd letter GC 48.07% 3rd letter GC 81.04% | | | | | | | | | | | |

**Table 2F: Codon Usage Table for Shigella flexneri (from www.kazusa.or.jp/codon/). Shigella flexneri [gbbct]: 706 CDS's (180312 codons)**

| fields: [triplet] [frequency: per thousand] ([number]) | | | | | | | | | | | |
|---|---|---|---|---|---|---|---|---|---|---|---|
| UUU | 25.8 | (4658) | UCU | 16.6 | (2986) | UAU | 21.9 | (3945) | UGU | 6.9 | (1252) |
| UUC | 15.1 | (2714) | UCC | 9.5 | (1717) | UAC | 11.0 | (1992) | UGC | 5.6 | (1011) |
| UUA | 20.8 | (3756) | UCA | 15.6 | (2821) | UVA | 2.0 | (362) | UGA | 1.4 | (254) |
| UUG | 13.4 | (2424) | UCG | 6.9 | (1241) | UAG | 0.5 | (91) | UGG | 13.1 | (2357) |
| | | | | | | | | | | | |
| CUU | 17.6 | (3169) | CCU | 9.2 | (1656) | CAU | 15.1 | (2725) | CGU | 15.0 | (2707) |
| CUC | 10.4 | (1878) | CCC | 5.9 | (1072) | CAC | 8.2 | (1472) | CGC | 12.6 | (2269) |
| CUA | 7.2 | (1295) | CCA | 9.7 | (1744) | CAA | 15.9 | (2861) | CGA | 5.8 | (1046) |
| CUG | 33.5 | (6045) | CCG | 12.2 | (2199) | CAG | 23.6 | (4255) | CGG | 9.0 | (1627) |
| | | | | | | | | | | | |
| AUU | 30.0 | (5417) | ACU | 13.8 | (2480) | AAU | 33.5 | (6044) | AGU | 15.3 | (2764) |
| AUC | 16.7 | (3018) | ACC | 13.4 | (2413) | AAC | 18.6 | (3348) | AGC | 12.7 | (2281) |
| AUA | 18.9 | (3402) | ACA | 16.2 | (2930) | AAA | 41.6 | (7507) | AGA | 10.3( | (1865) |
| AUG | 23.3 | (4198) | ACG | 10.0 | (1809) | AAG | 16.4 | (2961) | AGG | 5.7 | (1029) |
| | | | | | | | | | | | |
| GUU | 19.8 | (3576) | GCU | 19.6 | (3527) | GAU | 34.0 | (6123) | GGU | 19.2 | (3468) |
| GUC | 11.8 | (2126) | GCC | 18.5 | (3338) | GAC | 16.3 | (2939) | GGC | 15.3 | (2754) |
| GUA | 13.1 | (2370) | GCA | 22.2 | (4009) | GAA | 37.5 | (6763) | GGQ | 15.1 | (2727) |
| GUG | 16.1 | (2910) | GCG | 15.2 | (2732) | GAG | 21.7 | (3913) | GGG | 10.9 | (1970) |

| | | | | | | | | | | | |
|---|---|---|---|---|---|---|---|---|---|---|---|
| Coding GC 44.63% 1st letter GC 51.72% 2nd letter GC 38.85% 3rd letter GC 43.32% | | | | | | | | | | | |

In some embodiments of this disclosure at least about 10%, at least about, 25%, at least about 50%, or at least about 75% of the codons in a codon-optimized coding sequence are the most preferred codon for that amino acid used in the target organism. In other embodiments, 100% of the codons in the codon-optimized coding sequence are the most preferred codon for that amino acid in the target organism (i.e., the sequence is "fully codon-optimized"). For instance, in the Examples shown below, all of the codons of the sequences characterized as codon-optimized were the most frequently used codons for the target organism; however, any codon substitution that results in a more frequently used codon than the original (native) sequence can be considered "codon-optimized". Table 3, below shows the optimal codon usage in *Listerial monocytogenes* for each amino acid.

**Table 3: Optimal Codon Usage Table in Listeria monocytogenes.**

| **Amino Acid** | **One Letter Code** | **Optimal *Listeria* Codon** |
|---|---|---|
| Alanine | A | GCA |
| Arginine | R | CGU |
| Asparagine | N | AAU |
| Aspartate | D | GAU |
| Cysteine | C | UGU |
| Glutamine | Q | CAA |
| Glutamate | E | GAA |
| Glycine | G | GGU |
| Histidine | H | CAU |
| Isoleucine | I | AUU |
| Leucine | L | UUA |
| Lysine | K | AAA |
| Methionine | M | AUG |
| Phenylalanine | F | UUU |
| Proline | P | CCA |
| Serine | S | AGU |
| Threonine | T | ACA |
| Tryptophan | W | UGG |
| Tyrosine | Y | UAU |
| Valine | V | GUU |

In some embodiments, the codon-optimized polynucleotides encode a signal peptide. In some embodiments, the signal peptide is foreign to the bacterium for which the sequence is codon-optimized. In other embodiments, the signal peptide is native to the bacterium for which the sequence is codon-optimized. For instance, in some embodiments, the codon-optimized polynucleotide encodes a signal peptide selected from the group consisting of LLO signal peptide from *Listeria monocytogenes,* Usp45 signal peptide from *Lactococcus lactis,* Protective Antigen signal peptide from *Bacillus anthracis,* p60 signal peptide from *Listeria monocytogenes* and PhoD signal peptide from *B. subtilis* Tat signal peptide. In some embodiments, the codon-optimized polynucleotide encodes a signal peptide other than Protective Antigen signal peptide from *Bacillus anthracis.* In some embodiments, the polynucleotide encoding a signal peptide is codon-optimized for expression in *Listeria monocytogenes.*

In some embodiments, the codon-optimized polynucleotide encodes a (non-signal peptide) protein that is foreign to the bacterium for which the polynucleotide sequence has been codon-optimized. In some embodiments, the codon-optimized polynucleotide encodes a polypeptide comprising an antigen. For instance, in some embodiments, the codon-optimized polynucleotide encodes a polypeptide comprising an antigen that is a tumor associated antigen or an antigen that is derived from a tumor-associated antigen.

In some embodiments, codon-optimization of a polynucleotide encoding a signal peptide and/or other polypeptide enhances expression of a polypeptide (such as a fusion protein, protein chimera and/or a foreign polypeptide encoded by a recombinant nucleic acid molecule, expression cassette, or expression vector) comprising the signal peptide and/or other polypeptide in a bacterium, relative to the corresponding polynucleotide without codon-optimization. In some embodiments, the codon-optimization of the polynucleotide enhances expression by at least about 2-fold, by at least about 5-fold, by at least about 10-fold, or by at least about 20 fold (relative to the corresponding polynucleotide without codon-optimization). In some embodiments, codon-optimization of a polynucleotide encoding a signal peptide and/or other polypeptide enhances secretion of a polypeptide (such as a fusion protein, protein chimera and/or a foreign polypeptide) comprising the signal peptide and/or other polypeptide from a bacterium, relative to the corresponding polynucleotide without codon-optimization. In some embodiments, the codon-optimization enhances secretion by at least about 2-fold, by at least about 5-fold, by at least about 10-fold, or by at least about 20 fold (relative to the corresponding polynucleotide without codon-optimization). In some embodiments, both the level of expression and secretion is enhanced. Levels of expression and/or secretion can be readily assessed using techniques standard to those in the art such as Western blots of the various relevant bacterial culture fractions.

### VI. Expression cassettes

Expression cassettes are also provided by the present invention. For instance, in some embodiments, described herein is an expression cassette comprising any of the recombinant nucleic acid molecules described herein and further comprising promoter sequences operably linked to the coding sequences in the recombinant nucleic acid molecules (e.g., the first polynucleotide encoding a signal peptide and the second polynucleotide encoding the other polypeptide). In some embodiments, the expression cassette is isolated. In some other embodiments, the expression cassette is contained within an expression vector, which may be isolated or may be contained within a bacterium. In still further embodiments, the expression cassette is positioned in the chromosomal DNA of a bacterium. For instance, in some embodiments, the expression cassette has been integrated within the genome of a bacterium. In some embodiments, an expression cassette that is integrated within the genome of a bacterium comprises one or more elements from the genomic DNA. For instance, in some embodiments, a recombinant nucleic acid molecule is inserted in a site in the genomic DNA of a bacterium (e.g., via site-specific integration or homologous recombination) such that the recombinant nucleic acid is operably linked to a promoter already present in the genomic DNA, thereby generating a new expression cassette integrated within the genomic DNA. In some other embodiments, the expression cassette is integrated into the genomic DNA (e.g., via site-specific integration or homologous recombination) as an intact unit comprising both the promoter and the recombinant nucleic acid molecule.

In some embodiments, the expression cassettes are designed for expression of polypeptides in bacteria. In some embodiments, the expression cassettes are designed for the expression of heterologous polypeptides, such as heterologous antigens in bacteria. In some embodiments, the expression cassettes provide enhanced expression and/or secretion of the polypeptides.

Generally, an expression cassette comprises the following ordered elements: (1) a promoter and (2) a polynucleotide encoding a polypeptide. In some embodiments, an expression cassette comprises the following elements: (1) a promoter; (2) a polynucleotide encoding a signal peptide; and (3) a polynucleotide encoding a polypeptide (e.g., a heterologous protein). In still other embodiments, an expression cassette comprises the following elements: (1) prokaryotic promoter; (2) Shine-Dalgarno sequence; (3) a polynucleotide encoding a signal peptide; and, (4) a polynucleotide encoding a polypeptide (such as a heterologous protein). In some embodiments, an expression cassette comprises more than one promoter.

In some embodiments, the expression cassette may also contain a transcription termination sequence inserted downstream from the C-terminus of the translational stop codon related to the heterologous polypeptide. For instance, in some embodiments, a transcription termination sequence may be used in constructs designed for stable integration within the bacterial chromosome. While not required, inclusion of a transcription termination sequence as the final ordered element in a heterologous gene expression cassette may prevent polar effects on the regulation of expression of adjacent genes due to read-through transcription. Accordingly, in some embodiments, appropriate sequence elements known to those who are skilled in the art that promote either rho-dependent or rho-independent transcription termination can be placed in the heterologous protein expression cassette.

In one aspect, described herein is an expression cassette comprising the following: (a) a first polynucleotide encoding a signal peptide, wherein the first polynucleotide is codon-optimized for expression in a bacterium; (b) a second polynucleotide encoding a polypeptide, wherein the second polynucleotide is in the same translational reading frame as the first polynucleotide; and (c) a promoter operably linked to the first and second polynucleotides, so that the expression cassette encodes a fusion protein comprising the signal peptide and the polypeptide.

In another aspect, described herein is an expression cassette comprising (a) a first polynucleotide encoding a signal peptide native to a bacterium, wherein the first polynucleotide is codon-optimized for expression in the bacterium, (b) a second polynucleotide encoding a polypeptide, wherein the second polynucleotide is in the same translational reading frame as the first polynucleotide, and (c) a promoter operably linked to the first and second polynucleotides of the expression cassette, wherein the recombinant nucleic acid molecule encodes a fusion protein comprising the signal peptide and the polypeptide. In some embodiments, the polypeptide encoded by the second polynucleotide is heterologous to the signal peptide. In some embodiments, the second polynucleotide is heterologous to the first polynucleotide. In some embodiments, the polypeptide is heterologous to the bacterium to which the signal peptide is native (i.e., foreign to the bacterium). In some embodiments, the bacterium from which the signal peptide is derived is an intracellular bacterium. In some embodiments, the bacterium is selected from the group consisting of *Listeria, Bacillus, Yersinia pestis, Salmonella, Shigella, Brucelia*, mycobacteria and *E. coli.* In some embodiments the bacterium is a *Listeria* bacterium (e.g., *Listeria monocytogenes).* In some embodiments, the second polynucleotide is codon-optimized for expression in the bacterium.

In another aspect, described herein is an expression cassette, wherein the expression cassette comprises (a) a first polynucleotide encoding a signal peptide, wherein the first polynucleotide is codon-optimized for expression in a *Listeria* bacterium, (b) a second polynucleotide encoding a polypeptide, wherein the second polynucleotide is in the same translational reading frame as the first polynucleotide, and (c) a promoter operably linked to the first and second polynucleotides of the expression cassette, wherein the recombinant nucleic acid molecule encodes a fusion protein comprising the signal peptide and the polypeptide. In some embodiments, the expression cassette is polycistronic expression cassette. In some embodiments, the second polynucleotide is codon-optimized for expression in the *Listeria* bacterium. In some embodiments, the polypeptide encoded by the second polynucleotide is foreign to the *Listeria* bacterium (i.e., heterologous to the *Listeria* bacterium). In some embodiments, the polypeptide encoded by the second polynucleotide is heterologous to the signal peptide. In some embodiments, the expression cassette comprises more than one promoter.

In another aspect, described herein is an expression cassette comprising (a) a first polynucleotide encoding a non-secA1 bacterial signal peptide; (b) a second polynucleotide encoding a polypeptide in the same translational reading frame as the first polynucleotide; and (c) a promoter operably linked to the first and second polynucleotides, so that the expression cassette encodes a fusion protein comprising the signal peptide and the polypeptide. In some embodiments, the first polynucleotide and/or the second polynucleotide is codon-optimized for expression in a bacterium, such as *Listeria, Bacillus, Yersinia pestis*, *Salmonella, Shigella, Brucella,* mycobacteria or *E. coli.* In some embodiments, the polynucleotide(s) is codon-optimized for expression in *Listeria,* such as *Listeria monocytogenes.* In some embodiments, the signal peptide encoded by the codon-optimized first polynucleotide is native to the bacterium for which it is codon-optimized. In some embodiments, the first polynucleotide encoding the signal peptide is heterologous to the second polynucleotide. In some embodiments, the polypeptide encoded by the second polynucleotide is heterologous to the signal peptide. In some embodiments, the expression cassette is a polycistronic expression cassette. In some embodiments, the first polynucleotide, the second polynucleotide, or both the first and second polynucleotide is codon-optimized for expression in a *Listeria* bacterium (e.g., *Listeria monocytogenes*). In some embodiments, the first and second polynucleotides are heterologous to each other. In some embodiments, the polypeptide encoded by the second polynucleotide and the signal peptide are heterologous to each other. In some embodiments, the polypeptide encoded by the second polynucleotide is foreign to the *Listeria* bacterium (i.e., heterologous to the *Listeria* bacterium). In some embodiments, the expression cassette comprises more than one promoter.

This disclosure also provides an expression cassette comprising the following: (a) a polynucleotide encoding a polypeptide foreign to *Listeria,* wherein the polynucleotide is codon-optimized for expression in *Listeria*; and (b) a promoter, operably linked to the polynucleotide encoding the foreign polypeptide. In some embodiments, the polypeptide that is encoded by the expression cassette is an antigen (e.g., see description of some possible antigens above). In some embodiments, the expression cassette further comprises a polynucleotide encoding a signal peptide. The polynucleotide encoding the signal peptide is also operably linked with the promoter so that the expression cassette expresses a fusion protein comprising both the foreign polypeptide and the signal peptide. Polynucleotides encoding signal peptides suitable for use in the expression cassette include, but are not limited to, those described above. In some embodiments, the polynucleotide encoding a signal peptide that is included in the expression cassette is codon-optimized for expression in a bacterium such as *Listeria* (e.g., a *L. monocytogenes* bacterium) as described above.

This disclosure also provides an expression cassette comprising the following: (a) a first polynucleotide encoding a non-Listerial signal peptide; (b) a second polynucleotide encoding a polypeptide that is in the same translational reading frame as the first polynucleotide; and (c) a promoter operably linked to both the first and second polynucleotides, wherein the expression cassette encodes a fusion protein comprising both the non-Listerial signal peptide and the polypeptide. In some embodiments, the expression cassette is a polycistronic expression cassette. In some embodiments, the first polynucleotide, the second polynucleotide, or both the first and second polynucleotide is codon-optimized for expression in *Listeria* (e.g., *Listeria monocytogenes*). In some embodiments, the first and second polynucleotides are Heterologous to each other. In some embodiments, the polypeptide encoded by the second polynucleotide and the signal peptide are heterologous to each other. In some embodiments, the polypeptide encoded by the second polynucleotide is foreign to the *Listeria* bacterium (i.e., heterologous to the *Listeria* bacterium). In some embodiments, the expression cassette comprises more than one promoter.

This disclosure further provides an expression cassette, wherein the expression cassette comprises (a) a first polynucleotide encoding a bacterial autolysin, or a catalytically active fragment or catalytically active variant thereof; and (b) a second polynucleotide encoding a polypeptide, wherein the second polynucleotide is in the same translational reading frame as the first polynucleotide, and (c) a promoter operably linked to the first and second polynucleotides, wherein the expression cassette encodes a protein chimera comprising the polypeptide encoded by the second polynucleotide and the autolysin, or catalytically active fragment or catalytically active variant thereof, wherein in the protein chimera the polypeptide is fused to the autolysin, or catalytically active fragment or catalytically active variant thereof, or is inserted within the autolysin, or catalytically active fragment or catalytically active variant thereof. In some embodiments, the protein chimera is catalytically active as an autolysin. In some embodiments, the polypeptide is heterologous to the autolysin. In some embodiments, the bacterial autolysin is from an intracellular bacterium (e.g., *Listeria*). In some embodiments, the second polynucleotide encoding the polypeptide is inserted within the first polynucleotide encoding the autolysin, or catalytically active fragment or catalytically active variant thereof, and the expression cassette encodes a protein chimera in which the polypeptide is inserted within the autolysin, or catalytically active fragment or catalytically active variant thereof (i.e., the polypeptide is embedded within the autolysin or catalytically active fragment or catalytically active variant thereof). In alternative embodiments, the second polynucleotide is positioned outside of the first polynucleotide encoding the autolysin, or catalytically active fragment or catalytically active variant thereof, and the expression cassette encodes a protein chimera in which the polypeptide is fused to the autolysin, or catalytically active fragment or catalytically active variant thereof. In some embodiments, the polypeptide is heterologous to the autolysin. In some embodiments, the first polynucleotide and the second polynucleotide are heterologous to each other. In some embodiments, the autolysin is a SecA2-dependent autolysin. In some embodiments, the autolysin is a peptidoglycan hydrolase (e.g., N-acetylmuramidase or p60). In some embodiments, the expression cassette further comprises a polynucleotide encoding a signal peptide (e.g., a signal peptide normally associated with the autolysin or a signal peptide heterologous to the signal peptide). For instance, in some embodiments, the expression cassette encodes a protein chimera comprising a p60 signal peptide, the p60 protein (or catalytically active fragment or catalytically active variant thereof), and a polypeptide heterologous to p60, embedded within the p60 sequence. In some embodiments, the polypeptide encoded by the second polynucleotide is a non-Listerial polypeptide.

In another aspect, described herein is an expression cassette comprising (a) a first polynucleotide encoding a signal peptide, (b) a second polynucleotide encoding a secreted protein, or a fragment thereof, wherein the second polynucleotide is in the same translational reading frame as the first polynucleotide, (c) a third polynucleotide encoding a polypeptide heterologous to the secreted protein, or fragment thereof, wherein the third polynucleotide is in the same translational reading frame as the first and second polynucleotides, and (d) promoter operably linked to the first, second, and third polynucleotides, wherein the recombinant nucleic acid molecule encodes a protein chimera comprising the signal peptide, the polypeptide encoded by the second polynucleotide, and the secreted protein, or fragment thereof, and wherein the polypeptide encoded by the third polynucleotide is fused to the secreted protein, or fragment thereof, or is positioned within the secreted protein, or fragment thereof, in the protein chimera.

In some embodiments, the promoters in the expression cassettes described herein (or recombinant nucleic acid molecules described herein) are prokaryotic promoters. For instance, the prokaryotic promoters can be Listerial promoters. In some embodiments, the Listerial promoter is an *hly* promoter. In some embodiments, the promoters are prfA-dependent promoters (e.g., an *actA* promoter). In some embodiments, the promoters are constitutive promoters (e.g., a *p60* promoter). In some embodiments, the expression cassette comprising a recombinant nucleic acid molecule described herein comprises an *hly, actA,* or *p60* promoter operably linked to the polynucleotides of the recombinant nucleic acid molecule. One of ordinary skill in the art will be readily able to identify additional prokaryotic and/or Listerial promoters suitable for use in the expression cassettes in view of the intended use of the expression cassette and host bacteria into which the expression cassette will be placed.

For instance, a variety of mycobacterial promoters suitable for use in the recombinant expression cassettes within mycobacterial and other bacteria are known. These include the *Mycobacterium bovis* BCG promoters HSP60 and HSP70, and also include such promoters as the mycobactin promoters, α-antigen promoter and 45 KDa antigen promoter of *M. tuberculosis* and BCG, the superoxide dismutase promoter, MBP-70, the mycobacterial asd promoter, the mycobacterial 14 kDa and 12 kDa antigen promoters, mycobacteriophage promoters such as the Bxb1, Bxb2, and Bxb3 promoters, the L1 and L5 promoters, the D29 promoter and the TM4 promoters (see, e.g., U.S. Patent No. 6,566,121). Promoters suitable for use in *Bacillus anthracis* include, but are not limited to, the pagA promoter, the alpha-amylase promoter (Pamy), and Pntr (see, e.g., Gat et al., Infect. Immun., 71;801-13 (2003)). Promoters suitable for use in recombinant *Salmonella* expression cassettes and vaccines are also known and include the nirB promoter, the osmC promoter, P(pagC), and P(tac) (see, e.g., Bumann, Infect. Immun. 69:7493-500 (2001); Wang et al., Vaccine, 17:1-12 (1999); McSorley et al., Infect. Immun. 65:171-8 (1997)). A variety of *E. coli* promoters are also known to those of ordinary skill in the art.

In some embodiments, the promoter used in an expression cassette described herein is a constitutive promoter. In other embodiments, the promoter used in an expression cassettes described herein is an inducible promoter. The inducible promoter can be induced by a molecule (e.g., a protein) endogenous to the bacteria in which the expression cassette is to be used. Alternatively, the inducible promoter can be induced by a molecule (e.g. a small molecule or protein) heterologous to the bacteria in which the expression cassette is to be used. A variety of inducible promoters are well-known to those of ordinary skill in the art.

In some embodiments of the expression cassettes, at the 3'-end of the promoter is a poly-purine Shine-Dalgarno sequence, the element required for engagement of the 30S ribosomal subunit (via 16S rRNA) to the heterologous gene RNA transcript and initiation of translation. The Shine-Dalgarno sequence has typically the following consensus sequence: 5'-NAGGAGGU-N₅₋₁₀-AUG (start codon)-3' (SEQ ID NO:85). There are variations of the poly-purine Shine-Dalgarno sequence. Notably, the *Listeria hly* gene that encodes listerolysin O (LLO) has the following Shine-Dalgarno sequence: AAGGAGAGTGAAACCCATG (SEQ ID NO:70) (Shine-Dalgarno sequence is underlined, and the translation start codon is bolded).

The construction of expression cassettes for use in bacteria, and even the construction of expression cassettes specifically for use in recombinant bacterial vaccines, are known in the art. For instance, descriptions of the production and use of a variety of bacterial expression cassettes and/or recombinant bacterial vaccines can be found in the following references: Horwitz et al., Proc. Natl. Acad. Sci. USA, 97:13853-8 (2000); Garmory et al., J. Drug Target, 11:471-9 (2003); Kang et al., FEMS Immunol. Med. Microbiol., 37:99-104 (2003); Garmory et al., Vaccine, 21:3051-7 (2003); Kang et al., Infect. Immun., 1739-49 (2002); Russman, et al., J. Immunol., 167:357-65 (2001); Harth et al., Microbiology, 150:2143-51 (2004); Varaldo et al., Infect. Immun., 72:3336-43 (2004); Goonetilleke et al., J. Immunol., 171:1602-9 (2003); Uno-Furuta et al., Vaccine, 21:3149-56 (2003); Biet et al., Infect. Immun., 71:2933-7 (2003); Bao et al., Infect. Immun., 71:1656-61 (2003); Kawahara et al., Clin Immunol., 105:326-31 (2002); Anderson et al., Vaccine, 18:2193-202 (2000); Bumann, Infect. Immun., 69:7493-500 (2001); Wang et al., Vaccine, 17:1-12 (1999); McSorley et al., Infect. Immun., 65:171-8 (1997); Gat et al., Infect. Immun., 71:841-13 (2003); U.S. Patent No. 5,504,005; U.S. Patent No. 5,830,702; U.S. Patent No. 6,051,237; US Patent Publication No. 2002/0025323; US Patent Publication No. 2003/0202985; WO 04/062597; US Patent No. 6,566,121; and U.S. Patent No. 6,270,776.

In some embodiments, it is desirable to construct expression cassettes that utilize bicistronic, polycistronic (also known as multicistronic) expression of heterologous coding sequences. Such expression cassettes can utilize, for example, a single promoter that is operably linked to two or more independent coding sequences. These coding sequences can, for example, correspond to individual genes or can, alternatively, correspond to desired and/or selected sub-fragments of a whole designated gene. In this later example, a gene might contain a sequence encoding a hydrophobic trans-membrane domain, which may potentially inhibit efficient secretion from *Listeria.* Thus, it may be desirable to segregate in two sub-fragments the coding sequence of this gene from the hydrophobic domain; in this instance the two sub-fragments are then expressed as a bicistronic message. Utilization of polycistronic expression requires that the 30s ribosome subunit stay on the polycistronic RNA message following translation termination of the first coding sequence and release of the 50s ribosome sub-unit, and subsequently "read-through" the RNA message to the next initiation codon, during which the 50s ribosome sub-unit binds to the RNA-bound 30s ribosome subunit, and re-initiating translation.

*Listeria monocytogenes,* like other bacteria, utilizes polycistronic expression of its genomic repertoire. By way of example, the sequence of a *Listeria monocytogenes* intergenic region from a selected polycistronic message can be used to construct polycistronic expression cassettes for expression of a selected heterologous protein from recombinant *Listeria* species. For example, several of the prfA-dependent virulence factors from *Listeria monocytogenes* are expressed from polycistronic message. For instance, the *Listeria monocytogenes* ActA and PlcB proteins are expressed as a bicistronic message. The DNA sequence corresponding to the *Listeria monocytogenes* actA-plcB intergenic sequence (5'-3') is shown below:
5'-TAAAAACACAGAACGAAAGAAAAA**GTGAGG**TGAATGA-3' (SEQ ID NO:71)
(The Shine-Dalgarno sequence for translation initiation of plcB is shown in bold. The first 3 nucleotides of the sequence correspond to an Ochre stop codon.) For a non-limiting example of a bicistronic expression vector, a bicistronic hEphA2 expression vector for use in *Listeria monocytogenes,* see Example 28, below.

Alternatively, other known intergenic or synthetic sequences can be used to construct polycistronic expression cassettes for use in *Listeria* or other bacteria. Construction of intergenic regions which lead to substantial secondary RNA structure should be prevented, to avoid unwanted transcription termination by a rho-independent mechanism.

Importantly, if secretion of any or all translated proteins expressed from the polycistronic message is desired, signal peptides must be functionally linked to each coding region. In some embodiments, these signal peptides differ from each other.

Thus, in some embodiments, the expression cassettes described herein for use in *Listeria* or other bacteria arc polycistronic (e.g., bicistronic). Two or more polypeptides are encoded by the bicistronic or polycistronic expression cassettes as discrete polypeptides. In some embodiments, the bicistronic or polycistronic expression cassettes comprise an intergenic sequence (e.g., from a bicistronic or polycistronic gene) positioned between the coding sequences of the two polypeptides. In some embodiments, the intergenic sequence comprises a sequence which promotes ribosomal entry and initiation of translation. In some embodiments, the intergenic sequence comprises a Shine-Dalgarno sequence. In some embodiments, the intergenic sequence is the *Listeria monocytogenes* actA-plcB intergenic sequence. Typically, the intergenic sequence is positioned between a polynucleotide sequence encoding a first polypeptide (or a first fusion protein comprising a first polypeptide and a signal peptide) and a polynucleotide sequence encoding a second polypeptide (or a second fusion protein comprising a second polypeptide and signal peptide).

Accordingly, in one aspect, described herein is an expression cassette comprising the following: (a) a first polynucleotide encoding a first polypeptide; (b) a second polynucleotide encoding a second polypeptide; (c) an intergenic sequence positioned between the first and second polynucleotides; and (f) a promoter operably linked to the first and second polynucleotides, wherein the expression cassette encodes the first and second polypeptides as two discrete polypeptides. In some embodiments, the first and second polypeptides are polypeptides selected from any of the polypeptides described herein (e.g., in Section IV, above). In some embodiments, at least one of the first or second polypeptides comprises an antigen. In some embodiments, the first and second polynucleotides each comprise a (different or the same) fragment of the same antigen. In some embodiments, the antigen is a tumor-associated antigen or is derived from a tumor-associated-antigen.

This disclosure further provides an expression cassette comprising the following: (a) a first polynucleotide encoding a first signal peptide; (b) a second polynucleotide encoding a first (non-signal) polypeptide, wherein the second polynucleotide is in the same translational reading frame as the first polynucleotide; (c) a third polynucleotide encoding a second signal peptide; (d) a fourth polynucleotide encoding a second (non-signal) polypeptide, wherein the fourth polynucleotide is in the same translational reading frame as the third polynucleotide; (e) an intergenic sequence (typically positioned between the second polynucleotide and the third polynucleotide); and (f) a promoter operably linked to the first polynucleotide, second polynucleotide, third polynucleotide, and fourth polynucleotide, so that the expression cassette encodes both a first fusion protein comprising the first signal peptide and the first polypeptide and a second fusion protein comprising the second signal peptide and second polypeptide. In some embodiments, the one or more of the polynucleotides encoding a signal peptide is codon-optimized for expression in a bacterium. In some embodiments, the third and/or fourth polynucleotides are codon-optimized for expression in a bacterium (preferably in addition to codon-optimization of the polynucleotides encoding the signal peptides). In some embodiments, the first and/or second signal peptide is a non-secA1 bacterial signal peptide. In some embodiments, the intergenic sequence is the *Listeria monocytogenes* actA-plcB intergenic sequence. In some embodiments, the second and third polypeptides are polypeptides selected from any of the polypeptides described herein (e.g., in Section IV, above), such as polypeptides comprising antigens. In some embodiments, the first and second polypeptides are polypeptides selected from any of the polypeptides described herein (e.g., in Section IV, above). In some embodiments, at least one of the first or second polypeptides comprises an antigen. In some embodiments, the first and second polynucleotides each comprise a fragment of the same antigen. In some embodiments, the antigen is a tumor-associated antigen or is derived from a tumor-associated-antigen.

For instance, described herein is a polycistronic expression cassette for expression of heterologous polypeptides in *Listeria,* wherein the expression cassette encodes at least two discrete non-Listerial polypeptides. In some embodiments, the polycistronic expression cassette is a bicistronic expression cassette which encodes two discrete non-Listerial polypeptides. In some embodiments, the expression cassette comprises the following: (a) a first polynucleotide encoding a first non-Listerial polypeptide; (b) a second polynucleotide encoding a second non-Listerial polypeptide; (c) an intergenic sequence positioned between the first and second polynucleotides; and (d) a promoter operably linked to the first and second polynucleotides, wherein the expression cassette encodes the first and second polypeptides as two discrete polypeptides. If the expression cassette is a polycistronic expression cassette that encodes three polypeptides as discrete polypeptides, the expression cassette will comprise a third polynucleotide operably linked to the promoter and a second intergenic sequence positioned between the second and third polynucleotide. In some embodiments, at least one of the non-Listerial polypeptides comprises an antigen. In some embodiments, at least two of the non-Listerial polypeptides each comprises a fragment of the same antigen.

In some embodiments, the expression cassette comprises the following: (a) a first polynucleotide encoding a first signal peptide; (b) a second polynucleotide encoding a first (non-signal) non-Listerial polypeptide, wherein the second polynucleotide is in the same translational reading frame as the first polynucleotide; (c) a third polynucleotide encoding a second signal peptide; (d) a fourth polynucleotide encoding a second (non-signal) non-Listerial polypeptide, wherein the fourth polynucleotide is in the same translational reading frame as the third polynucleotide; (e) an intergenic sequence positioned between the second polynucleotide and the third polynucleotide; and (f) a promoter operably linked to the first polynucleotide, second polynucleotide, third polynucleotide, and fourth polynucleotide, so that the expression cassette encodes both a first fusion protein comprising the first signal peptide and the first polypeptide and a second fusion protein comprising the second signal peptide and second polypeptide. In some embodiments, at least one of the non-Listerial polypeptides is an antigen. In some embodiments, at least two of the non-Listerial polypeptides are each fragments of the same antigen.

This disclosure also provides a method of using any of the expression cassettes described herein to produce a recombinant bacterium (e.g. a recombinant *Listeria* bacterium). In some embodiments, the method of using an expression cassette described herein to make a recombinant bacterium comprises introducing the expression cassette into a bacterium. In some embodiments, the expression cassette is integrated into the genome of the bacterium. In some other embodiments, the expression cassette is on a plasmid which is incorporated within the bacterium. In some embodiments, incorporation of the expression cassette into the bacterium occurs by conjugation. The introduction of the expression cassette into the bacterium can be effected by any of the standard techniques known in the art. For instance, incorporation of the expression cassette into the bacterium can occur by conjugation, transduction (transfection), or transformation.

### VII. Vectors

This disclosure further provides vectors, such as expression vectors, which comprise any one of the expression cassettes and/or recombinant nucleic acid molecules described herein. In some embodiments, the vector is a plasmid. In some embodiments, the vector is linear. In some embodiments, the vector is circular. In some embodiments, the vector is an integration or homologous recombinant vector. In some embodiments, the vector is pAM401. In some embodiments, the vector is pPL2. In some embodiments, the vector is isolated.

As indicated above, in some embodiments, an expression cassette described herein is contained within an expression vector. In some embodiments, the vector is a plasmid. In other embodiments the vector is linear. In alternative embodiments, the expression cassette is inserted (i.e. integrated) within genomic DNA of a bacterium using an expression vector. In some embodiments, the expression vector is linear. In other embodiments, the expression vector is circular.

Expression vectors suitable for use in bacteria such as *Listeria* are known to those skill in the art. There are a variety of suitable vectors suitable for use as a plasmid construct backbone for assembly of the expression cassettes. A particular plasmid construct backbone is selected based on whether expression of the polynucleotide (i.e., a polynucleotide encoding a heterologous antigen) from the bacterial chromosome or from an extra-chromosomal episome is desired.

In some embodiments, incorporation of the expression cassette (and/or recombinant nucleic acid molecule) into the bacterial chromosome of *Listeria monocytogenes* (*Listeria*) is accomplished with an integration vector that contains an expression cassette for a listeriophage integrase that catalyzes sequence-specific integration of the vector into the *Listeria* chromosome. For example, the integration vectors known as pPL1 and pPL2 program stable single-copy integration of a heterologous protein expression cassette within an innocuous region of the bacterial genome, and have been described in the literature (Lauer et. al.2002 J. Bacteriol. 184:4177-4178; U.S. Patent Publication No. 20030203472). The integration vectors are stable as plasmids in *E. coli* and are introduced via conjugation into the desired *Listeria* background. Each vector lacks a *Listeria*-specific origin of replication and encodes a phage integrase, such that the vectors are stable only upon integration into a chromosomal phage attachment site. Starting with a desired plasmid construct, the process of generating a recombinant *Listeria* strain expressing a desired protein(s) takes approximately one week. The pPL1 and pPL2 integration vectors are based, respectively, on the U153 and PSA listeriophages. The pPL1 vector integrates within the open reading frame of the comK gene, while pPL2 integrates within the tRNAArg gene in such a manner that the native sequence of the gene is restored upon successful integration, thus keeping its native expressed function intact. The pPL1 and pPL2 integration vectors contain a multiple cloning site sequence in order to facilitate construction of plasmids containing a recombinant nucleic acid molecule or an expression cassette such as the heterologous protein expression cassette. Some specific examples of the use of the pPL2 integration vector are described in Example 2 and Example 3, below.

Alternatively, incorporation of the expression cassette (and/or recombinant nucleic acid molecule) into the *Listeria* chromosome can be accomplished through allelic exchange methods, known to those skilled in the art. In particular, compositions in which it is desired to not incorporate a gene encoding an antibiotic resistance protein as part of the construct containing the expression cassette, methods of allelic exchange are desirable. For example, the pKSV7 vector (Camilli et. al. Mol. Microbiol. (1993) 8,143-157), contains a temperature-sensitive *Listeria* Gram-positive replication origin which is exploited to select for recombinant clones at the non-permissive temperature that represent the pKSV7 plasmid recombined into the *Listeria* chromosome. The pKSV7 allelic exchange plasmid vector contains a multiple cloning site sequence in order to facilitate construction of plasmids containing the protein expression cassette, and also a chloramphenicol resistance gene. For insertion into the *Listeria* chromosome, the expression cassette construct may be flanked by approximately 1 kb of chromosomal DNA sequence that corresponds to the precise location of desired integration. The pKSV7-expression cassette plasmid may be introduced into a desired bacterial strain by electroporation, according to standard methods for electroporation of Gram positive bacteria. A non-limiting example of a method of effecting allelic exchange using the pKSV7 vector is provided in Example 16 below.

In other embodiments, it may be desired to express the polypeptide (including a fusion protein comprising a polypeptide) from a stable plasmid episome. Maintenance of the plasmid episome through passaging for multiple generations requires the co-expression of a protein that confers a selective advantage for the plasmid-containing bacterium. As non-limiting examples, the protein co-expressed from the plasmid in combination with the polypeptide may be an antibiotic resistance protein, for example chloramphenicol, or may be a bacterial protein (that is expressed from the chromosome in wild-type bacteria), that can also confer a selective advantage. Non-limiting examples of bacterial proteins include enzyme required for purine or amino acid biosynthesis (selected using defined media lacking relevant amino acids or other necessary precursor macromolecules), or a transcription factor required for the expression of genes that confer a selective advantage *in vitro* or *in vivo* (Gunn et. al. 2001 J. Immuol. 167:6471-6479). As a non-limiting example, pAM401 is a suitable plasmid for episomal expression of a selected polypeptide in diverse Gram-positive bacterial genera (Wirth et. al. 1986 J. Bacteriol 165:831-836). For further description of exemplary uses of pAM401, see Examples 3 and 13, below.

Incorporation of the expression cassette into the bacterial chromosome of *B*. *anthracis* can, for instance, be accomplished with an integration vector that contains an expression cassette for a phage integrase that catalyzes sequence-specific integration of the vector into the *B. anthracis* chromosome. The integrase and attachment site of a *B. anthracis* phage can be used to derive an integration vector, to incorporate desired antigen expression cassettes into the vaccine composition. As a non-limiting example, the integrase and attachment site from the *B. anthracis* temperate phage w-alpha is used to derive a *B. anthracis* specific integration vector (McCloy, E.W. 1951. Studies on a lysogenic Bacillus stain, I. A bacteriophage specific for Bacillus anthracis. J. Hyg. 49:114-125).

Alternatively, incorporation of an antigen expression cassette into the B. *anthracis* chromosome can be accomplished through allelic exchange methods, known to those skilled in the art. See, e.g., Gat et al., Infect. Immun., 71:801-13 (2003). In particular, compositions in which it is desired to not incorporate a gene encoding an antibiotic resistance protein as part of the construct containing the expression cassette, methods of allelic exchange are desirable. For example, the pKSV7 vector (Camilli et. al. Mol. Microbiol. 1993 8,143-157), contains a temperature-sensitive *Listeria*-derived Gram positive replication origin which is exploited to select for recombinant clones at the non-permissive temperature that represent the pKSV7 plasmid recombined into the bacterial chromosome. The pKSV7 allelic exchange plasmid vector contains a multiple cloning site sequence in order to facilitate construction of plasmids containing the expression cassette, and also a chloramphenicol resistance gene. For insertion into the *Bacillus anthracis* chromosome, the expression cassette construct may be flanked by approximately 1 kb of chromosomal DNA sequence that corresponds to the precise location of desired integration. The pKSV7-expression cassette plasmid may be introduced into a desired bacterial strain by electroporation, according to standard methods for electroporation of Gram positive bacteria. A non-limiting example of a method of effecting allelic exchange in *B. anthracis* is provided in U.S. patent application Serial No. 10/883,599. In particular, allelic exchange using the pKSV7 vector can be used in strains of *B. anthracis* to add a desired antigen expression cassette at any desired location within the bacterial chromosome.

The allelic exchange methods described above using pKSV7 are broadly applicable to use in gram positive bacteria. In addition, a variety of expression vectors useful in bacteria, including recombinant bacterial vectors, are known to those of ordinary skill in the art. Examples include those vectors described in the following references: Horwitz et al., Proc. Natl. Acad. Sci. USA, 97:13853-8 (2000); Garmory et al., J. Drug Target, 11:471-9 (2003); Kang et al., FEMS Immunol. Med. Microbiol., 37:99-104 (2003); Garmory et al., Vaccine, 21:3051-7 (2003); Kang et al., Infect. Immun., 1739-49 (2002); Russman, et al., J. Immunol., 167:357-65 (2001); Harth et al., Microbiology, 150:2143-51 (2004); Varaldo et al., Infect. Immun., 72:3336-43 (2004); Goonetilleke et al., J. Immunol, 171:1602-9 (2003); Uno-Furuta et al., Vaccine, 21:3149-56 (2003); Biet et al., Infect. Immun., 71:2933-7 (2003); Bao et al., Infect. Immun., 71:1656-61 (2003); Kawahara et al., Clin. Immunol, 105:126-31 (2002); Anderson et al., Vaccine, 18:2193-202 (2000); Bumann, Infect. Immun., 69:7493-500 (2001); Wang et al., Vaccine, 17:1-12 (1999); McSorley et al., Infect. Immun., 65:171-8 (1997); Gat et al., Infect. Immun., 71:801-13 (2003); U.S. Patent No. 5,504,005; U.S. Patent No. 5,830,702; U.S. PatentNo. 6,051,237; US Patent Publication No. 2002/0025323; US Patent Publication No. 2003/0202985; WO 04/062597; US Patent No. 6,566,121; and U.S. Patent No. 6,270,776.

This disclosure further provides expression vectors comprising expression cassettes comprising the following: (a) a first polynucleotide encoding a first signal peptide; (b) a second polynucleotide encoding a first polypeptide, wherein the second polynucleotide is in the same translational reading frame as the first polynucleotide; (c) an intergenic sequence; (d) a third polynucleotide encoding a second signal peptide; (e) a fourth polynucleotide encoding a second polypeptide, wherein the fourth polynucleotide is in the same translational reading frame as the third polynucleotide; and (f) a promoter operably linked to the first polynucleotide, second polynucleotide, third polynucleotide, fourth polynucleotide, and intergenic sequence, such that the expression cassette encodes both a first fusion protein comprising the first signal peptide and the first polypeptide and a second fusion protein comprising the second signal peptide and second polypeptide.

This disclosure further provides methods of using any of the expression vectors described herein to produce a recombinant bacterium (e.g. a recombinant *Listeria* bacterium). In some embodiments, the method of using an expression vector described herein to make a recombinant bacterium comprises introducing the expression vector into a bacterium.

### VIII. Bacteria and other host cells

This disclosure further provides host cells comprising the recombinant nucleic acid molecules, expression cassettes, and/or vectors described herein (see, e.g., the Summary of the Invention and Sections I, II, VI, and VII of the Detailed Description, above, as well as the specific Examples below). In some embodiments, the cells are prokaryotic. In some embodiments, the cells are eukaryotic. In some embodiments, the cells are mammalian. In some embodiments, the cells are antigen-presenting cells, such as dendritic cells. In some embodiments, the cells are bacterial cells. In some embodiments, the host cells are isolated.

For example, described herein are bacteria comprising the recombinant nucleic acid molecules, expression cassettes, and/or the vectors described herein (see, e.g., Summary of the Invention and Sections I, II, VI, and VII of the Detailed Description, above, as well as the specific examples below). The bacteria comprising these polynucleotides are alternatively referred to herein as "recombinant bacteria," and a bacterium comprising a recombinant nucleic acid molecule, expression cassette, or vector described herein is alternatively referred to herein as "a recombinant bacterium." In some embodiments, the bacteria comprising the recombinant nucleic acid molecules, expression cassettes, and/or expression vectors are isolated. In some embodiments, the recombinant bacteria comprising the recombinant nucleic acid molecules, expression cassettes, and/or expression vectors express the polypeptides or fusion proteins encoded by the recombinant nucleic acid molecules, expression cassettes, and/or expression vectors contained therein. In some embodiments, the recombinant bacteria secrete the polypeptides or fusion proteins encoded by the recombinant nucleic acid molecules, expression cassettes, and/or expression vectors contained therein. In some embodiments, the recombinant bacteria express and secrete the polypeptides and/or fusion proteins in an amount sufficient to generate an immune response in a host upon administration of the bacteria (or a composition comprising the bacteria) to a host (e.g., a human subject).

In some embodiments, the bacteria are selected from the group consisting of gram positive bacteria, Gram negative bacteria, intracellular bacteria and mycobacteria. In some embodiments, the bacteria are gram positive bacteria. In some embodiments of this disclosure the bacteria are intracellular bacteria (e.g., facultative intracellular bacteria). In some embodiments the bacteria belong to the genus *Listeria.* In other embodiments, the bacteria are members of the species *Listeria monocytagenes.* In some other embodiments the bacteria are members of the *Listeria ivanavii, Listeria seeligeri,* or *Listeria innocua* species. In some embodiments, the bacteria are members of the genus *Bacillus.* In another embodiment, the bacteria are *Bacillus anthracis.* In still another embodiment, the bacteria are *Yersinia pestis*. In other embodiments of this disclosure the bacteria are from the genus *Salmonella*. In some embodiments, the bacteria are *Salmonella typhimurium*. In some embodiments, the bacteria belong to the genus *Shigella.* For instance, in some embodiments, the bacteria are *Shigella flexneri*. In some embodiments, the bacteria are members of the genus *Brucella.* In an alternative embodiment, the bacteria are mycobacteria. The mycobacteria is optionally a member of the species *Mycobacterium tuberculosis*. In some embodiments, the bacteria are Bacillus Calmette-Guerin (BCG). In some embodiments, the bacteria are *E. coli*, for instance, an *E*. *coli* which has been modified to express Listeriolysin O (LLO). Accordingly, in some embodiments, the bacteria comprising the recombinant nucleic acid molecules, expression cassettes, and/or vectors described herein are selected from the group consisting of *Listeria, Bacillus anthracis, Yersinia pestis, Salmonella*, and mycobacteria. In some other embodiments, the bacteria comprising the recombinant nucleic acid molecules, expression cassettes, and/or vectors described herein are selected from the group consisting of *Listeria, Bacillus, Yersinia pestis, Salmonella, Shigella, Brucella*, mycobacteria and *E. coli.*

In some embodiments, the bacteria of this disclosure that are modified through the insertion of the recombinant nucleic acid molecules, expression cassettes, and/or vectors described herein (e.g., see the Summary of the Invention, Sections I, II, VI, and VII of the Detailed Description, above, and the Examples, below) to express polypeptides, and, in at least some embodiments, secrete the polypeptides, are wild-type bacteria. For instance, in some embodiments, the recombinant bacterium is a wild-type *Listeria* bacterium, such as a *Listeria monocytogenes* bacterium, which comprises the recombinant nucleic acid molecule, expression cassette, and/or vector. However, in some embodiments of this disclosure the bacteria comprising the expression cassettes and/or vectors is a mutant strain of the bacteria. In some embodiments, the bacteria are attenuated. In some embodiments, the bacteria are an attenuated mutant strain of bacteria. A mutant in which a gene *"xyz"* has been deleted is alternatively referred to herein as *Δxyz* or *xyz⁻* or an *xyz* deletion mutant. For instance, a bacterial strain in which the uvrA gene has been deleted is alternatively referred to herein as uvrA mutant, *ΔuvrA*, or *uvrA⁻*. In addition, it will be understood by one of ordinary skill in the art that a reference to a particular mutant or strain as an "*xyz*" mutant or "*xyz*" strain will sometimes refer to a mutant or strain in which the *xyz* gene has been deleted.

The mutation in a mutant bacterium comprising the expression cassettes and/or expression vectors may be a mutation of any type. For instance, the mutation may constitute a point mutation, a frame-shift mutation, an insertion, a deletion of part or all of a gene. In addition, in some embodiments of the modified strains, a portion of the bacterial genome has been replaced with a heterologous polynucleotide. In some embodiments, the mutations are naturally-occurring. In other embodiments, the mutations are the results of artificial mutation pressure. In still other embodiments, the mutations in the bacterial genome are the result of genetic engineering.

In some embodiments, the bacteria comprising any one of the recombinant nucleic acid molecules, expression cassettes and/or vectors described herein are attenuated for cell-to-cell spread, entry into non-phagocytic cells, or proliferation (relative to the wild-type bacteria). The bacteria may be attenuated by mutation or by other modifications. In some embodiments, the bacteria comprising any one of the recombinant nucleic acid molecules, expression cassettes and/or expression vectors described herein are attenuated for cell-to-cell spread (e.g., *Listeria monocytogenes actA* mutants). In some embodiments, the bacteria comprising any one of the recombinant nucleic acid molecules, expression cassettes and/or expression vectors described herein are attenuated for entry into non-phagocytic cells (e.g., *Listeria monocytogenes* internalin mutants, such as *inlB* deletion mutants). In some embodiments, the bacteria comprising any one of the recombinant nucleic acid molecules, expression cassettes and/or expression vectors described herein are attenuated for proliferation. In some embodiments, the bacteria are attenuated both for cell-to-cell spread and for entry into non-phagocytic cells.

In some embodiments, the bacteria comprising the expression cassettes and/or expression vectors described herein are attenuated for cell-to-cell spread. In some embodiments, the bacteria (e.g., *Listeria)* are defective with respect to ActA (relative to the non-mutant or wildtype bacteria), or its equivalent (depending on the organism). In some embodiments, the bacteria comprise one or more mutation in *actA.* For instance, the bacterium (e.g., *Listeria)* may be an *actA* deletion mutant. ActA is the actin polymerase encoded by the *actA* gene (Kocks, et al., Cell, 68:521-531 (1992); Genbank accession no. AL591974, nts 9456-11389). The actin polymerase protein is involved in the recruitment and polymerization of host F-actin at one pole of the *Listeria* bacterium. Subsequent polymerization and dissolution of actin results in *Listeria* propulsion throughout the cytosol and into neighboring cells. This mobility enables the bacteria to spread directly from cell-to-cell without further exposure to the extracellular environment, thus escaping host defenses such as antibody development. In some embodiments, the attenuated *Listeria* optionally comprises both a mutation in an internalin gene, such as *inlB*, and in *actA.* The *Listeria* strain of this embodiment of the present invention is attenuated for entry into non-phagocytic cells as well as attenuated for cell-to-cell spreading.

In some embodiments, the capacity of the attenuated bacterium for cell-to-cell spread is reduced by at least about 10%, at least about 25%, at least about 50%, at least about 75%, or at least about 90%, relative to a bacterium without the attenuating mutation (e.g., the wild type bacterium). In some embodiments, the capacity of the attenuated bacterium for cell-to-cell spread is reduced by at least about 25% relative to a bacterium without the attenuating mutation. In some embodiments, the capacity of the attenuated bacterium attenuated for cell-to-cell spread is reduced by at least about 50% relative to a bacterium without the attenuating mutation.

*In vitro* assays for determining whether or not a bacterium such as a *Listeria* bacterium is attenuated for cell-to-cell spread are known to those of ordinary skill in the art. For example, the diameter of plaques formed over a time course after infection of selected cultured cell monolayers can be measured. Plaque assays within L2 cell monolayers can be performed as described previously in Sun, A., A. Camilli, and D.A. Portnoy. 1990, Isolation of Listeria monocytogenes small-plaque mutants defective for intracellular growth and cell-to-cell spread. Infect. Immun. 58:3770-3778, with modifications to the methods of measurement, as described by in Skoble, J., D.A. Portnoy, and M.D. Welch. 2000, Three regions within ActA promote Arp2/3 complex-mediated actin nucleation and Listeria monocytogenes motility. J. Cell Biol. 150:527-538. In brief, L2 cells are grown to confluency in six-well tissue culture dishes and then infected with bacteria for 1 h. Following infection, the cells are overlayed with media warmed to 40°C that is comprised of DME containing 0.8% agarose, Fetal Bovine Serum (e.g., 2%), and a desired concentration of Gentamicin. The concentration of Gentamicin in the media dramatically affects plaque size, and is a measure of the ability of a selected *Listeria* strain to effect cell-to-cell spread (Glomski, I J., M. M. Gedde, A. W. Tsang, J. A. Swanson, and D. A. Portnoy. 2002. J. Cell Biol. 156:1029-1038). For example, at 3 days following infection of the monolayer the plaque size of *Listeria* strains having a phenotype of defective cell-to-cell spread is reduced by at least 50% as compared to wild-type *Listeria,* when overlayed with media containing Gentamicin at a concentration of 50 µg/ml. On the otherhand, the plaque size between *Listeria* strains having a phenotype of defective cell-to-cell spread and wild-type *Listeria* is similar, when infected monolayers are overlayed with media + agarose containing only 5 µg/ml gentamicin. Thus, the relative ability of a selected strain to effect cell-to-cell spread in an infected cell monolayer relative to wild-type *Listeria* can be determined by varying the concentration of gentamicin in the media containing agarose. Optionally, visualization and measurement of plaque diameter can be facilitated by the addition of media containing Neutral Red (GIBCO BRL; 1:250 dilution in DME + agarose media) to the overlay at 48 h. post infection. Additionally, the plaque assay can be performed in monolayers derived from other primary cells or continuous cells. For example HepG2 cells, a hepatocyte-derived cell line, or primary human hepatocytes can be used to evaluate the ability of selected *Listeria* mutants to effect cell-to-cell spread, as compared to wild-type *Listeria.* In some embodiments, *Listeria* comprising mutations or other modifications that attenuate the *Listeria* for cell-to-cell spread produce "pinpoint" plaques at high concentrations of gentamicin (about 50 µg/ml).

In some embodiments, the bacteria comprising the expression cassettes and/or expression vectors described herein are mutant bacteria that are attenuated for nucleic acid repair (relative to wildtype such as bacteria without the attenuating genetic mutation). For instance, in some embodiments, the bacteria are defective with respect to at least one DNA repair enzyme (e.g., *Listeria monocytogenes uvrAB* mutants). In some embodiments, the bacteria are defective with respect to PhrB, UvrA, UvrB, UvrC, UvrD, and/or RecA, or one of their equivalents (depending on the genus and species of the bacteria). In some embodiments, the bacteria are defective with respect to UvrA, UvrB, and/or UvrC. In some embodiments, the bacteria comprise attenuating mutations in phrB, uvrA, uvrB, uvrC, uvrD, and/or recA genes. In some embodiments, the bacteria comprise one or more mutations in the *uvrA*, *uvrB*, and/or *uvrC* genes. In some embodiments, the bacteria are functionally deleted in UvrA, UvrB, and/or UvrC. In some embodiments, the bacteria are deleted in functional UvrA and UvrB. In some embodiments, the bacteria are *uvrAB* deletion mutants. In some embodiments, the bacteria are *ΔuvrABΔactA* mutants. In some embodiments, the nucleic acid of the bacteia which are attenuated for nucleic acid repair and/or are defective with respect to at least one DNA repair enzyme are modified by reaction with a nucleic acid targeting compound (see below). Nucleic acid repair mutants, such as *ΔuvrAB Listeria monocytogenes* mutants, and methods of making the mutants, are described in detail in U.S. Patent Publication No. 2004/0197343 (see, e.g., Example 7 of U.S. 2004/0197343).

In some embodiments, the capacity of the attenuated bacterium for nucleic acid repair is reduced by at least about 10%, at least about 25%, at least about 50%, at least about 75%, or at least about 90%, relative to a bacterium without the attenuating mutation (e.g., the wild type bacterium). In some embodiments, the capacity of the attenuated bacterium for nucleic acid repair is reduced by at least about 25% relative a bacterium without the attenuating mutation. In some embodiments, the capacity of the attenuated bacterium attenuated for nucleic acid repair is reduced by at least about 50% relative a bacterium without the attenuating mutation.

Confirmation that a particular mutation is present in a bacterial strain can be obtained through a variety of methods known to those of ordinary skill in the art. For instance, the relevant portion of the strain's genome can be cloned and sequenced. Alternatively, specific mutations can be identified via PCR using paired primers that code for regions adjacent to a deletion or other mutation. Southern blots can also be used to detect changes in the bacterial genome. Also, one can analyze whether a particular protein is expressed by the strain using techniques standard to the art such as Western blotting. Confirmation that the strain contains a mutation in the desired gene may also be obtained through comparison of the phenotype of the strain with a previously reported phenotype. For example, the presence of a nucleotide excision repair mutation such as deletion of *uvrAB* can be assessed using an assay which tests the ability of the bacteria to repair its nucleic acid using the nucleotide excision repair (NER) machinery and comparing that ability against wild-type bacteria. Such functional assays are known in the art. For instance, cyclobutane dimer excision or the excision of UV-induced (6-4) products can be measured to determine a deficiency in an NER enzyme in the mutant (see, e.g., Franklin et al., Proc. Natl. Acad. Sci. USA, 81: 3821-3824 (1984)). Alternatively, survival measurements can be made to assess a deficiency in nucleic acid repair. For instance, the bacteria can be subjected to psoralen/UVA treatment and then assessed for their ability to proliferate and/or survive in comparison to wild-type.

In some embodiments, the bacterium is attenuated for entry into non-phagocytic cells (relative or a non-mutant or wildtype bacterium). In some embodiments, the bacterium (e.g., *Listeria*) is defective with respect to one or more internalins (or equivalents). In some embodiments, the bacterium is defective with respect to internalin A. In some embodiments, the bacterium is defective with respect to internalin B. In some embodiments, the bacterium comprises a mutation in *inlA.* In some embodiments, the bacterium comprises a mutation in *inlB*. In some embodiments, the bacterium comprises a mutation in both *actA* and *inlB*. In some embodiments, the bacterium is deleted in functional ActA and internalinB. In some embodiments, the bacterium is an *ΔactAΔinlB* double deletion mutant. In some embodiments, the bacterium is defective with respect to both ActA and internalin B.

In some embodiments, the capacity of the attenuated bacterium for entry into non-phagocytic cells is reduced by at least about 10%, at least about 25%, at least about 50%, at least about 75%, or at least about 90%, relative to a bacterium without the attenuating mutation (e.g., the wild type bacterium). In some embodiments, the capacity of the attenuated bacterium for entry into non-phagocytic cells is reduced by at least about 25% relative to a bacterium without the attenuating mutation. In some embodiments, the capacity of the attenuated bacterium for entry into non-phagocytic cells is reduced by at least about 50% relative to a bacterium without the attenuating mutation. In some embodiments, the capacity of the attenuated bacterium for entry into non-phagocytic cells is reduced by at least about 75% relative to a bacterium without the attenuating mutation.

In some embodiments, the attenuated bacteria, such as a mutant *Listeria* strain, are not attenuated for entry into more than one type of non-phagocytic cell. For instance, the attenuated strain may be attenuated for entry into hepatocytes, but not attenuated for entry into epithelial cells. As another example, the attenuated strain may be attenuated for entry into epithelial cells, but not hepatocytes. It is also understood that attenuation for entry into a non-phagocytic cell of particular modified bacteria is a result of mutating a designated gene, for example a deletion mutation, encoding an invasin protein which interacts with a particular cellular receptor, and as a result facilitates infection of a non-phagocytic cell. For example, *Listeria ΔinlB* mutant strains are attenuated for entry into non-phagocytic cells expressing the hepatocyte growth factor receptor (c-met), including hepatocyte cell lines (e.g., HepG2), and primary human hepatocytes.

In some embodiments, even though the bacteria (e.g., mutant *Listeria*) are attenuated for entry into non-phagocytic cells, the *Listeria* are still capable of uptake by phagocytic cells, such as at least dendritic cells and/or macrophages. In one embodiment the ability of the attenuated bacteria to enter phagocytic cells is not diminished by the modification made to the strain, such as the mutation of an invasin (i.e. approximately 95% or more of the measured ability of the strain to be taken up by phagocytic cells is maintained post-modification). In other embodiments, the ability of the attenuated bacteria to enter phagocytic cells is diminished by no more than about 10%, no more than about 25%, no more than about 50%, or no more than about 75%.

In some embodiments of this disclosure the amount of attenuation in the ability of the bacterium (e.g., a *Listeria* bacterium) to enter non-phagocytic cells ranges from a twofold reduction to much greater levels of attenuation. In some embodiments, the attenuation in the ability of the bacteria to enter non-phagocytic cells is at least about 0.3 log, about 1 log, about 2 log, about 3 log, about 4 log, about 5 log, or at least about 6 log. In some embodiments, the attenuation is in the range of about 0.3 to > 8 log, about 2 to >8 log, about 4 to >8 log, about 6 to >8 log, about 0.3-8 log, also about 0.3-7 log, also about 0.3-6 log, also about 0.3-5 log, also about 0.3-4 log, also about 0.3-3 log, also about 0.3-2 log, also about 0.3-1 log. In some embodiments, the attenuation is in the range of about 1 to >8 log, 1-7 log, 1-6 log, also about 2-6 log, also about 2-5 log, also about 3-5 log.

A number of internalins have been identified in *L. monocytogenes* (Boland, et al., Clinical Microbiology Reviews, 2001, 14: 584-640). These internalins include, but are not limited to, InlA, InlB, InlC, InlC2, InlD, InlE, InlF, InlG, and InlH (Dramsi, et al., Infection and Immunity, 65: 1615-1625 (1997); Raffelsbauer et al., Mol. Gen. Genet. 260:144-158 (1988)). The gene sequences encoding these proteins have been previously reported. For instance, the sequences for both *inlA* and *inlB* have been reported in Gaillard et al., Cell, 65:1127-1141 (1991) and as GenBank accession number M67471. Genes encoding additional members of the internalin-related protein family are identified in Web Table 2 of the Supplementary Web material of Glaser et al., Science, 294:849-852 (2001), (www.sciencemag.org/cgi/content/fulll294/5543/849/DC1), including *lmo0327, lmo0331, Imo0514, lmo0610, lmo0732, lmo1136, Imo1289, lmo2396, lmo0171, lmo0333, lmo0801, lmo1290, lmo2026,* and *lmo2821*. (The sequences of each member of the internalin-related protein family can be found in the *L. monocytogenes* strain EGD genome, GenBank Accession no. AL591824, and/or in the *L. monocytogenes* strain EGD-e genome, Genbank Accession no. NC_003210. Locations of the various internalin-related genes are indicated in Glaser et al.).

InlA (internalin A) (Gaillard et al., Cell, 65:1127-1141 (1991); Genbank accession no. NC_003210) directs the uptake of *Listeria* by epithelial cells such as those of the intestines.

InlB (internalin B) (Gaillard et al., Cell, 65:1127-1141 (1991); Genbank accession number AL591975 (*Listeria monocytogenes* strain EGD, complete genome, segment 3/12, *inlB* gene region: nts. 97008-98963); and Genbank accession number NC_003210 (*Listeria monocytogenes* strain EGD, complete genome, *inlB* gene region: nts. 457008-458963)), directs the uptake of *Listeria* by hepatocytes or by endothelial cells such as the vascular endothelial cells of the brain microvasculature that comprise the blood brain barrier. (For further descriptions of internalin B, see Ireton, et al., J. of Biological Chemistry, 274: 17025-17032 (1999); Dramsi, et al., Molecular Microbiology, 16:251-261 (1995); Mansell et al., J. of Biological Chemistry, 276: 43597-43603 (2001); and Bierne et al., J of Cell Science 115:3357-3367 (2002)).

In some embodiments, the bacterium is deficient with respect to ActA, internalin B, or both Act A and internalin B. In some embodiments, the bacterium is deleted in functional ActA, internalin B, or both ActA and internalin B. In some embodiments, the bacterium is deleted in functional ActA. In some embodiments, the bacterium is deleted in functional internalin B. In some embodiments, the bacterium is deleted in functional ActA and internalin B.

*In vitro* assays for determining whether or not a bacterium (e.g., a mutant *Listeria* strain) is attenuated for entry into non-phagocytic cells are known to those of ordinary skill in the art. For instance, both Dramsi et al., Molecular Microbiology 16:251-261 (1995) and Gaillard et al., Cell 65:1127-1141 (1991) describe assays for screening the ability of mutant *L. monocytogenes* strains to enter certain cell lines. For instance, to determine whether a *Listeria* bacterium with a particular modification is attenuated for entry into a particular type of non-phagocytic cells, the ability of the attenuated *Listeria* bacterium to enter a particular type of non-phagocytic cell is determined and compared to the ability of the identical *Listeria* bacterium without the modification to enter non-phagocytic cells. Likewise, to determine whether a *Listeria* strain with a particular mutation is attenuated for entry into a particular type of non-phagocytic cells, the ability of the mutant *Listeria* strain to enter a particular type of non-phagocytic cell is determined and compared to the ability of the *Listeria* strain without the mutation to enter non-phagocytic cells. In addition, confirmation that the strain is defective with respect to internalin B may also be obtained through comparison of the phenotype of the strain with the previously reported phenotypes for internalin B mutants.

In some embodiments, the attenuation of bacteria can be measured in terms of biological effects of the *Listeria* on a host. The pathogenicity of a bacterial strain can be assessed by measurement of the LD₅₀ in mice or other vertebrates. The LD₅₀ is the amount, or dosage, of *Listeria* injected into vertebrates necessary to cause death in 50% of the vertebrates. The LD₅₀ values can be compared for bacteria having a particular modification (e.g., mutation) versus the bacteria without the particular modification as a measure of the level of attenuation. For example, if the bacterial strain without a particular mutation has an LD₅₀ of 10³ bacteria and the bacterial strain having the particular mutation has an LD₅₀ of 10⁵ bacteria, the strain has been attenuated so that is LD₅₀ is increased 100-fold or by 2 log.

Alternatively, the degree of attenuation of the ability of a bacterium (e.g., a *Listeria* bacterium) to infect non-phagocytic cells can be assessed much more directly *in vitro.* For instance, the ability of a modified *Listeria* bacterium to infect non-phagocytic cells, such as hepatocytes, can be compared to the ability of non-modified *Listeria* or wild type *Listeria* to infect phagocytic cells. In such an assay, the modified and non-modified *Listeria* are typically added to the non-phagocytic cells *in vitro* for a limited period of time (for instance, an hour), the cells are then washed with a gentamicin-containing solution to kill any extracellular bacteria, the cells are lysed and then plated to assess titer. Examples of such an assay are found in U.S. patent publication no. 2004/0228877.

As a further example, the degree of attenuation may also be measured qualitatively by other biological effects, such as the extent of tissue pathology or serum liver enzyme levels. Alanine aminotransferase (ALT), aspartate aminotransferase (AST), albumin and bilirubin levels in the serum are determined at a clinical laboratory for mice injected with *Listeria* (or other bacteria). Comparisons of these effects in mice or other vertebrates can be made for *Listeria* with and without particular modifications/mutations as a way to assess the attenuation of the *Listeria.* Attenuation of the *Listeria* may also be measured by tissue pathology. The amount of *Listeria* that can be recovered from various tissues of an infected vertebrate, such as the liver, spleen and nervous system, can also be used as a measure of the level of attenuation by comparing these values in vertebrates injected with mutant versus non-mutant *Listeria.* For instance, the amount of *Listeria* that can be recovered from infected tissues such as liver or spleen as a function of time can be used as a measure of attenuation by comparing these values in mice injected with mutant vs. non-mutant *Listeria.*

Accordingly, the attenuation of the *Listeria* can be measured in terms of bacterial load in particular selected organs in mice known to be targets by wild-type *Listeria.* For example, the attenuation of the *Listeria* can be measured by enumerating the colonies (Colony Forming Units; CFU) arising from plating dilutions of liver or spleen homogenates (homogenized in H₂0 + 0.2% NP40) on BHI agar media. The liver or spleen cfu can be measured, for example, over a time course following administration of the modified *Listeria* via any number of routes, including intravenous, intraperitoneal, intramuscular, and subcutaneous. Additionally, the *Listeria* can be measured and compared to a drug-resistant, wild type *Listeria* (or any other selected *Listeria* strain) in the liver and spleen (or any other selected organ) over a time course following administration by the competitive index assay, as described.

The degree of attenuation in uptake of the attenuated bacteria by non-phagocytic cells need not be an absolute attenuation in order to provide a safe and effective vaccine. In some embodiments, the degree of attenuation is one that provides for a reduction in toxicity sufficient to prevent or reduce the symptoms of toxicity to levels that are not life threatening.

In some embodiments of this disclosure the bacterium that comprises a recombinant nucleic acid molecule, expression cassette and/or expression vector described herein is a mutant strain of *Listeria.* In further embodiments, the bacterium is an attenuated mutant strain of *Listeria monocytogenes.* A variety of exemplary mutant strains of *Listeria* that are attenuated are described in the U.S. Patent Application Nos. 60/446,051 (filed February 6, 2003), 60/449,153 (filed February 21, 2003), 60/511,719 (filed October 15, 2003), 60/511,919 (filed October 15, 2003), 60/511,869 (filed October 15, 2003), 60/541,515 (filed February 2, 2004), and 10/883,599 (filed June 30, 2004), as well as in U.S. Patent Publication Nos. 2004/0197343 and US 2004/0228877. Mutant strains of *Listeria* are also described in the International Application No. PCT/US2004/23881, filed July 23, 2004. For instance, the bacterium that comprise the expression cassette and/or vector is optionally a mutant strain of *Listeria monocytogenes* that is defective with respect to ActA or internalin B, or both ActA and internalin B. In some embodiments, the bacterium is a mutant strain of *Listeria monocytogenes* that is *actA⁻* (e.g., DP-L4029 (the DP-L3078 strain described in Skoble et al., J. of Cell Biology, 150: 527-537 (2000), which has been cured of its prophage as described in (Lauer et al., J. Bacteriol. 184:4177 (2002); U.S. Patent Publication No. 2003/0203472)), *actA⁻inlB⁻* (e.g., DP-L4029*inlB*, deposited with the American Type Culture Collection (ATCC), 10801 University Blvd., Manassas, Virginia 20110-2209, United States of America, on October 3,2003, under the provisions of the Budapest Treaty on the International Recognition of the Deposit of Microorganisms for the Purposes of Patent Procedure, and designated with accession number PTA-5562), *actA⁻uvrAB⁻* (e.g., DP-*L4029uvrAB,* deposited with the American Type Culture Collection (ATCC), 10801 University Blvd., Manassas, Virginia 20110-2209, United States of America, on October 3, 2003, under the provisions of the Budapest Treaty on the International Recognition of the Deposit of Microorganisms for the Purposes of Patent Procedure, and designated with accession number PTA-5563), or *actA⁻inlB⁻uvrAB⁻.* In some embodiments, the attenuated *Listeria* bacterium (e.g., a *Listeria monocytogenes* bacterium) is an *ΔactAΔinlB* double deletion mutant.

Bacterial mutations can be achieved through traditional mutagenic methods, such as mutagenic chemicals or radiation followed by selection of mutants. Bacterial mutations can also be achieved by one of skill in the art through recombinant DNA technology. For instance, the method of allelic exchange using the pKSV7 vector described in Camilli et al., Molecular Micro. 8:143-157 (1993) and described above with respect to the introduction of heterologous expression cassettes in bacterial genomes is suitable for use in generating mutants including deletion mutants. (Camilli et al. (1993)) One exemplary example of the production of a *Listeria monocytogenes* internalin B mutant using the pKSV7 vector is provided in Example 24, below. Alternatively, the gene replacement protocol described in Biswas et al., J. Bacteriol. 175:3628-3635 (1993), can be used. Other similar methods are known to those of ordinary skill in the art.

The construction of a variety of bacterial mutants is described in U.S. patent application Serial No. 10/883,599, U.S. Patent Publication No. 2004/0197343, and U.S. Patent Publication no. 2004/0228877.

In some embodiments of this disclosure the bacterium that comprises the recombinant nucleic acid molecule, expression cassette and/or expression vector is a mutant strain of *Bacillus anthracis.* In some embodiments, the bacterium is the Sterne strain. In some embodiments, the bacterium is the Ames strain. In some embodiments, the *Bacillus anthracis* bacterium is a *uvrAB* mutant. In some embodiments, the *Bacillus anthracis* strain is a *uvrC* mutant In some embodiments, the *Bacillus anthracis* mutant is a *recA* mutant. In some embodiments, the bacterium is a *ΔuvrAB* mutant of the *Bacillus anthracis* (e.g., the *Bacillus anthracis* Sterne *ΔuvrAB* mutant deposited with the American Type Culture Collection (ATCC), 108011 University Blvd., Manassas, Virginia 20110-2209, United States of America, on February 20, 2004, under the provisions of the Budapest Treaty on the International Recognition of the Deposit of Microorganisms for the Purposes of Patent Procedure, and designated with accession number PTA-5825).

Methods of altering the genome of *Bacillus anthracis* are known to those skilled in the art. One method of generating mutations in *Bacillus anthracis* is by allelic exchange using an allelic exchange vector known to those in the art. An exemplary allelic exchange plasmid is pKSV7 described in Camilli et al., Molecular Microbiology, 8:143-147 (1993). As a first step in generating a mutant *Bacillus anthracis,* the region of the genome to be deleted or otherwise mutated and approximately 1000 bps both upstream and downstream of the *B. anthracis* genome is PCR-amplified and then cloned into the pKSV7 plasmid vector (or an analogous vector). (A *Bacillus* genera-specific or B. *anthracis-specific* temperature (ts) replicon may be substituted for the *Listeria* ts replicon present in the pKSV7 allelic exchange plasmid vector.) Restriction endonuclease recognition sites in the region to be deleted or mutated may be used to delete the desired portion of the targeted gene in the region. Alternatively, a portion of the targeted gene within the region may be removed and replaced with sequences containing the desired mutation or other alteration. The region of the *B. anthracis* genome that is amplified can be altered, for instance, using restriction enzymes or a combination of restriction enzymes and synthetic gene sequences, before or after cloning into the allelic exchange plasmid. In some embodiments, the sequence may be altered as a PCR amplicon and then cloned into pKSV7. In alternative embodiments, the amplicon is first inserted into another plasmid first and then altered, excised, and inserted into pKSV7. Alternatively, the PCR amplicon is inserted directly into the pKSV7 plasmid and then altered, for instance, using convenient restriction enzymes. The pKSV7 plasmid containing the altered sequence is then introduced into *B. anthracis.* This can be done via electroporation. The bacteria are then selected on media at a permissive temperature in the presence of chloramphenicol. This is followed by selection for single cross-over integration into the bacterial chromosome by passaging for multiple generations at a non-permissive temperature in the presence of chloramphenicol. Lastly, colonies are passaged for multiple generations at the permissive temperature in media not containing the antibiotic to achieve plasmid excision and curing (double cross-over). The U.S. Provisional Application Serial Nos. 60/584,886, and 60/599,522, and U.S. Patent Publication No. 2004/0197343, provide additional description regarding the construction of different types of *Bacillus anthracis* mutants.

In some embodiments of this disclosure the bacterium that comprises the recombinant nucleic acid molecule, expression cassette, and/or expression vector is a bacterium that has been modified so that the bacterium is attenuated for proliferation (relative to the non-modified bacterium). Preferably, the modified bacterium maintains a sufficient level of gene expression despite the modification. For instance, in some embodiments the gene expression level is substantially unaffected by the modification so that an antigen is expressed at a level sufficient to stimulate an immune response to the antigen upon administration of the bacterium expressing the antigen to a host. In some embodiments, the nucleic acid of the bacterium has been modified by reaction with a nucleic acid targeting compound. In some embodiments, the nucleic acid of the modified bacterium has been modified by reaction with a nucleic acid targeting compound that reacts directly with the nucleic acid so that proliferation of the bacterium is attenuated. In some embodiments, the nucleic-acid targeting compound is a nucleic acid alkylator, such as β-alanine, N-(acridin-9-yl), 2-[bis(2-chloroethyl)amino]ethyl ester. In some embodiments, the nucleic acid targeting compound is activated by irradiation, such as UVA irradiation. In some embodiments, the bacterium has been treated with a psoralen compound. For instance, in some embodiments, the bacterium has been modified by treatment with a psoralen, such as 4'-(4-amino-2-oxa)butyl-4,5',8-trimethylpsoralen ("S-59"), and UVA light. In some embodiments, the nucleic acid of the bacterium has been modified by treatment with a psoralen compound and UVA irradiation. Descriptions of methods of modifying bacteria to attenuate them for proliferation using nucleic acid targeting compounds are described in each of the following U.S. patent applications or publications: 60/446,051 (filed February 6, 2003), 60/449,153 (filed February 21,2003), 60/490,089 (filed July 24, 2003), 60/511,869 (filed October 15, 2003), 60/541,515 (filed February 2,2004), 10/883,599 (filed June 30, 2004), and US 2004/0197343. Modified bacteria and their uses are also described in International Application No. PCT/US2004/23881, filed July23, 2004.

For example, for treatment of *ΔactAΔuvrAB L. monocytogenes*, in some embodiments, S-59 psoralen can be added to 200 nM in a log-phase culture of (approximately) OD₆₀₀=0.5, followed by inactivation with 6 J/m² of UVA light when the culture reaches an optical density of one. Inactivation conditions are optimized by varying concentrations of S-59, UVA dose, the time of S-59 exposure prior to UVA treatment as well as varying the time of treatment during bacterial growth of the *Listeria actAluvrAB* strain. The parental *Listeria* strain is used as a control. Inactivation of *Listeria* (log-kill) is determined by the inability of the bacteria to form colonies on BHI (Brain heart infusion) agar plates. In addition, one can confirm the expression of a heterologous protein and virulence factors, such as LLO and p60, of the S-59/UVA inactivated *Listeria* using ³⁵S-pulse-chase experiments to determine the synthesis and secretion of newly expressed proteins post S-59 / UVA inactivation. Expression of LLO and p60 using ³⁵S-metabolic labeling can be routinely determined. S-59/UVA inactivated *Listeria actAluvrAB* can be incubated for I hour in the presence of ³⁵S-Methionine. Antigen expression and secretion of the heterologous protein, endogenous LLO, and p60 can be determined of both whole cell lysates, and TCA precipitation of bacterial culture fluids. LLO-, p60- and heterologous protein-specific monoclonal antibodies can be used for immunoprecipitation to verify the continued expression and secretion from recombinant *Listeria* post inactivation.

In some embodiments, the bacteia attenuated for proliferation are also attenuated for nucleic acid repair and/or are defective with respect to at least one DNA repair enzyme. For instance, in some embodiments, the bacterium in which nucleic acid has been modified by a nucleic acid targeting compound such as a psoralen (combined with UVA treatment) is a *uvrAB* deletion mutant.

In some embodiments, the proliferation of the bacteria is attenuated by at least about 0.3 log, also at least about 1 log, about 2 log, about 3 log, about 4 log, about 6 log, or at least about 8 log. In another embodiment, the proliferation of the bacteria is attenuated by about 0.3 to > 10 log, about 2 to >10 log, about 4 to >10 log, about 6 to >10 log, about 0.3-8 log, about 0.3-6 log, about 0.3-5 log, about 1-5 log, or about 2-5 log. In some embodiments, the expression of an antigen by the bacteria are at least about 10%, about 25%, about 50%, about 75%, or at least about 90% of the expression of the antigen by bacteria in which the bacterial nucleic acid is not modified.

In some embodiments, the nucleic acid of the bacterium has not been modified by reaction with a nucleic acid targeting compound. In some embodiments, the recombinant bacterium has not been attenuated for proliferation. In some embodiments, the recombinant bacterium is not attenuated in its ability for nucleic acid repair. In some embodiments, the recombinant bacterium is not deficient with respect to at least one DNA repair enzyme.

In some embodiments, the signal peptide encoded by first polynucleotide in the recombinant nucleic acid molecule, expression cassette, and/or expression vector contained within the recombinant bacterium is native to the recombinant bacterium. In some embodiments, the polynucleotide encoding the signal peptide that is native to the recombinant bacterium has been codon-optimized for expression in the recombinant bacterium. In some embodiments, the polynucleotide has been fully codon-optimized. In some embodiments, the signal peptide encoded by the first polynucleotide of the recombinant nucleic acid molecule, expression cassette, and/or expression vector contained within the recombinant bacterium is foreign to the host recombinant bacterium. In some embodiments, the polynucleotide encoding the signal peptide that is foreign to the host recombinant bacterium has been codon-optimized for expression in the recombinant bacterium.

In some embodiments, the second polynucleotide in the recombinant nucleic acid molecule, expression cassette, and/or expression vector within the recombinant bacterium has been codon-optimized for expression in the recombinant bacterium. In some embodiments, the second polynucleotide has been fully codon-optimized for expression in the recombinant bacterium. In some embodiments, both the first and second polynucleotides within the recombinant bacterium have been codon-optimized for expression in the recombinant bacterium. In some embodiments, both the first and second polynucleotides within the recombinant bacterium have been fully codon-optimized for expression in the recombinant bacterium.

In one aspect, described herein is a bacterium comprising an expression cassette, wherein the expression cassette comprises (a) a first polynucleotide encoding a signal peptide, wherein the first polynucleotide is codon-optimized for expression in the bacterium; (b) a second polynucleotide encoding a polypeptide, wherein the second polynucleotide is in the same translational reading frame as the first polynucleotide; and (c) a promoter operably linked to the first and second polynucleotides, wherein the expression cassette encodes a fusion protein comprising the signal peptide and the polypeptide. As described herein, e.g., in Section III, in some embodiments, the signal peptide that is encoded is a derived from bacteria. In some embodiments, the bacterial signal peptide encoded by the expression cassette is derived from the bacteria of the same genus and/or species as the bacterium comprising the expression cassette. In some embodiments, the signal peptide is native to the host recombinant bacterium. In other embodiments, the bacterial signal peptide encoded by the expression cassette is derived from bacteria of a different genus and/or species as the bacterium comprising the expression cassette. In some embodiments, the signal peptide is foreign to the host recombinant bacterium. In some embodiments the signal peptide is a secA1, secA2, or a Tat signal peptide. In some embodiments the polypeptide encoded by the second polynucleotide is heterologous (i.e., foreign) to the bacterium.

In another aspect, described herein is a bacterium comprising a recombinant nucleic acid molecule, comprising (a) a first polynucleotide encoding a signal peptide native to the bacterium, wherein the first polynucleotide is codon-optimized for expression in the bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the bacterium is an intracellular bacterium. In some embodiments, the recombinant nucleic acid molecule is part of an expression cassette that further comprises a promoter operably linked to both the first and second polynucleotides. In some embodiments, the bacterium is selected from the group consisting of *Listeria, Bacillus, Yersinia pestis, Salmonella, Shigella, Brucella*, mycobacteria and *E*. *coli.* In some embodiments, the bacterium is *Listeria* (e.g., *Listeria monocytogenes*).

In another aspect, described herein is a recombinant *Listeria* bacterium (e.g., *Listeria monocytogenes)* comprising a recombinant nucleic acid molecule, wherein the recombinant nucleic acid molecule comprises (a) a first polynucleotide encoding a signal peptide, wherein the first polynucleotide is codon-optimized for expression in the *Listeria* bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the recombinant nucleic acid molecule is part of an expression cassette that further comprises a promoter operably linked to both the first and second polynucleotides. In some embodiments, the second polynucleotide is codon-optimized for expression in the *Listeria* bacterium. In some embodiments, the polypeptide encoded by the second polynucleotide is foreign to the *Listeria* bacterium (i.e., heterologous to the *Listeria* bacterium). In some embodiments, the *Listeria* bacterium is attenuated. For instance, the *Listeria* may be attenuated for cell-to-cell spread, entry into non-phagocytic cells, or proliferation. In some embodiments, the recombinant *Listeria* bacterium is deficient with respect to ActA, Internalin B, or both Act A and Internalin B (e.g., an *ΔactAΔinlB* double deletion mutant). In some embodiments, the nucleic acid of the recombinant bacterium has been modified by reaction with a nucleic acid targeting compound (e.g., a psoralen compound).

In another aspect, described herein is a bacterium comprising a recombinant nucleic acid molecule, wherein the recombinant nucleic acid molecule comprises a first polynucleotide encoding a non-secA1 bacterial signal peptide, and a second polynucleotide encoding a polypeptide (e.g., an antigen), wherein the second polynucleotide is in the same translational reading frame as the first polynucleotide, and wherein the recombinant nucleic acid molecule encodes a fusion protein comprising the signal peptide and the polypeptide. In some embodiments, the polypeptide encoded by the second polynucleotide is heterologous to the signal peptide. In some embodiments, the recombinant nucleic acid molecule is part of an expression cassette that further comprises a promoter operably linked to both the first and second polynucleotides. In some embodiments, the bacterium is a bacterium selected from the group consisting of *Listeria, Bacillus, Yersinia pestis, Salmonella, Shigella, Brucella*, mycobacteria or *E*. *coli.* In some embodiments, the polypeptide encoded by the second polynucleotide is foreign to the bacterium (i.e., heterologous to the bacterium).

In another aspect, described herein is a bacterium comprising an expression cassette, wherein the expression cassette comprises (a) a first polynucleotide encoding a non-secA1 bacterial signal peptide; (b) a second polynucleotide encoding a polypeptide (e.g., a polypeptide heterologous to the bacterium) in the same translational reading frame as the first polynucleotide; and (c) a promoter operably linked to the first and second polynucleotides, wherein the expression cassette encodes a fusion protein comprising the signal peptide and the polypeptide. As described herein, e.g., in Section III, above, in some embodiments, the non-secA1 bacterial signal peptide is a secA2 signal peptide. In some other embodiments, the non-secA1 bacterial signal peptide is a Tat signal peptide. In some embodiments, the bacterial signal peptide encoded by the expression cassette is derived from the bacteria of the same genus and/or species as the bacterium comprising the expression cassette. In other embodiments, the bacterial signal peptide encoded by the expression cassette is derived from bacteria of a different genus and/or species as the bacterium comprising the expression cassette.

In another aspect, described herein is a recombinant *Listeria* bacterium comprising a recombinant nucleic acid molecule, wherein the recombinant nucleic acid molecule comprises (a) a first polynucleotide encoding a non-secA1 bacterial signal peptide, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide. In some embodiments, the recombinant nucleic acid molecule is part of an expression cassette that further comprises a promoter operably linked to both the first and second polynucleotides. In some embodiments, the *Listeria* bacterium is attenuated. In some embodiments, the *Listeria* bacterium is a *Listeria monocytogenes* bacterium. For instance, the *Listeria* may be attenuated for cell-to-cell spread, entry into non-phagocytic cells, or proliferation. In some embodiments, the recombinant *Listeria* bacterium is deficient with respect to ActA, Internalin B, or both Act A and Internalin B (e.g., an *ΔactAΔinlB* double deletion mutant). In some embodiments, the nucleic acid of the recombinant bacterium has been modified by reaction with a nucleic acid targeting compound (e.g., a psoralen compound).

In an another aspect, described herein is a recombinant *Listeria* bacterium comprising a recombinant nucleic acid molecule, wherein the recombinant nucleic acid molecule comprises a polynucleotide encoding a polypeptide foreign to the *Listeria* bacterium, wherein the polynucleotide is codon-optimized for expression in *Listeria*

In an additional aspect, described herein is a recombinant *Listeria* bacterium comprising an expression cassette, wherein the expression cassette comprises the following: (a) a polynucleotide encoding a polypeptide foreign to the *Listeria* bacterium, wherein the polynucleotide is codon-optimized for expression in *Listeria;* and (b) a promoter, operably linked to the polynucleotide encoding the foreign polypeptide. Again, in some embodiments, the *Listeria* is *Listeria monocytogenes.* In other embodiments the *Listeria* bacterium belongs to the *Listeria ivanovii, Listeria seeligeri*, or *Listeria innocua* species. In some embodiments, the *Listeria* bacterium is an attenuated strain of *Listeria* bacterium as described above.

In a further aspect, described herein is a recombinant *Listeria* bacterium (e.g., *Listeria monocytogenes)* comprising a recombinant nucleic acid molecule, wherein the recombinant nucleic acid molecule comprises (a) a first polynucleotide encoding a non-*Listeria* signal peptide; and (b) a second polynucleotide encoding a polypeptide that is in the same translational reading frame as the first polynucleotide, wherein the recombinant nucleic acid molecule encodes a fusion protein comprising both the non-Listerial signal peptide and the polypeptide. In some embodiments, the *Listeria* bacterium is attenuated. For instance, the *Listeria* may be attenuated for cell-to-cell spread, entry into non-phagocytic cells, or proliferation. In some embodiments, the recombinant *Listeria* bacterium is deficient with respect to ActA, Internalin B, or both Act A and Internalin B (e.g., an *ΔactAΔinlB* double deletion mutant). In some embodiments, the nucleic acid of the recombinant bacterium has been modified by reaction with a nucleic acid targeting compound (e.g., a psoralen compound).

In still another aspect, described herein is a recombinant *Listeria* bacterium (for instance, from the species *Listeria monocytogenes*) comprising an expression cassette which comprises a first polynucleotide encoding a non*-Listerial* signal peptide, a second polynucleotide encoding a polypeptide (e.g., a non-Listerial polypeptide) that is in the same translational reading frame as the first polynucleotide, and a promoter operably linked to both the first and second polynucleotides. The expression cassette encodes a fusion protein comprising both the non*-Listerial* signal peptide and the polypeptide. In some embodiments, the *Listeria* bacterium is attenuated for cell-to-cell spread, entry into non-phagocytic cells, or proliferation. In some embodiments, the *Listeria* bacterium is deficient with respect to ActA, Internalin B, or both ActA and Internalin B. In some embodiments, the nucleic acid of the recombinant bacterium has been modified by reaction with a nucleic acid targeting compound (e.g., a psoralen compound). In some embodiments, the first polynucleotide, the second polynucleotide, or both the first and second polynucleotides are codon-optimized for expression in *Listeria.* In some embodiments, the first polynucleotide and/or second polynucleotide is codon-optimized for expression in *Listeria monocytogenes.* In some embodiments, the polypeptide encoded by the second polynucleotide is an antigen, which, in some instances, may be a non-bacterial antigen. For instance, the polypeptide is, in some embodiments a tumor-associated antigen or is derived from such a tumor-associated antigen. For instance, in some embodiments, the polypeptide is K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA, or is derived from K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, or CEA. For instance, in some embodiments, the polypeptide is mesothelin, or is a fragment or variant of mesothelin. In some other embodiments, the polypeptide is NY-ESO-1, or a fragment or variant of mesothelin. In some embodiments, the antigen is an infectious disease antigen or is derived from an infectious disease antigen. In preferred embodiments, the signal peptide is bacterial. In some embodiments, the signal peptide is from a bacterium belonging to the genus *Bacillus, Staphylococcus*, or *Lactococcus.* For instance, in some embodiments, the signal peptide is from *Bacillus anthracis, Bacillus subtilis, Staphylococcus aureus*, or *Lactococcus lactis.* In some embodiments, the signal peptide is a secA1 signal peptide, such as a Usp45 signal peptide from *Lactococcus lactis* or a Protective Antigen signal peptide from *Bacillus anthracis.* In some embodiments, the signal peptide is a secA2 signal peptide. In still further embodiments, the signal peptide is a Tat signal peptide, such as a *B*. *subtilis* Tat signal peptide (e.g., PhoD).

This disclosure further provides a recombinant bacterium comprising a recombinant nucleic acid molecule, wherein the recombinant nucleic acid molecule comprises: (a) a first polynucleotide encoding a bacterial autolysin, or a catalytically active fragment or catalytically active variant thereof; and (b) a second polynucleotide encoding a polypeptide, wherein the second polynucleotide is in the same translational reading frame as the first polynucleotide, wherein the recombinant nucleic acid molecule encodes a protein chimera comprising the polypeptide encoded by the second polynucleotide and the autolysin, or catalytically active fragment or catalytically active variant thereof, wherein, in the protein chimera, the polypeptide is fused to the autolysin, or catalytically active fragment or catalytically active variant thereof, or is positioned within the autolysin, or catalytically active fragment or catalytically active variant thereof. In some embodiments, the recombinant bacterium is an intracellular bacterium, such as a *Listeria* bacterium (e.g., *Listeria monocytogenes*). In some embodiments, the polypeptide encoded by the second polynucleotide is foreign to the recombinant bacterium.

In yet another aspect, described herein is a recombinant *Listeria* bacterium comprising a polycistronic expression cassette, wherein the polycistronic expression cassette encodes at least two discrete non-Listerial polypeptides. For instance, in some embodiments, the expression cassette comprises a first polynucleotide encoding the first non-Listerial polypeptide, a second polynculeotide encoding the second non-Listerial polypeptide, and a promoter operably linked to the first and second polynucleotides. In some embodiments, the recombinant *Listeria* bacterium belongs to the species *Listeria monocytogenes.* In some embodiments, the first and/or second non-Listerial polypeptides comprise antigens (or fragments thereof).

In some embodiments, described herein is a recombinant bacterium (e.g., *Listeria)* comprising an expression cassette comprising the following: (a) a first polynucleotide encoding a first signal peptide; (b) a second polynucleotide encoding a first polypeptide, wherein the second polynucleotide is in the same translational reading frame as the first polynucleotide; (c) an intergenic sequence; (d) a third polynucleotide encoding a second signal peptide; (e) a fourth polynucleotide encoding a second polypeptide, wherein the fourth polynucleotide is in the same translational reading frame as the third polynucleotide; and (f) a promoter operably linked to the first polynucleotide, second polynucleotide, third polynucleotide, fourth polynucleotide, and intergenic sequence, such that the expression cassette encodes both a first fusion protein comprising the first signal peptide and the first polypeptide and a second fusion protein comprising the second signal peptide and second polypeptide. In some embodiments, the one or more of the polynucleotides encoding a signal peptide is codon-optimized for expression in the bacterium. In some embodiments, the third and/or fourth polynucleotides are codon-optimized for expression in the bacterium. In some embodiments, the first and/or second polypeptides are heterologous to the recombinant bacterium (e.g., heterologous antigens). In some embodiments, the first and/or second signal peptide is a non-secA1 bacterial signal peptide. The first and/or second signal peptide may be native or foreign to the recombinant bacterium. In some embodiments, the recombinant bacterium is a *Listeria* bacterium and the first and/or second signal peptide is non-Listerial. In some embodiments, the intergenic sequence is the *Listeria monocytogenes* actA-plcB intergenic sequence. In some embodiments, the bacterium is *Listeria monocytogenes.*

In other aspects, described herein is a bacterium comprising a recombinant nucleic acid molecule, comprising (a) a first polynucleotide encoding a signal peptide, (b) a second polynucleotide encoding a secreted protein, or a fragment thereof, wherein the second polynucleotide is in the same translational reading frame as the first polynucleotide, and (c) a third polynucleotide encoding a polypeptide heterologous to the secreted protein, or fragment thereof, wherein the third polynucleotide is in the same translational reading frame as the first and second polynucleotides, and wherein the recombinant nucleic acid molecule encodes a protein chimera comprising the signal peptide, the polypeptide encoded by the second polynucleotide, and the secreted protein, or fragment thereof, and wherein the polypeptide is fused to the secreted protein, or fragment thereof, or is positioned within the secreted protein, or fragment thereof, in the protein chimera. In some embodiments, the bacterium is a *Listeria* bacterium. In some embodiments, where the bacterium is a *Listeria* bacterium, the polypeptide encoded by the third polynucleotide is foreign to the *Listeria.* In some embodiments, the bacterium is *Listeria monocytogenes.*

In some embodiments (for instance, in some embodiments of each of the aforementioned aspects), the expression cassette contained within the bacterium is integrated into the genome of the bacterium. In other embodiments, the expression cassette contained within the bacterium is on a plasmid within the bacterium.

Generally, the promoter that is used in the expression cassette will be an expression cassette that is suitable for effecting heterologous expression with the particular bacterial host chosen. One of ordinary skill in the art can readily discern which promoters are suitable for use in which bacteria. In some embodiments, the promoter is a bacterial promoter. In additional embodiments, the promoter on the expression cassette in the bacterium is a promoter from bacteria belonging to the same genus as the bacterium which contains the expression cassette. In other embodiments, the promoter on the expression cassette in the bacterium is a promoter from bacteria belonging to the same species as the bacterium which contains the expression cassette. For instance, if the bacterium which contains the expression cassette belongs to the species *Listeria monocytogenes,* then the promoter that is used on the expression cassette is optionally derived from a *Listeria* gene such as *hly.* In other embodiments, the promoter is a constitutive promoter (e.g., a p60 promoter) or is prfA-dependent promoter (e.g. an actA promoter). Again, as described above, the promoter of the expression cassette is, in some embodiments, a constitutive promoter. In other embodiments, the promoter of the expression cassette is an inducible promoter, as also described above.

In some embodiments (for instance, in some embodiments of each of the aforementioned aspects), the polypeptides or fusion proteins comprising the polypeptides that are encoded by the expression cassettes in the bacteria are antigens or other proteins of therapeutic value, as described, for instance, above in Section IV. In some embodiments, the polypeptide or a protein comprising the polypeptide is secreted from the bacterium. In some embodiments the polypeptide that is expressed and/or secreted from the bacterium is heterologous to the bacterium.

In some embodiments, therefore, described herein are recombinant *Listeria* comprising an expression cassette, wherein the expression cassette comprises (a) a first polynucleotide encoding a bacterial (either *Listerial* or non-*Listerial*) signal peptide, wherein the first polynucleotide is codon-optimized for expression in *Listeria;* (b) a second polynucleotide encoding a non*-Listerial* antigen, wherein the second polynucleotide is in the same translational reading frame as the first polynucleotide; and (c) a promoter operably linked to the first and second polynucleotides, wherein the expression cassette encodes a fusion protein comprising the signal peptide and the antigen. In further embodiments, the *Listeria* is a strain of *Listeria monocytogenes,* such as an *actA⁻ïnlB⁻* strain. In some embodiments, the expression cassette has been integrated into the genome of the recombinant *Listeria.* In some embodiments, the second polynucleotide is codon-optimized for expression in *Listeria.*

This disclosure also provides *Listeria* comprising an expression cassette, wherein the expression cassette comprises (a) a first polynucleotide encoding a secA2 or Tat bacterial signal peptide; (b) a second polynucleotide encoding an antigen in the same translational reading frame as the first polynucleotide; and (c) a promoter operably linked to the first and second polynucleotides, wherein the expression cassette encodes a fusion protein comprising the signal peptide and the antigen. In some embodiments, the bacterial signal peptide is *Listerial.* In other embodiments, the bacterial signal peptide is non*-Listerial.* In further embodiments, the *Listeria* is a strain of *Listeria monocytogenes,* such as an *actA⁻inlB⁻*strain. In some embodiments, the expression cassette has been integrated into the genome of the recombinant *Listeria.* In some embodiments, either the polynucleotide encoding the signal peptide (even if the signal peptide is a *Listeria* signal peptide) and/or the polynucleotide encoding the antigen is codon-optimized for expression in *Listeria.*

In further embodiments, described herein are recombinant *Listeria* comprising an expression cassette, where the expression cassette comprises the following:
(a) a polynucleotide encoding a non*-Listerial* antigen, wherein the polynucleotide is codon-optimized for expression in *Listeria;* and (b) a promoter, operably linked to the polynucleotide encoding the foreign polypeptide. In some embodiments, the expression cassette further comprises a polynucleotide encoding a bacterial signal peptide, which is also codon-optimized for expression in *Listeria.* In one embodiment, the bacterial signal peptide is *Listerial.* In another embodiment, the bacterial signal peptide is non*-Listerial.* In some embodiments the bacterial signal peptide is a secA1 signal peptide, a secA2 signal peptide, or a Tat signal peptide. In further embodiments, the *Listeria* is a strain of *Listeria monocytogenes*, such as an *actA⁻inlB⁻* strain. In some embodiments, the expression cassette has been integrated into the genome of the recombinant *Listeria.*

In still another embodiment, described herein is a recombinant *Listeria* bacterium, comprising (a) a first polynucleotide encoding a bacterial (either *Listeria* or non-*Listerial*) signal peptide, wherein the first polynucleotide is codon-optimized for expression in *Listeria;* (b) a second polynucleotide encoding an non*-Listerial* antigen, wherein the second polynucleotide is also codon-optimized for expression in *Listeria* and is in the same translational reading frame as the first polynucleotide; and (c) a promoter operably linked to the first and second polynucleotides, wherein the expression cassette encodes a fusion protein comprising the signal peptide and the antigen. In some embodiments, the *Listeria* bacterium belongs to the species *Listeria monocytogenes.* In some embodiments, the *Listeria* bacterium is an *actA⁻inlB⁻* mutant strain of *Listeria monocytogenes.*

The present disclosure further provides bacteria such as *Listeria* comprising more than one expression cassette described herein. In particular compositions, the molecular mass of a given protein can inhibit its expression from recombinant bacteria, such as recombinant *Listeria.* One approach to address this problem is to molecularly "divide" the gene encoding a protein of interest and fuse each division functionally to a sequence that will program its secretion from the bacterium (e.g., secA1, secA2, or Tat elements). One approach is to individually derive recombinant *Listeria* expressing each division of the heterologous gene. Alternatively, the individually components of the molecularly divided gene (also including appropriate elements for secretion) can be introduced into intergenic regions throughout the bacterial chromosome, using methods well established in the art, for example by allelic exchange. Another example is to express the molecularly divided gene as a single polycistronic message. According to this composition, interspersed between the protein-encoding sequence of the molecularly divided gene would be the Shine-Dalgarno ribosome binding sequence, in order to re-initiate protein synthesis on the polycistronic message.

In additional aspects, described herein are methods of improving expression and/or secretion of heterologous polypeptides in recombinant bacteria such as *Listeria.* Any of the polynucleotides, expression cassettes and/or expression vectors described herein may be used in these methods. For instance, described herein is a method of improving expression and/or secretion of a heterologous polypeptide fused to a signal peptide in *Listeria,* comprising codon-optimizing either the polypeptide-encoding sequence on the expression cassette, the signal peptide-encoding sequence of the expression cassette, or both. This disclosure also provides a method of improving expression and/or secretion of a heterologous polypeptide fused to a signal peptide in *Listeria,* comprising using a signal peptide from a non-Listerial source and/or from a secretory pathway other than secA1.

This disclosure also provides a method of producing a recombinant bacterium (e.g. a recombinant *Listeria* bacterium) comprising introducing a recombinant nucleic acid molecule, expression cassette, and/or expression vector described herein into a bacterium to produce the recombinant bacterium. For instance, in some embodiments, a recombinant nucleic acid molecule comprising (a) a first polynucleotide encoding a signal peptide native to a bacterium, wherein the first polynucleotide is codon-optimized for expression in the bacterium, and (b) a second polynucleotide encoding a polypeptide, wherein the second polynucleotide is in the same translational reading frame as the first polynucleotide, wherein the recombinant nucleic acid molecule encodes a fusion protein comprising the signal peptide and the polypeptide, is introduced into a bacterium to produce the recombinant bacterium. In some embodiments, a recombinant nucleic acid molecule, comprising (a) a first polynucleotide encoding a non-secA1 bacterial signal peptide, and (b) a second polynucleotide encoding a polypeptide, wherein the second polynucleotide is in the same translational reading frame as the first polynucleotide, and wherein the recombinant nucleic acid molecule encodes a fusion protein comprising the signal peptide and the polypeptide, is introduced into a bacterium to produce the recombinant bacterium. In some embodiments, the recombinant nucleic acid molecule that is introduced into a bacterium to produce the recombinant bacterium is a recombinant nucleic acid molecule, wherein the recombinant nucleic acid molecule comprises (a) a first polynucleotide encoding a non-Listerial signal peptide, and (b) a second polynucleotide encoding a polypeptide that is in the same translational reading frame as the first polynucleotide, wherein the recombinant nucleic acid molecule encodes a fusion protein comprising both the non-Listerial signal peptide and the polypeptide. The recombinant nucleic acid molecule used to produce the recombinant bacterium is, in some embodiments, a recombinant nucleic acid molecule, comprising (a) a first polynucleotide encoding a bacterial autolysin, or catalytically active fragment or catalytically active variant thereof, and (b) a second polynucleotide encoding a polypeptide, wherein the second polynucleotide is in the same translational reading frame as the first polynucleotide, wherein the recombinant nucleic acid molecule encodes a protein chimera in which the non-Listerial polypeptide is fused to the autolysin, or catalytically active fragment or catalytically active variant thereof, or is inserted within the autolysin, or catalytically active fragment or catalytically active variant thereof. In some other embodiments, a method of producing a recombinant *Listeria* bacterium is provided, which comprises introducing a polycistronic expression cassette, wherein the expression cassette encodes at least two discrete non-Listerial polypeptides, into a *Listeria* bacterium to produce the recombinant *Listeria* bacterium.

### IX. Pharmaceutical, immunogenic, and/or vaccine compositions

A variety of different compositions such as pharmaceutical compositions, immunogenic compositions, and vaccines are also provided by this disclosure These compositions comprise any of the recombinant bacteria described herein (see, e.g., the Summary of the Invention, Sections I and VIII of the Detailed Description, above, and elsewhere in the specification, including the Examples, below). In some embodiments, the compositions are isolated.

For instance, described herein is a pharmaceutical composition comprising the following: (i) a pharmaceutically acceptable carrier; and (ii) a recombinant bacterium described herein.

For example, described herein is a pharmaceutical composition comprising the following (i) a pharmaceutically acceptable carrier; and (ii) a recombinant bacterium comprising an expression cassette comprising a first polynucleotide encoding a signal peptide, wherein the first polynucleotide is codon-optimized for expression in a bacterium, a second polynucleotide encoding a polypeptide, wherein the second polynucleotide is in the same translational reading frame as the first polynucleotide, and a promoter operably linked to the first and second polynucleotides, so that the expression cassette encodes a fusion protein comprising the signal peptide and the polypeptide.

This disclosure also provides a pharmaceutical composition comprising: (i) a pharmaceutically acceptable carrier; and (ii) a recombinant bacterium comprising an expression cassette, where the expression cassette comprises a first polynucleotide encoding a non-secA1 bacterial signal peptide, a second polynucleotide encoding a polypeptide in the same translational reading frame as the first polynucleotide, and a promoter operably linked to the first and second polynucleotides, so that the expression cassette encodes a fusion protein comprising the signal peptide and the polypeptide.

This disclosure further provides a pharmaceutical composition comprising: (i) a pharmaceutically acceptable carrier; and (ii) a recombinant *Listeria* bacterium comprising an expression cassette, wherein the expression cassette comprises the following: (a) a polynucleotide encoding a polypeptide foreign to *Listeria,* wherein the polynucleotide is codon-optimized for expression in *Listeria*; and (b) a. promoter, operably linked to the polynucleotide encoding the foreign polypeptide.

This disclosure also provides a pharmaceutical composition comprising: (i) a pharmaceutically acceptable carrier; and (ii) a recombinant *Listeria* bacterium comprising an expression cassette which comprises:(a) a first polynucleotide encoding a non-Listerial signal peptide; (b) a second polynucleotide encoding a polypeptide that is in the same translational reading frame as the first polynucleotide; and (c) a promoter operably linked to both the first and second polynucleotides, wherein the expression cassette encodes a fusion protein comprising both the non-Listerial signal peptide and the polypeptide.

As used herein, "carrier" includes any and all solvents, dispersion media, vehicles, coatings, diluents, antifungal agents, isotonic and absorption delaying agents, buffers, carrier solutions, suspensions, colloids, and the like. Pharmaceutically acceptable carriers are well known to those of ordinary skill in the art, and include any material which, when combined with an active ingredient, allows the ingredient to retain biological activity and is non-reactive with the subject's immune system. For instance, pharmaceutically acceptable carriers include, but are not limited to, water, buffered saline solutions (e.g., 0.9% saline), emulsions such as oil/water emulsions, and various types of wetting agents. Possible carriers also include, but are not limited to, oils (e.g., mineral oil), dextrose solutions, glycerol solutions, chalk, starch, salts, glycerol, and gelatin.

While any suitable carrier known to those of ordinary skill in the art may be employed in the pharmaceutical compositions, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. In some embodiments, for parenteral administration, such as subcutaneous injection, the carrier comprises water, saline, alcohol, a fat, a wax or a buffer. In some embodiments, any of the above carriers or a solid carrier, such as mannitol, lactose, starch, magnesium stearate, sodium saccharine, talcum, cellulose, glucose, sucrose, and magnesium carbonate, are employed for oral administration.

Compositions comprising such carriers are formulated by well known conventional methods (see, for example, Remington's Pharmaceutical Sciences, 18th edition, A. Gennaro, ed., Mack Publishing Co., Easton, PA, I. 990; and Remington, The Science and Practice of Pharmacy 20th Ed. Mack Publishing, 2000).

In addition to pharmaceutical compositions, immunogenic compositions are provided. For instance, described herein is an immunogenic composition comprising a recombinant bacterium described herein (see, e.g., the recombinant bacterium described above in the Summary of the Invention, Sections I and VIII of the Detailed Description above, and elsewhere in the specification, including the Examples, below). In some embodiments, the immunogenic composition comprises a recombinant bacterium, wherein the polypeptide sequence that is part of the polypeptide expressed by the recombinant bacterium as a discrete protein, as part of a fusion protein, or embedded in a protein chimera (depending on the recombinant nucleic acid molecule or expression cassette used) is an antigen or comprises an antigen. In other words, in some embodiments, the immunogenic composition comprises a recombinant bacterium which comprises a recombinant nucleic acid molecule or expression cassette encoding a polypeptide that comprises an antigen. Suitable antigens include, but are not limited to, any of those described herein (e.g., above in Section IV). In some embodiments, the recombinant bacterium in the immunogenic composition expresses the polypeptide comprising the antigen at a level sufficient to induce an immune response to the antigen upon administration of the composition to a host (e.g., a mammal such as a human). In some embodiments, the immune response stimulated by the immunogenic composition is a cell-mediated immune response. In some embodiments, the immune response stimulated by the immunogenic composition is a humoral immune response. In some embodiments, the immune response stimulated by the immunogenic composition comprises both a humoral and cell-mediated immune response.

For instance, in one aspect, described herein is an immunogenic composition comprising a recombinant bacterium, where the bacterium comprises an expression cassette comprising the following: (a) a first polynucleotide encoding a signal peptide, wherein the first polynucleotide is codon-optimized for expression in a bacterium; (b) a second polynucleotide encoding an antigen, wherein the second polynucleotide is in the sametranslational reading frame as the first polynucleotide; and (c) a promoter operably linked to the first and second polynucleotides, so that the expression cassette encodes a fusion protein comprising the signal peptide and the antigen.

In another aspect, described herein is an immunogenic composition comprising a recombinant bacterium, where the bacterium comprises an expression cassette that comprises the following: (a) a first polynucleotide encoding a non-secA1 bacterial signal peptide; (b) a second polynucleotide encoding an antigen in the same translational reading frame as the first polynucleotide; and (c) a promoter operably linked to the first and second polynucleotides, so that the expression cassette encodes a fusion protein comprising the signal peptide and the antigen.

In still another aspect, described herein is immunogenic composition comprising a recombinant *Listeria* bacterium, wherein the recombinant *Listeria* bacterium comprises an expression cassette, wherein the expression cassette comprises the following:
(a) a polynucleotide that encodes a non-*Listerial* antigen and that is codon-optimized for expression in *Listeria*; and (b) a promoter, operably linked to the polynucleotide encoding the antigen.

This disclosure also provides an immunogenic composition comprising a recombinant *Listeria* bacterium comprising an expression cassette which comprises:(a) a first polynucleotide encoding a non-Listerial signal peptide; (b) a second polynucleotide encoding an antigen that is in the same translational reading frame as the first polynucleotide; and (c) a promoter operably linked to both the first and second polynucleotides, wherein the expression cassette encodes a fusion protein comprising both the non-Listerial signal peptide and the antigen.

In another aspect, described herein is an immunogenic composition (or vaccine) comprising a recombinant bacterium comprising a recombinant nucleic acid molecule, wherein the recombinant nucleic acid molecule comprises (a) a first polynucleotide encoding a signal peptide native to a bacterium, wherein the first polynucleotide is codon-optimized for expression in the bacterium, and (b) a second polynucleotide encoding a polypeptide comprising an antigen, wherein the second polynucleotide is in the same translational reading frame as the first polynucleotide, wherein the recombinant nucleic acid molecule encodes a fusion protein comprising the signal peptide and the polypeptide.

In another aspect, described herein is an immunogenic composition (or vaccine) comprising a recombinant *Listeria* bacterium, wherein the recombinant bacterium comprises a recombinant nucleic acid molecule which comprises (a) a first polynucleotide encoding a signal peptide, wherein the first polynucleotide is codon-optimized for expression in *Listeria,* and (b) a second polynucleotide encoding a polypeptide comprising an antigen, wherein the second polynucleotide is in the same translational reading frame as the first polynucleotide, wherein the recombinant nucleic acid molecule encodes a fusion protein comprising the signal peptide and the polypeptide.

In another aspect, described herein is an immunogenic composition (or vaccine) comprising a recombinant bacterium comprising a recombinant nucleic acid molecule, wherein the recombinant nucleic acid molecule comprises a first polynucleotide encoding a non-secA1 bacterial signal peptide, and a second polynucleotide encoding a polypeptide comprising an antigen, wherein the second polynucleotide is in the same translational reading frame as the first polynucleotide, and wherein the recombinant nucleic acid molecule encodes a fusion protein comprising the signal peptide and the polypeptide.

In still another aspect, described herein is an immunogenic composition (or vaccine) comprising a recombinant *Listeria* bacterium comprising a recombinant nucleic acid molecule, wherein the recombinant nucleic acid molecule comprises (a) a first polynucleotide encoding a non-secA1 bacterial signal peptide, and (b) a second polynucleotide encoding a polypeptide either heterologous to the signal peptide or foreign to the bacterium, wherein the second polynucleotide is in the same translational reading frame as the first polynucleotide, and wherein the recombinant nucleic acid molecule encodes a fusion protein comprising the signal peptide and the polypeptide. In some embodiments, the polypeptide encoded by the first polynucleotide comprises an antigen.

In another aspect, described herein is an immunogenic composition (or vaccine) comprising a recombinant *Listeria* bacterium, wherein the recombinant *Listeria* bacterium comprises a recombinant nucleic acid molecule, wherein the recombinant nucleic acid molecule comprises a polynucleotide encoding a polypeptide foreign to *Listeria,* wherein the polynucleotide encoding the foreign polypeptide is codon-optimized for expression in *Listeria.* In some embodiments, the foreign polypeptide comprises an antigen.

In another aspect, described herein is an immunogenic composition (or vaccine) comprising a recombinant *Listeria* bacterium, wherein the recombinant bacterium comprises a recombinant nucleic acid molecule comprising (a) a first polynucleotide encoding a non-Listerial signal peptide, and (b) a second polynucleotide encoding a polypeptide comprising an antigen, wherein the second polynucleotide is in the same translational reading frame as the first polynucleotide, and wherein the recombinant nucleic acid molecule encodes a fusion protein comprising both the non-Listerial signal peptide and the polypeptide.

This disclosure also provides an immunogenic composition (or vaccine) comprising a recombinant bacterium, wherein the recombinant bacterium comprises a nucleic acid molecule comprising (a) a first polynucleotide encoding a bacterial autolysin, or a catalytically active fragment or catalytically active variant thereof, and (b) a second polynucleotide encoding a polypeptide, wherein the second polynucleotide is in the same translational reading frame as the first polynucleotide, wherein the recombinant nucleic acid molecule encodes a protein chimera comprising the polypeptide encoded by the second polynucleotide and the autolysin, or catalytically active fragment or catalytically active variant thereof, wherein, in the protein chimera, the polypeptide is fused to or is positioned within the autolysin, or catalytically active fragment or catalytically active variant thereof In some embodiments, the polypeptide encoded by the second polynucleotide comprises an antigen.

In another aspect, described herein is an immunogenic composition (or vaccine) comprising a recombinant *Listeria* bacterium, wherein the recombinant *Listeria* bacterium comprises a recombinant nucleic acid molecule, wherein the recombinant nucleic acid molecule encodes at least two discrete non-Listerial polypeptides, at least one of which comprises an antigen.

In other aspects, described herein is an immunogenic composition (or vaccine) comprising a recombinant bacterium, which comprises a recombinant nucleic acid molecule comprising (a) a first polynucleotide encoding a signal peptide, (b) a second polynucleotide encoding a secreted protein, or a fragment thereof, wherein the second polynucleotide is in the same translational reading frame as the first polynucleotide, and (c) a third polynucleotide encoding a polypeptide heterologous to the secreted protein, or fragment thereof, wherein the third polynucleotide is in the same translational reading frame as the first and second polynucleotides, wherein the recombinant nucleic acid molecule encodes a protein chimera comprising the signal peptide, the polypeptide encoded by the third polynucleotide, and the secreted protein, or fragment thereof, and wherein the polypeptide encoded by the third polynucleotide is fused to the secreted protein, or fragment thereof, or is positioned within the secreted protein, or fragment thereof, in the protein chimera. In some embodiments, the heterologous polypeptide encoded by the third polynucleotide comprises an antigen.

It can be determined if a particular form of recombinant bacteria (and/or a particular expression cassette) is useful in an immunogenic composition (or as a vaccine) by testing the ability of the recombinant bacteria to stimulate an immune response *in vitr o* or in a model system.

These immune cell responses can be measured by *both in vitro* and *in vivo* methods to determine if the immune response of a particular recombinant bacterium (and/or a particular expression cassette) is effective. One possibility is to measure the presentation of the protein or antigen of interest by an antigen-presenting cell that has been mixed with a population of the recombinant bacteria. The recombinant bacteria may be mixed with a suitable antigen presenting cell or cell line, for example a dendritic cell, and the antigen presentation by the dendritic cell to a T cell that recognizes the protein or antigen can be measured. If the recombinant bacteria are expressing the protein or antigen at a sufficient level, it will be processed into peptide fragments by the dendritic cells and presented in the context of MHC class I or class II to T cells. For the purpose of detecting the presented protein or antigen, a T cell clone or T cell line responsive to the particular protein or antigen may be used. The T cell may also be a T cell hybridoma, where the T cell is immortalized by fusion with a cancer cell line. Such T cell hybridomas, T cell clones, or T cell lines can comprise either CD8+ or CD4+ T cells. The dendritic cell can present to either CD8+ or CD4+ T cells, depending on the pathway by which the antigens are processed. CD8+ T cells recognize antigens in the context of MHC class I while CD4+ recognize antigens in the context of MHC class II. The T cell will be stimulated by the presented antigen through specific recognition by its T cell receptor, resulting in the production of certain proteins, such as IL-2, tumor necrosis factor-α (TNF-α), or interferon-γ (IFN-γ), that can be quantitatively measured (for example, using an ELISA assay, ELISPOT assay, or Intracellular Cytokine Staining (ICS)). These are techniques that are well known in the art and that are also exemplified below in the Examples (see, e.g., Example 21, below).

Alternatively, a hybridoma can be designed to include a reporter gene, such as β-galactosidase, that is activated upon stimulation of the T cell hybridoma by the presented antigens. The increase in the production of β-galactosidase can be readily measured by its activity on a substrate, such as chlorophenol red-B-galactoside, which results in a color change. The color change can be directly measured as an indicator of specific antigen presentation.

Additional *in vitro* and *in vivo* methods for assessing the antigen expression of recombinant bacteria vaccines of the present invention are known to those of ordinary skill in the art. It is also possible to directly measure the expression of a particular heterologous antigen by recombinant bacteria. For example, a radioactively labeled amino acid can be added to a cell population and the amount of radioactivity incorporated into a particular protein can be determined. The proteins synthesized by the cell population can be isolated, for example by gel electrophoresis or capillary electrophoresis, and the amount of radioactivity can be quantitatively measured to assess the expression level of the particular protein. Alternatively, the proteins can be expressed without radioactivity and visualized by various methods, such as an ELISA assay or by gel electrophoresis and Western blot with detection using an enzyme linked antibody or fluorescently labeled antibody.

Example 21, below, provides some specific exemplary examples of how some of the techniques known to those of ordinary skill in the art can be used to assess immunogenicity. For instance, Elispot assay, Intracellular Cytokine Staining Assay (ICS), measurement of cytokine expression of stimulated spleen cells, and assessment of cytotoxic T cell activity *in vitro* and *in vivo* are all techniques for assessing immunogenicity known to those in the art. Exemplary descriptions of these techniques with model antigens are provided in Example 21. Exemplary assays are also described in Examples 31A and 31E, below.

In addition, therapeutic efficacy of the vaccine composition can be assessed more directly by administration of the immunogenic composition or vaccine to an animal model such as a mouse model, followed by an assessment of survival or tumor growth. For instance, survival can be measured following administration and challenge (e.g., with a tumor or pathogen). See, e.g., the assays described in Examples 20 and 31B-D, below.)

Mouse models useful for testing the immunogenicity of an immunogenic composition or vaccine expressing a particular antigen can be produced by first modifying a tumor cell so that it expresses the antigen of interest or a model antigen and then implanting the tumor cells expressing the antigen of interest into mice. The mice can be vaccinated with the candidate immunogenic composition or vaccine comprising a recombinant bacterium expressing a polypeptide comprising the antigen of interest or a model antigen prior to implantation of the tumor cells (to test prophylactic efficacy of the candidate composition) or following implantation of the tumor cells in the mice (to test therapeutic efficacy of the candidate composition).

As an example, CT26 mouse murine colon carcinoma cells can be transfected with an appropriate vector comprising an expression cassette encoding the desired antigen or model antigen using techniques standard in the art. Standard techniques such as flow cytometry and Western blots can then be used to identify clones expressing the antigen or model antigen at sufficient levels for use in the immunogenicity and/or efficacy assays.

Alternatively, candidate compositions can be tested which comprise a recombinant bacterium expressing an antigen that corresponds to or is derived from an antigen endogenous to a tumor cell line (e.g., the retroviral gp70 tumor antigen AH1 endogenous to CT26 mouse murine colon carcinoma cells, or the heteroclitic epitope AH1-A5). In such assays, the tumor cells can be implanted in the animal model without further modification to express an additional antigen. Candidate vaccines comprising the antigen can then be tested.

As indicated, vaccine compositions comprising the bacteria described herein are also provided. For instance, described herein is a vaccine comprising a recombinant bacterium described herein (see, e.g., the recombinant bacterium described above in the Summary of the Invention, Sections I and VIII of the Detailed Description above, and elsewhere in the specification, including the Examples, below) where the polypeptide sequence that is part of the polypeptide expressed by the recombinant bacterium as a discrete protein, as part of a fusion protein, or embedded in a protein chimera (depending on the recombinant nucleic acid molecule or expression cassette used) is an antigen. Suitable antigens include any of those described herein (e.g., above in Section IV).

In one aspect, described herein is a vaccine that comprises a bacterium, wherein the bacterium comprises an expression cassette comprising the following: (a) a first polynucleotide encoding a signal peptide, wherein the first polynucleotide is codon-optimized for expression in a bacterium; (b) a second polynucleotide encoding an antigen, wherein the second polynucleotide is in the same translational reading frame as the first polynucleotide; and (c) a promoter operably linked to the first and second polynucleotides, so that the expression cassette encodes a fusion protein comprising the signal peptide and the antigen.

In another aspect, described herein is a vaccine that comprises a bacterium, where the bacterium comprises an expression cassette that comprises the following: (a) a first polynucleotide encoding a non-secA1 bacterial signal peptide; (b) a second polynucleotide encoding an antigen in the same translational reading frame as the first polynucleotide; and (c) a promoter operably linked to the first and second polynucleotides, so that the expression cassette encodes a fusion protein comprising the signal peptide and the antigen.

In still another aspect, described herein is a vaccine that comprises a recombinant *Listeria* bacterium comprising a nucleic acid molecule, wherein the nucleic acid molecule comprises the following: (a) a polynucleotide that encodes a non-*Listerial* antigen and that is codon-optimized for expression in *Listeria*; and (b) a promoter, operably linked to the polynucleotide encoding the antigen.

In another aspect, described herein is a vaccine comprising a recombinant *Listeria* bacterium comprising an expression cassette which comprises: (a) a first polynucleotide encoding a non-Listerial signal peptide; (b) a second polynucleotide encoding an antigen that is in the same translational reading frame as the first polynucleotide; and (c)
a promoter operably linked to both the first and second polynucleotides, wherein the expression cassette encodes a fusion protein comprising both the non-Listerial signal peptide and the antigen.

In some embodiments, the vaccine compositions comprise antigen-presenting cells (APC) which have been infected with any of the recombinant bacteria described herein. In some embodiments the vaccine (or immunogenic or pharmaceutical composition) does not comprise antigen-presenting cells (i.e., the vaccine or composition is a bacteria-based vaccine or composition, not an APC-based vaccine or composition).

Methods of administration suitable for administration of vaccine compositions (and pharmaceutical and immunogenic compositions) are known in the art, and include oral, intraveneous, intradermal, intraperitoneal, intramuscular, intralymphatic, intranasal and subcutaneous routes of administration.

Vaccine formulations are known in the art and in some embodiments may include numerous additives, such as preservatives (e.g., thimerosal, 2-phenyoyx ethanol), stabilizers, adjuvants (e.g. aluminum hydroxide, aluminum phosphate, cytokines), antibiotics (e.g., neomycin, streptomycin), and other substances. In some embodiments, stabilizers, such as lactose or monosodium glutamate (MSG), are added to stabilize the vaccine formulation against a variety of conditions, such as temperature variations or a freeze-drying process. In some embodiments, vaccine formulations may also include a suspending fluid or diluent such as sterile water, saline, or isotonic buffered saline (e.g., phosphate buffered to physiological pH). Vaccine may also contain small amount of residual materials from the manufacturing process.

For instance, in some embodiments, the vaccine compositions are lyophilized (i.e., freeze-dried). The lyophilized preparation can be combined with a sterile solution (e.g., citrate-bicarbonate buffer, buffered water, 0.4% saline, or the like) prior to administration.

In some embodiments, the vaccine compositions may further comprise additional components known in the art to improve the immune response to a vaccine, such as adjuvants or co-stimulatory molecules. In addition to those listed above, possible adjuvants include chemokines and bacterial nucleic acid sequences, like CpG. In some embodiments, the vaccines comprise antibodies that improve the immune response to a vaccine, such as CTLA4. In some embodiments, co-stimulatory molecules comprise one or more factors selected from the group consisting of GM-CSF, IL-2, IL-12, IL-14, IL-15, IL-18, B7.1, B7.2, and B7-DC are optionally included in the vaccine compositions of the present invention. Other co-stimulatory molecules are known to those of ordinary skill in the art.

In additional aspects, described herein are methods of improving a vaccine or immunogenic composition comprising *Listeria* that express an antigen. Any of the polynucleotides, expression cassettes and/or expression vectors described herein may be used in these methods. For instance, described herein is a method of improving a vaccine or immunogenic composition comprising a *Listeria* bacterium, wherein the *Listeria* bacterium expresses a heterologous antigen fused to a signal peptide, comprising codon-optimizing either the polypeptide-encoding sequence on the expression cassette, the signal peptide-encoding sequence of the expression cassette, or both. Described herein is a method of improving a vaccine or immunogenic composition comprising *Listeria* bacterium, wherein the *Listeria* bacterium expresses a heterologous antigen fused to a signal peptide, comprising using a signal peptide from a non-Listerial source and/or from a secretory pathway other than secA1.

Methods of producing the vaccines of the present invention are also provided. For instance, in one embodiment, a method of producing a vaccine comprising a recombinant bacterium (e.g. a recombinant *Listeria* bacterium) comprises introducing a recombinant nucleic acid molecule into the bacterium, expression cassette, or expression vector described herein into a bacterium, wherein the recombinant nucleic acid molecule, expression cassette, or expression vector encodes an antigen. For instance, in some embodiments, a recombinant nucleic acid molecule comprising (a) a first polynucleotide encoding a signal peptide native to a bacterium, wherein the first polynucleotide is codon-optimized for expression in the bacterium, and (b) a second polynucleotide encoding an antigen, wherein the second polynucleotide is in the same translational reading frame as the first polynucleotide, wherein the recombinant nucleic acid molecule encodes a fusion protein comprising the signal peptide and the antigen, is introduced into a bacterium to produce the vaccine. In some embodiments, a recombinant nucleic acid molecule, comprising (a) a first polynucleotide encoding a non-secA1 bacterial signal peptide, and (b) a second polynucleotide encoding an antigen, wherein the second polynucleotide is in the same translational reading frame as the first polynucleotide, and wherein the recombinant nucleic acid molecule encodes a fusion protein comprising the signal peptide and the antigen, is introduced into the bacterium to produce the vaccine. In some embodiments, the recombinant nucleic acid molecule that is introduced into the bacterium to produce the vaccine is a recombinant nucleic acid molecule, wherein the recombinant nucleic acid molecule comprises (a) a first polynucleotide encoding a non-Listerial signal peptide, and (b) a second polynucleotide encoding an antigen that is in the same translational reading frame as the first polynucleotide, wherein the recombinant nucleic acid molecule encodes a fusion protein comprising both the non-Listerial signal peptide and the antigen. The recombinant nucleic acid molecule used to produce the vaccine is, in some embodiments, a recombinant nucleic acid molecule, comprising (a) a first polynucleotide encoding a bacterial autolysin, or a catalytically active fragment or catalytically active variant thereof, and (b) a second polynucleotide encoding a polypeptide, wherein the second polynucleotide is in the same translational reading frame as the first polynucleotide, wherein the recombinant nucleic acid molecule encodes a protein chimera in which the non-Listerial polypeptide is fused to the autolysin, or catalytically active fragment or catalytically active variant thereof, or is inserted within the autolysin, or catalytically active fragment or catalytically active variant thereof. In some other embodiments, a method of producing a vaccine comprising a recombinant *Listeria* bacterium is provided, which comprises introducing a polycistronic expression cassette, wherein the polycistronic expression cassette encodes at least two discrete non-Listerial polypeptides, where at least one of the polypeptides is an antigen, into a *Listeria* bacterium to produce vaccine.

Kits comprising any of the recombinant nucleic acid molecules, expression cassettes, vectors, bacteria and/or compositions of this disclosure are also provided.

### X. Methods of use

A variety of methods of using the recombinant bacteria or pharmaceutical, immunogenic, or vaccine compositions described herein for inducing immune responses, and/or preventing or treating conditions in a host are provided. In some embodiments, the condition that is treated or prevented is a disease. In some embodiments, the disease is cancer. In some embodiments, the disease is an infectious disease. In addition, the recombinant bacteria are also useful in the production and isolation of heterologous proteins, such as mammalian proteins.

As used herein, "treatment" or "treating" (with respect to a condition or a disease) is an approach for obtaining beneficial or desired results including and preferably clinical results. For purposes of this invention, beneficial or desired results with respect to a disease include, but are not limited to, one or more of the following: improving a condition associated with a disease, curing a disease, lessening severity of a disease, delaying progression of a disease, alleviating one or more symptoms associated with a disease, increasing the quality of life of one suffering from a disease, and/or prolonging survival. Likewise, for purposes of this invention, beneficial or desired results with respect to a condition include, but are not limited to, one or more of the following: improving a condition, curing a condition, lessening severity of a condition, delaying progression of a condition, alleviating one or more symptoms associated with a condition, increasing the quality of life of one suffering from a condition, and/or prolonging survival. For instance, in those embodiments where the compositions described herein are used for treatment of cancer, the beneficial or desired results include, but are not limited to, one or more of the following: reducing the proliferation of (or destroying) neoplastic or cancerous cells, reducing metastasis of neoplastic cells found in cancers, shrinking the size of a tumor, decreasing symptoms resulting from the cancer, increasing the quality of life of those suffering from the cancer, decreasing the dose of other medications required to treat the disease, delaying the progression of the cancer, and/or prolonging survival of patients having cancer.

As used herein, the terms "preventing" disease or "protecting a host" from disease (used interchangeably herein) encompass, but are not limited to, one or more of the following: stopping, deferring, hindering, slowing, retarding, and/or postponing the onset or progression of a disease, stabilizing the progression of a disease, and/or delaying development of a disease. The terms "preventing" a condition or "protecting a host" from a condition (used interchangeably herein) encompass, but are not limited to, one or more of the following: stopping, deferring, hindering, slowing, retarding, and/or postponing the onset or progression of a condition, stabilizing the progression of a condition, and/or delaying development of a condition. The period of this prevention can be of varying lengths of time, depending on the history of the disease or condition and/or individual being treated. By way of example, where the vaccine is designed to prevent or protect against an infectious disease caused by a pathogen, the terms "preventing" disease or "protecting a host" from disease encompass, but are not limited to, one or more of the following: stopping, deferring, hindering, slowing, retarding, and/or postponing the infection by a pathogen of a host, progression of an infection by a pathogen of a host, or the onset or progression of a disease associated with infection of a host by a pathogen, and/or stabilizing the progression of a disease associated with infection of a host by a pathogen. Also, by way of example, where the vaccine is an anti-cancer vaccine, the terms "preventing" disease or "protecting the host" from disease encompass, but are not limited to, one or more of the following: stopping, deferring, hindering, slowing, retarding, and/or postponing the development of cancer or metastasis, progression of a cancer, or a reoccurrence of a cancer.

In one aspect, described herein is a method of inducing an immune response in a host to an antigen, comprising administering to the host an effective amount of a recombinant bacterium described herein or an effective amount of a composition (e.g., a pharmaceutical composition, immunogenic composition, or vaccine) comprising a recombinant bacterium described herein (see, e.g., the Summary of the Invention, Sections I, VIII, and IX of the Detailed Description above, or the Examples below). In some embodiments, the polypeptide encoded by the recombinant nucleic acid, expression cassette, and/or expression vector in the recombinant bacterium comprises the antigen or is a fusion protein or protein chimera comprising the antigen.

For instance, in one aspect, described herein is a method of inducing an immune response in a host to an antigen comprising administering to the host an effective amount of a composition comprising a recombinant bacterium, wherein the recombinant bacterium comprises an expression cassette comprising the following: (a) a first polynucleotide encoding a signal peptide, wherein the first polynucleotide is codon-optimized for expression in the bacterium; (b) a second polynucleotide encoding the antigen, wherein the second polynucleotide is in the same translational reading frame as the first polynucleotide; and (c) a promoter operably linked to the first and second polynucleotides, so that the expression cassette encodes a fusion protein comprising the signal peptide and the antigen.

In another aspect, described herein is a method of inducing an immune response in a host to an antigen comprising administering to the host an effective amount of a composition comprising a recombinant bacterium comprising an expression cassette, where the expression cassette comprises the following: (a) a first polynucleotide encoding a non-secA1 bacterial signal peptide; (b) a second polynucleotide encoding the antigen in the same transitional reading frame as the first polynucleotide; and (c) a promoter operably linked to the first and second polynucleotides, so that the expression cassette encodes a fusion protein comprising the signal peptide and the antigen.

In yet another aspect, described herein is a method of inducing an immune response in a host to a non-*Listerial* antigen comprising administering to the host an effective amount of a compositions comprising a recombinant *Listeria* bacterium comprising a nucleic acid molecule, wherein the nucleic acid molecule comprises the following: (a) a polynucleotide which encodes the non-*Listerial* antigen and that is codon-optimized for expression in *Listeria*; and (b) a promoter, operably linked to the polynucleotide encoding the antigen.

In another aspect, described herein is a method of inducing an immune response in a host to an antigen comprising administering to the host an effective amount of a recombinant *Listeria* bacterium comprising an expression cassette which comprises the following: (a) a first polynucleotide encoding a non-Listerial signal peptide; (b) a second polynucleotide encoding the antigen that is in the same translational reading frame as the first polynucleotide; and (c) a promoter operably linked to both the first and second polynucleotides, wherein the expression cassette encodes a fusion protein comprising both the non-Listerial signal peptide and the polypeptide.

In some embodiments of the methods of inducing immune responses described herein, the bacterium is administered in the form of a pharmaceutical composition, an immunogenic composition and/or vaccine composition.

In some embodiments, the immune response is an MHC Class I immune response. In other embodiments, the immune response is an MHC Class II immune response. In still other embodiments, the immune response that is induced by administration of the bacteria or compositions is both an MHC Class I and an MHC Class II response. Accordingly, in some embodiments, the immune response comprises a CD4+ T-cell response. In some embodiments, the immune response comprises a CD8+ T-cell response. In some embodiments, the immune response comprises both a CD4+ T-cell response and a CD8+ T-cell response. In some embodiments, the immune response comprises a B-cell response and/or a T-cell response. B-cell responses may be measured by determining the titer of an antibody directed against the antigen, using methods known to those of ordinary skill in the art. In some embodiments, the immune response which is induced by the compositions described herein is a humoral response. In other embodiments, the immune response which is induced is a cellular immune response. In some embodiments, the immune response comprises both cellular and humoral immune responses. In some embodiments, the immune response is antigen-specific. In some embodiments, the immune response is an antigen-specific T-cell response.

In addition to providing methods of inducing immune responses, the present invention also provides methods of preventing or treating a condition in a host (e.g., a subject such as human patient). In some embodiments, the condition is a disease. The methods comprise administration to the host of an effective amount of a recombinant bacterium described herein, or a composition comprising a recombinant bacterium described herein (see, e.g., the Summary of the Invention, Sections I, VIII, and IX of the Detailed Description above, or the Examples below). In some embodiments, the disease is cancer. In some embodiments, the disease is an infectious disease.

For instance, in one aspect, described herein is a method of preventing or treating disease (or condition) in a host comprising administering to the host an effective amount of composition comprising a bacterium, wherein the bacterium comprises an expression cassette comprising the following: (a) a first polynucleotide encoding a signal peptide, wherein the first polynucleotide is codon-optimized for expression in a bacterium; (b) a second polynucleotide encoding a polypeptide (e.g., an antigen and/or a therapeutic mammalian protein), wherein the second polynucleotide is in the same translational reading frame as the first polynucleotide; and (c) a promoter operably linked to the first and second polynucleotides, so that the expression cassette encodes a fusion protein comprising the signal peptide and the antigen.

In another aspect, described herein is a method of preventing or treating disease (or condition) in a host comprising administering to the host an effective amount of a composition comprising a recombinant bacterium, where the bacterium comprises an expression cassette, and where the expression cassette comprises the following: (a) a first polynucleotide encoding a non-secA1 bacterial signal peptide; (b) a second polynucleotide encoding a polypeptide (e.g., an antigen and/or mammalian protein) in the same translational reading frame as the first polynucleotide; and (c) a promoter operably linked to the first and second polynucleotides, so that the expression cassette encodes a fusion protein comprising the signal peptide and the antigen.

In still another aspect, described herein is a method of preventing or treating disease (or a condition) in a host comprising administering to the host an effective amount of a composition comprising a recombinant *Listeria* bacterium comprising a nucleic acid molecule, wherein the nucleic acid molecule comprises the following: (a) a polynucleotide which encodes a non-*Listerial* polypeptide (e.g., an antigen and/or a therapeutic mammalian protein) and that is codon-optimized for expression in *Listeria*; and (b) a promoter, operably linked to the polynucleotide encoding the antigen.

In another aspect, described herein is a method of preventing or treating disease (or a condition) in a host comprising administering to the host an effective amount of a composition comprising a recombinant *Listeria* bacterium comprising an expression cassette which comprises: (a) a first polynucleotide encoding a non-Listerial signal peptide; (b) a second polynucleotide encoding a polypeptide (e.g., an antigen and/or a therapeutic mammalian protein) that is in the same translational reading frame as the first polynucleotide; and (c) a promoter operably linked to both the first and second polynucleotides, wherein the expression cassette encodes a fusion protein comprising both the non-Listerial signal peptide and the polypeptide.

In some embodiments, the disease is cancer. In some embodiments, where the condition being treated or prevented is cancer, the disease is melanoma, breast cancer, pancreatic cancer, liver cancer, colon cancer, colorectal cancer, lung cancer, brain cancer, testicular cancer, ovarian cancer, squamous cell cancer, gastrointestinal cancer, cervical cancer, kidney cancer, thyroid cancer or prostate cancer. In some embodiments, the cancer is melanoma. In some embodiments, the cancer is pancreatic cancer. In some embodiments, the cancer is colon cancer. In some embodiments, the cancer is prostate cancer. In some embodiments, the cancer is metastatic.

In other embodiments, the disease is an autoimmune disease. In still other embodiments, the disease is an infectious disease or another disease caused by a pathogen such as a virus, bacterium, fungus, or protozoa. In some embodiments, the disease is an infectious disease.

In some embodiments, the use of the recombinant bacteria in the prophylaxis or treatment of a cancer comprises the delivery of the recombinant bacteria to cells of the immune system of an individual to prevent or treat a cancer present or to which the individual has increased risk factors, such as environmental exposure and/or familial disposition. In other embodiments, the use of the recombinant bacteria in the prophylaxis or treatment of a cancer comprises delivery of the recombinant bacteria to an individual who has had a tumor removed or has had cancer in the past, but is currently in remission.

In some embodiments, administration of composition comprising a recombinant bacterium described herein to a host elicits a CD4+ T-cell response in the host. In some other embodiments, administration of a composition comprising a recombinant bacterium described herein to a host elicits a CD8+ T-cell response in the host. In some embodiments, administration of a composition comprising a recombinant bacterium described herein elicits both a CD4+ T-cell response and a CD8+ T-cell response in the host.

The efficacy of the vaccines or other compositions for the treatment of a condition can be evaluated in an individual, for example in mice. A mouse model is recognized as a model for efficacy in humans and is useful in assessing and defining the vaccines of the present invention. The mouse model is used to demonstrate the potential for the effectiveness of the vaccines in any individual. Vaccines can be evaluated for their ability to provide either a prophylactic or therapeutic effect against a particular disease. For example, in the case of infectious diseases, a population of mice can be vaccinated with a desired amount of the appropriate vaccine of this disclosure where the recombinant bacterium expresses an infectious disease associated antigen. The mice can be subsequently infected with the infectious agent related to the vaccine antigen and assessed for protection against infection. The progression of the infectious disease can be observed relative to a control population (either non vaccinated or vaccinated with vehicle only or a bacterium that does not contain the appropriate antigen).

In the case of cancer vaccines, tumor cell models are available, where a tumor cell line expressing a desired tumor antigen can be injected into a population of mice either before (therapeutic model) or after (prophylactic model) vaccination with a composition comprising a bacterium of this disclosure containing the desired tumor antigen. Vaccination with a recombinant bacterium containing the tumor antigen can be compared to control populations that are either not vaccinated, vaccinated with vehicle, or with a recombinant bacterium that expresses an irrelevant antigen. The effectiveness of the vaccine in such models can be evaluated in terms of tumor volume as a function of time after tumor injection or in terms of survival populations as a function of time after tumor injection (e.g., Example 31D). In one embodiment, the tumor volume in mice vaccinated with a composition comprising the recombinant bacterium is about 5%, about 10%, about 25%, about 50%, about 75%, about 90% or about 100% less than the tumor volume in mice that are either not vaccinated or are vaccinated with vehicle or a bacterium that expresses an irrelevant antigen. In another embodiment, this differential in tumor volume is observed at least about 10, about 17, or about 24 days following the implant of the tumors into the mice. In one embodiment, the median survival time in the mice vaccinated with the composition comprising a recombinant bacterium is at least about 2, about 5, about 7 or at least about 10 days longer than in mice that are either not vaccinated or are vaccinated with vehicle or bacteria that express an irrelevant antigen.

The host (i.e., subject) in the methods described herein, is any vertebrate, preferably a mammal, including domestic animals, sport animals, and primates, including humans. In some embodiments, the host is a mammal. In some embodiments, the host is a human.

The delivery of the recombinant bacteria, or a composition comprising the strain, may be by any suitable method, such as intradermal, subcutaneous, intraperitoneal, intravenous, intramuscular, intralymphatic, oral or intranasal, as well as by any route that is relevant for any given malignant or infectious disease or other condition.

The compositions comprising the recombinant bacteria and an immunostimulatory agent may be administered to a host simultaneously, sequentially or separately. Examples of immunostimulatory agents include, but are not limited to IL-2, IL-12, GMCSF, IL-15, B7.1, B7.2, and B7-DC and IL-14. Additional examples of stimulatory agents are provided in Section IX, above

As used herein, an "effective amount" of a bacterium or composition (such as a pharmaceutical composition or an immunogenic composition) is an amount sufficient to effect beneficial or desired results. For prophylactic use, beneficial or desired results includes results such as eliminating or reducing the risk, lessening the severity, or delaying the outset of the disease, including biochemical, histologic and/or behavioral symptoms of a disease, its complications and intermediate pathological phenotypes presenting during development of the disease. For therapeutic use, beneficial or desired results includes clinical results such as inhibiting or suppressing a disease, decreasing one or more symptoms resulting from a disease (biochemical, histologic and/or behavioral), including its complications and intermediate pathological phenotypes presenting during development of a disease, increasing the quality of life of those suffering from a disease, decreasing the dose of other medications required to treat the disease, enhancing effect of another medication, delaying the progression of the disease, and/or prolonging survival of patients. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an effective amount may be considered in the context of administering one or more therapeutic agents, and a single agent may be considered to be given in an effective amount if, in conjunction with one or more other agents, a desirable result may be or is achieved.

In some embodiments, for a therapeutic treatment of a cancer, an effective amount includes an amount that will result in the desired immune response, wherein the immune response either slows the growth of the targeted tumors, reduces the size of the tumors, or preferably eliminates the tumors completely. The administration of the vaccine may be repeated at appropriate intervals, and may be administered simultaneously at multiple distinct sites in the vaccinated individual. In some embodiments, for a prophylactic treatment of a cancer, an effective amount includes a dose that will result in a protective immune response such that the likelihood of an individual to develop the cancer is significantly reduced. The vaccination regimen may be comprised of a single dose, or may be repeated at suitable intervals until a protective immune response is established.

In some embodiments, the therapeutic treatment of an individual for cancer may be started on an individual who has been diagnosed with a cancer as an initial treatment, or may be used in combination with other treatments. For example, individuals who have had tumors surgically removed or who have been treated with radiation therapy or by chemotherapy may be treated with the vaccine in order to reduce or eliminate any residual tumors in the individual, or to reduce the risk of a recurrence of the cancer. In some embodiments, the prophylactic treatment of an individual for cancer, would be started on an individual who has an increased risk of contracting certain cancers, either due to environmental conditions or genetic predisposition.

The dosage of the pharmaceutical compositions or vaccines that are given to the host will vary depending on the species of the host, the size of the host, and the condition or disease of the host. The dosage of the compositions will also depend on the frequency of administration of the compositions and the route of administration. The exact dosage is chosen by the individual physician in view of the patient to be treated.

In some embodiments, the pharmaceutical compositions, immunogenic compositions, or vaccines used in the methods comprise recombinant bacteria which comprise the recombinant nucleic acid molecules, expression cassettes and/or expression vectors described herein. In some embodiments, the recombinant bacteria are modified and/or mutant bacteria such as those described in U.S. patent application Serial No. 10/883,599, entitled "Modified Free-Living Microbes, Vaccine Compositions and Methods of Use Thereof," by Thomas W. Dubensky, Jr. et al., filed June 30,2004, U.S. Patent Publication No. 2004/0228877 and U.S. Patent Publication No.2004/0197343. In some embodiments, a single dose of the pharmaceutical composition or vaccine comprising such modified and/or mutant bacteria or any of the other recombinant bacteria described herein comprises from about 10² to about 10¹² of the bacterial organisms. In another embodiment, a single dose comprises from about 10⁵ to about 10¹¹ of the bacterial organisms. In another embodiment, a single dose comprises from about 10⁶ to about 10¹¹ of the bacterial organisms. In still another embodiment, a single dose of the pharmaceutical composition or vaccine comprises from about 10⁷ to about 10¹⁰ of the bacterial organisms. In still another embodiment, a single dose of the pharmaceutical composition or vaccine comprises from about 10⁷ to about 10⁹ of the bacterial organisms.

In some embodiments, a single dosage comprises at least about 1 x 10² bacterial organisms. In some embodiments, a single dose of the composition comprises at least about 1 x 10⁵ organisms. In another embodiment, a single dose of the composition or vaccine comprises at least about 1 x 10⁶ bacterial organisms. In still another embodiment, a single dose of the composition or vaccine comprises at least about 1 x 10⁷ of the bacterial organisms.

In some embodiments, a single dose of the pharmaceutical composition, immunogenic composition, or vaccine comprising recombinant, modified and/or mutant bacteria described herein comprises from about 1 CFU/kg to about 1 x 10¹⁰ CFU/kg (CFU = colony forming units). In some embodiments, a single dose of the composition comprises from about 10 CFU/kg to about 1 x 10⁹ CFU/kg. In another embodiment, a single dose of the composition or vaccine comprises from about 1 x 10² CFU/kg to about 1 x 10⁸ CFU/kg. In still another embodiment, a single dose of the composition or vaccine comprises from about 1 x 10³ CFU/kg to about 1 x 10⁸ CFU/kg. In still another embodiment, a single dose of the composition or vaccine comprises from about 1 x 10⁴ CFU/kg to about 1 x 10⁷ CFU/kg. In some embodiments, a single dose of the composition comprises at least about 1 CFU/kg. In some embodiments, a single dose of the composition comprises at least about 10 CFU/kg. In another embodiment, a single dose of the composition or vaccine comprises at least about 1 x 10² CFU/kg. In still another embodiment, a single dose of the composition or vaccine comprises at least about 1 x 10³ CFU/kg. In still another embodiment, a single dose of the composition or vaccine comprises from at least about 1 x 10⁴ CFU/kg.

In some embodiments, the proper (i.e., effective) dosage amount for one host, such as human, may be extrapolated from the LD₅₀ data for another host, such as a mouse, using methods known to those in the art.

In some embodiments, the pharmaceutical composition, immunogenic composition, or vaccine comprises antigen-presenting cells, such as dendritic cells, which have been infected with recombinant bacteria comprising the recombinant nucleic acid molecules, expression cassettes and/or expression vectors described herein. In some embodiments, the bacteria have been modified and/or are mutants such as those described in U.S. patent application Serial No. 10/883,599, filed June 30, 2004, and U.S. Patent Publication Nos. 2004/0228877 and US 2004/0197343. Such antigen-presenting cell based vaccines are described, for instance, in the following: International Application No. PCT/US2004/23881, entitled "Antigen-Presenting Cell Vaccines and Methods of Use Thereof," by Thomas W. Dubensky, Jr. et al., filed July 23,2004; U.S. patent application Serial No. 10/883,599, filed June 30, 2004; U.S. Patent Publication No. 2004/0228877; and U.S. Patent Publication No. US 2004/0197343. In some embodiments, an individual dosage of an antigen-presenting cell based vaccine comprising bacteria such as those described herein comprises between about 1x10³ to about 1x10¹⁰ antigen-presenting cells. In some embodiments, an individual dosage of the vaccine comprises between about 1x10⁵ to about 1x10⁹ antigen-presenting cells. In some embodiments, an individual dosage of the vaccine comprises between about 1x10⁷ to about 1x10⁹ antigen-presenting cells.

In some embodiments, multiple administrations of the dosage unit are preferred, either in a single day or over the course of a week or month or year or years. In some embodiments, the dosage unit is administered every day for multiple days, or once a week for multiple weeks.

This disclosure further provides the use of any recombinant bacterium described herein (i.e., any bacterium comprising a recombinant nucleic acid molecule, expression cassette, or vector described herein) in the manufacture of a medicament for inducing an immune response in a host to an antigen, wherein a polypeptide encoded by the recombinant nucleic acid molecule, expression cassette, and/or vector in the bacterium comprises the antigen. In some embodiments, the antigen is a heterologous antigen. This disclosure also provides the use of a recombinant bacterium described herein in the manufacture of a medicament for preventing or treating a condition in a host (e.g., a disease such as cancer or an infectious disease). This disclosure further provides the recombinant bacteria described herein for use in inducing an immune response in a host to an antigen, wherein a polypeptide encoded by the recombinant nucleic acid molecule, expression cassette, and/or vector in the bacterium comprises the antigen. This disclosure further provides the recombinant bacteria described herein for use in the prevention or treatment of a condition (such as a disease) in a host.

This disclosure also provides a method of inducing MHC class I antigen presentation or MHC class II antigen presentation on an antigen-presenting cell comprising contacting a bacterium described herein with an antigen-presenting cell.

This disclosure further provides a method of inducing an immune response in a host to an antigen comprising, the following steps: (a) contacting a recombinant bacterium described herein with an antigen-presenting cell from the host, under suitable conditions and for a time sufficient to load the antigen-presenting cells; and (b) administering the antigen-presenting cell to the host.

Other possible uses of the recombinant nucleic acid molecules, expression cassettes, and bacteria will be recognized by those of ordinary skill in the art. For instance, the recombinant nucleic acid molecules, expression cassettes, vectors, and recombinant bacteria (and other host cells) described herein are useful for the production and isolation of heterologous polypeptides. Accordingly in an alternative aspect, described herein is a method of expressing a polypeptide in a bacterium, comprising (a) introducing an expression cassette or vector described herein into bacteria (e.g., via transfection, transformation, or conjugation); and (b) growing the bacteria in culture under conditions suitable for protein expression. In another alternative aspect, described herein is a method of producing an isolated polypeptide comprising the following: (a) introducing an expression cassette or vector described herein into bacteria (e.g., via transfection, transformation, or conjugation); (b) growing the bacteria in cell culture under conditions suitable for protein expression; and (c) isolating the protein from the bacterial cell culture. Suitable methods of transformation, transfection, and conjugation are well known to those of ordinary skill in the art, as are methods of culturing and growing bacteria and isolating secreted or non-secreted protein from cell culture.

### EXAMPLES

The following examples are provided to illustrate, but not to limit, the present disclosure.

### Example 1. Preparation of exemplary mutant Listeria strains.

*Listeria* strains were derived from 10403S (Bishop et al., J. Immunol. 139:2005 (1987)). *Listeria* strains with in-frame deletions of the indicated genes were generated by SOE-PCR and allelic exchange with established methods (Camilli, et al, Mol. Microbiol. 8:143 (1993)). The mutant strain LLO L461T (DP-L4017) was described in Glomski, et al, J. Cell. Biol. 156: 1029 (2002). The *actA⁻*mutant (DP-L4029) is the DP-L3078 strain described in Skoble et al., J. of Cell Biology, 150: 527-537 (2000), which has been cured of its prophage. (Prophage curing is described in (Lauer et al., J. Bacteriol. 184:4177 (2002); U.S. Patent Publication No. 2003/0203472).) Construction of an *actA⁻uvrAB⁻* strain is described in the U.S. provisional application 60/446,051, filed February 6, 2003, as L4029/uvrAB (see, e.g. Example 7 of that application), as well as in U.S. Patent Publication No. 2004/0197343. DP-L4029*uvrAB* (a *Listeria monocytogenes actA⁻*/*uvrAB⁻* double deletion mutant) was deposited with the American Type Culture Collection (ATCC), at 10801 University Blvd, Manassas, Virginia, 20110-2209, United States of America, on October 3, 2003, under the provisions of the Budapest Treaty on the International Recognition of the Deposit of Microorganisms for the Purposes of Patent Procedure, and designated PTA-5563. Additional descriptions regarding mutant *Listeria* are provided in the following applications or publications: U.S. Patent Publication No. 2004/0228877; U.S. Patent Publication No. US 2004/0197343; the PCT International Application No. PCT/US2004/23881, filed July 23, 2004; and U.S. patent application Serial No. 10/883,599, filed June 30, 2004. In addition, an exemplary *Listeria monocytogenes* ΔactAΔinlB double deletion mutant has been deposited with the American Type Culture Collection ((ATCC), at 10801 University Blvd, Manassas, Virginia, 20110-2209, United States of America, on October 3, 2003, under the provisions of the Budapest Treaty on the International Recognition of the Deposit of Microorganisms for the Purposes of Patent Procedure, and designated PTA-5562.

One non-limiting example of a method of deleting a gene in *Listeria monocytogenes* to generate an attenuated mutant is provided in Example 24, below.

### Example 2. Construction of Listeria strains expressing AH1/OVA or AH1-A5/OVA

Mutant *Listeria* strains expressing a truncated form of a model antigen ovalbumin (OVA), the immunodominant epitope from mouse colorectal cancer (CT26) known as AH1 (SPSYVYHQF (SEQ ID NO:72)), and the altered epitope AH1-A5 (SPSYAYHQF (SEQ ID NO:73); Slansky et al., Immunity, 13:529-538 (2000)) were prepared. The pPL2 integrational vector (Lauer et al., J. Bacteriol. 184:4177 (2002); U.S. Patent Publication No. 2003/0203472) was used to derive OVA and AH1-A5/OVA recombinant *Listeria* strains containing a single copy integrated into an innocuous site of the *Listeria* genome.

### A. Construction of OVA-expressing Listeria (DP-L4056).

An antigen expression cassette consisting of hemolysin-deleted LLO fused with truncated OVA and contained in the pPL2 integration vector (pPL2/LLO-OVA) is first prepared. The *Listeria*-OVA vaccine strain is derived by introducing pPL2/LLO-OVA into the phage-cured *L. monocytogenes* strain DP-L4056 at the PSA (Phage from ScottA) attachment site tRNA^{Arg}*-attBB'*.

PCR is used to amplify the hemolysin-deleted LLO using the following template and primers:
Source: DP-L4056 genomic DNA
Primers:
   Forward (*kpnI*-LLO nts. 1257-1276):
      5'-CTCTGGTACCTCCTTTGATTAGTATATTC (SEQ ID NO:74)
      (Tₘ: LLO-spec:52°C. Overall:80°C.)
   Reverse (*BamHI*-*XhoI-*LLO nts. 2811-2792):
      5'-CAATGGATCCCTCGAGATCATAATTTACTTCATCCC (SEQ ID NO:75)
      (Tₘ: LLO-spec: 52°C. Overall: 102°C.)

PCR is also used to amplify the truncated OVA using the following template and primers:
Source: pDP3616 plasmid DNA from DP-E3616 *E. coli* (Higgins et al., Mol. Molbiol. 31:1631-1641 (1999)).
Primers: Forward (*XhoI- NcoI* OVA cDNA nts. 174-186):
   5'-ATTTCTCGAGTCCATGGGGGGTTCTCATCATC (SEQ ID NO:76)
   (Tₘ: OVA-spec: 60°C. Overall: 88°C.)
Reverse (*XhoI-NotI-HindIII*):
   5'-GGTGCTCGAGTGCGGCCGCAAGCTT (SEQ ID NO:77)
   (Tₘ: Overall: 82°C.)

One protocol for completing the construction process involves first cutting the LLO amplicon with KpnI and BamHI and inserting the KpnI/BamHI vector into the pPL2 vector (pPL2-LLO). The OVA amplicon is then cut with *XhoI* and *NotI* and inserted into the pPL2-LLO which has been cut with *XhoI*/*NotI*. (Note: The pPL2 vector does not contain any *XhoI* sites; pDP-3616 contains one *XhoI* site, that is exploited in the OVA reverse primer design.) The construct pPL2/LLO-OVA is verified by restriction analysis (*KpnI*-LLO-*XhoI-*OVA-*NotI*) and sequencing. The plasmid pPL2/LLO-OVA is introduced into *E. coli* by transformation, followed by introduction and integration into *Listeria* (DP-L4056) by conjugation, exactly as described by Lauer et al. (or into another desired strain of *Listeria*, such as an *inlB⁻* mutant or an *inlB⁻actA⁻* double mutant).

### B. Construction of Listeria strains expressing AH1/OVA or AH1-A5/OVA.

To prepare *Listeria* expressing either the AH1/OVA or the AH1-A5/OVA antigen sequences, inserts bearing the antigen are first prepared from oligonucleotides and then ligated into the vector pPL2/LLO-OVA (prepared as described above).

The following oligonucleotides are used in preparation of the AH1 or AH1-A5 insert:
*AH1 epitope insert (ClaI-PstI compatible ends):*
   Top strand oligo (AH1 Top):
   5'-CGATTCCCCTAGTTATGTTTACCACCAATTTGCTGCA (SEQ ID NO:78)
   Bottom strand oligo (AH1 Bottom):
   5'-GCAAATTGGTGGTAAACATAACTAGGGGAAT (SEQ ID NO:79)
*AHI-A5 epitope insert (ClaI-AvaII compatible ends):*
   The sequence of the AH1-A5 epitope is SPSYAYHQF (SEQ ID NO:73) (5'-AGT CCA AGT TAT GCA TAT CAT CAA TTT-3' (SEQ ID NO:80)).
   Top: 5'-**CGAT**AGTCCAAGTTATGCATATCATCAATTTGC (SEQ ID NO:81)
   Bottom: 5'-**GTCG**CAAATTGATGATATGCATAACTTGGACTAT (SEQ ID NO:82)

The oligonucletide pair for a given epitope are mixed together at an equimolar ratio, heated at 95 °C for 5 min. The oligonucleotide mixture is then allowed to slowly cool. The annealed oligonucleotide pairs are then ligated at a 200 to 1 molar ratio with pPL2-LLO/OVA plasmid prepared by digestion with the relevant restriction enzymes. The identity of the new construct can be verified by restriction analysis and/or sequencing.

The plasmid can then be introduced into E. coli by transformation, followed by introduction and integration into *Listeria* (DP-L4056) by conjugation, exactly as described by Lauer et al., or into another desired strain of *Listeria,* such as an actA⁻ mutant strain (DP-L0429), LLO L461 T strain (DP-L4017), or *actA⁻*/*uvrAB⁻* strain (DP-L4029uvrAB).

### Example 3. Construction of Listeria polynucleotides and expression cassette elements

### A. Cloning vectors

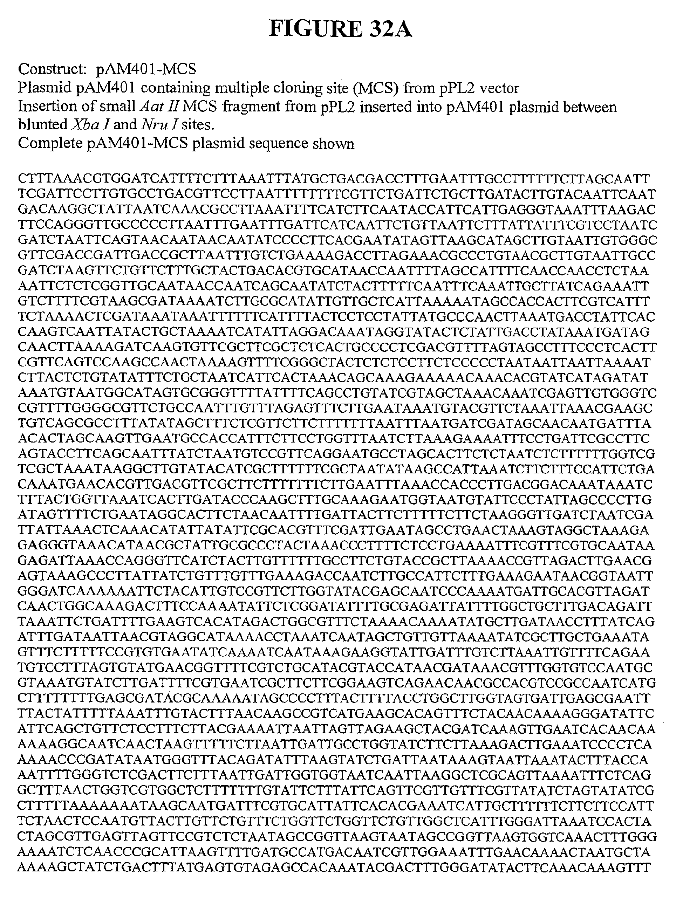

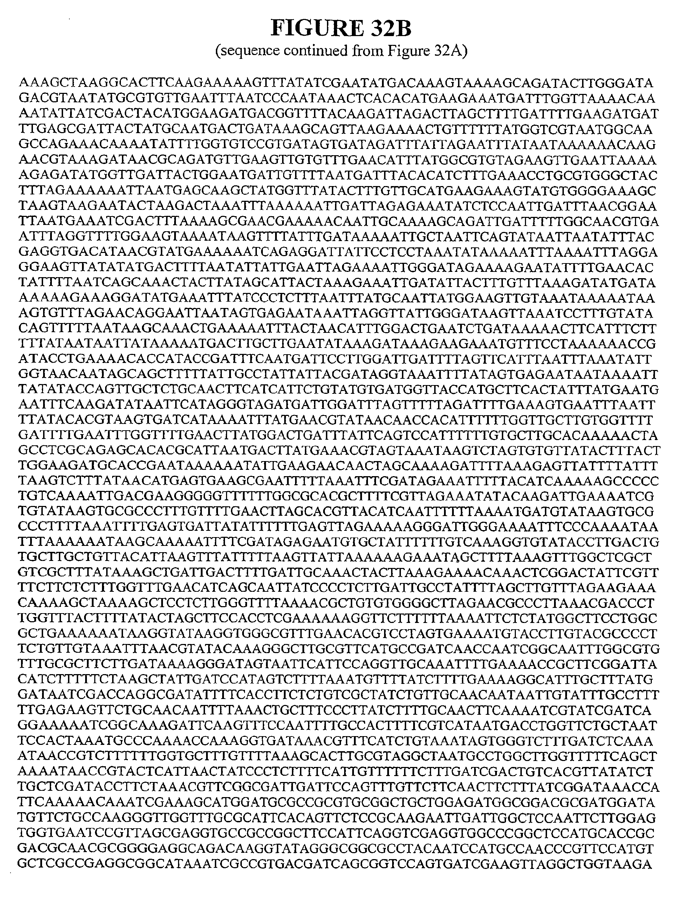

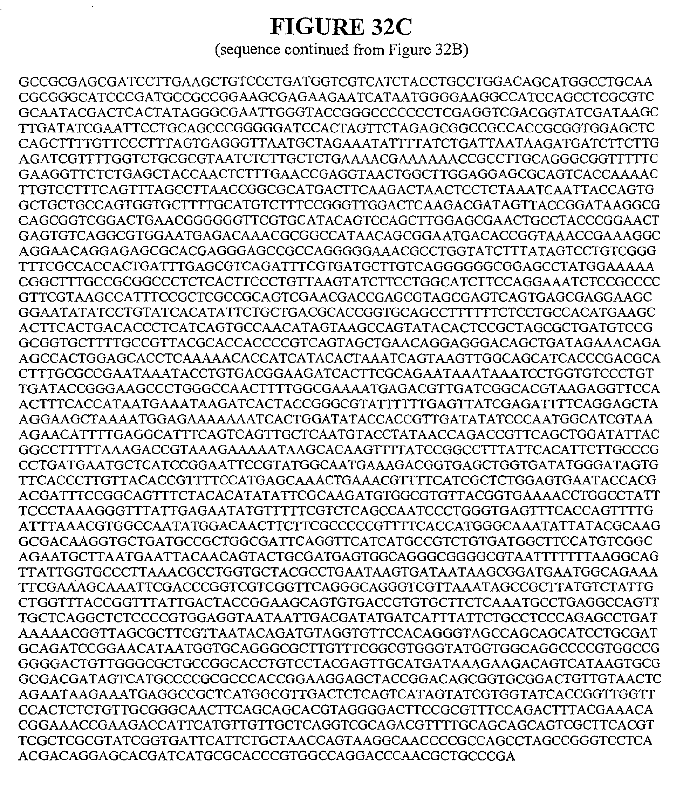

Selected heterologous antigen expression cassette molecular constructs were inserted into pPL2 (Lauer et. al. J. Bacteriol. 2002), or pAM401 (Wirth et. al., J. Bacteriol. 165:831-836), modified to contain the multiple cloning sequence of pPL2 (*Aat II* small fragment, 171 bps), inserted between blunted *Xba* I and *Nru I* recognition sites, within the tetracycline resistance gene (pAM401-MCS, Figure 32). In general, the *hly* promoter and (selected) signal peptide sequence was inserted between the unique *Kpn I* and *Bam HI* sites in the pPL2 or pAM401-MCS plasmid vectors. Selected EphA2 genes (sometimes modified to contain N-terminal and C-terminal epitope tags; see description below) were cloned subsequently into these constructs between unique *Bam HI* and *Sac I* sites. Molecular constructs based on the pAM401-MCS plasmid vector were introduced by electroporation into selected *Listeria monocytogenes* strains also treated with lysozyme, utilizing methods common to those skilled in the art. The expected plasmid structure in *Listeria*-transfectants was verified by isolating DNA from colonies that formed on chloramphenicol-containing BHI agar plates (10 µg/ml) by restriction enzyme analysis. Recombinant *Listeria* transformed with various pAM401-MCS based heterologous protein expression cassette constructs were utilized to measure heterologous protein expression and secretion, as described below.

The pPL2 based heterologous protein expression cassette constructs were incorporated into the tRNA^{Arg} gene in the genome of selected *Listeria* strains, according to the methods as described previously [Lauer et. al., J. Bacteriol. 184, 4177-4186 (2002)]. Briefly, the pPL2 heterologous protein expression cassette constructs plasmid was first introduced into the *E. coli* host strain SM10 (Simon et. al., Bio/Technology 1:784-791 (1983)] by electroporation or by chemical means. Subsequently, the pPL2-based plasmid was transferred from transformed SM10 to the selected *Listeria* strains by conjugation. Following incubation on drug-selective BHI agar plates containing 7.5 µg of chloramphenicol per ml and 200 µg of streptomycin per ml as described, selected colonies are purified by passaging 3 times on plates with the same composition. To verify integration of the pPL2 vector at the phage attachment site, individual colonies are picked and screened by PCR using the primer pair of forward primer NC16 (5'-gtcaaaacatacgctcttatc-3' (SEQ ID NO:94)) and reverse primer PL95 (5'-acataatcagtccaaagtagatgc-3' (SEQ ID NO:95)). Selected colonies having the pPL2-based plasmid incorporated into the tRNA^{Arg} gene in the genome of selected *Listeria* strains yielded a diagnostic DNA amplicon of 499 bps.

### B. Promoter

Heterologous protein expression cassettes contained the prfA-dependent *hly* promoter, which drives the transcription of the gene encoding Listeriolysin O (LLO), and is activated within the microenvironment of the infected cell. Nucleotides 205586-206000 (414 bps) were amplified by PCR from *Listeria monocytogenes*, strain DP-L4056, using the primer pair shown below. The region amplified includes the *hly* promoter and also the first 28 amino acids of LLO, comprising the secA1 signal peptide (see above) and PEST domain. The expected sequence of this region for *Listeria monocytogenes*, strain EGD can be found in (GenBank (Accession number: gi|16802048|ref|NC_003210.1|[16802048]). The primers used in the PCR reaction are as follows:
Primer Pair:
   Forward (*KpnI*-LLO nts. 1257-1276):
      5'-CTCTGGTACCTCCTTTGATTAGTATATTC (SEQ ID NO:74)
   Reverse (*Bam HI*-LLO nts.):
      5'-CTCTGGATCCATCCGCGTGTTTCTTTTCG (SEQ ID NO:84)

### (Restriction endonuclease recognition sites are underlined.)

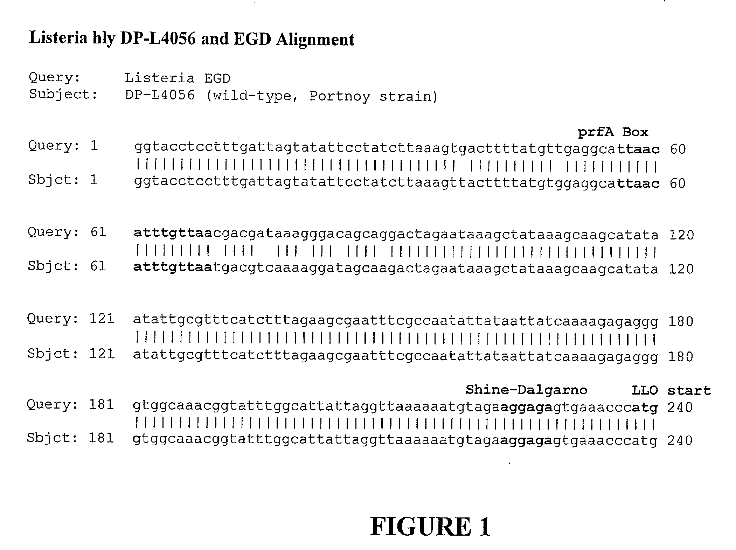

The 422 bp PCR amplicon was cloned into the plasmid vector pCR-XL-TOPO (Invitrogen, Carlsbad, CA), according to the manufacturer's specifications. The nucleotide sequences of *Listeria*-specific bases in the pCR-XL-TOPO-*hly* promoter plasmid clone was determined. *Listeria monocytogenes* strain DP-L4056 contained eight nucleotide base changes flanking the prfA box in the hly promoter, as compared to the EGD strain. The *hly* promoter alignment for the *Listeria monocytogenes* DP-L4056 and EGD strains is shown in Figure 1 below.

The 422 bp DNA corresponding to the hly promoter and secA1 LLO signal peptide were liberated from the pCR-XL-TOPO-*hly* promoter plasmid clone by digestion with Kpn I and Bam HI, and cloned into the pPL2 plasmid vector (Lauer et. al. 2002 J. Bact.), according to conventional methods well-known to those skilled in the art. This plasmid is known as pPL2-*hly*P (native).

### C. Shine-Dalgarno Sequence

At the 3' end of the promoter is contained a poly-purine Shine-Dalgarno sequence, the element required for engagement of the 30S ribosomal subunit (via 16S rRNA) to the heterologous gene RNA transcript and initiation of translation. The Shine-Dalgarno sequence has typically the following consensus sequence: 5'-NAGGAGGU-N₅₋₁₀-AUG (start codon)-3' (SEQ ID NO:85). There are variations of the poly-purine Shine-Dalgarno sequence Notably, the *Listeria* hly gene that encodes listerolysin O (LLO) has the following Shine-Dalgarno sequence: AAGGAGAGTGAAACCCATG (SEQ ID NO:70) (Shine-Dalgarno sequence is underlined, and the translation start codon is bolded).

### Example 4. Polynucleotides encoding a fusion protein comprising a secA1 signal peptide (LLO and human EphA2

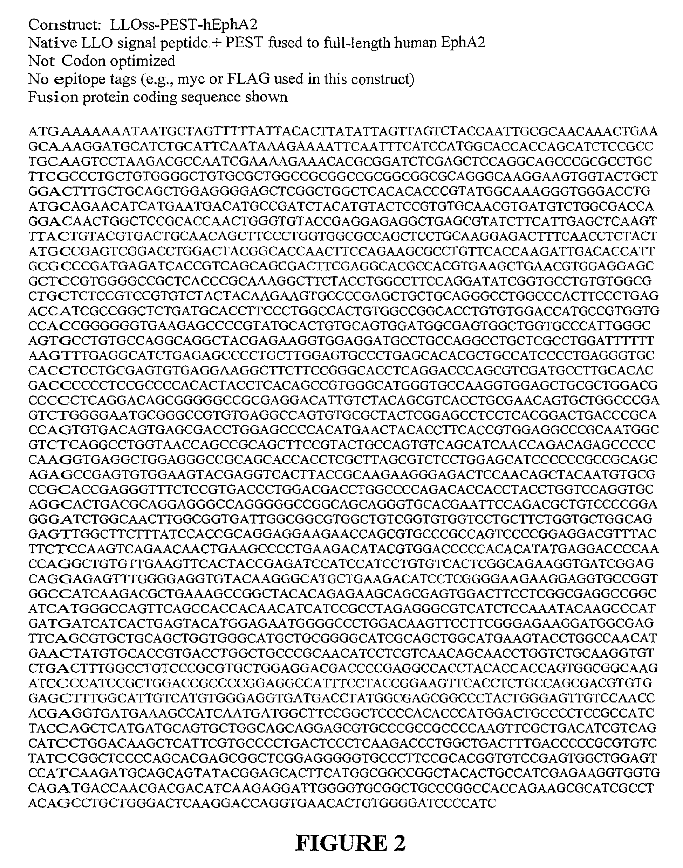

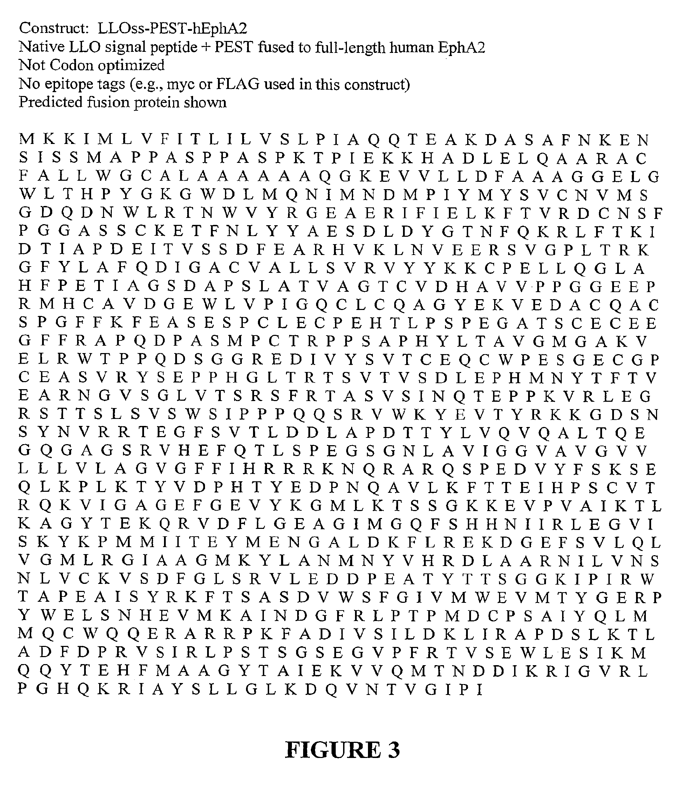

The sequence of an expression cassette encoding the full-length human EphA2 antigen fused to a secA1 signal peptide (LLO signal peptide), plus the LLO PEST sequence, is shown in Figure 2. The amino acid sequence of the fusion protein encoded by this expression cassette is shown in Figure 3.

### Example 5. Codon-optimization of the extracellular domain of human EphA2 (EX2)

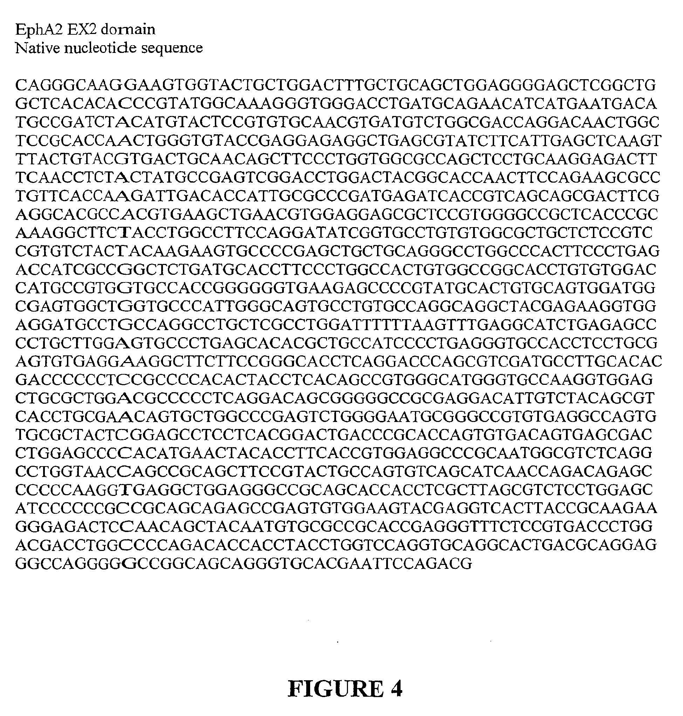

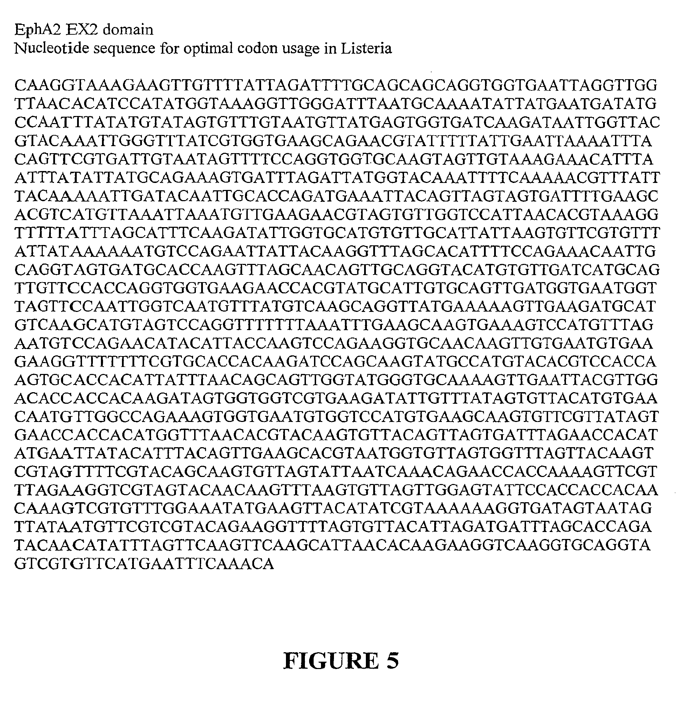

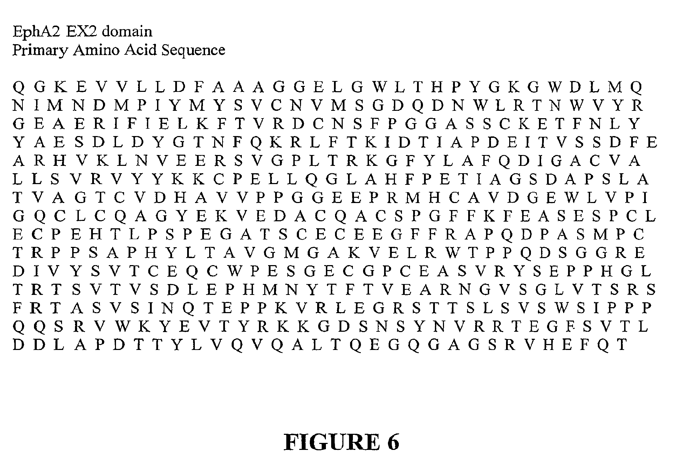

The sequence encoding the extracellular domain of human EphA2 (amino acids 25-526) has been codon-optimized for expression in *Listeria monocytogenes.* The native nucleotide sequence encoding the extracellular domain of human EphA2 is shown in Figure 4. The nucleotide sequence for optimal codon usage in *Listeria* is shown in Figure 5. The amino acid sequence of the extracellular domain of human EphA2 is shown in Figure 6.

### Example 6. Polynucleotides encoding an fusion proteins comprising a secA1 signal peptide (LLO) and the extracellular domain of huEphA2 (EX2)

### A. Polynucleotide without codon-optimization

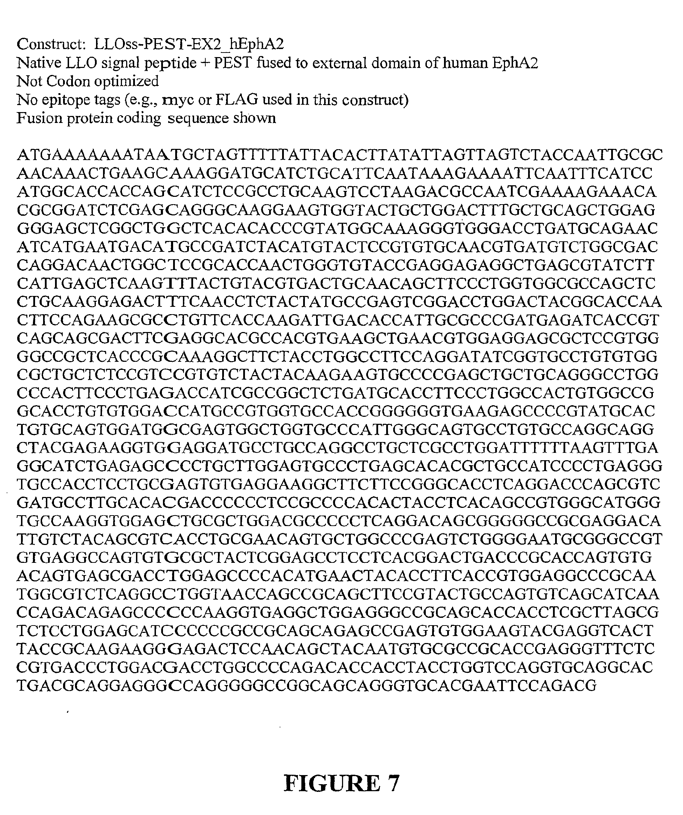

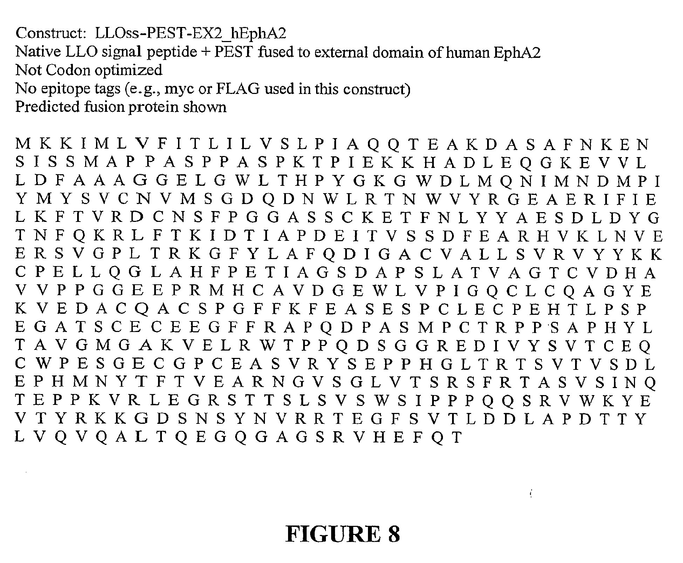

The sequence of a polynucleotide encoding the extracellular domain of human EphA2 antigen fused to a secA1 signal peptide (LLO signal peptide), plus the LLO PEST sequence, is shown in Figure 7. The amino acid sequence of the fusion protein encoded by this expression cassette is shown in Figure 8.

### B. Expression cassette with codon-optimized extracellular domain of human EphA2

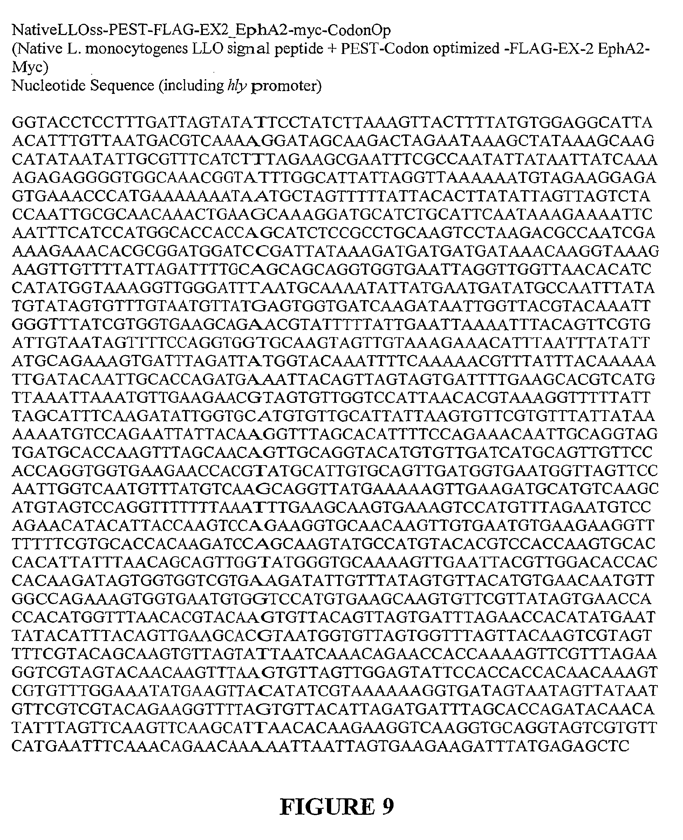

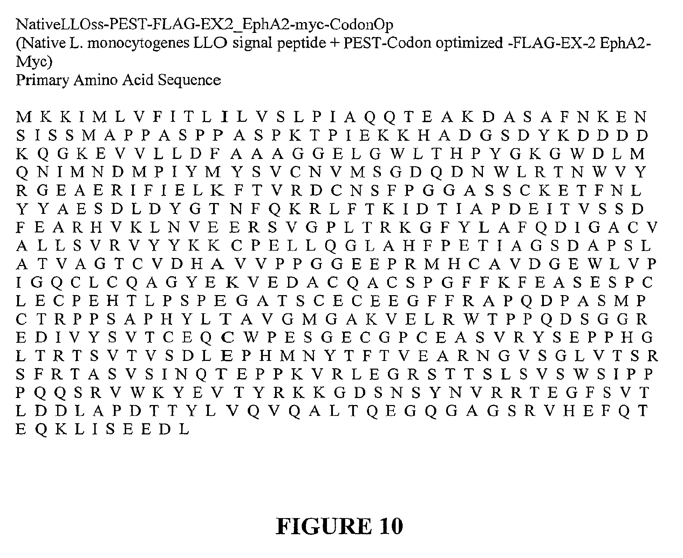

The sequence of an expression cassette encoding the extracellular domain of human EphA2 antigen fused to a secA1 signal peptide (LLO signal peptide), plus the LLO PEST sequence, in which the sequence encoding the extracellular domain of EphA2 is codon-optimized for expression in *Listeria monocytogenes*, is shown in Figure 9. The amino acid sequence of the fusion protein encoded by this expression cassette is shown in Figure 10.

### C. Expression cassette with codon-optimized secA1 Signal peptide and codon-optimized extracellular domain of human EphA2

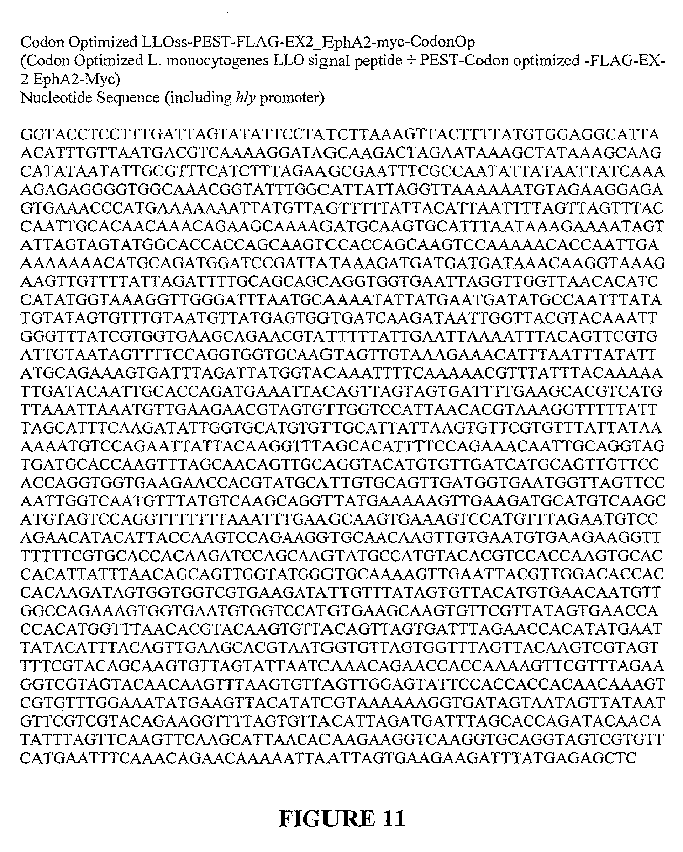

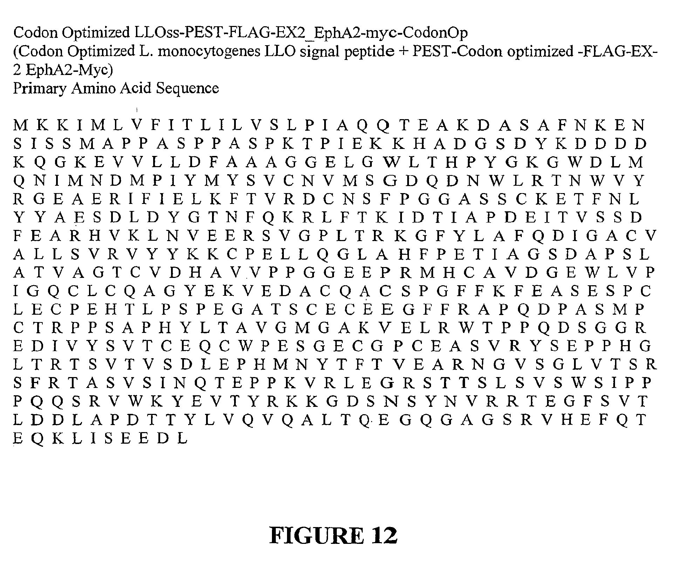

The sequence of an expression cassette encoding the extracellular domain of human EphA2 antigen fused to a secA1 signal peptide (LLO signal peptide), plus the LLO PEST sequence, where the sequences encoding the extracellular domain of EphA2, signal peptide and PEST sequence are all codon-optimized for expression in *Listeria monocytogenes*, is shown in Figure 11. The amino acid sequence of the fusion protein encoded by this expression cassette is shown in Figure 12.

### Example 7. Codon-optimized expression cassette encoding a fusion protein comprising a Tat signal peptide (B. subtilis phoD) and extracellular domain of huEphA2 (EX2)

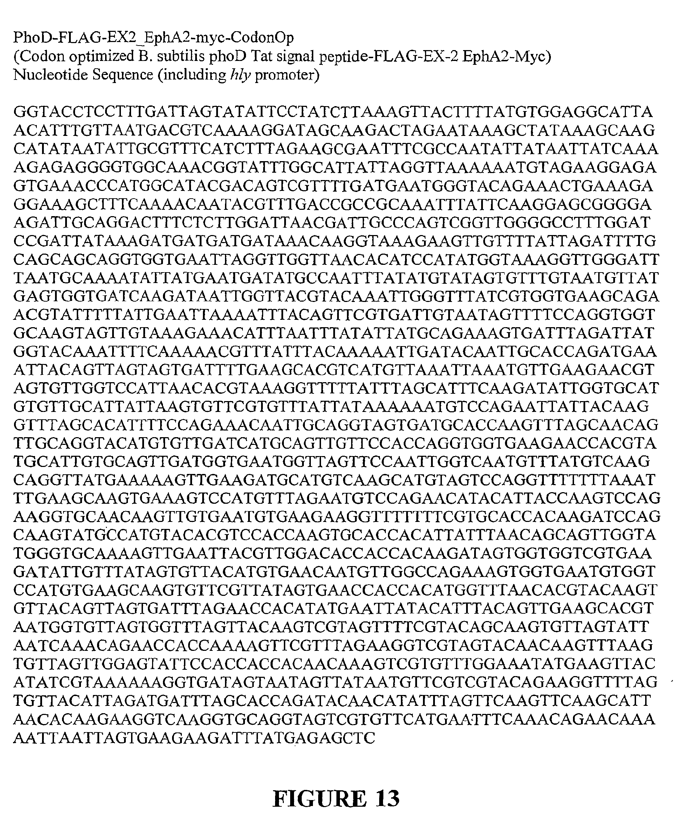

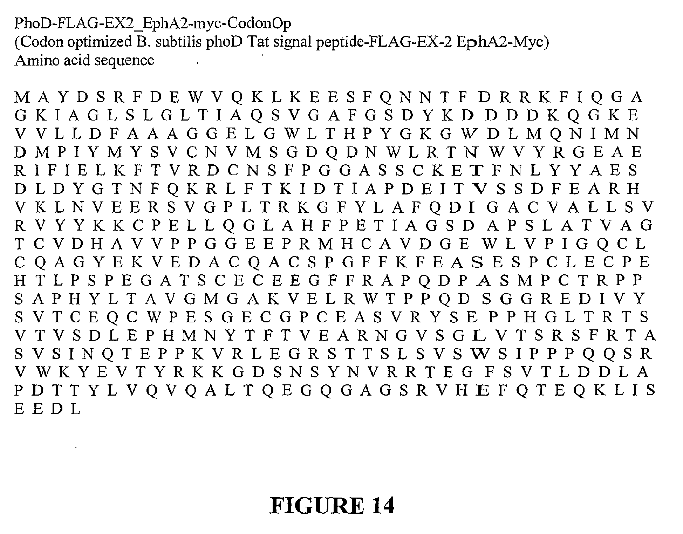

The sequence of an expression cassette encoding the extracellular domain of EphA2 antigen fused to a Tat signal peptide (*B. subtilis* phoD) where the sequences encoding the extracellular domain of EphA2 and the signal peptide are all codon-optimized for expression in *Listeria monocytogenes*, is shown in Figure 13. The amino acid sequence of the fusion protein encoded by this expression cassette is shown in Figure 14.

### Example 8. Codon-optimization of the intracellular domain of human EphA2 (CO)

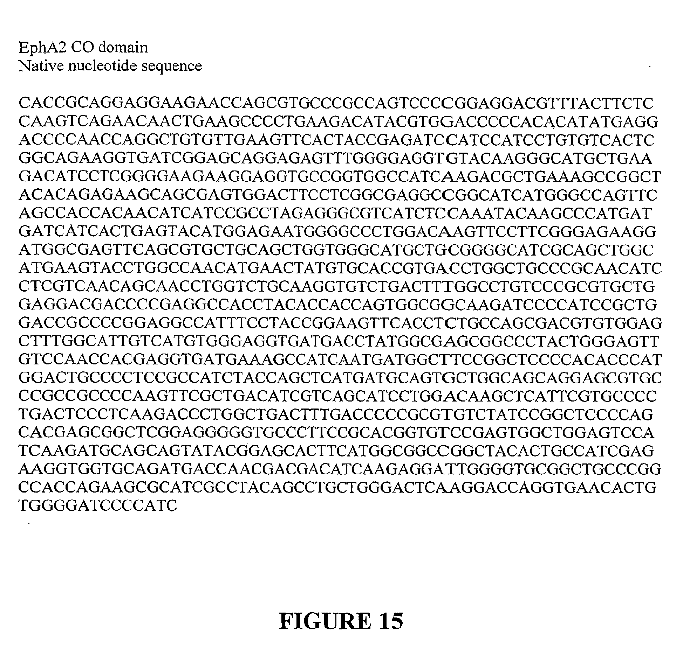

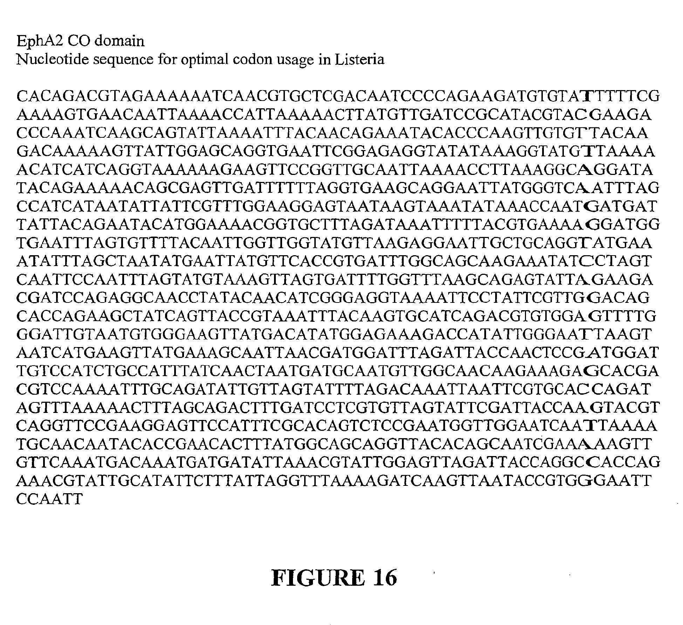

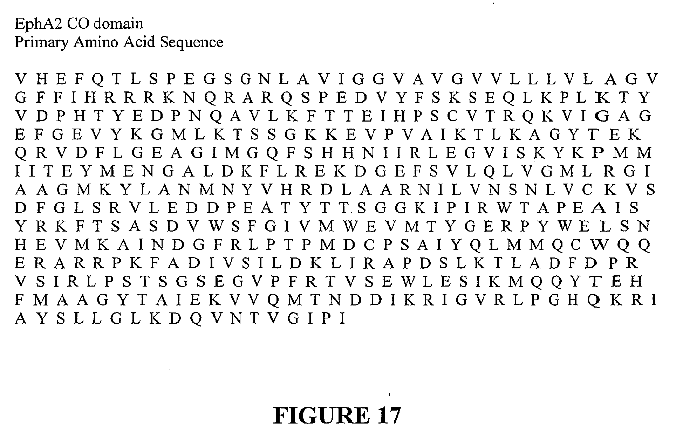

The sequence encoding the intracellular domain of human EphA2 (amino acids 558-975) has been codon-optimized for expression in *Listeria monocytogenes.* The native nucleotide sequence encoding the extracellular domain of human EphA2 is shown in Figure 15. The nucleotide sequence for optimal codon usage in *Listeria* is shown in Figure 16. The amino acid sequence of the extracellular domain of human EphA2 is shown in Figure 17.

### Example 9. Polynucleotides encoding fusion proteins comprising a secA1 signal peptide (LLO) and intracellular domain of huEphA2 (CO)

### A. Polynucleotide without codon-optimization

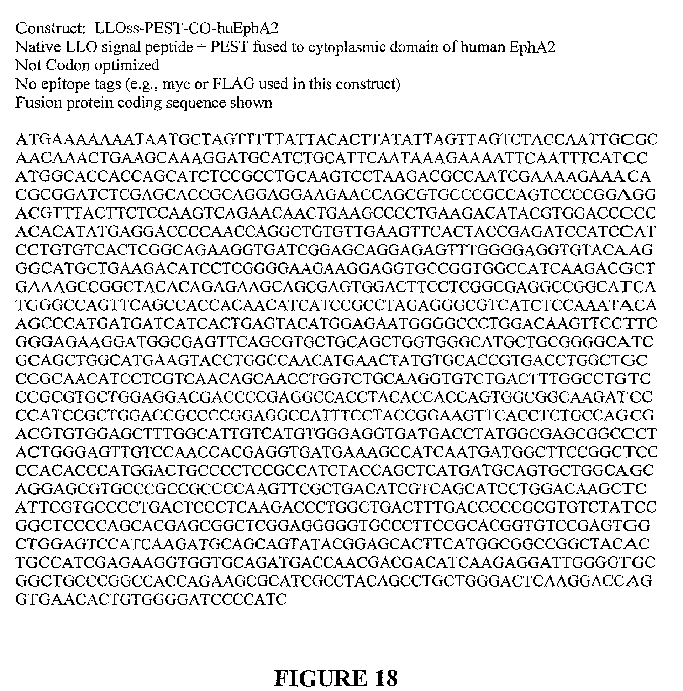

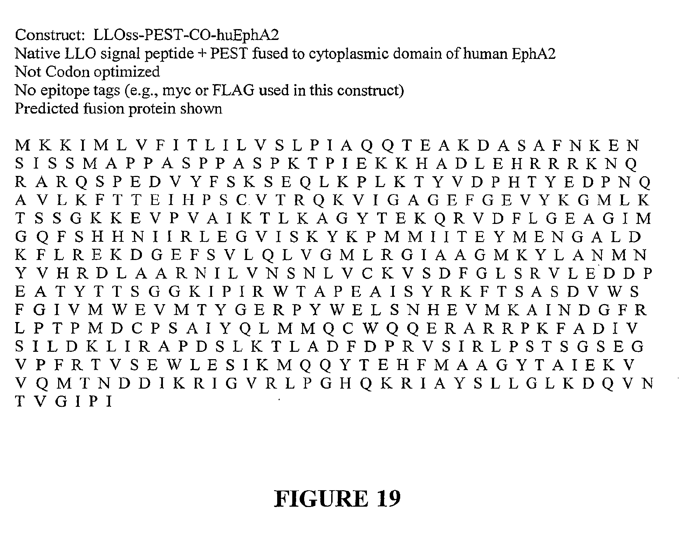

The sequence of a polynucleotide encoding the intracellular domain of human EphA2 antigen fused to a secA1 signal peptide (LLO), plus the LLO PEST sequence, is shown in Figure 18. The amino acid sequence of the fusion protein encoded by this expression cassette is shown in Figure 19.

### B. Expression cassette with codon-optimized intracellular domain of human EphA2

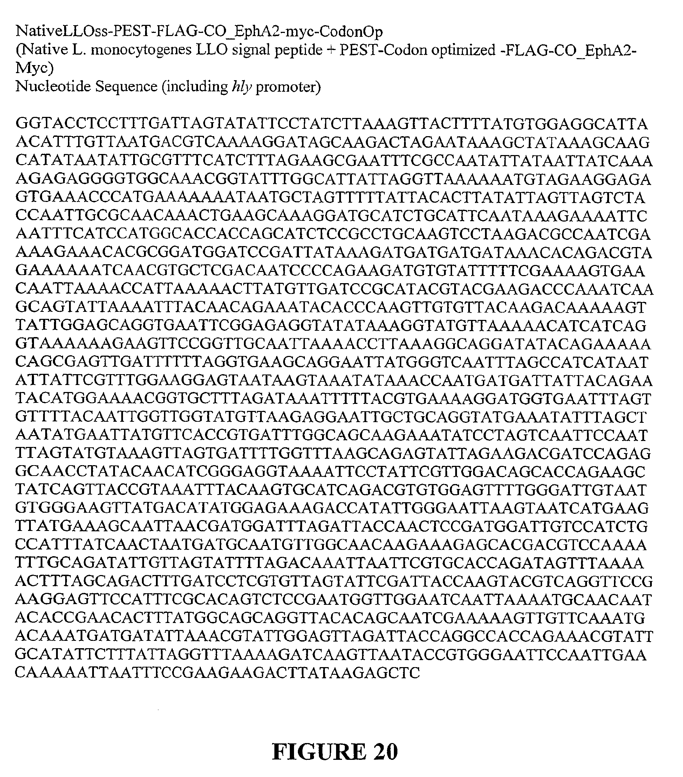

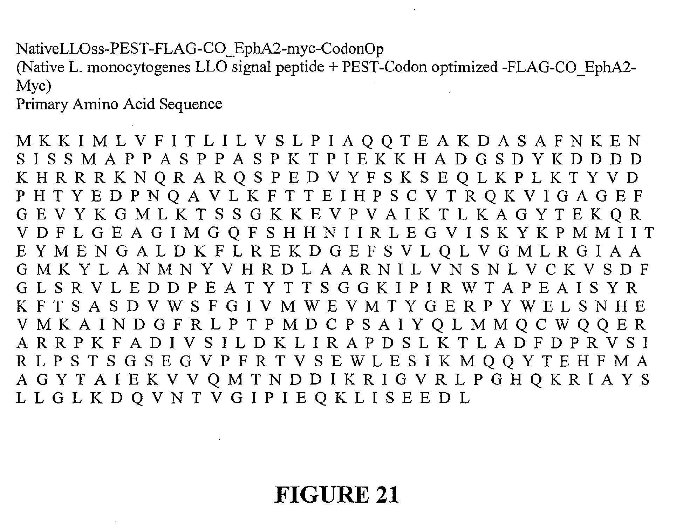

The sequence of an expression cassette encoding the intracellular domain of huEphA2 antigen fused to a secA1 signal peptide (LLO signal peptide), plus the LLO PEST sequence, in which the sequence encoding the intracellular domain of EphA2 is codon-optimized for expression in *Listeria monocytogenes*, is shown in Figure 20. The amino acid sequence of the fusion protein encoded by this expression cassette is shown in Figure 21.

### C. Expression cassette with codon-optimized secA1 signal peptide and codon-optimized intracellular domain of human EphA2

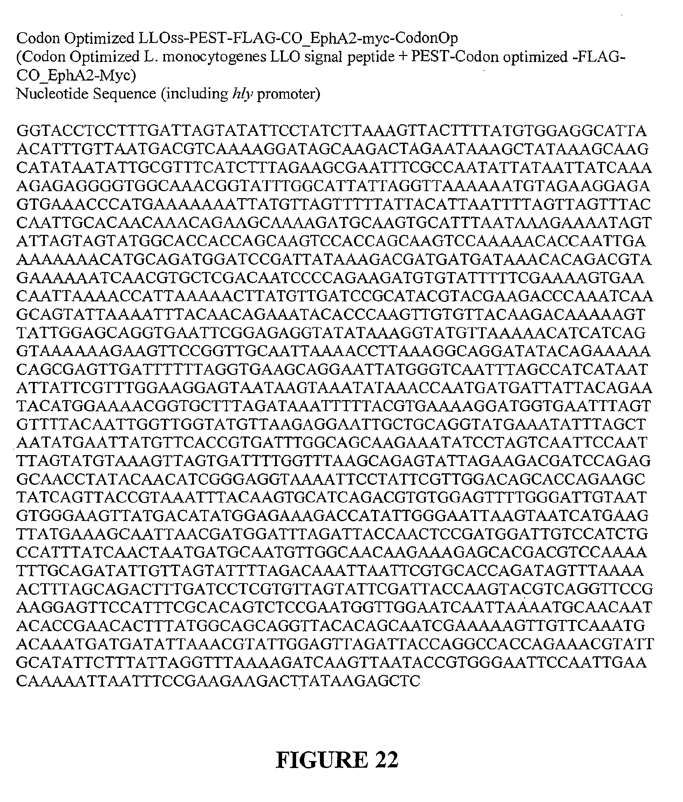

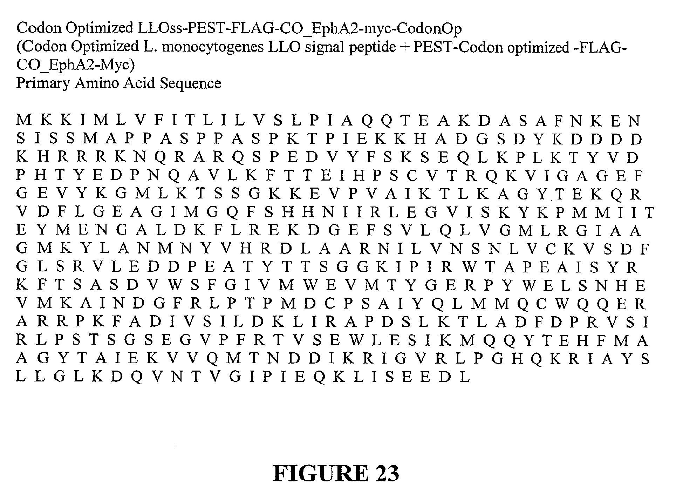

The sequence of an expression cassette encoding the intracellular domain of EphA2 antigen fused to a secA1 signal peptide (LLO signal peptide), plus the LLO PEST sequence, where the sequences encoding the intracellular domain of EphA2, signal peptide and PEST sequence are all codon-optimized for expression in *Listeria monocytogenes*, is shown in Figure 22. The amino acid sequence of the fusion protein encoded by this expression cassette is shown in Figure 23.

### Example 10. Codon-optimized expression cassette encoding a fusion protein comprising B. subtilis phoD signal peptide and intracellular domain of huEphA2 (CO)

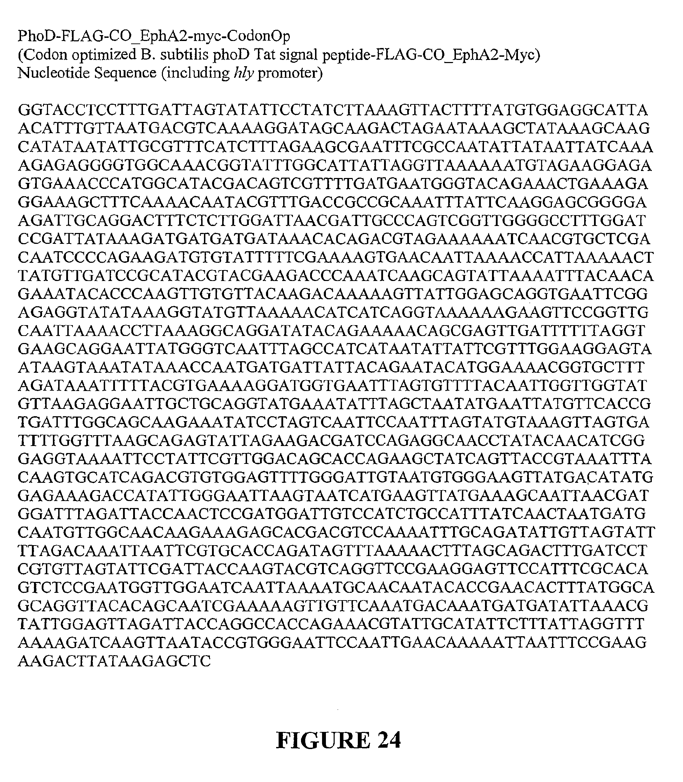

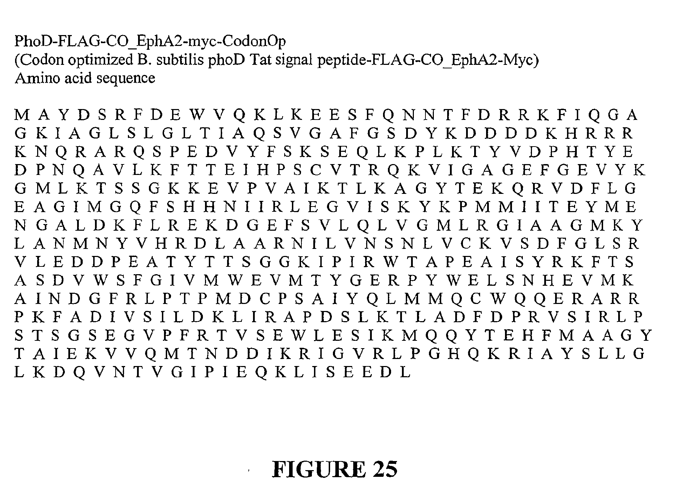

The sequence of an expression cassette encoding the intracellular domain of EphA2 antigen fused to a Tat signal peptide (*B. subtilis* phoD) where the sequences encoding the intracellular domain of EphA2 and the signal peptide are all codon-optimized for expression in *Listeria monocytogenes*, is shown in Figure 24. The amino acid sequence of the fusion protein encoded by this expression cassette is shown in Figure 25.

### Example 11. Codon-optimized expression cassette encoding a fusion protein comprising LLO signal peptide and NY-ESO-1

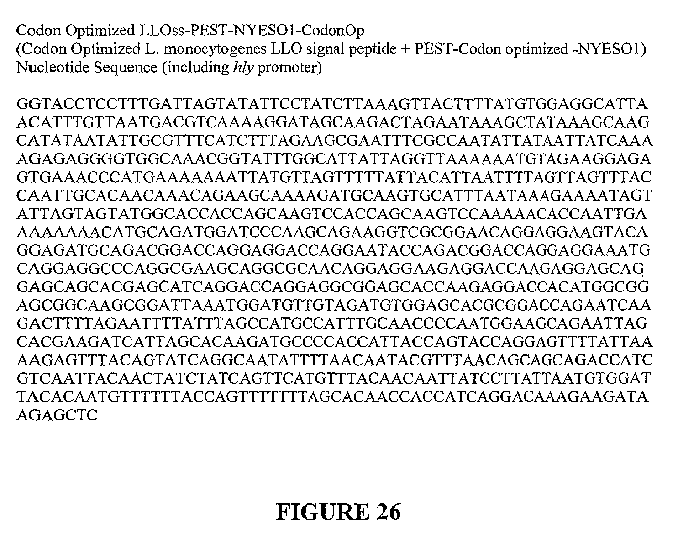

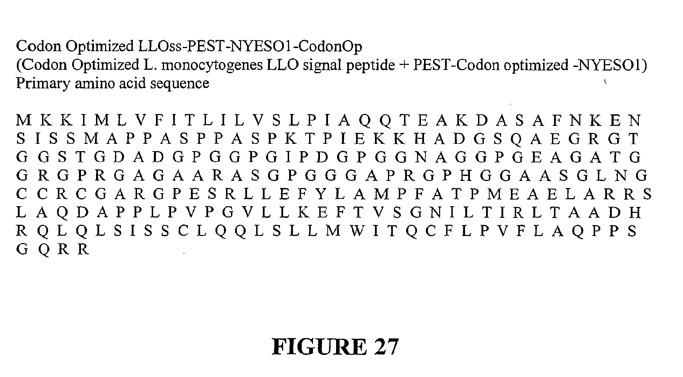

An expression cassette was designed for expression of the human testis cancer antigen NY-ESO-1 ((Genbank Accession No. NM_001327) in *Listeria monocytogenes.* The sequence of the expression cassette encoding the NY-ESO-1 fused to a secA1 signal peptide (LLO), plus the LLO PEST sequence, is shown in Figure 26. The sequences coding for the antigen as well as the signal peptide in the expression cassette were codon-optimized for expression in *Listeria monocytogenes.* The amino acid sequence of the fusion protein encoded by this expression cassette is shown in Figure 27.

### Example 12. Codon-optimized expression cassette for encoding antigens fused to a non-Listerial secA1 signal peptide (L. Lactis usp45)

An expression cassette was designed for expression of heterologous antigens in *Listeria monocytogenes* using a non-Listerial secA1 signal peptide. The amino acid sequence of the usp45 signal peptide from *Lactococcus lactis* (Steidler et al., Nature Biotechnology, 21:755-9 (2003)), its native coding sequence, and the coding sequence optimized for expression in *Listeria monocytogenes* is shown below.

Amino acid sequence:
MKKKIISAILMSTVILSAAAPLSGVYA'DT (SEQ ID NO:46)
Signal peptidase recognition site: VYA-DT (SEQ ID NO:55)

Native nucleotide sequence:

5'ATGAAAAAAAGATTATCTCAGCTATTTAATGTCTACAGTGATACTTTCTGCT
GCAGCCCCGTTGTCAGGTGTTTACGCTGACACA3' (SEQ ID NO:86)

Codons optimized for expression in *Listeria monocytogenes:*

5'ATGAAAAAAAAATTATTAGTGCAATTTTAATGAGTACAGTTATTTTAAGTGCA
GCAGCACCATTAAGTGGTGTTTATGCAGATACA3' (SEQ ID NO:87)

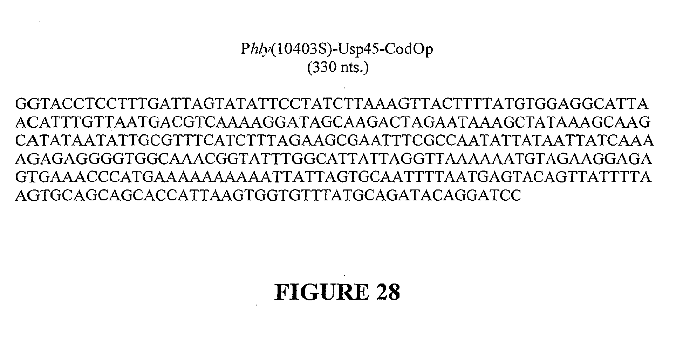

The sequence of a partial expression cassette comprising the *hly* promoter from *Listeria monocytogenes* operably linked to the codon-optimized sequence encoding the Usp45 signal peptide is shown in Figure 28. This sequence can be combined with either a codon-optimized or non-codon-optimized antigen sequence for expression of a fusion protein comprising the Usp45 signal peptide and the desired antigen.

### Example 13. Codon-optimized expression cassette and vector for encoding antigens fused to a secA2 signal peptide (p60)

### A. Design of codon-optimized expression cassette

An expression cassette was designed for expression of heterologous antigens in *Listeria monocytogenes* using the secA2 secretion pathway. The amino acid sequence of the p60 signal peptide from *Listeria monocytogenes,* its native coding sequence, and the coding sequence optimized for expression in *Listeria monocytogenes* is shown below.

Amino acid sequence:
MNMKKATIAATAGIAVTAFAAPTIASA'ST (SEQ ID NO:48)
Signal peptidase recognition site: ASA-ST (SEQ ID NO:57)

Native nucleotide sequence:

5'ATGAATATGAAAAAAGCAACTATCGCGGCTACAGCTGGGATTGCGGTAACAGC
ATTTGCTGCGCCAACAATCGCATCCGCAAGCACT3' (SEQ ID NO:90)

Codons optimized for expression in *Listeria monocytogenes:*

5'ATGAATATGAAAAAAGCAACAATTGCAGCAACAGCAGGTATTGCAGTTACAGC
ATTTGCAGCACCAACAATTGCAAGTGCAAGTACA3' (SEQ ID NO:91)

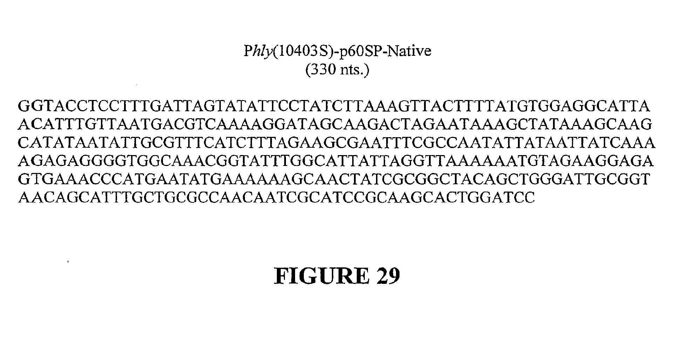

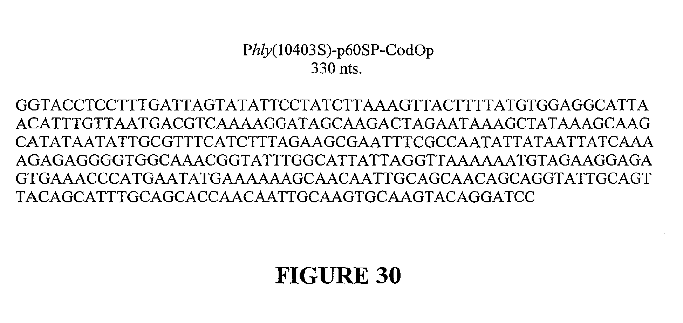

The sequence of a partial expression cassette comprising the *hly* promoter from *Listeria monocytogenes* operably linked to the native sequence encoding the p60 signal peptide is shown in Figure 29. The sequence of a partial expression cassette comprising the *hly* promoter from *Listeria monocytogenes* operably linked to the codon-optmized sequence encoding the p60 signal peptide is shown in Figure 30.

### B. Construction of pPL2-hhpro_p60.

An expression cassette can also be constructed in which the antigen-encoding sequence is inserted in frame in one or more sites within the coding sequence of the p60 gene. A description of the construction of a partial expression cassette useful for inserting antigen sequences in frame within the p60 sequence is described below. This partial expression cassette contains an *hly* promoter.

Individual primary PCR reactions using Pfx or Vent polymerase are performed using the following primers and pPL2-hlyP-OVA (identical to pPL2/LLO-OVA described above in Example 2A) as a first template:
pPL2-5F: 5'-GACGTCAATACGACTCACTATAG (SEQ ID NO:92)
p60-hlyP-237R: 5'-CTTTTTTCATATTCATGGGTTTCACTCTCCTTCTAC (SEQ ID NO:93)
The size of the resulting amplicon is 285 bps.

Individual primary PCR reactions using Pfx or Vent polymerase are also performed using the following primers and pCR-TOPO-p60 as a second template. (The vector pCR-TOPO-p60 is made from a pCR-TOPO vector obtained from Invitrogen, Carlsbad, California in which the *genomic p60* sequence from *Listeria monocytogenes* has been inserted. Any other of the many alternative sources of the p60 coding sequence that are available could be used as a template instead.) The primers used in this PCR reaction are as follows:
hlyP-p60-1F: 5'-AAGGAGAGTGAAACCCATGAATATGAAAAAAGCAAC (SEQ ID NO:88)
pCR-TOPO-2283R: 5'-GTGTGATGGATATCTGCAGAATTC (SEQ ID NO:89)
The size of the resulting amplicon is 1510 bps. The PCR reactions are then cleaned with S6 columns (Bio-Rad Laboratories, Hercules, California).

A secondary PCR reaction is then performed, using approximately 5 µl of each primary PCR reaction as template. The secondary PCR reaction uses the following primers:
*KpnI-*LLO 1257F (primer used previously):
   5'CTCTGGTACCTCCTTTGATTAGTATATTC (SEQ ID NO:74) and pCR-TOPO-2258R: 5'-CCCTTGGGGATCCTTAATTATACG (SEQ ID NO:83). The size of the resulting amplicon is 1715 bps. The expected amplicon sizes in all PCR reactions are verified by agarose gel analysis. The secondary PCR reaction is cleaned, digested with *BamHI,* cleaned again, and digested with *KpnI.* The hlyP-p60 gene fragment (KpnI-BamHI) (Figure 30) is then ligated between the *BamHI* and *KpnI* sites of both pPL2 and modified pAM401 (pAM401-MCS; Figure 32) plasmids.

The construction of pPL2-p60 plasmid is then confirmed with *BanaHI* /*KpnI* (1697, 6024 bps) and HindIII (210, 424, 3460, 3634 bps) digests. The *PstI* site in pPL2-p60 plasmid is also confirmed as unique. (Also, *KpnI*/ *PstI* digest will yield fragments of 736 and 6985 bps.) The construction of the pAM401-p60 plasmid (KpnI / PstI, and KpnI / BamHI fragments from p60 region is the same as that for the pPL2 construct.

Large prep isolations of each plasmid are then prepared using methods known to those of ordinary skill in the art.

The desired antigen-encoding sequences can then be inserted within the p60 sequence and in the same translational frame as the p60 sequence using techniques well known to those of ordinary skill in the art. Typically, the insertion or insertions should leave the N-terminal signal peptide sequence of p60 intact. The C-terminal autolysin sequence of p60 should also be left intact.

### Example 14. Codon-optimization of human mesothelin-encoding sequences for expression in Listeria monocytogenes

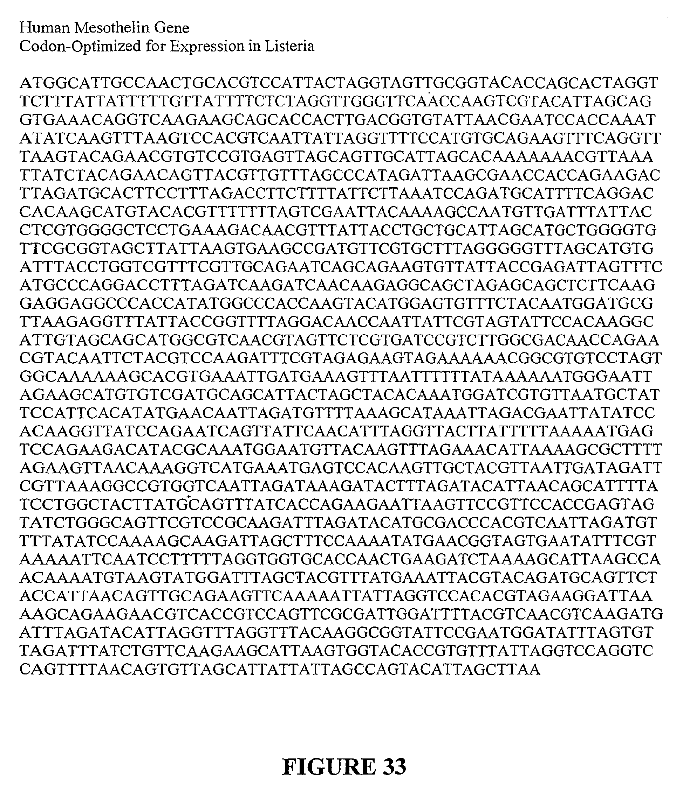

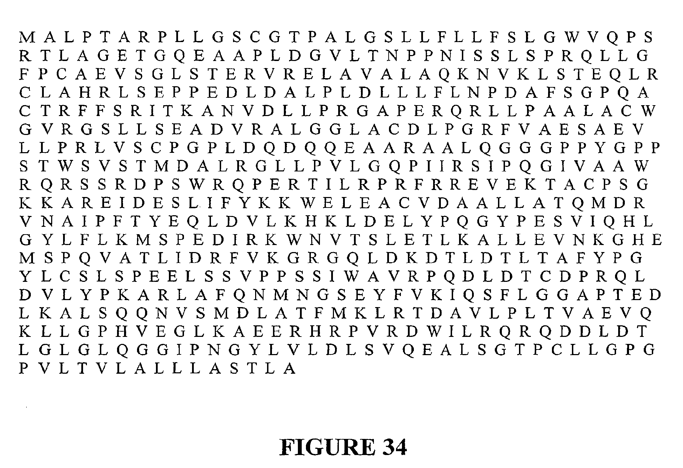

A codon-optimized polynucleotide sequence encoding human mesothelin, a cancer antigen, is shown in Figure 33. The sequence shown in Figure 32 has been codon-optimized for expression in *Listeria monocytogenes.* The polypeptide sequence encoded by the sequence in Figure 33 is shown in Figure 34.

### Example 15. Codon-optimization of murine mesothelin-encoding sequences for expression in Listeria monocytogenes

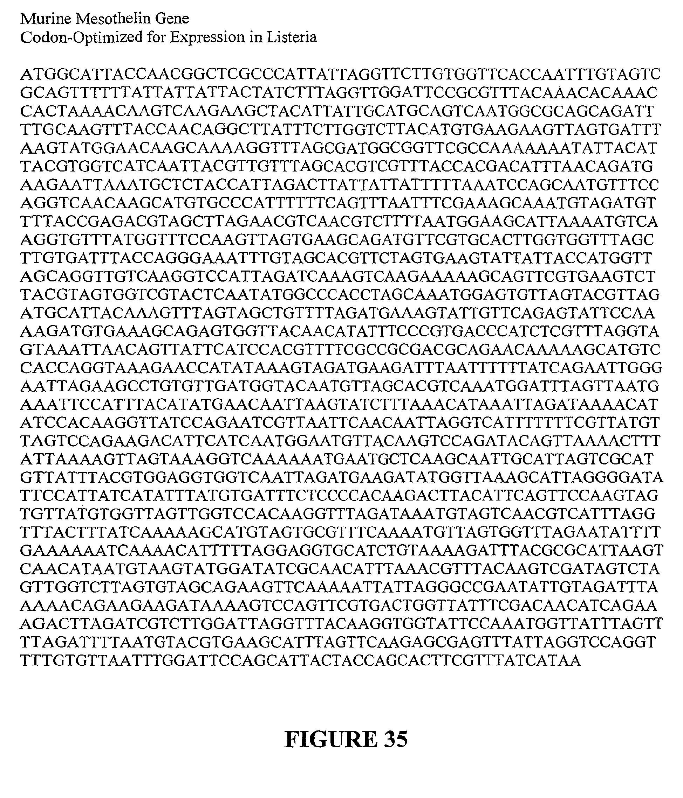

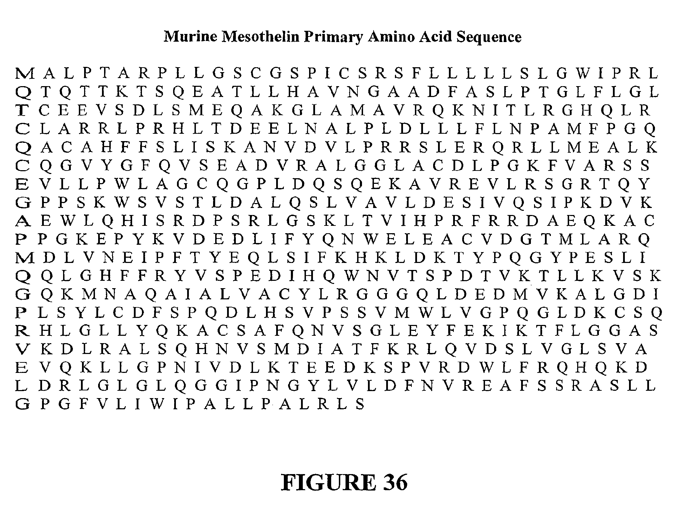

A codon-optimized polynucleotide sequence encoding human mesothelin, a cancer antigen, is shown in Figure 35. The sequence shown in Figure 35 has been codon-optimized for expression in *Listeria naonocytogenes.* The polypeptide sequence encoded by the sequence in Figure 35 is shown in Figure 36.

### Example 16. Integration of an expression cassette into the Listeria chromosome via allelic exchange

As one possible alternative to using an integration vector such as pPL2 to insert the heterologous gene expression cassette into the chromosome of *Listeria,* allelic exchange may be used.

Briefly, bacteria electroporated with the pKSV7-heterologous protein expression cassette plasmid are selected by plating on BHI agar media containing chloramphenicol (10 µg/ml), and incubated at the permissive temperature of 30°C. Single cross-over integration into the bacterial chromosome is selected by passaging several individual colonies for multiple generations at the non-permissive temperature of 41 °C in media containing chloramphenicol. Finally, plasmid excision and curing (double cross-over) is achieved by passaging several individual colonies for multiple generations at the permissive temperature of 30°C in BHI media not containing chloramphenicol. Verification of integration of the heterologous protein expression cassette into the bacteria chromosome is verified by PCR, utilizing a primer pair that amplifies a region defined from within the heterologous protein expression cassette to the bacterial chromosome targeting sequence not contained in the pKSV7 plasmid vector construct.

### Example 17. Cloning and insertion of EphA2 into pPL2 vectors for expression in selected recombinant Listeria monocytogenes strains.

The external (EX2) and cytoplasmic (CO) domains of EphA2 which flank the EphA2 transmembrane helix were cloned separately for insertion into various pPL2-signal peptide expression constructs. Genes corresponding to the native manunalian sequence or codon-optimized for expression in *Listeria monocytogenes* of EphA2 EX2 and CO domains were used. The optimal codons in *Listeria* (see Table 3, above) for each of the 20 amino acids were utilized for codon-optimized EphA2 EX2 and EphA2 CO. The codon-optimized EphA2 EX2 and CO domains were synthesized by extension of overlapping oligonucleotides, using techniques common to those skilled in the art. The expected sequence of all synthesized EphA2 constructs was verified by nucleotide sequencing.

The primary amino acid sequences, together with the native and codon-optimized nucleotide sequences for the EX2 and CO domains of EphA2 are shown in Figures 4-6 (EX2 sequences) and Figures 15-17 (CO domain sequences)

Additonally, FLAG (Stratagene, La Jolla, CA) and myc epitope tags were inserted, respectively, in-frame at the amino and carboxy termini of synthesized EphA2 EX2 and CO genes for detection of expressed and secreted EphA2 by Western blot analysis using antibodies specific for the FLAG or proteins. Thus, the expressed protein had the following ordered elements: NH₂-Signal Peptide-FLAG-EphA2-myc-CO₂. Shown below are the FLAG and myc epitope tag amino acid and codon-optimized nucleotide sequences:
FLAG:
   5'-GATTATAAAGATGATGATGATAAA (SEQ ID NO:96) NH₂-DYKDDDDK-CO₂ (SEQ ID NO:97)
Myc:
   5'-GAAGA14AAATTAATTAGTGAAGAAGATTTA (SEQ ID NO:98) NH₂-EQKLISEEDL-CO₂(SEQ ID NO:99)

### Example 18. Detection of synthesized and secreted heterologous proteins by Western blot analysis.

Synthesis of EphA2 protein and secretion from various selected recombinant *Listeria-*EphA2 strains was determined by Western blot analysis of trichloroacetic acid (TCA) precipitated bacterial culture fluids. Briefly, mid-log phase cultures of *Listeria* grown in BHI media were collected in a 50 mL conical centrifuge tube, the bacteria were pelleted, and ice-cold TCA was added to a final [6%] concentration to the bacterial culture supernatant and incubated on ice minimally for 90 min or overnight. The TCA-precipitated proteins were collected by centrifugation at 2400 X g for 20 min at 4°C. The pellet was then resuspended in 300-600 µl volume of TE, pH 8.0 containing 15 µg/ml phenol red. Sample dissolution was facilitated by vortexing. Sample pH was adjusted by NH₄OH addition if necessary until color was pink. All samples were prepared for electrophoresis by addition of 100 µl of 4X SDS loading buffer and incubating for 10 min. at 90°C. The samples were then centrifuged from 5 min at 14,000 rpm in a micro-centrifuge, and the supernatants collected and stored at -20°C. For Western bolt analysis, 20 µl of prepared fractions (the equivalent of culture-fluids from of 1-4 x 10⁹ bacteria), were loaded on the 4-12% SDS-PAGE gel, electrophoresed, and the proteins were transferred to PDDF membrane, according to common methods used by those skilled in the art. Transferred membranes were prepared s for incubation with antibody, by incubating in 5% dry milk in PBS for 2 hr. at room temperature with agitation. Antibodies were used under the following dilutions in PBST buffer (0.1% Tween 20 in PBS): (1) Rabbit anti-Myc polyclonal antibody (ICL laboratories, Newberg, Oregon) at 1:10,000; (2) murine anti-FLAG monoclonal antibody (Stratagene, La Jolla, CA) at 1:2,000; and, (3) Rabbit anti-EphA2 (carboxy terminus-specific) polyclonal antibody (sc-924, Santa Cruz Biotechnology, Inc., Santa Cruz, CA). Specific binding of antibody to protein targets was evaluated by secondary incubation with goat anti-rabbit or anti-mouse antibody conjugated with horseradish peroxidase and detection with the ECL chemilumenescence assay kit (Amersham), and exposure to film.

### Example 19. Secretion of EphA2 protein by recombinant Listeria encoding various forms of EphA₂.

### A. Listeria: [strains DP-L4029 (actA) or DP-L4017 (LLO L461T)] Expression cassette construct: LLOss-PEST-CO-EphA2

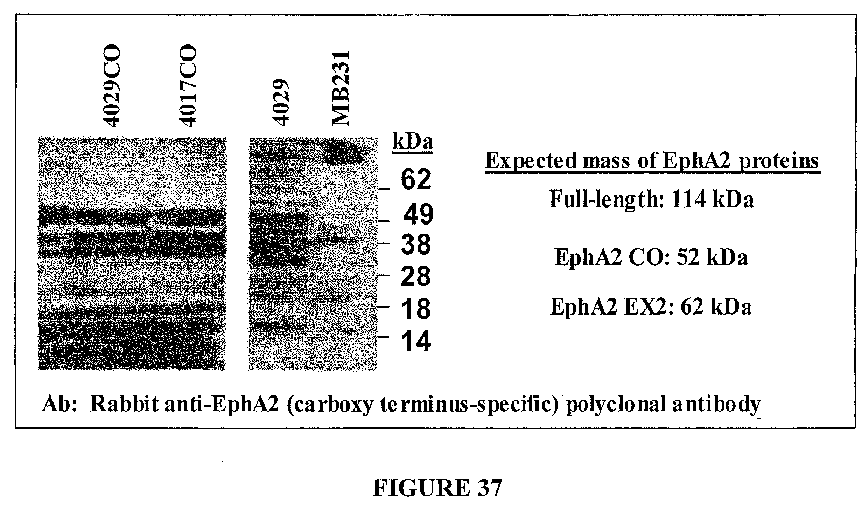

The native sequence of the EphA2 CO domain was genetically fused to the native secA1 LLO sequence, and the heterologous antigen expression cassette under control of the *Listeria hly* promoter was inserted into the pPL2 plasmid between the *Kpn I* and *Sac I* sites as described above. The pPL2-EphA2 plasmid constructs were introduced by conjugation into the *Listeria* strains DP-L4029 (actA⁻) and DP-L4017 (L461T LLO) as described above. Figure 37 shows the results of a Western blot analysis of TCA-precipitated bacterial culture fluids of 4029-EphA2 CO and 4017-EphA2 CO. This analysis demonstrated that recombinant *Listeria* engineered to contain a heterologous protein expression cassette comprised of native sequences corresponding to the secA1 and EphA2 CO fusion protein secreted multiple EphA2-specific fragments that were lower than the 52 kDa expected molecular weight, demonstrating the need for modification of the expression cassette.

### B. Listeria: [DP-L4029 (actA⁻)]

*Expression cassette constructs:*
*1. Native LLOss-PEST-FLAG-EX2_EphA2-myc-CodonOp*
*2. (CodonOp) LLOss-PEST-(CodonOp)FLAG-EX2 EpIxA2-myc*

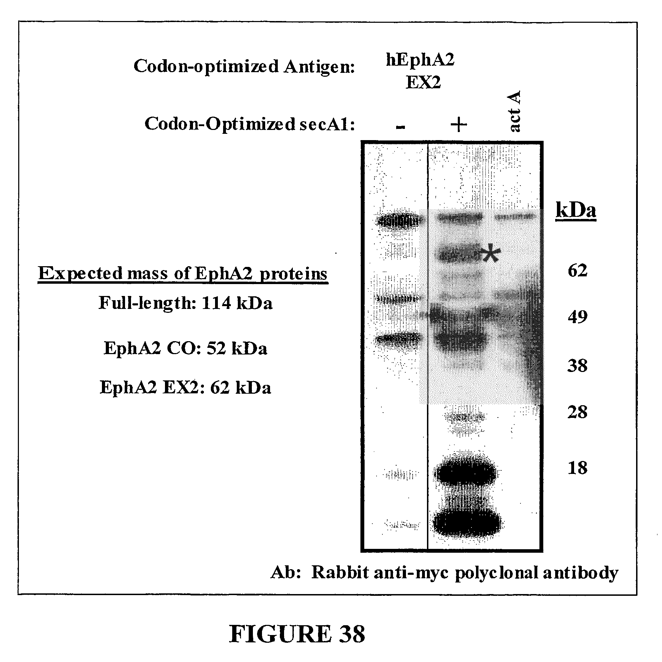

The native secA1 LLO signal peptide sequence or secA1 LLO signal peptide sequence codon-optimized for expression in *Listeria* was fused genetically with the EphA2 EX2 domain sequence codon-optimized for expression in *Listeria,* and the heterologous antigen expression cassette under control of the *Listeria hly* promoter was inserted into the pPL2 plasmid between the *Kin I* and *Sac I* sites as described above. The pPL2-EphA2 plasmid constructs were introduced by conjugation into the *Listeria* strain DP-L4029 (actA) as described above. Figure 38 shows the results of a Western blot analysis of TCA-precipitated bacterial culture fluids of *Listeria* actA encoding either the native or codon-optimized secA1 LLO signal peptide fused with the codon-optimized EphA2 EX2 domain. This analysis demonstrated that the combination of utilizing sequence for both signal peptide and heterologous protein optimized for the preferred codon usage in *Listeria monocytogenes* resulted in expression of the expected full-length EphA2 EX2 domain protein. Expression of full-length EphA2 EX2 domain protein was poor with codon-optimization of the EphA2 coding sequence alone. The level of heterologous protein expression (fragmented or full-length) was highest when utilizing the *Listeria monocytogenes* LLO secA1 signal peptide, codon-optimized for expression in *Listeria monocytogenes.*

### C. Listeria: [DP-L4029 (actA)]

*Expression cassette constructs:*
*3. Native LLOss-PEST-(CodonOp) FLAG*-*EphA2*_*CO*-*myc*
*4. CodonOp LLOss-PEST-(CodonOp) FLAG- EphA2*_*CO*-*myc*
*5. CodonOp PhoD-(CodonOp) FLAG- EphA2_CO-myc*

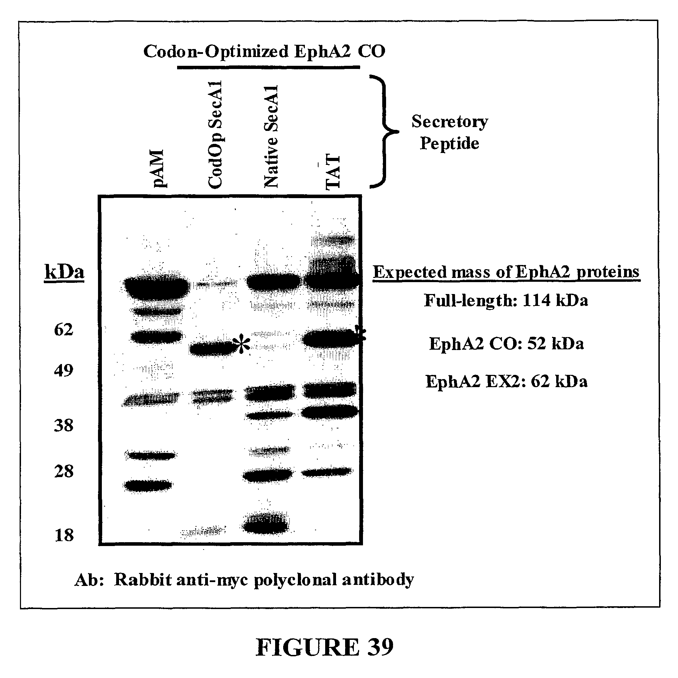

The native secA1 LLO signal peptide sequence or the secA1 LLO signal peptide sequence codon-optimized for expression in *Listeria,* or, alternatively, the Tat signal peptide of the phoD gene from *Bacillus subtilis* codon-optimized for expression in *Listeria,* was fused genetically with the EphA2 CO domain sequence codon-optimized for expression in *Listeria,* and the heterologous antigen expression cassette under control of the *Listeria hly* promoter was inserted into the pAM401-MCS plasmid between the *Kpn I and Sac I* sites as described above. The pAM401-EphA2 plasmid constructs were introduced by electroporation into the *Listeria* strain DP-L4029 (actA) as described above. Figure 39 shows the results of a Western blot analysis of TCA-precipitated bacterial culture fluids of *Listeria,* actA encoding either the native or codon-optimized secA1 LLO signal peptide, or codon-optimized *Bacillus subtilis phoD* Tat signal peptide fused with the codon-optimized EphA2 CO domain. This analysis demonstrated once again that the combination of utilizing sequence for both signal peptide and heterologous protein optimized for the preferred codon usage in *Listeria monocytogenes* resulted in expression of the expected full-length EphA2 CO domain protein. Furthermore, expression and secretion of the expected full-length EphA2 CO domain protein resulted from recombinant *Listeria* encoding codon-optimized *Bacillus subtilis phoD* Tat signal peptide fused with the codon-optimized EphA2 CO domain. This result demonstrates the novel and unexpected finding that signal peptides from distinct bacterial species can be utilized to program the secretion of heterologous proteins from recombinant *Listeria.* Expression of full-length EphA2 CO domain protein was poor with codon-optimization of just the EphA2 sequence. The level of heterologous protein expression was highest when utilizing signal peptides codon-optimized for expression in *Listeria monocytogenes.*

### D. Transfection of 293 cells with pCDNA4 plasmids encoding full-length EphA2 Expression cassette constructs:

### 6. pCDNA4-EphA2

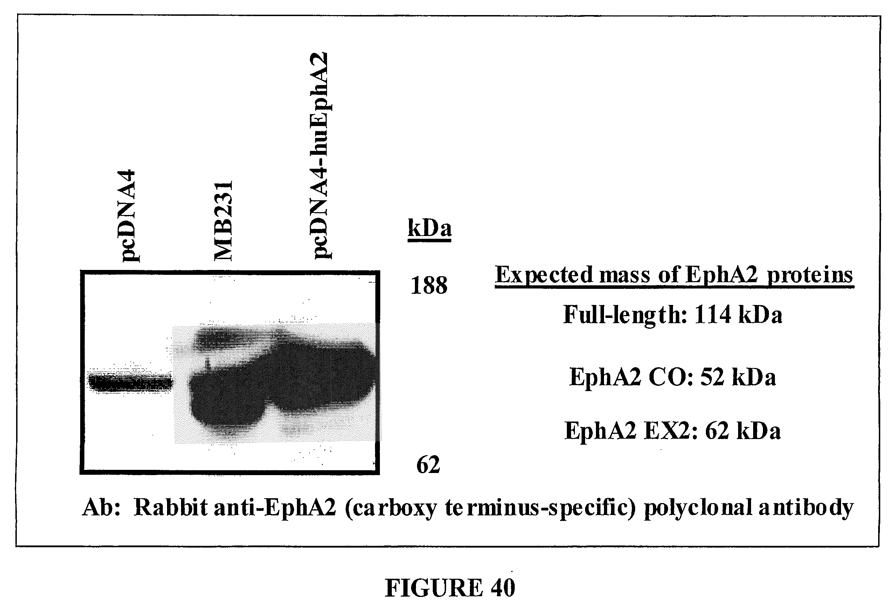

The native full-length EphA2 gene was cloned into the eukaryotic CMV promoter-based expression plasmid pCDNA4 (Invitrogen, Carlsbad, CA). Figure 40 shows the results of a Western blot analysis of lysates prepared from 293 cells transfected with the pCDNA4-EphA2 plasmid, and demonstrates the abundant expression in mammalian cells of full-length EphA2 protein.

### Example 20. Therapeutic efficacy in Balb/C mice bearing CT26 tumors encoding human EphA2 immunized with recombinant Listeria encoding codon-optimized EphA2.

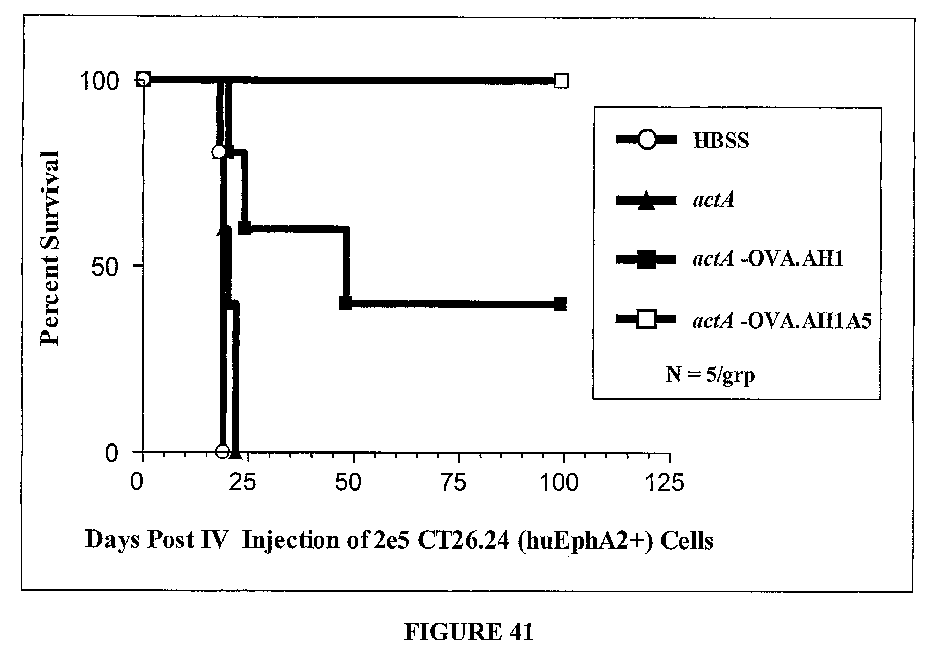

The following data presented in Figures 41-44 demonstrated the following:

Immunization of Balb/C mice bearing CT26.24 (huEphA2+) lung tumors with recombinant *Listeria* encoding OVA.AH1 (MMTV gp70 immunodominant epitope) or OVA.AH1-A5 (MMTV gp70 immunodominant epitope, with heteroclitic change for enhanced T-cell receptor binding) confers long-term survival (Figure 41).

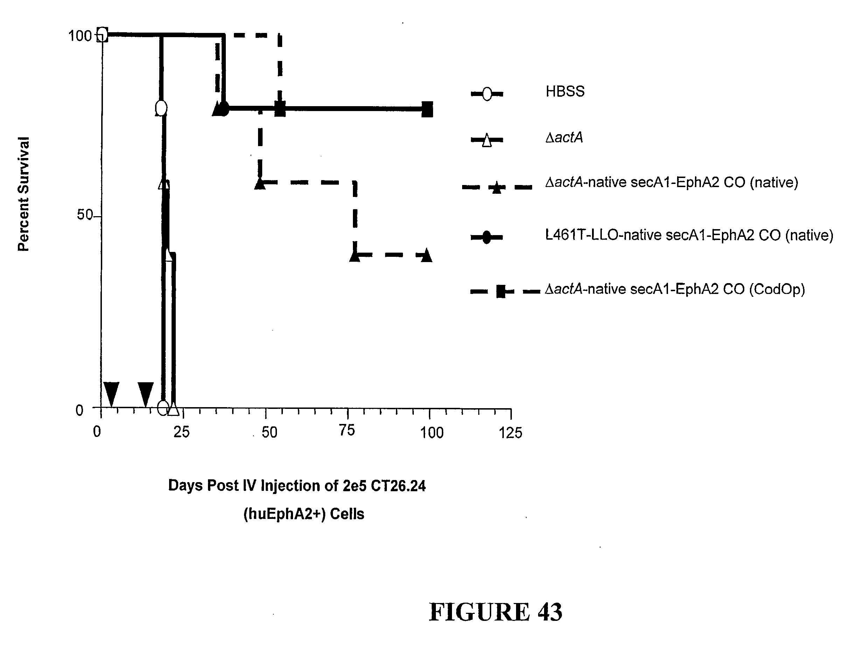

The EphA2 CO domain is strongly immunogenic, and a significant long term increase in survival of Balb/C mice bearing CT26.24 (huEphA2+) lung tumors was observed when immunized with recombinant *Listeria* encoding codon-optimized or native EphA2 CO domain sequence (Figure 43).

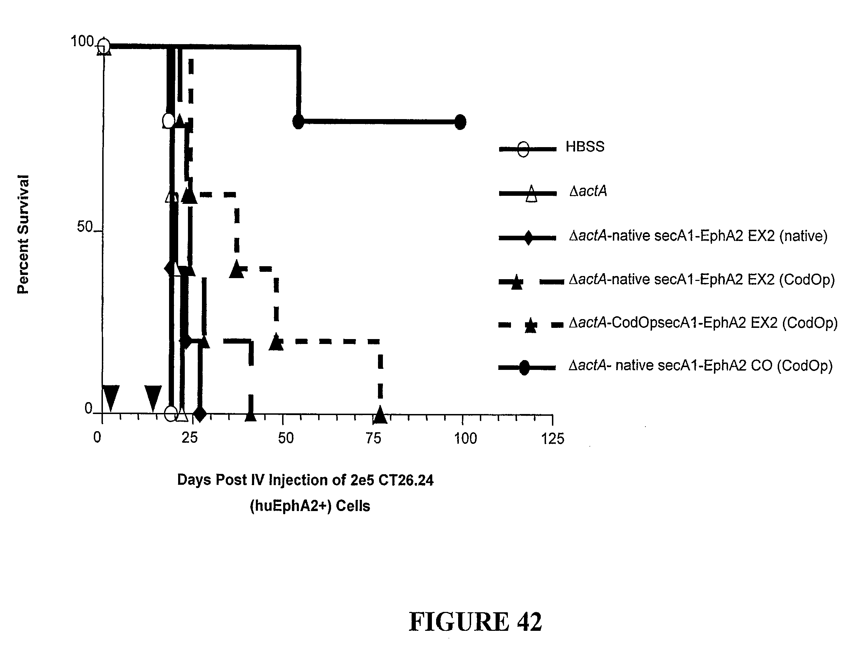

The EphA2 EX2 domain is poorly immunogenic, and increased survival of Balb/C mice bearing CT26.24 (huEphA2+) lung tumors was observed only when immunized with recombinant *Listeria* encoding codon-optimized secA1 signal peptide fused with the codon-optimized EphA2 EX2 domain sequence. Therapeutic efficacy was not observed in mice when immunized with recombinant *Listeria* encoding native secA1 signal peptide fused with the codon-optimized EphA2 EX2 domain sequence (Figure 42). The desirability of using both codon-optimized secA1 signal peptide and EphA2 EX2 domain sequences was supported by statistically significant therapeutic anti-tumor efficacy, as shown in Table 4, below.

**Table 4. Comparison by log-rank test of survival curves shown in Figure 42.**

| Experimental Group | Median Survival (Days) | Significance versus HBSS cohort (p value) | Significance versus actA-native secAl/EphA2 EX2 cohort (p value) |
|---|---|---|---|
| HBSS | **19** | - | - |
| *actA* | **20** | **NS** | **NS** |
| *actA*-native secA1-EphA2 EX2 (native) | **19** | **NS** | **-** |
| *actA*-native secA1-EphA2 EX2 (CodOp) | **24** | **0.0035** | **NS** |
| *actA-*CodOp secA1-EphA2 EX2 (CodOp) | **37** | **0.0035** | **0.0162** |
| *actA*-native secAl-EphA2 CO (CodOp) | **>99** | **0.0035** | **0.0015** |

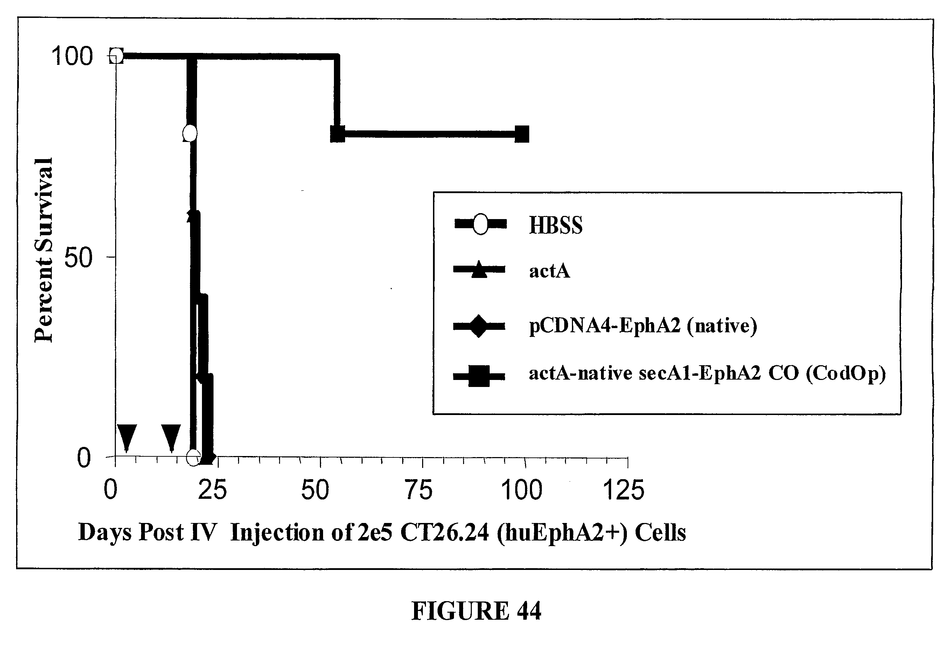

Significantly, even though pCDNA4-EphA2 plasmid transfected 293 cells yielded very high levels of protein expression, immunization of Balb/C mice bearing CT26.24 (huEphA2+) lung tumors with the pCDNA4-EphA2 plasmid did not result in any observance of therapeutic anti-tumor efficacy (Figure 44).

For therapeutic *in vivo* tumor studies, female Balb/C mice were implanted IV with 5 x 10⁵ CT26 cells stably expressing EphA2. Three days later, mice were randomized and vaccinated IV with various recombinant *Listeria* strains encoding EphA2. In some cases (noted in figures) mice were vaccinated with 100 µg of pCDNA4 plasmid or pCDNA4-EphA2 plasmid in the *tibialis* anterior muscle. As a positive control, mice were vaccinated IV with recombinant *Listeria* strains encoding OVA.AHI or OVA.AH1-A5 protein chimeras. Mice were vaccinated on days 3 and 14 following tumor cell implantation. Mice injected with Hanks Balanced Salt Solution (HBSS) buffer or unmodified *Listeria* served as negative controls. All experimental cohorts contained 5 mice. For survival studies mice were sacrificed when they started to show any signs of stress or labored breathing.

### Example 21. Assessment of antigen-specific immune responses after vaccination.

The vaccines of the present invention can be assessed using a variety of *in vitro* and *in vivo* methods. Some assays involve the analysis of antigen-specific T cells from the spleens of mice that have been vaccinated. Provided in this example are non-limiting examples of methods of assessing *in vitro* and *in vivo* immune responses. The antigens recited in these exemplary descriptions of assays are model antigens, not necessarily antigens produced using the recombinant nucleic acid molecules, expression cassettes, and/or expression vectors described herein. One of ordinary skill in the art will readily recognize that the assays described in this example can readily be applied for use in assessing the *in vitro* or *in vivo* immune responses of bacteria comprising the recombinant nucleic acid molecules, expression cassettes, and/or expression vectors described herein.

For example C57B1/6 or Balb/c are vaccinated by intravenous injection of 0.1 LD₅₀ of a *Listeria* strain expressing OVA (or other appropriate antigen). Seven days after the vaccination, the spleen cells of the mice are harvested (typically 3 mice per group) by placing the spleens into ice cooled RPMI 1640 medium and preparing a single cell suspension from this. As an alternative, the lymph nodes of the mice could be similarly harvested, prepared as a single cell suspension and substituted for the spleen cells in the assays described below. Typically, spleen cells are assessed for intravenous or intraperitoneal administration of the vaccine while spleen cells and cells from lymph nodes are assessed for intramuscular, subcutaneous or intradermal administration of the vaccine.

Unless otherwise noted, all antibodies used in these examples can be obtained from Pharmingen, San Diego, CA.

*ELISPOT Assay:* Using a *Listeria* strain having an OVA antigen as an example, the quantitative frequency of antigen-specific T cells generated upon immunization in a mouse model is assessed using an ELISPOT assay. The antigen-specific T cells evaluated are OVA specific CD8+ or LLO specific CD8+ or CD4+ T cells. This OVA antigen model assesses the immune response to a heterologous tumor antigen inserted into the vaccine and could be substituted with any antigen of interest. The LLO antigen is specific to *Listeria.* The specific T cells are assessed by detection of cytokine release (e.g. IFN-γ) upon recognition of the specific antigen. PVDF-based 96 well plates (BD Biosciences, San Jose, CA) are coated overnight at 4°C with an anti-murine IFN-γ monoclonal antibody (mAb R4; 5 µg/ml). The plates are washed and blocked for 2 hours at room temperature with 200 µL of complete RPMI. Spleen cells from vaccinated mice (or non vaccinated control mice) are added at 2 x 10⁵ cells per well and incubated for 20 to 22 hours at 37°C in the presence of various concentrations of peptides ranging from 0.01 to 10 µM. The peptides used for OVA and LLO are either SL8, an MHC class I epitope for OVA, LLO₁₉₀ (NEKYAQAYPNVS (SEQ ID NO:100) Invitrogen) an MHC class II epitope for listeriolysin O (*Listeria* antigen), LLO₂₉₆ (VAYGRQVYL (SEQ ID NO:101) an MHC class I epitope for listeriolysin O, or LLO₉₁ (GYKDGNEYI (SEQ ID NO:102)), an MHC class I epitope for listeriolysin O. LLO₁₉₀ and LLO₂₉₆ are used in a C57B1/6 model, while LLO₉₁ is used in a Balb/c model. After washing, the plates are incubated with secondary biotinylated antibodies specific for IFN-γ (XMG1.2) diluted in PBS to 0.5µg/ml. After incubation at room temperature for 2 hours, the plates are washed and incubated for 1 hour at 3 7 °C with a 1 nm gold goat anti-biotin conjugate (GAB-1; 1:200 dilution; Ted Pella, Redding, CA) diluted in PBS containing 1 % BSA. After thorough washing, the plates are incubated at room temperature for 2 to 10 minutes with substrate (Silver Enhancing Kit; 30 ml/well; Ted Pella) for spot development. The plates are then rinsed with distilled water to stop the substrate reaction. After the plates have been air-dried, spots in each well are counted using an automated ELISPOT plate reader (CTL, Cleveland, OH). The cytokine response is expressed as the number of IFN-γ spot-forming cells (SFCs) per 2 x 10⁵ spleen cells for either the OVA specific T cells or the *Listeria* specific T cells

*Intracellular Cytokine Staining Assay (ICS):* In order to further assess the number of antigen-specific CD8+ or CD4+ T cells and correlate the results with those obtained from ELISPOT assays, ICS is performed and the cells evaluated by flow cytometry analysis. Spleen cells from vaccinated and control groups of mice are incubated with SL8 (stimulates OVA specific CD8+ cells) or LLO₁₉₀ (stimulates LLO specific CD4+ cells) for 5 hours in the presence of Brefeldin A (Pharmingen). The Brefeldin A inhibits secretion of the cytokines produced upon stimulation of the T cells. Spleen cells incubated with an irrelevant MHC class I peptide are used as controls. PMA (phorbol-12-myristate-13-acetate, Sigma) 20 ng/ml and ionomycin (Sigma) 2 µg/ml stimulated spleen cells are used as a positive control for IFN-γ and TNF-α intracellular cytokine staining. For detection of cytoplasmic cytokine expression, cells are stained with FITC-anti-CD4 mAb (RM 4-5) and PerCP-anti-CD8 mAb (53-6.7), fixed and permeabilized with Cytofix/CytoPerm solution (Pharmingen), and stained with PE-conjugated anti-TNF-α mAb (MP6-XT22) and APC-conjugated anti-IFN-γ mAb (XMG1.2) for 30 minutes on ice. The percentage of cells expressing intracellular IFN-γ and/or TNF-α was determined by flow cytometry (FACScalibur, Becton Dickinson, Mountain View, CA) and data analyzed using CELLQuest software (Becton Dickinson Immunocytometry System). As the fluorescent labels on the various antibodies can all be distinguished by the FACScalibur, the appropriate cells are identified by gating for those CD8+ and CD4+ that are stained with either or both of the anti-IFN-γ or anti-TNF-α.

*Cytokine Expression of Stimulated Spleen Cells:* The level of cytokine secretion by the spleen cells of mice can also be assessed for control and vaccinated C57B1/6 mice. Spleen cells are stimulated for 24 hours with SL8 or LLO₁₉₀. Stimulation with irrelevant peptide HSV-gB2 (Invitrogen, SSIEFARL, SEQ ID NO:4) is used as a control. The supernatants of the stimulated cells are collected and the levels of T helper-1 and T helper 2 cytokines are determined using an ELISA assay (eBiosciences, CO) or a Cytometric Bead Array Kit (Pharmingen).

*Assessment of Cytotoxic T cell Activity:* The OVA specific CD8+ T cells can be further evaluated by assessing their cytotoxic activity, either *in vitro* or directly in C57B1/6 mouse *in vivo.* The CD8+ T cells recognize and lyse their respective target cells in an antigen-specific manner. *In vitro* cytotoxicity is determined using a chromium release assay. Spleen cells of naive and *Listeria*-OVA (internal) vaccinated mice are stimulated at a 10:1 ratio with either irradiated EG7.OVA cells (EL-4 tumor cell line transfected to express OVA, ATCC, Manassas, VA) or with 100 nM SL8, in order to expand the OVA specific T cells in the spleen cell population. After 7 days of culture, the cytotoxic activity of the effector cells is determined in a standard 4-hour 51Cr-release assay using EG7.OVA or SL8 pulsed EL-4 cells (ATCC, Manassas, VA) as target cells and EL-4 cells alone as negative control. The YAC-1 cell line (ATCC, Manassas, VA) is used as targets to determine NK cell activity, in order to distinguish the activity due to T cells from that due to NK cells. The percentage of specific cytotoxicity is calculated as 100 x (experimental release - spontaneous release) / (maximal release - spontaneous release). Spontaneous release is determined by incubation of target cells without effector cells. Maximal release is determined by lysing cells with 0.1 % Triton X-100. Experiments are considered valid for analysis if spontaneous release is < 20% of maximal release.

For the assessment of cytotoxic activity of OVA-specific CD8+ T cells *in vivo,* spleen cells from naïve C57B1/6 mice are split into two equivalent aliquots. Each group is pulsed with a specific peptide, either target (SL8) or control (HSV-gB2), at 0.5 µg/ml for 90 minutes at 37 °C. Cells are then washed 3 times in medium, and twice in PBS + 0.1% BSA. Cells are resuspended at 1 x 10⁷ per ml in warm PBS + 0.1% BSA (10 ml or less) for labeling with carboxyfluorescein diacetate succinimidyl ester (CFSE, Molecular Probes, Eugene, OR). To the target cell suspension, 1.25 µL of a 5mM stock of CFSE is added and the sample mixed by vortexing. To the control cell suspension, a ten-fold dilution of the CFSE stock is added and the sample mixed by vortexing. The cells are incubated at 37 °C for 10 minutes. Staining is stopped by addition of a large volume (>40 ml) of ice-cold PBS. The cells are washed twice at room temperature with PBS, then resuspended and counted. Each cell suspension is diluted to 50 x 10⁶ per ml, and 100 µL of each population is mixed and injected via the tail vein of either naive or vaccinated mice. After 12-24 hours, the spleens are harvested and a total of 5 x 10⁶ cells are analyzed by flow cytometry. The high (target) and low (control) fluorescent peaks are enumerated, and the ratio of the two is used to establish the percentage of target cell lysis. The *in vivo* cytotoxicity assay permits the assessment of lytic activity of antigen-specific T cells without the need of *in vitro* re-stimulation. Furthermore, this assays assesses the T cell function in their native environment.

### Example 22. Human EphA2-specific immunity induced by vaccination of Balb/c mice with Listeria strains expressing EphA2.

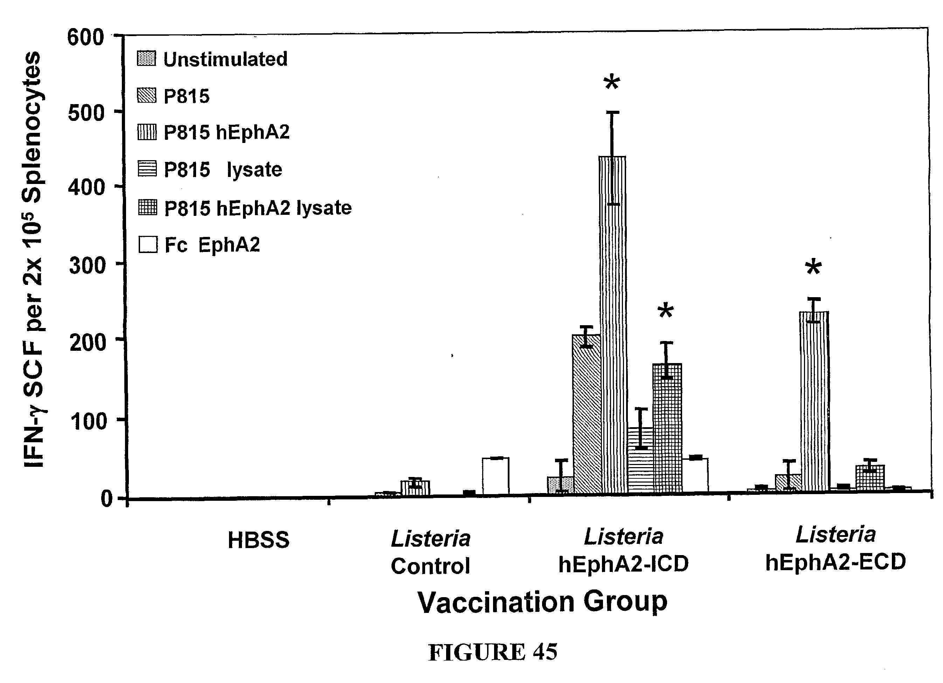

Balb/c mice (n=3) were immunized with *Listeria* L461 T expressing the intracellular domain of hEphA2 *(Listeria* hEphA2-ICD in Figure 45) or an Δ*actA* (*actA*⁻) strain of *Listeria* expressing the extracellular domain of hEphA2 from a sequence codon-optimized for expression in *L. monocytogenes* (*Listeria* hEphA2-ECD in Figure 45) two weeks apart. (The intracellular domain of hEphA2 is alternatively referred to herein as hEphA2-ICD, hEphA2 ICD, EphA2 CO, or CO. The extracellular domain of hEphA2 is alternatively referred to herein as hEphA2-ECD, hEphA2 ECD, EphA2 EX2, or EX2.) Mice were euthanized, and spleens harvested and pooled 6 days after the last immunization. For the ELISPOT assay, the cells were re-stimulated *in vitro* with P815 cells expressing full-length hEphA2 or cell lysates prepared from these cells. The parental P815 cells or cell lysates served as a negative control. Cells were also stimulated with recombinant hEphA2 Fc fusion protein. IFN-gamma positive spot forming colonies (SFCs) were measured using a 96 well spot reader. As shown in Figure 45, increased IFN-gamma SFCs were observed with spleen cells derived from mice vaccinated with *Listeria*-hEphA2. Both hEphA2 expressing cells or cell lysates stimulation resulted in an increase in IFN-gatrima SFC which suggests an EphA2-specific CD8+ as well as CD4+ T cell response. Spleen cells from mice vaccinated with the parental *Listeria* control did not demonstrate an increase in IFN-gamma SFC.

### Example 23. CD4+ and CD8+ T cell responses are required for EphA2 specific anti-tumor efficacy.

Balb/c mice (n=10) were inoculated i.v. with 2 x 10⁵ CT26-hEphA2 on day 0. CD4+ cells and CD8+ T-cells were depleted by injecting 200 µg anti-CD4 (ATCC hybridoma G1.5) or anti-CD8 (ATCC hybridoma 2.4-3) on Days 1 and 3, which was confirmed by FACS analysis (data not shown). Mice were then immunized i.v. with 0.1 LD₅₀ *Listeria* L461 T expressing hEphA2 ICD on Day 4 and monitored for survival.

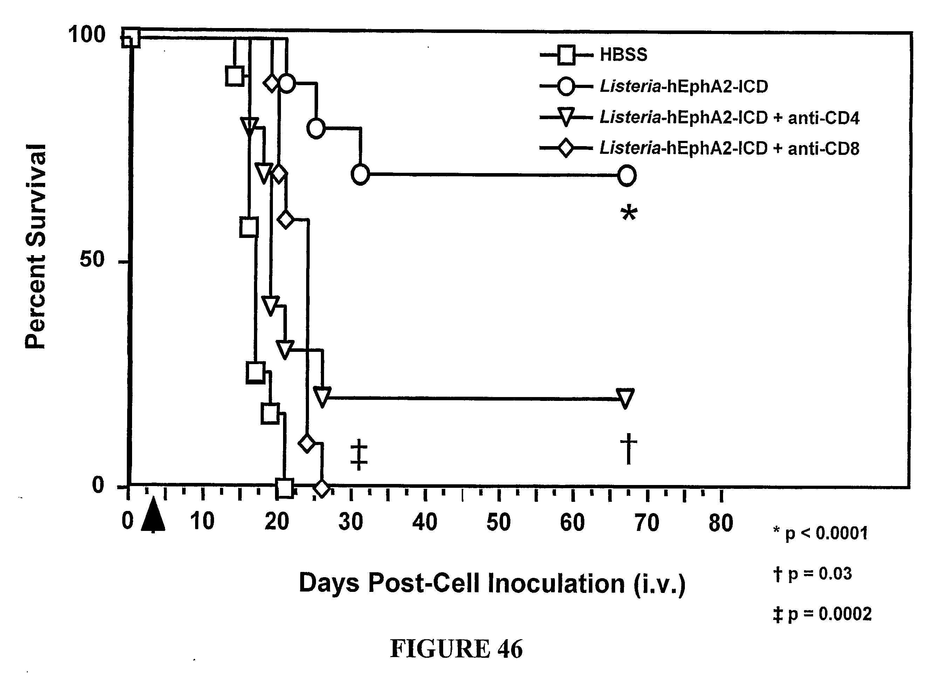

As shown in Figure 46, both CD4+ and CD8+ depleted groups failed to demonstrate the anti-tumor response seen in the non-T cell depleted animals. The data are summarized in Table 5 below:

**Table 5**

| Vaccination Group | Median Survival (Days) | P vs. HBSS | # Survivors (Day 67) |
|---|---|---|---|
| HBSS | 17 | - | 0 |
| *Listeria-*hEphA2-ICD | >67 | <0.0001 | 7 |
| *Listeria-*hEphA2-ICD + anti-CD4 | 19 | 0.03 | 2 |
| *Listeria-*hEphA2-ICD + anti-CD8 | 24 | 0.0002 | 0 |

The foregoing data indicate a requirement for both CD4+ and CD8+ T cells in optimal suppression of tumor growth.

### Example 24. Deletion of inlB from Listeria by allelic exchange.

Bacteria comprising the recombinant nucleic acid molecules and expression cassettes described herein are, in some embodiments, mutant *Listeria.* For instance, in some embodiments, the bacteria comprising the recombinant nucleic acid molecules and expression cassettes are *Listeria monocytogenes* strains in which the *actA* gene, the *in*/*B* gene, or both, have been deleted. One exemplary method for generating a deletion mutant in *Listeria* is described below.

Deletion of the internalin B gene (*inIB*) from *Listeria* DP-L4029 (or from other selected mutant strains or from wild-type *Listeria*) can be effected by allelic exchange, as described by Camilli et al., Mol. Microbiol. 8:143-147 (1993). Splice Overlap Extension (SOE) PCR can be used to prepare the construct used in the allelic exchange procedure. The source of the internalin B gene is the sequence listed as Genbank accession number AL591975 (*Listeria monocytogenes* strain EGD, complete genome, segment 3/12; *inlB* gene region: nts. 97008-98963), and/or the sequence listed as Genbank accession number NC_003210 (*Listeria monocytogenes* strain EGD, complete genome, *inlB* gene region: nts. 457008-458963).

In the primary PCR reactions, approximately 1000 bps of sequence upstream and downstream from the *Listeria inIB* gene 5' and 3' ends, respectively, are amplified using the following template and primers:
*Template:* DP-L4056 or DP-L4029 genomic DNA
*Primer pair 1*(For amplification of region upstream from 5' end of *inlB*):
   Lm-9603 IF: 5'-GTTAAGTTTCATGTGGACGGCAAAG (SEQ ID NO:103) (Tₘ: 72°C)
   Lm-(3' *inlB-*R +) 97020R: 5'-AGGTCTTTTTCAGTTAACTATCCTCTCCTTGATTCTAGTTAT (SEQ ID NO:104) (Tₘ: 114°C)
   (The underlined sequence complementary to region downstream of *InlB* carboxy terminus.)
   Amplicon Size (bps): 1007
*Primer pair* 2 (For amplification of region downstream from 3' end of *inlB):*
   Lm-(5' *inlB-*F +) 98911F: 5'-CAAGGAGAGGATAGTTAACTGAAAAAGACCTAAAAAAGAA GGG (SEQ ID NO:105)(Tₘ: 118°C)
   (The underlined sequence complementary to region upstream of *InlB* amino terminus.)
   Lm-99970R: 5'-TCCCCTGTTCCTATAATTGTTAGCTC (SEQ ID NO:106) (Tₘ: 74°C)
Amplicon size (bps): 1074

In the secondary PCR reaction, the primary PCR amplicons are fused through SOE PCR, taking advantage of complementarity between reverse primer from pair 1 and the forward primer of pair 2. This results in precise deletion of inlB coding sequence: nts. 97021-98910=1889 bps. The following template and primers were utilized in the secondary PCR reaction:
*Template:* Cleaned primary PCR reactions
*Primer pair:*
   Lm-96043F: 5'-GTGGACGGCAAAGAAACAACCAAAG (SEQ ID NO: 107) (Tₘ: 74°C)
   Lm-99964R: 5'-GTTCCTATAATTGTTAGCTCATTTTTTTC (SEQ ID NO:108) (Tₘ: 74°C)
   (Amplicon size (bps): 2033)

A protocol for completing the construction process is as follows:

The primary PCR reactions (3 temperature cycle) are performed using Vent DNA polymerase (NEB) and 10 µl of a washed 30°C *Listeria* DP-L4056 OR DP-L4029 overnight culture. The expected size of *Listeria* amplicons by 1% agarose gel (1007 bps and 1074 bps). The primary PCR reactions are gel purified and the DNA eluted with GeneClean (BIO 101).

A secondary PCR reaction is performed, utilizing approximately equal amounts of each primary reaction as template (ca. 5 µl). The expected size of the *Listeria*, amplicon from the secondary PCR reaction is verified by 1% agarose gel (2033 bps). Adenosine residue are added at the 3' ends of *Listeria dl inlB* amplicon with Taq polymerase.

The *Listeria dl inlB* amplicon is then inserted into a pCR2.1-TOPO vector. The pCR2.1-TOPO-dl inlB plasmid DNA is digested with *XhoI* and *KpnI* and the 2123 bp fragment is gel purified. The *KpnI*/*XhoI* 2123 bp fragment is inserted into a pKSV7 vector that has been prepared by digestion with *KpnI* and *XhoI* and treatment with CIAP (pKSV7-dl inlB). The fidelity of dl inlB sequence in pKSV7-dl inlB is then verified. The inlB gene is deleted from desired *Listeria* strains by allelic exchange with pKSV7-dl inlB plasmid.

### Example 25. Codon-optimized signal peptides for construction of recombinant Listeria.

Some exemplary codon-optimized signal peptides that can be used in the expression cassettes in the recombinant Listeria are provided in Table 6, below.

**Table 6. Exemplary signal peptides for construction of recombinant Listeria**

| Secretion Pathway | Signal Peptide Amino Acid Sequence | Signal peptidase Site (') | Native Sequence | Sequence codon-optimized for expression in *Lm* | Gene [Genus/species] |
|---|---|---|---|---|---|
| **secA1** | MKKIMLV FITLILVSL PIAQQTEA KDASAFN KENSISSM APPASPPA SPKTPIEK KHAD (SEQ ID NO:109)¹ | TEA'KD (SEQ ID NO:54) | ATGAAAAAAATAATG CTAGTTTTTATTACAC TTATATTAGTTAGTCT ACCAATTGCGCAACA AACTGAAGCAAAGGA TGCATCTGCATTCAAT AAAGAAAATTCAATT TCATCCATGGCACCA CCAGCATCTCCGCCTG CAAGTCCTAAGACGC CAATCGAAAAGAAAC ACGCGGAT (SEQ ID NO:110) | ATGAAAAAATTATGTT AGTTTTTATTACATTAAT TTTAGTTAGTTTACCAAT TGCACAACAAACAGAAG CAAAAGATGCAAGTGCA TTTAATAAAGAAAATAG TATTAGTAGTATGGCACC ACCAGCAAGTCCACCAG CAAGTCCAAAAACACCA ATTGAAAAAAAACATGC AGAT (SEQ ID NO:113) | *hly* (LLO) |
| | | | | | [*Listeria monocytogenes*] |
| | MKKKIISA ILMSTVILS AAAPLSG VYADT (SEQ ID NO:46) | VYA'DT (SEQ ID NO:55) | ATGAAAAAAAAGATT ATCTCAGCTATTTTAA TGTCTACAGTGATACT TTCTGCTGCAGCCCCG TTGTCAGGTGTTTACG CTGACACA (SEQ ID NO:86) | ATGAAAAAAAAAATTAT TAGTGCAATTTTAATGAG TACAGTTATTTTAAGTGC AGCAGCACCATTAAGTG GTGTTTATGCAGATACA (SEQ ID NO:87) | Usp45 |
| | | | | | *[Lactococcus lactis]* |
| | MKKRKVL IPLMALSTI LVSSTGNL EVIQAEV (SEQ ID NO:47) | IQA'EV (SEQ ID NO:56) | ATGAAAAAACGAAAA GTGTTAATACCATTAA TGGCATTGTCTACGAT ATTAGTTTCAAGCAC AGGTAATTTAGAGGT GATTCAGGCAGAAGT T (SEQ ID NO:111) | ATGAAAAAACGTAAAGT TTTAATTCCATTAATGGC ATTAAGTACAATTTTAGT TAGTAGTACAGGTAATTT AGAAGTTATTCAAGCAG AAGTT (SEQ ID NO:114) | *pag* (Protective Antigen) |
| | | | | | *[Bacillus anthracis]* |
| | | | | | |
| **secA2** | MNMKKAT IAATAGIA VTAFAAPT IASAST (SEQ ID NO:48) | ASA'ST (SEQ ID NO:57) | ATGAATATGAAAAAA GCAACTATCGCGGCT ACAGCTGGGATTGCG GTAACAGCATTTGCT GCGCCAACAATCGCA TCCGCAAGCACT (SEQ ID NO:90) | ATGAATATGAAAAAAGC AACAATTGCAGCAACAG CAGGTATTGCAGTTACAG CATTTGCAGCACCAACA ATTGCAAGTGCAAGTAC A (SEQ ID NO:91) | *iap* invasion-associated protein p60 |
| | | | | | *[Listeria monocytogenes*] |
| | | | | | |
| **Tat** | MAYDSRF DEWVQKL KEESFQNN TFDRRKFI QGAGKIA GLSLGLTI AQSVGAF (SEQ ID NO:53) | VGA'F (SEQ ID NO:62) | ATGGCATACGACAGT CGTTTTGATGAATGG GTACAGAAACTGAAA GAGGAAAGCTTTCAA AACAATACGTTTGAC CGCCGCAAATTTATTC AAGGAGCGGGAAGA TTGCAGGACTTTCTCT TGGATTAACGATTGC CCAGTCGGTTGGGGC CTTT (SEQ ID NO:112) | ATGGCATATGATAGTCGT TTTGATGAATGGGTTCAA AAATTAAAAGAAGAAAG TTTTCAAAATAATACATT TGATCGTCGTAAATTTAT TCAAGGTGCAGGTAAAA TTGCAGGTTTAAGTTTAG GTTTAACAATTGCACAAA GTGTTGGTGCATTT (SEQ ID NO:115) | PhoD alkaline phosphatase |
| | | | | | [*Bacillus subtilis*] |

| | | | | | |
|---|---|---|---|---|---|
| ¹The sequence shown includes the PEST sequence from LLO. | | | | | |

### Example 26. Codon-optimized expression cassette comprising Bacillus anthracis Protective Antigen (PA) signal peptide.

An expression cassette was designed for expression of heterologous antigens in *Listeria monocytogenes* using a non-Listerial secA1 signal peptide. The amino acid sequence of the Protective Antigen (PA) signal peptide from *Bacillus anthracis* (Ba) (GenBank accession number NC_007322), its native coding sequence, and the coding sequence optimized for expression in *Listeria monocytogenes* are shown below.

Amino acid sequence:
MKKRhVLIPLMALSTILVSSTGNLEVIQAEV (SEQ ID NO:47)
Signal peptidase recognition site: IQA'EV (SEQ ID NO:56)

Native nucleotide sequence:

ATGAAAAACGAAAAGTGTTAATACCATTAATGGCATTGTCTACGATATTAGTTT
CAAGCACAGGTAATTTAGAGGTGATTCAGGCAGAAGTT (SEQ ID NO:111)

Codons optimized for expression in *Listeria monocytogenes:*

ATGAAAAAACGTAAAGTTTTAATTCCATTAATGGCATTAAGTACAATTTTAGTTA
GTAGTACAGGTAATTTAGAAGTTATTCAAGCAGAAGTT (SEQ ID NO:114)

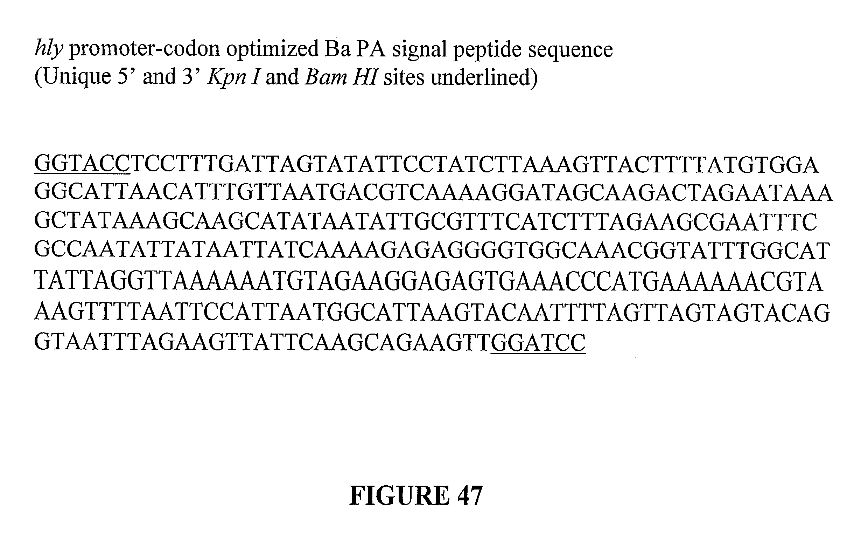

The sequence of a partial expression cassette comprising the hly promoter from *Listeria monocytogenes* operably linked to the codon-optimized sequence encoding the Ba PA signal peptide is shown in Figure 47. This sequence can be combined with either a codon-optimized or non-codon-optimized antigen sequence for expression of a fusion protein comprising the *Bacillus anthracis* PA signal peptide and the desired antigen.

### Example 27. Expression and secretion of antigens from recombinant Listeria comprising codon-optimized expression cassettes.

*Codon optimization of both signal peptide and tumor antigen provides efficient expression and secretion from recombinant Listeria:* Codon-optimization of both signal peptide- and heterologous protein-encoding genetic elements provides optimal secretion from recombinant *Listeria-*based vaccines of human tumor antigens that contain hydrophobic domains. Efficient antigen secretion from cytosolic bacteria is required for efficient presentation via the MHC class I pathway and CD8+ T-cell priming, and is thus linked directly to the potency of *Listeria-*based vaccines. Secretion from recombinant *Listeria* of two malignant cell membrane-bound human tumor antigens, mesothelin and NY-ESO-1, which are immune targets related to pancreatic and ovarian cancer (mesothelin), and melanoma (NY-ESO-1), among other solid tumors, has been optimized through codon-optimization of the combination of both the antigen and signal peptide coding sequences.

A variety of expression cassettes were constructed comprising the *hly* promoter linked to either native or codon-optimized sequences encoding signal peptides related to secA1 or alternative secretion pathways including secA2 and Twin-Arg Translocation (Tat), fused in frame with a selected human tumor antigen - human NY-ESO-1 or human mesothelin. (See Examples 11-14 and 25, above, for the antigen sequences and/or signal sequences.) Western blot analysis of TCA-precipitated culture fluids of *Listeria* grown in BHI broth was used to assess the synthesis and secretion of the heterologous proteins from the recombinant *Listeria.* (Methods analogous to those described in Example 18, above, were used for the Western blot analyses.)

The results of these experiments are shown in Figure 48A-C. Efficient expression and secretion of full-length tumor antigens from recombinant *Listeria* was observed when both signal peptide coding sequences, including when derived from *Listeria monocytogenes,* and operably linked foreign antigen coding sequences were optimized for codon usage in *Listeria monocytogenes.* Figure 48A shows the expression/secretion of human mesothelin by Δ*actA Listeria monocytogenes* with a construct comprising an LLO signal peptide fused with human mesothelin, using native codons for both LLO and mesothelin. By Western analysis of TCA-precipitated bacterial culture fluids, secretion of expected full-length mesothelin (62 kDa) was not observed with these constructs, and only secretion of several small fragments was observed (Figure 48A).

Figure 48B shows a Western blot analysis of the expression/secretion of human mesothelin by *Listeria monocytogenes* Δ*actA* comprising plasmids (pAM401) containing constructs encoding various signal peptides fused with human mesothelin. In each construct, the mesothelin coding sequence was codon-optimized for expression in *Listeria monocytogenes.* Where indicated, the signal peptide coding sequences used contained either the native sequence ("native") or were codon-optimized ("CodOp") for expression in *Listeria monocytogenes.* Secreted mesothelin was detected using an affinity-purified polyclonal antihuman/mouse antibody, prepared by injection of rabbits with selected peptides together with IFA.

Significantly, as shown in lanes 3-5, and 8-9 of Figure 48B, secretion of full-length mesothelin (62 kDa) was observed only when both signal peptide and mesothelin coding sequences were codon-optimized for expression in *Listeria.* This observation significantly also included the *Listeria*-derived signal peptides from the bacterial LLO and p60 proteins, related to the secA1 and secA2 secretion pathways, respectively, both of which contain infrequently-used codons. (The LLO PEST sequence is also included with the LLO signal peptide and its coding sequence is also codon-optimized.) Efficient secretion of full-length mesothelin (62 kDa) was observed when the codon-optimized *Listeria* LLO signal peptide was linked with codon-optimized mesothelin (Lane 8, Figure 48B), but NOT when the native coding sequence of the *Listeria* LLO signal peptide was used (Lane 7, Figure 48B). Furthermore, secretion of full-length mesothelin (62 kDa) was observed when the codon-optimized *Listeria* p60 signal peptide was linked with codon-optimized mesothelin (Lane 3, Figure 48B), but NOT when the native coding sequence of the *Listeria* p60 signal peptide was used (Lane 6, Figure 48B). Finally, secretion of full-length mesothelin (62 kDa) was observed when codon-optimized optimized signal peptides from bacterial species different from *Listeria monocytogenes* were operably linked to codon-optimized mesothelin (Figure 48B). The signal peptide from *Bacillus anthracis* protective antigen (*Ba* PA), or the signal peptide from *Lactococcus lactis* Usp45 protein (*Ll* Usp45) programmed the efficient secretion of full-length mesothelin (62 kDa) from the recombinant *Listeria* strains (Figure 48B, lanes 4 and 5). The *Bacillus subtilis* phoD signal peptide (Bs phoD) also programmed the efficient secretion of full-length mesothelin from *Listeria* (Figure 48B, lane 9). The bands with a molecular weight of about 62,000 correspond to mesothelin and the pairs of double bands probably correspond to non-cleaved plus cleaved mesothelin polypeptides (i.e., to partial cleavage).

Figure 48C shows the expression/secretion of NY-ESO-1 from *Listeria monocytogenes* Δ*act*Δ*inlB* with constructs comprising a sequence encoding LLO signal peptide which was fused with a sequence encoding human NY-ESO-1, both of which were codon-optimized for expression in *Listeria.* Secreted NY-ESO-1 was detected using a NY-ESO-1 monoclonal antibody.

In this example, signal peptide and tumor antigen domains were synthesized to utilize the most preferred codon for each amino acid, as defined by frequency of occurrence per 1000 codons in coding sequences from the *Listeria* genome (http://www.kazusa.or.jp/codon/cgi-bin/showcodon.cgi?species=Listeria+monocytogenes+[gbbct]). Signal peptides related to secA1, secA2, or twin-Arg translocation (Tat) secretion pathways from *Listeria* and other Gram-positive bacterial genera programmed the efficient secretion of human tumor antigens from recombinant *Listeria.* Surprisingly, the signal peptides from *Listeria* proteins LLO and p60 each contain rare codons (frequency of <10 per 1000 codons), and optimization of these sequences was required for efficient secretion of mesothelin and NY-ESO-1 from recombinant *Listeria* (Figure 48B). Mesothelin secretion was also observed when linked to secA1 signal peptides from *B. anthracis* protective antigen (pagA) and *Lactococcus lactis* Usp45, and the Tat signal peptide from the phosphodiesterase/alkaline phosphatase D gene (PhoD) of *B. subtilis.*

Signal peptides from distinct secretion pathways were used to determine whether a particular pathway would be favored for optimal secretion of heterologous proteins. For example, the Tat pathway is utilized for secretion of proteins folded within the bacterium, and the *B. subtilis* phoD protein is secreted via this mechanism. It had originally been hypothesized that secretion of tumor antigens containing significant hydrophobic domains, such as NY-ESO-1, might be facilitated by folding prior to transport. However, these results indicated that codon-optimization of both the signal peptide and tumor antigen encoding sequences, and not secretion pathway, is the primary requirement for efficient secretion of mammalian proteins.

Importantly, the phenotype of recombinant vaccines utilizing any pathway for tumor antigen secretion was not significantly affected, as compared to the parental *Listeria* Δ*act*/Δ*inlB* strain. The median lethality (LD₅₀) of *Listeria* Δ*actA*/Δ*inlB* is 1 x 10⁸ cfu in C57BL/6 mice. Stable single copy site-specific incorporation of tumor antigen expression cassettes into an innocuous site on the chromosome of *Listeria* Δ*actAl*Δ*inlB,* was accomplished using the pPL2 integration vector. The LD₅₀ of tumor antigen encoding *Listeria* Δ*actA*/Δ*inlB* was within 5-fold of *Listeria* Δ*act*A*l*Δ*inlB.*

### Example 28. Construction of bicistronic hEphA2 expression vectors.

As a non-limiting example, construction of an antigen expression cassette, in which expression of the external (EX2) and internal (CO; kinase dead) domains of hEphA2 occurs from a bicistronic message, is given. Secretion of the EX2 and CO domains is accomplished by functional linkage of the Ba PA and Bs PhoD signal peptides with the EX2 and CO domains, respectively.

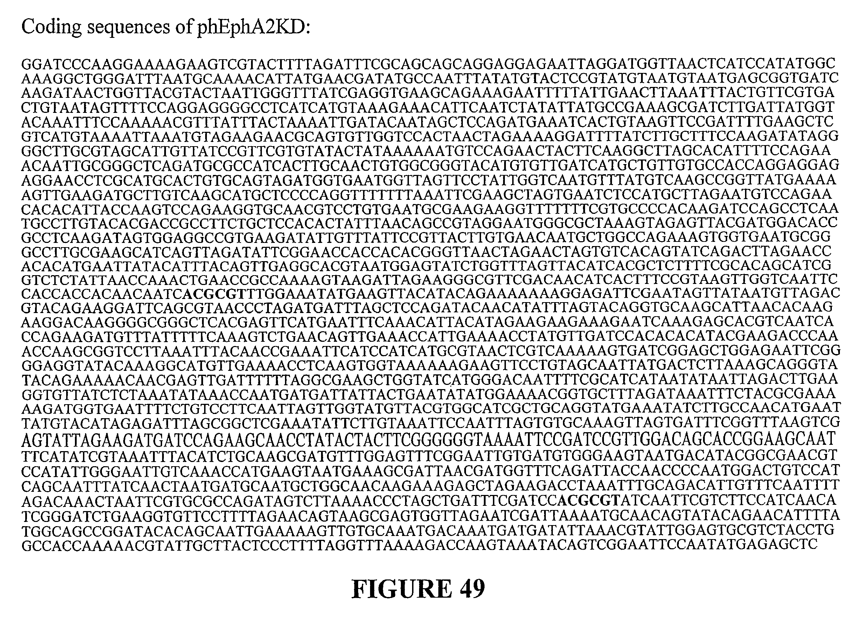

A codon-optimized human EpliA2 kinase dead plasmid, known as phEphA2KD, is used in the construction of a bicistronic hEphA2 expression vector. (EphA2 is a receptor tyrosine kinase, but the kinase activity is ablated by a mutation from K to M at the active site of the enzyme.) The coding sequences of phEphA2KD are shown in Figure 49. The phEphA2KD sequence in Figure 49 comprises the codon-optimized coding sequence for hEphA2 deleted of the transmembrane domain, and contains unique 5' and 3' Bam HI and Sac I restriction sites to facilitate construction of functional antigen expression cassettes. Mlu I recognition sequences are shown bolded in the sequence shown in Figure 49.

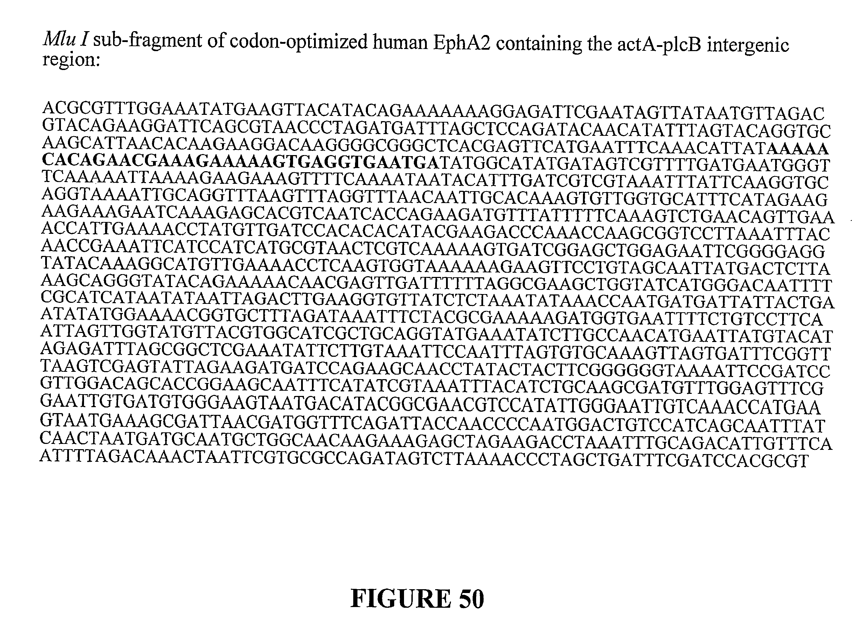

A sub-fragment of the human EphA2 (trans-membrane domain deleted, kinase-dead) between the two Mlu I restriction enzyme recognition sequences is synthesized (by a gene synthesis method known in the art, e.g., by oligonucleotide synthesis, PCR, and/or Klenow fill-in, or the like). The actA-plcB intergenic region is inserted during the synthesis precisely at the junction between the EphA2 extracellular and intracellular domains, which are separated by the hydrophobic trans-membrane domain in the native protein. The sequence of the Mlu I sub-fragment of codon-optimized human EphA2 containing the actA-plcB intergenic region is shown in Figure 50 (the intergenic region is shown in bold). Additionally, the codon-optimized Bs phoD signal peptide is placed at the 3' end of the actA-plcB intergenic sequence and is fused in-frame with the downstream EphA2 CO domain coding region.

The functional human EphA2 bicistronic cassette is assembled by substitution of the Mlu I fragment containing the actA-plcB intergenic region and Bs phoD signal peptide for the corresponding region in the trans-membrane deleted kinase dead human EphA2 sequence shown in Figure 49. This resulting sequence contains unique Bam HI and Sac I restriction enzyme recognition sites at its 5' and 3' ends, respectively, to facilitate insertion and functional linkage to the hly promoter and initial signal peptide, for example Ba PA.

Thus, the seven ordered functional elements of the bicistronic human EphA2 antigen expression cassette are the following: hly promoter-Ba PA signal peptide-EX domain EphA2-termination codon-actA-plcB intergenic region (with Shine-Dalgarno sequence)-Bs PhoD signal peptide-CO domain EphA2-termination codon. All EphA2 and signal peptide coding sequences are preferably codon-optimized.

Recombinant *Listeria* strains that express and secrete the EphA2 EX and CO domains can be derived by methods illustrated in this application, utilizing the pAM401, pKSV7, or pPL1 and pPL2 integration vectors. Expression and secretion of the EphA2 proteins is detected by Western analysis of desired bacterial fractions, using methods described herein and/or known to those skilled in the art.

### Example 29. Expression and secretion of antigens from recombinant Listeria comprising antigen-bacterial protein chimeras.

In some embodiments of this disclosure both the sequences encoding the signal peptide and its heterologous protein fusion partner are codon-optimized. In some embodiments, it is desirable to place the codon-optimized heterologous protein sequence within a defined region of a protein, whose native form is secreted from *Listeria.* The heterologous protein sequence is functionally placed within a defined sequence of the selected secreted *Listeria* protein sequence such that a protein chimera is synthesized and secreted that corresponds to the combined molecular weights of the secreted proteins. Secretion of the heterologous protein can be facilitated by exploiting the machinery of the host *Listeria* bacterium that is required for optimal secretion of autologous bacterial proteins. Molecular chaperones facilitate secretion of selected bacterial proteins.

As a non-limiting example, protein chimeras between the *L. monocytogenes* protein p60 and the human tumor antigen, mesothelin, were generated. The protein chimeras were generated by precise placement of the human tumor antigen, mesothelin, into *L*. *monocytogenes* protein p60 at amino acid position 70 (although it is understood that any desired heterologous protein encoding sequence can be selected to generate a protein chimera). The protein chimera contained optimal codons for expression in *Listeria* in the p60 amino acids 1-70 and the entire mesothelin coding sequence. Furthermore, the p60-human mesothelin protein chimeria was functionally linked to the *L. manocytogenes hly* promoter, incorporated into the pPL2 vector, which was used subsequently as described herein to generate recombinant *L. monocytogenes* strains expressing and secreting human mesothelin. The experimental methods used to construct a recombinant *Listeria* strain that optimally expresses and secretes a p60-human mesothelin protein chimera are described below.

In some embodiments, an important feature of protein chimeras between a selected *L. monocytogenes* gene and a selected heterologous protein sequence is appropriate functional placement of the selected heterologous protein sequence within the selected *L*. *monocytogenes* gene to retain optimal secretion of the protein chimera through interaction of the *L. monocytogenes* expressed protein with the bacterial chaperones and secretion apparatus, as well as to retain functional activity of the *L. monocytogenes* protein in the context of the protein chimera. In some embodiments, functional placement of a heterologous sequence within the *L. monocytogenes* secA2-dependent proteins NamA and p60 is desired to retain the peptidoglycan cell wall hydrolase activites of these said proteins. (See Lenz et. al. (2003 PNAS, 100:12432-12437*),* for instance, for descriptions of the SecA2-dependent NamA and p60 proteins.) In some embodiments, the functional placement of the heterologous protein coding sequence is desired between the signal sequence (SS) and the cell wall binding domains (LySM) and catalytic domains Lyz-2 (NamA) and p60-dom (p60) (Lenz et. al. (2003)).

In some embodiments, expression of antigens or heterologous proteins is functionally linked to a prfA-dependent promoter. As such, expression of the heterologous protein is induced within the microenvironment of the recombinant *Listeria* infected cell.

The first step in the construction of a p60-Mesothelin protein chimera involved the DNA synthesis of the prfA-dependent *hly* promoter linked functionally to a DNA sequence encoding the first 70 amino acids of p60, with codons for optimal secretion in *Listeria.* (In some embodiments, the codon usage can be modified further to avoid regions of excessive RNA secondary structure, which may inhibit protein translation efficiency.) The DNA sub-fragment corresponding to the *hly* promoter-70 N-terminal p60 amino acids was synthesized. (This can generally be done by a gene synthesis method known in the art, e.g., by oligonucleotide synthesis, PCR, and/or Klenow fill-in, or the like.)

The sequence of the first 70 amino acids of p60 from *L. monocytogenes,* strain 10403S, is shown below: 
M N M K K A T I A A T A G I A V T A F A A P T I A S A S T V V V E
A G D T L W G I A Q S K G T T V D A I K K A N N L T T D K I V P
G Q K L Q (SEQ ID NO:116)
 It can be appreciated to those skilled in the art that there exists multiple laboratory and field isolates of *L. monocytogenes* encoding genes, including p60, that may contain variability at both the nucleotide sequence and amino acid level, but are nevertheless essentially the same gene and protein. Furthermore, it can be appreciated by those skilled in the art that protein chimeras can be constructed utilizing genes from any laboratory or field isolate (including food-borne or clinical strain) of *L. monocytogenes.*

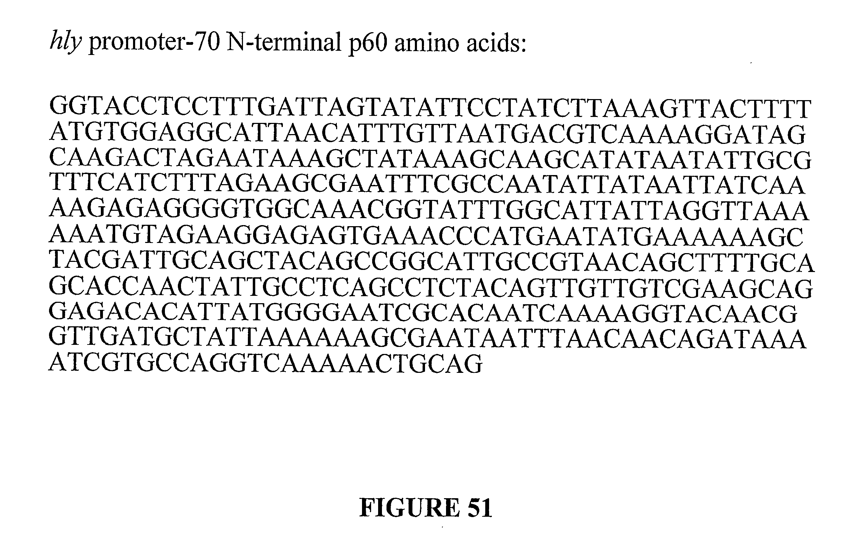

The synthesized DNA sequence corresponding to the *hly* promoter-70 N-terminal p60 amino acids is shown in Figure 51. Furthermore, the codons encoding p60 amino acid residues 69 (L) and 70 (Q), were modified to contain a unique Pst I enzyme recognition sequence, to facilitate functional insertion of a heterologous sequence. Furthermore, the 5' end of the synthesized sub-fragment contains a unique *KpnI* enzyme recognition sequence.

The 447 bp *KpnI* and *PstI* digested sub-fragment fragment was ligated into the corresponding *KpnI* and *Pstl* sites of the pPL2 vector, and treated by digestion with *KpnI* and *PstI* enzymes and digestion with calf intestinal alkaline phosphatase (CIAP). This plasmid is known as pPL2-hlyP-Np60 CodOp. Subsequently, the remainder of the native p60 gene was cloned into the pPL2-hlyP-Np60 CodOp plasmid, between the unique *Pst I* and *BamHI* sites. The remainder of the p60 gene was cloned by PCR, using a proof-reading containing thermostable polymerase, and the following primer pair:
Forward primer:
   5'- CGC CTGCAGGTAAATAATGAGGTTGCTG (SEQ ID NO:117)
Reverse primer:
   5'-CGCGGATCCTTAATTATACGCGACCGAAG (SEQ ID NO:118)

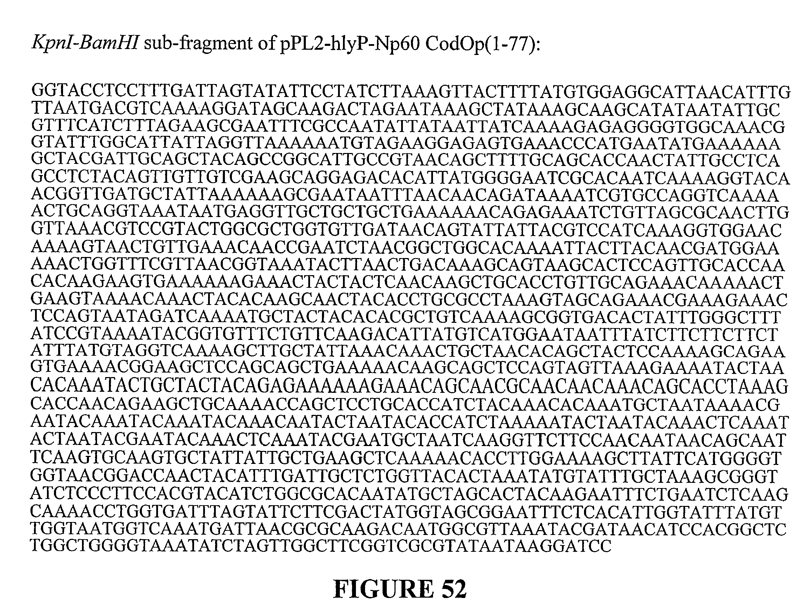

The 1241 bp amplicon was digested with PstI and BamHI, and the purified 1235 bp was ligated into the pPL2-hlyP-Np60 CodOp plasmid, digested with *PstI* and *BanaHI*, and treated with CIAP. This plasmid contains the full *L. monocytogenes* p60 gene with optimal codons corresponding to amino acids 1-77, and native codons corresponding to amino acids 78-478, and is linked functional to the *L. monocytogenes hly* promoter. This plasmid is known as pPL2-hlyP-Np60 CodOp(1-77), and the sequence of the *KpnI-BamHI* sub-fragment that contains the hlyP linked functionally to the p60 encoding sequence is shown in Figure 52. The expected sequence of the pPL2-hlyP-Np60 CodOp(1-77) plasmid was confirmed by sequencing.

The next step in the construction was the functional insertion of a heterologous protein encoding sequence at the unique *PstI* site of plasmid as pPL2-hlyP-Np60 CodOp(1-77), which is between the N-terminal signal sequence and the first LysM cell wall binding domain of p60, thus retaining the normal biological function of the *L. monocytogenes* protein.

As a non-limiting example, human mesothelin that was codon-optimized for optimal expression in *L. monocytogenes* protein was inserted into the unique *PstI* site of plasmid as pPL2-hlyP-Np60 CodOp(1-77). Specifically, full-length mesothelin, or mesothelin that was deleted of the signal peptide and GPI linker domains (Mesothelin ΔSP/AGPI) was cloned from the plasmid described in Example 27 that contains the full-length human mesothelin, containing optimal codons for expression in *L. monocytogenes,* using a thermostable polymerase with proof-reading activity, and the following primer pair:

### 1. Full Length

Forward Primer (huMeso 3F):
5'-AAACTGCAGGCATTGCCAACTGCACGTCC (SEQ ID NO:119)

Reverse Primer (hMeso 1935R):
5'-AAACTGCAGAGCTAATGTACTGGCTAATAATAATGCTAAC (SEQ ID NO:120)

### 2. Δ Signal Peptide, Δ GPI Anchor

Forward Primer (huMeso 133F):
5'- CGCCTGCAGCGTACATTAGCAGGTGAAACAGG (SEQ ID NO:121)

Reverse Primer (huMeso 1770R):
5'-CGCCTGCAGGCCTTGTAAACCTAAACCTAATGTATC (SEQ ID NO:122)

The PCR amplicons of 1932 bps (full-length mesothelin) and 1637 bps (Mesothelin ΔSP/ΔGPI) were purified, digested with *PstI,* purified, and ligated into the unique *PstI site* of plasmid pPL2-hlyP-Np60 CodOp(1-77), treated by digestion with *PstI,* and treatment with CIAP. The consistent N-CO orientation of the p60 and mesothelin domains was confirmed by restriction endonuclease mapping. These plasmids are known as pPL2-hlyP-Np60 CodOp(1-77)-Mesothelin and pPL2-hlyP-Np60 CodOp(1-77)- Mesothelin ΔSP/ΔGPI, and were introduced into selected *L. monocytogenes* strains (such as Δ*actA*Δ*inlB* double deletion mutants), as described throughout the examples contained herein.

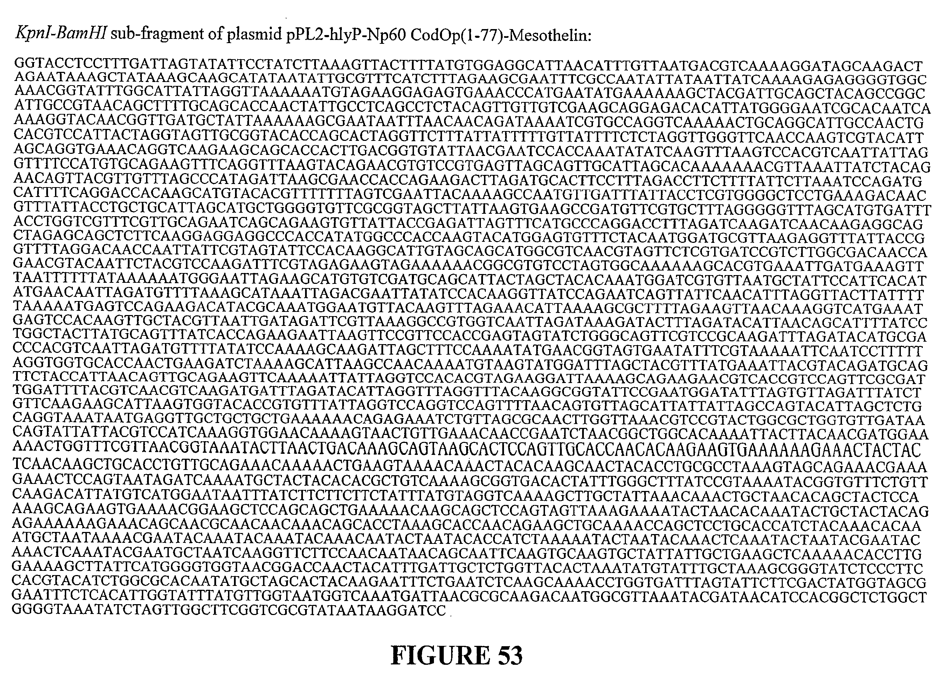

The sequence of the *KpnI-BamHI* sub-fragment of plasmid pPL2-hlyP-Np60 CodOp(1-77)-Mesothelin containing the *hly* promoter linked functionally to the p60-human Mesothelin protein chimera encoding gene is shown in Figure 53.

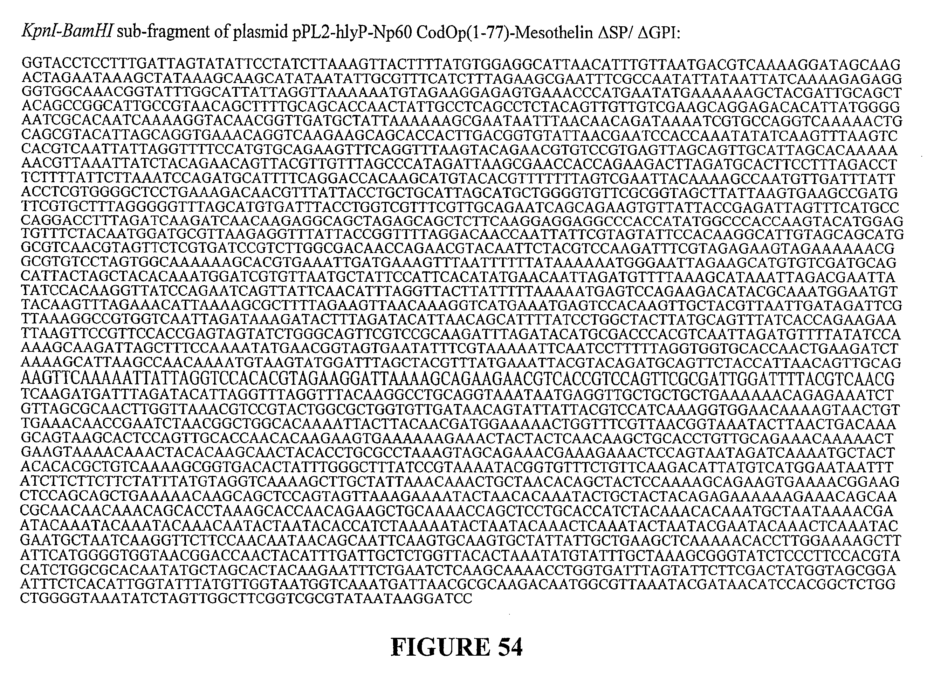

The sequence of the *KpnI-BamHI* sub-fragment of plasmid pPL2-hlyP-Np60 CodOp(1-77)-Mesothelin ΔSP/ΔGPI containing the *hly* promoter linked functionally to the p60-human mesothelin ΔSP/ΔGPI protein chimera encoding gene is shown in Figure 54.

### Western analysis of expression and secretion of p60-mesothelin protein chimeras:

As discussed throughout the examples, expression and secretion of a selected heterologous antigen results in potent priming of MHC class I-restricted CD8+ T cell responses. The expression and secretion of the protein chimeras into the media by recombinant *L. monocytogenes ΔactAΔinlB* double deletion mutants containing tRNA-Arg chromosomal insertions of the pPL2-hlyP-Np60 CodOp(1-77)-Mesothelin or pPL2-hlyP-Np60 CodOp(1-77)- Mesothelin ΔSP/ΔGPI plasmids, generated by methods described herein, were tested by Western analysis by methods described in the Examples contained herein, using a mesothelin-specific polyclonal antibody.

The indicated engineered deletions in hMesothelin (ΔSPAGPI, also referred to herein as ΔSSAGPI, ΔSP/AGPI, ΔSS/AGPI, etc.) for the proteins shown in some of the lanes were as follows: The deleted signal sequence (ΔSP) corresponds to the N-terminal 34 amino acids of hMesothelin (for sequences of human mesothelin, see, e.g., Fig. 34 or GenBank Acc. No. BC009272). The deleted GPI (ΔGPI) domain corresponds to the C-terminal 42 amino acids, beginning with the amino acid residues Gly-Ile-Pro and ending with the amino acid residues Thr-Leu-Ala (see, e.g., Fig. 34).

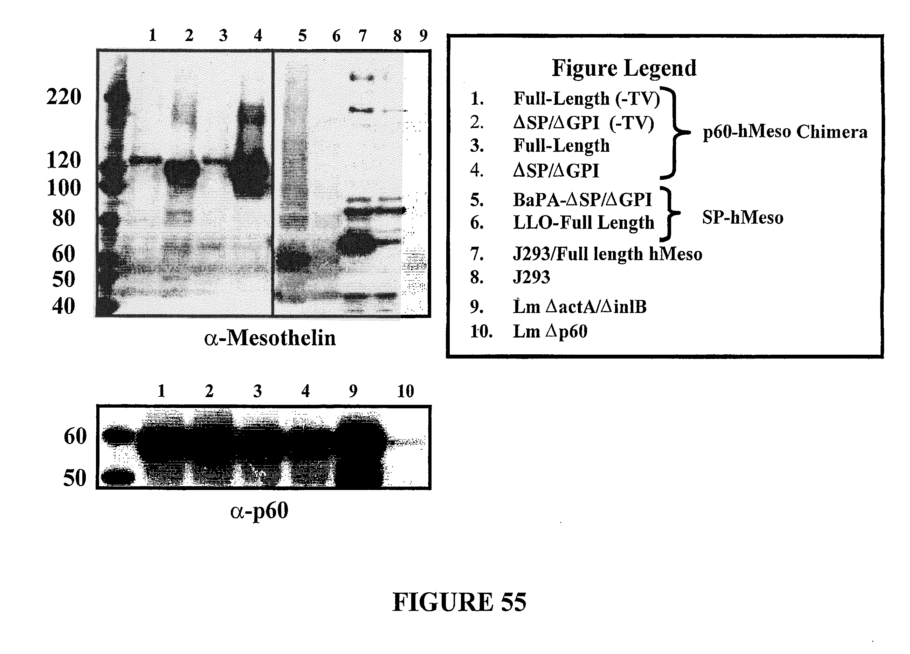

The results of this analysis demonstrated that protein chimeras comprised of p60 with precise insertion of human mesothelin or human mesothelin ΔSP/ΔGPI (inserted in frame at amino acid 70 of p60 between the N-terminal signal sequence and the first of two LysM cell wall binding domains) were efficiently expressed and secreted from the recombinant *L. monocytogenes.* See Figure 55. (The Y-axis of Figure 55 shows the molecular weight (in kDa) of proteins in the ladder run in the far left lane.) Specifically, lanes 1-4 in Figure 55 demonstrate the expression and secretion of the expected protein chimeras containing human mesothelin or human mesothelin ΔSP/ΔGPI. The increased efficiency of expression and secretion of human mesothelin ΔSP/ΔGPI relative to the full-length mesothelin is evident in lanes 2 and 4. In the protein chimeras shown in lanes 3 and 4, the authentic N-terminal p60 amino acids were used. In the chimeras run in lanes 1 and 2 in the Figure 55, the nucleotides encoding amino acids T and V at positions 29 and 64, respectively, were deleted. Lane 5 shows expression and secretion of *Bacillus anthracis* PA signal peptide fused to human ΔSPΔGPI-mesothelin (where both the signal peptide and the mesothelin coding sequences were codon-optimized for expression in *L. monocytogenes*), and lane 6 shows the expression and secretion of LLO fused to full-length human mesothelin (where both the signal peptide and the mesothelin coding sequences were codon-optimized for expression in *L. monocytogenes*). Lane 8 shows protein expression by J293, a human cell line, while lane 7 shows protein expressed and secreted by J293 containing a plasmid encoding full-length human mesothelin ("J293/Full Length"). Lane 10 shows protein expression and secretion from *Listeria* which has been deleted of endogenous p60. The lower panel in Figure 55 shows the Western analysis of *L.monocytogenes* p60 secretion using a polyclonal α-p60 antibody. The results demonstrate that equivalent amounts of Lm-secreted protein were loaded on the gel.

The results demonstrate that p60 can be used as a molecular chaperone to secrete heterologous proteins and facilitate presentation to the MHC class I pathway.

### Example 30: Additional examples of antigen expression and secretion by recombinant Listeria monocytogenes

### A. Expression of the Intracellular domain (ICD) of EphA2 from a bicistronic construct using a non-Listerial signal peptide.

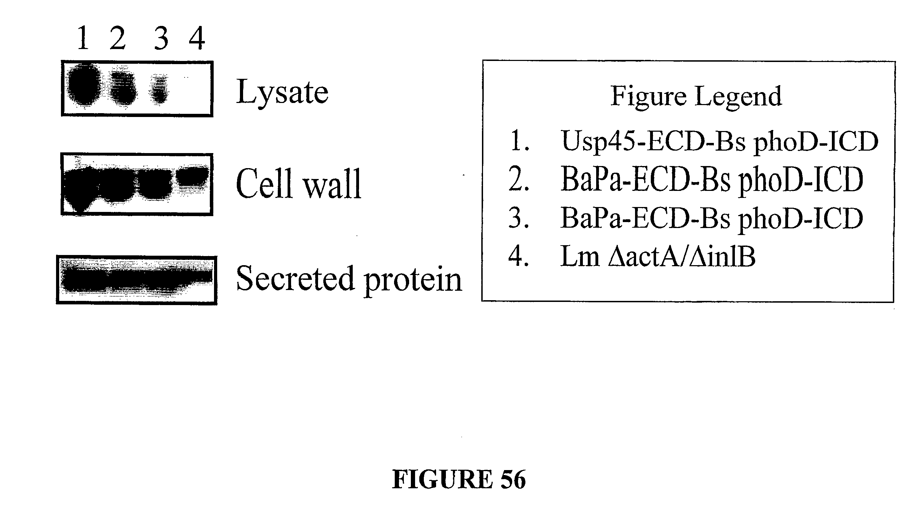

Figure 56 shows the Western blot analysis of the expression and secretion of the intracellular domain (ICD) of EphA2 from bicistronic messages using a non-Listerial, non-secA1 signal sequence.

EphA2 is a protein comprised of an extracellular domain (ECD) and an intracellular domain (ICD). *Listeria ΔactAΔinlB* were engineered to express a bicistronic mRNAs, where the bicistronic mRNAs encoded the extracellular domain and intracellular domain of Epha2 as discrete polypeptides. All of the sequences encoding the signal sequences used in the constructs (*B. subtilis* phoD signal peptide, *B. anthracis* Protective Antigen signal peptide, and *L. lactis* Usp45 signal peptide) were codon-optimized for expression in *L. monocytogenes.* The sequences encoding the ECD and ICD domains were also codon-optimized for expression in *L. monocytogenes.* The Listerial promoter *hly* from the LLO gene was used as the promoter in these constructs.

The expression cassettes encoding the bicistronic mRNA were integrated into the *Listeria* genome using the integration vector pPL2. Western blot analysis of various bacterial fractions using standard techniques was used to detect and measure the accumulated intracellular EphA2 domain. The results demonstrated that the intracellular domain of Epha2 was expressed and secreted from bicistronic constructs using non-Listerial signal peptides encoded by codon-optimized sequences.

The expression constructs comprised: (1) a codon-optimized sequence encoding the *L. lactis* Usp45 secretory sequence operably (functionally) linked with the coding sequence for the extracellular domain of EphA2 (first polypeptide) and a codon-optimized sequence encoding the *B. subtilis* phoD secretory signal operably linked with an intracellular domain of EphA2 (second polypeptide) (lane 1); and (2) a codon-optimized sequence encoding the *B. anthracis* Protective Antigen secretory sequence operably linked with the coding sequence for the extracellular domain of EphA2 (first polypeptide) and a codon-optimized sequence encoding the *B. subtilis* phoD secretory sequence operably linked with the coding sequence for the intracellular domain of EphA2 (second polypeptide) (lanes 2-3 (two different clones); see description of construction of this expression cassette in Example 28, above). Control studies (lane 4) with the attenuated parent *Listeria ΔactAΔnlB* strain demonstrated a variable amount of detectable cross reactivity in some control blots. Lanes 1-3 show a slow migrating band and a fast moving band, where the fast moving band corresponds to the intracellular domain (ICD). Expressed intracellular domain of EphA2 from all of the constructs (lanes 1-3) was observed in all three bacterial fractions. Lane 4 (control) shows only the slow migrating band. Because no antibody was available for the extracellular domain, expression/secretion of the extracellular domain was not assayed.

### B. Plasmid based expression and Secretion of murine mesothelin as a function of N-terminal fusion with various codon-optimized signal peptides.

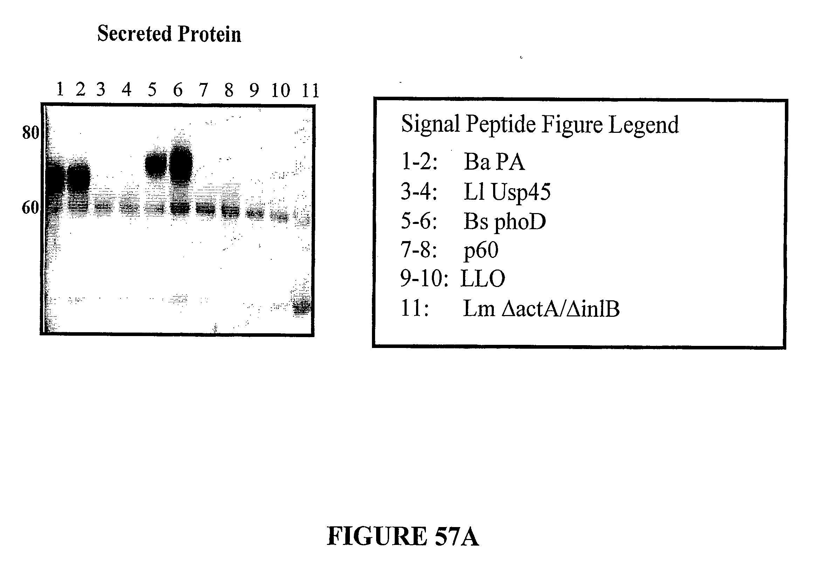

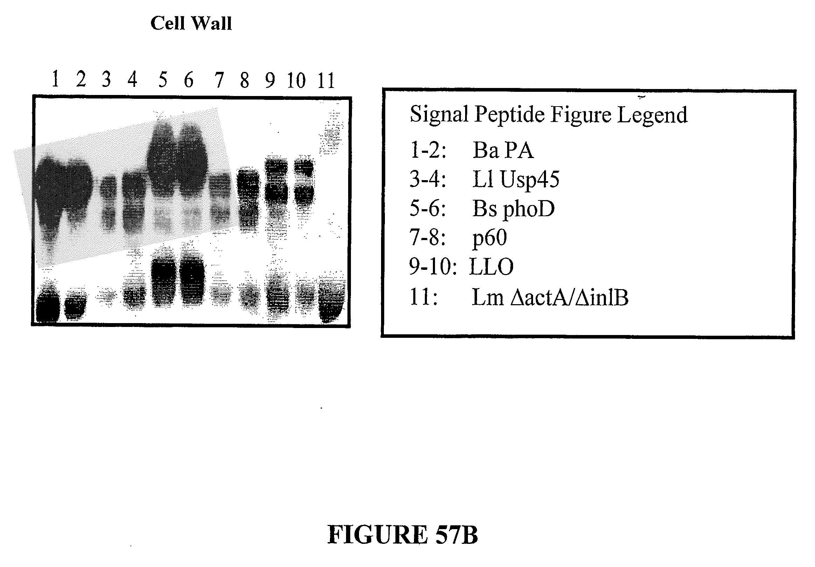

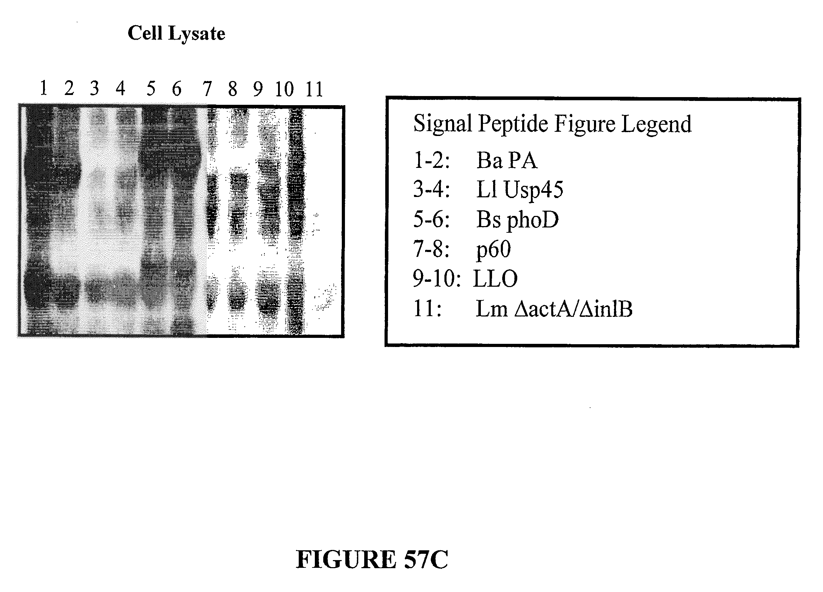

Figure 57 shows plasmid based expression and secretion of murine mesothelin expressed from a codon-optimized mesothelin coding sequence using various signal peptides, including non-Listerial signal sequences and non-secA1 signal sequences. Plasmid based expression and secretion of murine mesothelin is shown as a function of N-terminal fusion with various signal peptides encoded by codon-optimized sequences. In all cases, the sequences encoding the signal peptides of the mesothelin fusion proteins were codon-optimized as well as the murine mesothelin coding sequence was codon-optimized for expression in *L. monocytogenes.* Expression and secretion of murine mesothelin from *L. monocytogenes* was measured, where the *Listeria* harbored a pAM401 plasmid, and where the plasmid encoded the mesothelin. Various plasmid-based constructs where tested, where the signal sequence was varied. Western blots were performed with proteins recovered from the various fractions of secreted proteins (A), the cell wall (B), and the cell lysate (C). For each fraction, lanes 1-2 show murine mesothelin expressed as a fusion with the *B. anthracis* Protective Antigen signal sequence, lanes 3-4 show murine mesothelin expressed as a fusion with the *Lactococcus lactis* Usp45 signal sequence, lanes 5-6 show murine mesothelin expressed as a fusion with the *B. subtilis* phoD signal sequence, lanes 7-8 show murine mesothelin expressed as a fusion with the p60 signal sequence, lanes 9-10 show murine mesothelin expressed as a fusion with the LLO signal sequence, and lane 11 shows protein expressed by the control host *Listeria ΔactAΔinlB.* The results demonstrate that the highest expression and secretion was found where the signal sequence comprised *B. anthracis* Protective Antigen signal sequence (lanes 1-2) and *B. subtilis* phoD signal sequence (lanes 5-6).

### C. Listeria monocytogenes chromosomal-based expression and secretion of human Mesothelin.

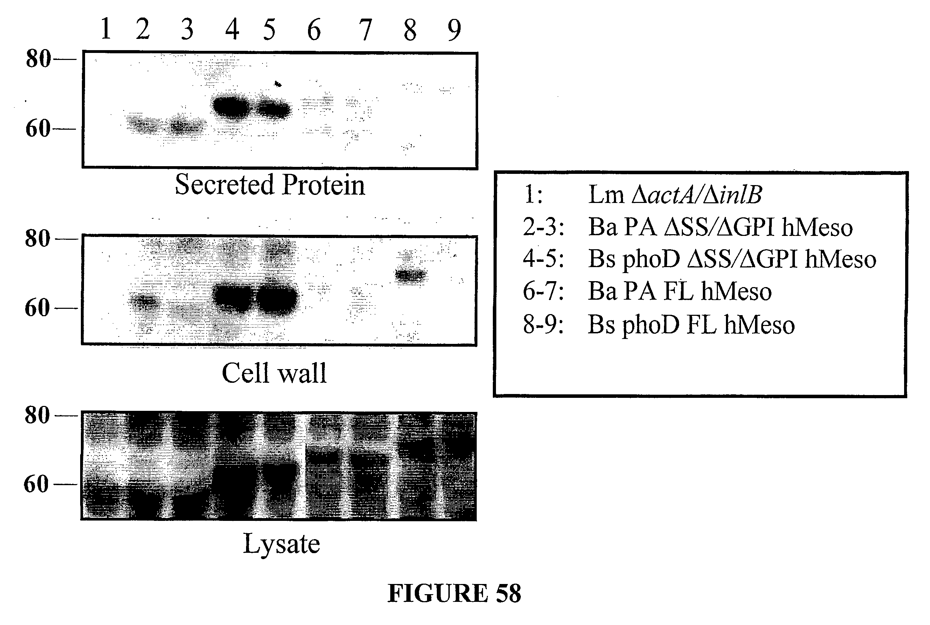

Figure 58 shows the Western blot analysis *of Listeria monocytogenes* chromosomal-based expression and secretion of human mesothelin in various bacterial cell fractions (i.e., secreted protein, cell wall, and lysate). Expression and secretion of human mesothelin was tested when fused to a non-Listerial secA1 and non-secA1 signal peptides. The *Listeria* bacteria tested were all *ΔactA*/*ΔinlB Listeria* and were as follows: *Listeria ΔactA*/*ΔnlB* (control *Listeria* that was not engineered to express mesothelin) (Lane 1); *Listeria* encoding *B. anthracis* Protective Antigen signal sequence fused to ΔSS/ΔGPI hMesothelin (Lanes 2-3); *Listeria* encoding *B. subtilis* phoD signal sequence fused to ΔSS/ΔGPI hMesothelin (Lanes 4-5); *Listeria* encoding *B. anthracis* Protective Antigen signal sequence fused with full-length hMesothelin (Lanes 6-7); *Listeria* encoding *B. subtilis* phoD signal sequence fused to full-length hMesothelin (Lanes 8-9).

The sequences encoding the signal sequences fused to mesothelin in all of the above *Listeria* were codon-optimized for expression in *L. monocytogenes.* In addition, the mesothelin coding sequences (ΔSS/ΔGPI and full-length) were codon-optimized for expression in *L. monocytogenes* in each of the constructs. In each of the above *Listeria,* expressing mesothelin, the mesothelin expression cassettes were inserted in the *Listeria,* chromosome via integration with pPL2.

Highest expression occurred with the *B. subtilis* phoD secretory sequence where human mesothelin was engineered to delete its signal sequence and to delete a hydrophobic region (gpi region) (Lanes 4-5).

### Example 31: Additional examples of immunogenicity and anti-tumor efficacy of recombinant Listeria monocytogenes vaccines

The following examples disclose results of vaccination with the *Listeria* of the present invention, e.g., vaccine-dependent stimulation of cytokine expression, vaccine-dependent survival of an animal with tumors, vaccine-dependent reduction in tumor metastasis, and vaccine-dependent reduction in tumor volume.

### A. Immunogenicity of Listeria vaccine comprising P-60-model antigen chimera

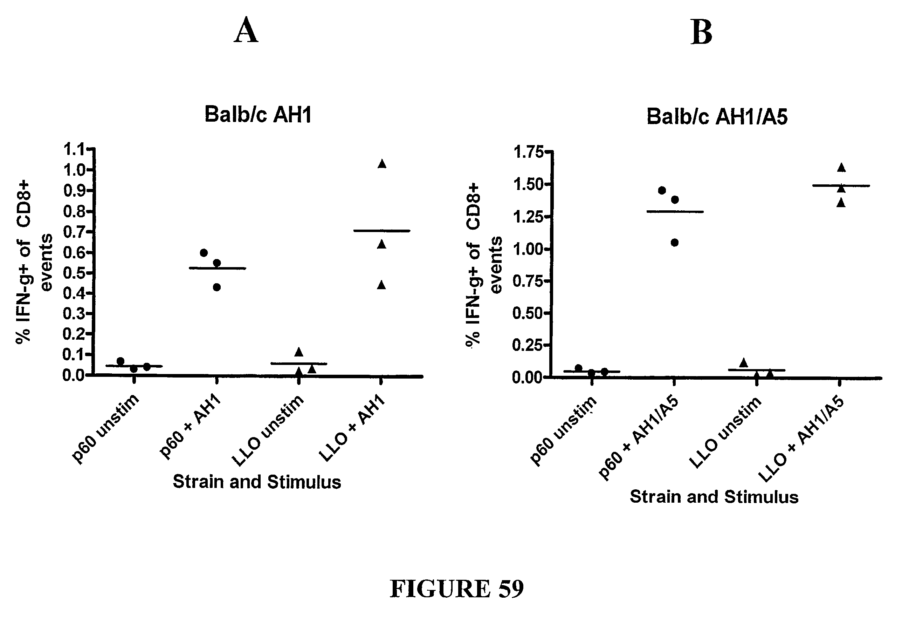

Figure 59A and B show delivery of a heterologous antigen to the MHC Class I pathway by *Listeria* expressing either a p60-antigen chimera or an LLO signal peptide-antigen fusion protein. The heterologous antigen used in this experiment was AH1-A5. Vaccination was with *Listeria* engineered to comprise a p60 protein chimera expression cassette encoding AH1-A5 (fused to the OVA SL8 peptide) inserted within the p60 polypeptide sequence including the N-terminal p60 signal peptide sequence ("p60-based construct"), or *Listeria* engineered to encode an LLO signal peptide linked to a nucleic acid encoding the same antigen, AH1-A5 embedded within OVA ("LLO-based construct"). Both of these constructs used the Listerial promoter *hly*. p60 is a Listerial peptidoglycan autolysin that is secreted by a secA2 pathway, while LLO is listeriolysin.

To generate the p60-based construct, the nucleic acid encoding p60 was engineered to contain a PstI cloning site, where the PstI cloning site represented a silent mutation, i.e., resulting in no change in the encoded amino acid sequence. The PstI site was located between the N-terminal signal sequence and the first of two LysM cell wall binding domains in the p60 sequence. A polynucleotide encoding a heterologous polypeptide comprising the AH1-A5 epitope (SPSYAYHQF (SEQ ID NO:73)) and SL8 epitope (SIINFEKL (SEQ ID NO:123)) was inserted in frame into the PstI cloning site. The coding sequences for these epitopes were separated by a unique XhoI site and codon-optimized for expression in *L. monocytogenes.* The insertion into the PstI site occurred at the equivalent of nucleotide base number 199 of p60. The first 1-70 amino acids of the p60 coding sequence were codon-optimized for expression in *L. monocytogenes.* Accordingly, the first 27 amino acids corresponding to the signal peptide were expressed from optimal codons for expression in *L. monocytogenes.* The antigen expression cassette further contained unique 5' and 3' KpnI and SacI sites, respectively for insertion into the MCS of the pPL2 plasmid, for site-specific integration adjacent to the tRNA^{Arg} gene of the *L. monocytogenes* genome. The LLO-based construct comprised a sequence encoding an LLO signal sequence operably linked to a nucleic acid encoding AH1-A5 within OVA (without use of any codon-optimization). Thus, in the present study, the signal peptide was either from *Listeria* LLO or from *Listeria* p60.

The constructs were placed into pPL2, a vector that mediates site-specific recombination with *Listeria* genome, and inserted into the *Listeria* genome.

Figure 59A and B show the immune response to a vaccination (tail vein) of *Listeria* expressing the AH1-A5 antigen with p60 signal sequence/autolysin as a p60 chimera, and immune response to vaccination *of Listeria* expressing AH1-A5 antigen linked with the LLO signal sequence. In the x-axis of the figure, "Unstim" means that no peptide was added to the wells (i.e., the cells were unstimulated), while "AH1" means that the AH1 nonapeptide was added to the wells, and "AH1-A5" means that the AH1-A5 nonapeptide was added to the wells. All bacterial vaccines were engineered to contain an integrated nucleic acid encoding AH1-A5 (the bacterial vaccines did not encode AH1) (see, e.g., Slansky, et al. (2000) Immunity 13:529-538). Where the vaccination was done with the *Listeria* comprising the p60-based constructs, the strain is indicated on the x-axis of the figure as "p60." Where the vaccination was done with *Listeria* comprising the LLO-based constructs, the strain is indicated on the x-axis of the figure as "LLO."

The overall protocol for vaccination with *Listeria* expressing the P60-based construct was as follows: (1) Mice were vaccinated with *Listeria* (tail vein (i.v.)) containing an integrated nucleic acid, where the integrated nucleic acid encoded p60 containing a nucleic acid encoding AH1-A5 inserted at nucleotide 199 of p60. In other words, the nucleic acid encoding AH1-A5 antigen was in frame with and operably linked with p60 signal sequence and with p60 autolysin. The nucleic acid encoding AH1-A5 was codon optimized for expression in *L. monocytogenes*; (2) Seven days post infection, the spleens were removed; (3) Spleen cells were dissociated, placed in wells, and the spleen cells were incubated with either no added peptide (Figure 59A and 59B), with added AH1 (Figure 59A), or with added AH1-A5 (Figure 59B), as indicated on the x-axis; (4) After adding the peptide, cells were incubated for five hours, followed by assessment of the percent of IFNgamma expressing CD8⁺ T cells by FACS analysis. An analogous protocol was used for vaccination with *Listeria* expressing the LLO-based construct.

The results demonstrate that the *Listeria* vaccines stimulated CD8⁺ T cell expression of IFNgamma, where the added peptide was AH1 (Figure 59A) or where the added peptide was AH1-A5 (Figure 59B). Stimulation was somewhat higher where integrated AH1-A5 was operably linked with LLO signal sequence, and stimulation was somewhat lower when integrated AH1-A5 was operably linked with p60 signal sequence (Figure 59A and B).

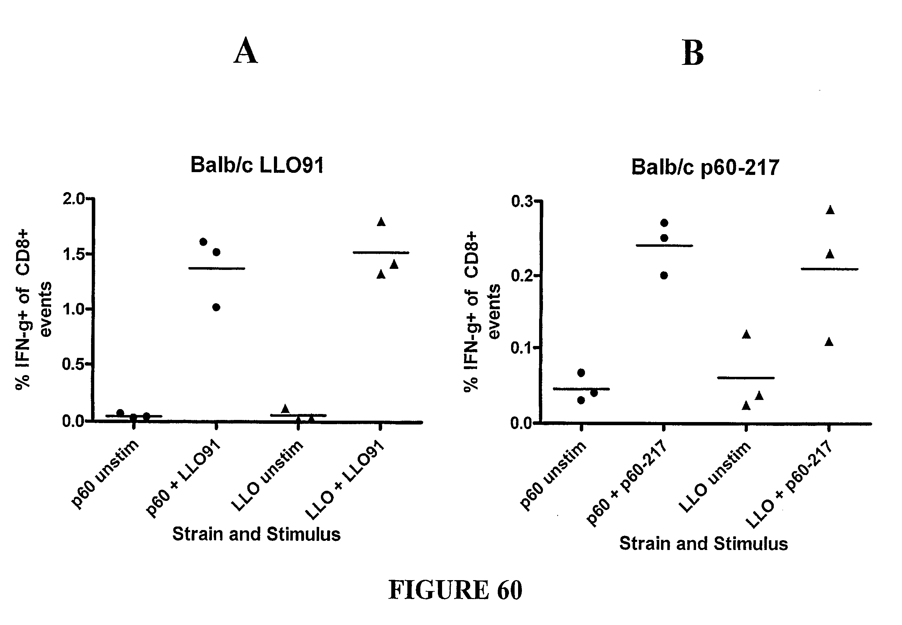

Figure 60A and B show experiments conducted with the same two *Listeria* vaccines as described above, i.e., as shown in Figs.59A and B. Figure 60A shows results where mice were vaccinated with the *Listeria* engineered to contain the p60-based construct ("p60")or with the *Listeria* engineered to contain the LLO-based construct ("LLO"). As indicated on the x-axis of Figure 60A, the cell based assays were supplemented with no peptide (unstimulated; "unstim") or with LLO₉₁₋₉₉ peptide ("LLO91"; Badovinac and Harty (2000) J. Immunol. 164:6444-6452). The results demonstrated a similar immune response (IFNgamma expression) where the *Listeria* vaccine contained the p-60 based construct or the LLO-based construct..The stimulated immune response in Fig. 60A, as reflected in the results from the cell-based assay, is due to the *Listeria's* endogenous expression of native LLO.

Figure 60B shows results where mice were vaccinated with *Listeria* engineered to contain the p60-based construct, where the *hly* promoter and signal peptide sequences were operably linked with a nucleic acid encoding AH1-A5, or with *Listeria* engineered to contain the LLO-based construct, where the *hly* promoter and signal peptide were operably linked with a nucleic acid encoding AH1-A5. The added peptides were either no peptide (unstimulated; "unstim") or p60₂₁₇₋₂₂₅ ("p60-217"; Sijts, et al. (1997) J. Biol. Chem. 272:19261-19268), as indicated on the x-axis. The stimulated immune response in Fig. 60B, as reflected in the results from the cell based assay, is due to the *Listeria*'*s* expression of endogenous p60 for the LLO-based construct and the combination of endogenous p60 and the expressed p60 protein chimera sequence for the p60-based construct.

### B. Therapeutic efficacy of Listeria expressing human mesothelin

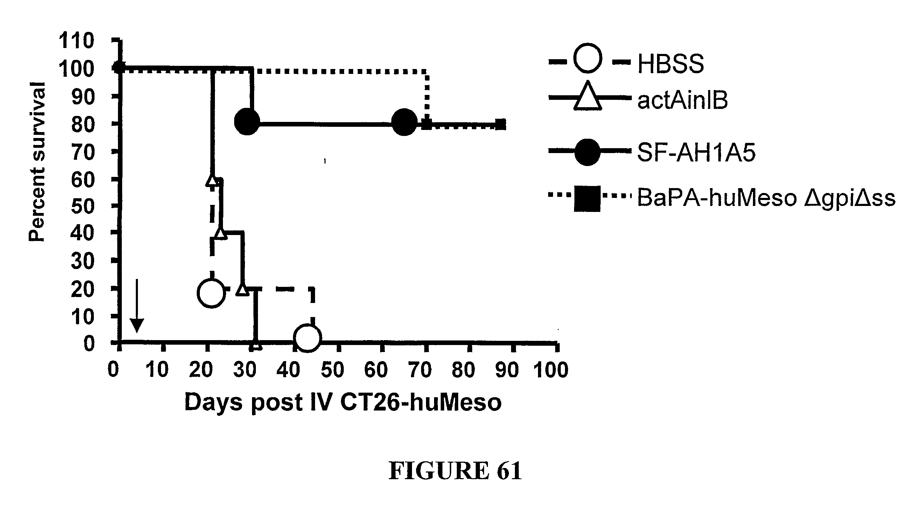

The results depicted in Figure 61 reveal that vaccination with *Listeria* expressing human mesothelin (huMesothelin) prolongs survival in tumor-bearing mice, where the tumor cells in the mice had been engineered to express human mesothelin. The tumor cells were CT26 cells expressing human mesothelin and the mice were Balb/c mice. (All CT26 tumor studies described herein involved Balb/c mice.) In one of the expression cassettes, a sequence encoding a non-Listerial signal sequence was operably linked in frame with a codon-optimized sequence encoding human mesothelin (deleted of its signal sequence and GPI anchor). The expression cassette encoding a signal peptide fused with human mesothelin (ΔGPIΔSS) was administered to tumor-bearing mice in a *Listeria* vaccine in studies on the effect of the fusion protein on immune response to tumors. The expression cassette encoding the mesothelin fusion protein had been integrated into the *Listeria,* chromosome. On Day 0, 2 x 10⁵ CT26 cells expressing human mesothelin (CT.26 huMeso+) were injected intravenously into the Balb/c mice. Vaccination of the mice was in the tail vein (i.v.). Inoculation with 1 e 7 colony forming units (CFU) *Listeria* (i.v.) occurred at day 3.

Figure 61 shows the percent survival (shown on y-axis) of the mice to CT26 tumor expressing human mesothelin, where the vaccine comprises Hank's Balanced Salt Solution (HBSS) (a sham vaccine; "HBSS"); *Listeria ΔactAΔinlB* expressing SF-AH1A5 from an integrated expression cassette (positive control vaccine; "SF-AH1A5"); or *Listeria ΔactAΔinlB* comprising an expression cassette encoding *B. anthracis* Protective Antigen signal sequence (encoded by a non-codon optimized sequence) fused with huMesothelin (encoded by a codon-optimized sequence), where the hMesothelin had a deleted signal sequence and a deleted region encoding the hydrophobic gpi-anchoring peptide ("BaPA-huMeso ΔgpiΔss"). *Listeria* bearing the SF-AH1A5 construct and the BaPA-huMeso ΔgpiΔss construct contained these constructs as chromosomally integrated constructs. The nucleic acid molecule encoding SF-AH1A5 and the nucleic acid molecule encoding the BaPA-huMeso ΔgpiΔss construct had been integrated into the *Listeria* genome using pPL2. SF is shorthand for an eight amino acid peptide derived from ovalbumin, also known as SL8 (see, e.g., Shastri and Ganzalez (1993) J. Immunol. 150:2724-2736). The abbreviations "SF-AH1A5," "SF-AH1-A5," and "OVA/AH1-A5" refer to AH1-A5 connected to an ovalbumin scaffold. "SF AH1-A5" refers to the AH1-A5 (SPSYAYHQF (SEQ ID NO:73)) and the SF peptide fused to the N-terminus of amino acids 138 to 386 of GenBank Accession. No. P01012 (ovalbumin). The polynucleotides encoding "SF-AH1A5," in this example, comprised a codon-optimized nucleic acid encoding AH1-A5 and a non-codon optimized nucleic acid encoding the ovalbumin-derived sequence.

The results demonstrate that a single immunization with *Listeria* expressing huMesothelin prolongs survival of mice containing huMesothelin-expressing tumors. The survival percentage was highest with the chromosomally integrated *B. anthracis* Protective Antigen signal sequence fused with the Δsignal sequence/Δgpi huMesothelin (BaPA-huMeso ΔgpiΔss; closed squares). Survival was lowest where "vaccination" was with the control salt solution.

### C. Reduction in lung tumor nodule level in tumor-bearing mice vaccinated with Listeria expressing human Mesothelin due to mesothelin-specific anti-tumor- efficacy

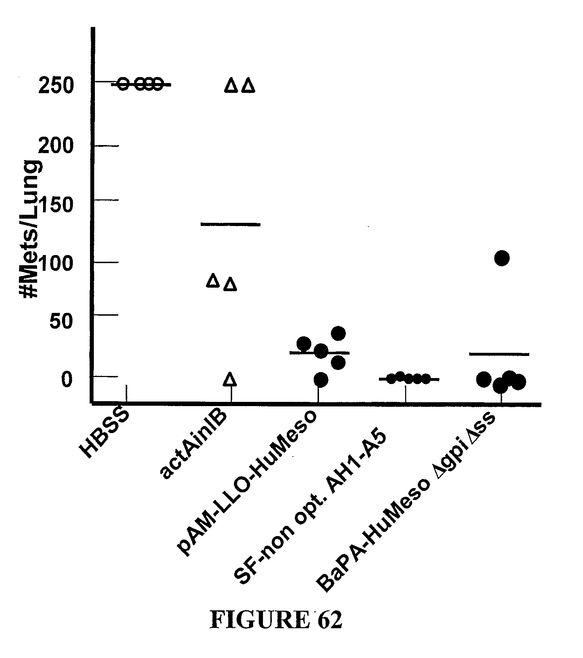

The data in Figure 62 demonstrate that the level of lung tumor nodules is reduced by vaccination with *Listeria ΔactAΔinlB* expressing human mesothelin, where the tumor cells were engineered to express human mesothelin. The mouse strain was Balb/c and the lung tumor cells were CT26 cells harboring a vector expressing human mesothelin. On Day 0, 2 x 10⁵ CT26 cells expressing human mesothelin were administered intravenously to the Balb/c mice. Sequences encoding various signal sequences were operably linked in frame with codon-optimized sequences encoding human mesothelin in expression cassettes. The expression cassettes encoding various signal peptides fused with human mesothelin were administered to the tumor-bearing mice via *Listeria* vaccines comprising the expression cassettes. On Day 3, 1 x 10⁷ CFU/100 µL of the *Listeria* vaccines were administered to the tumor-bearing mice intravenously. Negative control vaccinations were with HBSS or *Listeria ΔactAΔinlB.* Positive control vaccinations were with *Listeria* expressing an OVA fusion protein comprising AH1 A5 (in frame with the OVA sequence). (The OVA fusion protein comprising AH1A5 was encoded by a non-codon optimized expression cassette.) On Day 19, the mice were sacrificed, their lungs harvested, and the lung tumor nodules counted.

The *Listeria* vaccines reduced the number of metastases in the lungs. Control vaccines involving only HBBS or *Listeria ΔactAΔinlB* resulted in a detected consistent 250 metastases per lung and an average of 135 metastasis per lung, respectively. *Listeria* bearing plasmid (pAM401) encoding LLO signal peptide fused to human mesothelin ("pAM-LLO-HuMeso") showed about 25 metastases per lung. The polynucleotide sequences of the pAM-LLO-HuMeso plasmid that encoded the LLO signal peptide and the human mesothelin sequence were codon-optimized for expression in *L. monocytogenes. Listeria* bearing integrated sequences encoding *B. anthnacis* Protective Antigen signal sequence (BaPA) fused with huMesothelin (Δgpi/Δsignal sequence) ("BaPA-HuMeso ΔgpiΔss") also showed on the average about 25 metastases per lung on average. The polynucleotide in BaPA-HuMeso ΔgpiΔss that encoded the *B. anthracis* Protective Antigen signal sequence was not codon-optimized, whereas the polynucleotide that encoded the human mesothelin sequence deleted of the mesothelin signal peptide and GPI anchor was codon-optimized for expression in *L. monocytogenes.*

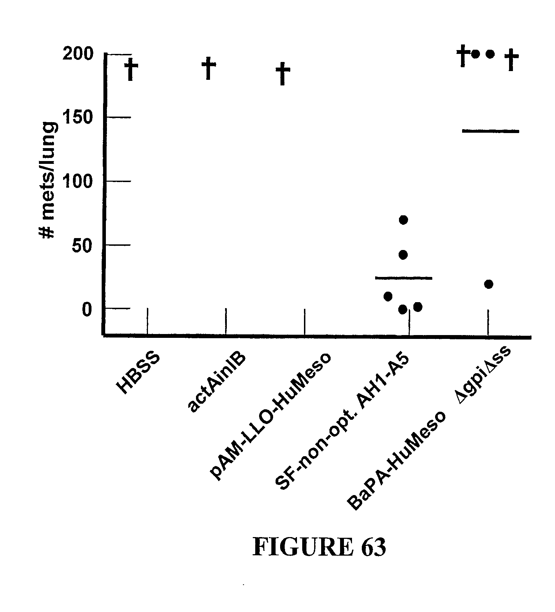

Figure 63 shows the results of a control study using mice comprising lung tumor nodules generated using CT.26 parental target cells. Balb/c mice were used, but wt CT26 was instead injected (2 x 10⁵ cells (i.v.) on Day 0). The study demonstrates that the anti-tumor efficacy of vaccination with the *Listeria* vaccine expressing mesothelin fusion proteins is mesothelin specific. Sequences encoding various signal sequences were operably linked in frame with codon-optimized sequences encoding human mesothelin in expression cassettes. (The constructs used in this experiment were identical to those used in the experiments above to generate the data shown in Figure 62.) The expression cassettes encoding various signal peptides fused with human mesothelin were administered to the tumor-bearing mice via *Listeria* vaccines comprising the expression cassettes. Vaccination was in the tail vein (1 x 10⁷ CFU/100 µL i.v. on Day 3). In this particular study, the tumor cells did not express human mesothelin. Survival was determined. Where the data was available, the number of lung metastases was also measured. There were a total of five mice in each vaccination group. Negative control inoculation involved HBSS or *Listeria ΔactAΔinlB.* Positive control inoculation involved *Listeria* expressing an OVA fusion comprising AH1A5 (not codon-optimized).

The results are shown in Figure 63. Crosses indicate failure to survive and each vaccination group contained 5 mice. With the positive control inoculation, the mice survived, and the number of detected metastases in the lung was on the average about 25 per lung. As the tumor cells were not engineered to express human mesothelin, the mice inoculated with *Listeria* harboring a plasmid expressing LLO signal peptide fused with human mesothelin ("pAM-LLO-HuMeso") did not survive. Where mice were inoculated with *Listeria* bearing chromosomally integrated *B. anthracis* Protective Antigen secretory sequence (BaPA; encoded by a non-codon optimized nucleotide sequence) fused with with human mesothelin (Δgpi/Δsignal sequence) ("BaPA-HuMeso ΔgpiΔss"), some survived but others failed to survive.

### D. Vaccination witch Listeria expressing codon-optimized human mesothelin reduces tumor volume

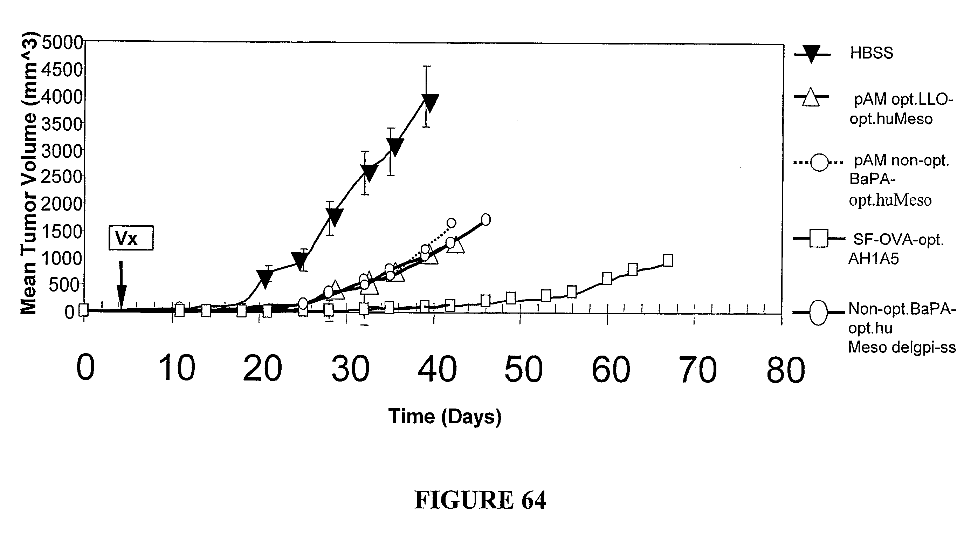

Figure 64 shows vaccination with *Listeria* (*ΔactAΔinlB*) expressing human mesothelin from expression cassettes comprising codon-optimized mesothelin codon sequences reduces tumor volume.

Sequences encoding various signal sequences were operably linked in frame with codon-optimized sequences encoding human mesothelin in expression cassettes. The expression cassettes encoding various signal peptides fused with human mesothelin were administered to tumor-bearing mice via *Listeria* vaccines comprising the expression cassettes. The *Listeria* vaccines expressing human mesothelin that were used for vaccination of the tumor-bearing mice in this study include the following: *Listeria* (*ΔactAΔinlB L. monocytogenes*) bearing a pAM401 plasmid expressing and secreting LLO signal peptide (encoded by a sequence codon-optimized for expression in *L. monocytogenes*) fused with human mesothelin ("pAM opt.LLO-opt.huMeso"); *Listeria* bearing a pAM401 plasmid expressing *B. anthracis* Protective Antigen signal sequence (encoded by a non-codon optimized expression cassette) fused with huMesothelin ("pAM non-opt.BaPA-opt.huMeso"); and *Listeria* comprising an integrated expression cassette encoding *B. anthracis* Protective Antigen signal peptide (encoded by a non-codon optimized sequence) fused with huMesothelin, where the huMesothelin had a deleted signal sequence and a deleted region encoding the hydrophobic gpi-anchoring peptide ("Non-opt.BaPA-opt.huMeso delgpi-ss").

In the study, Balb/c mice were implanted subcutaneously with 2 x 10⁵ cells of CT26 murine colon tumor cells engineered to expression human mesothelin (Day 0). Five mice were included in each vaccination group. On Day 3 following injection with the CT26 cells, the mice were vaccinated with non-Listerial control or 1 x 10⁷ colony forming units (CFU) of the *Listeria* vaccine intravenously. Negative control inoculation involved HBSS. Positive control inoculation involved *Listeria* expressing SF-AH1A5 (codon optimized). (SF is an eight amino acid peptide derived from ovalbumin, also known as SL8 (see, e.g., Shastri and Ganzalez (1993) J. Immunol. 150:2724-2736).) At various time points, the mean tumor volume was determined.

The results of this study are shown in Figure 64. The results demonstrated that vaccination with *Listeria* expressing human mesothelin fused to various signal peptides reduces tumor volume. Vaccination with *Listeria* expressing a *B. anthracis* Protective Antigen signal peptide fused with human mesothelin was protective (open circles with dotted line). Vaccination with *Listeria* expressing plasmid-encoded human mesothelin fused to LLO signal peptide was protective (open triangles). Vaccination with *Listeria* comprising a chromosomally integrated expression cassette encoding *B. anthracis* Protective Antigen (non-codon optimized nucleic acid) signal peptide fused with human mesothelin (Δgpi/Δsignal sequence) (open ovals with solid line) was also protective. Regarding the positive controls, *Listeria* expressing chromosomally integrated SF-AH1A5 (open squares) were also protective. The highest tumor volume, and earliest time of tumor growth onset, occurred in mice receiving the sham vaccine (HBSS).

### E. Immunogenicity of a Listeria vaccine expressing human mesothelin fused to a non-Listerial signal sequence

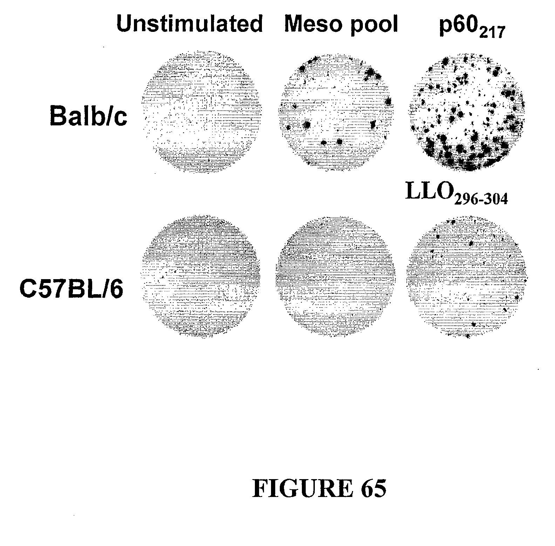

Figure 65 depicts the immunogenicity of *a Listeria* Δ*actA*/*ΔinlB*-hMesothelin strain, where the *Listeria* contained a chromosomally integrated nucleic acid encoding hMesothelin fused to a *Bacillus anthracis* signal peptide (optimized Ba PA hMeso ΔGPIΔSS). ELISPOT assays were used to assess immune response, where the assays were sensitive to expression of interferon-gamma.

The study comprised the following steps: (1) Mice (Balb/c mice or C57BL/6 mice) were vaccinated (i.v.) with the *Listeria* comprising an integrated expression cassette encoding *B. anthracis* Protective Antigen signal peptide (encoded by a non-codon optimized sequence) fused with huMesothelin (encoded by a codon-optimized sequences in which the mesothelin signal sequence and hydrophobic gpi-anchoring sequences had been deleted); (2) After 7 days, the spleens were removed; (3) The cells removed from the spleens were dispersed in wells. Each well received about 200,000 spleen cells; (4) One of three kinds of medium were added to the wells, as indicated. Spleen cells from studies with Balb/c mice received medium only ("Unstimulated"), mesothelin peptide pool ("Meso pool"), or p60₂₁₇₋₂₂₅ ("p60₂₁₇")- Spleen cells from studies with C57BL/6 received medium only ("Unstimulated"), mesothelin peptide pool ("Meso pool"), or LLO₂₉₆₋₃₀₄ ("LLO₂₉₆₋₃₀₄''); (5) ELISPOT assays were performed to determine number of immune cells responding to the added peptide(s). The mesothelin peptide pool comprised 153 different peptides, where these peptides spanned the entire sequence of hMesothelin, where each peptide was 15 amino acids long, overlapping the adjacent peptides by 11 amino acids.

The results of the ELISPOT assays are shown in Figure 65. The results indicated that the *Listeria* vaccine expressing human mesothelin fused to *B. anthracis* signal peptide was capable of inducing an immune response to mesothelin in Balb/c mice. A higher IFN-gamma response to *Listeria*-expressed hMesothelin was observed with the Balb/c mouse immune system than with the C57BL/6 immune system. ELISPOT signal to p60 or LLO was in response to the *Listeria's* naturally occurring p60 and LLO proteins.

## Claims

1. A recombinant *Listeria* bacterium comprising a recombinant nucleic acid molecule, wherein the recombinant nucleic acid molecule comprises:
(a) a first polynucleotide encoding a secretory signal peptide that is a signal peptide native to *Listeria*; wherein the first polynucleotide is codon-optimized for expression in the *Listeria* bacterium such that the codons encoding said signal peptide sequence are the codons of Table 3:
| Amino Acid | Optimal *Listeria* Codon |
|---|---|
| Alanine | GCA |
| Arginine | CGT |
| Asparagine | AAT |
| Aspartate | GAT |
| Cysteine | TGT |
| Glutamine | CAA |
| Glutamate | GAA |
| Glycine | GGT |
| Histidine | CAT |
| Isoleucine | ATT |
| Leucine | TTA |
| Lysine | AAA |
| Methionine | ATG |
| Phenylalanine | TTT |
| Proline | CCA |
| Serine | AGT |
| Threonine | ACA |
| Tryptophan | TGG |
| Tyrosine | TAT |
| Valine | GTT |
and
(b) a second polynucleotide sequence encoding a polypeptide heterologous to the signal peptide, wherein the second polynucleotide sequence is in the same translational reading frame as the first polynucleotide sequence,
wherein the recombinant nucleic acid molecule encodes a fusion protein comprising the signal peptide and the polypeptide.

2. The recombinant *Listeria* bacterium of claim 1, which comprises an expression cassette comprising the recombinant nucleic acid molecule, wherein the expression cassette further comprises a promoter operably linked to both the first and second polynucleotides of the recombinant nucleic acid molecule.

3. The recombinant *Listeria* bacterium of claim 2, wherein the expression cassette is a polycistronic expression cassette.

4. The recombinant *Listeria* bacterium of claim 2, wherein the promoter is a prfA-dependent promoter.

5. The recombinant *Listeria* bacterium of claim 1, wherein the signal peptide is a LLO signal peptide from *Listeria monocytogenes* or is a p60 signal peptide from *Listeria monocytogenes.*

6. The recombinant *Listeria* bacterium of claim 1, wherein both the first and second polynucleotides are codon-optimized for expression in the *Listeria* bacterium.

7. The recombinant *Listeria* bacterium of claim 1, wherein the bacterium is *Listeria monocytogenes.*

8. The recombinant *Listeria* bacterium of claim 1, wherein the signal peptide is a signal peptide that utilizes a secA1 secretion pathway.

9. The recombinant *Listeria* bacterium of claim 1, wherein the polypeptide encoded by the second polynucleotide comprises is an antigen selected from the group consisting of a tumor-associated antigen, a polypeptide derived from a tumor-associated antigen, an infectious disease antigen, and a polypeptide derived from an infectious disease antigen.

10. The recombinant *Listeria* bacterium of claim 1, wherein the polypeptide encoded by the second polynucleotide comprises an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA, or comprises a polypeptide derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, and CEA.

11. The recombinant *Listeria* bacterium of claim 10, wherein the polypeptide encoded by the second polynucleotide comprises mesothelin, or an antigenic fragment or antigenic variant thereof.

12. The recombinant *Listeria* bacterium of claim 11, wherein the polypeptide encoded by the second polynucleotide comprises human mesothelin deleted of its signal peptide and GPI anchor.

13. The recombinant *Listeria* bacterium of claim 1, which is attenuated for cell-to-cell spread, entry into non-phagocytic cells, or proliferation.

14. The recombinant *Listeria* bacterium of claim 1, which is deficient with respect to ActA, Internalin B, or both ActA and Internalin B.

15. The recombinant *Listeria* bacterium of claim 1, wherein the nucleic acid of the recombinant bacterium has been modified by reaction with a nucleic acid targeting alkylator or a psoralen so that proliferation of the bacterium is attenuated.

16. An immunogenic composition or vaccine comprising the recombinant *Listeria* bacterium of claim 1, 10, 14, or 15.

17. The recombinant *Listeria* bacterium of claim 1, 10, 14, or 15 for use in a method of treatment of a human or mammalian host.

18. The recombinant *Listeria* bacterium for use according to claim 17, wherein the treatment is of a cancer or of an infectious disease.

19. The recombinant *Listeria* bacterium for use according to claim 17, wherein the treatment induces an immune response in a host to an antigen, and wherein the polypeptide encoded by the second polynucleotide comprises the antigen.

20. A method of making a recombinant *Listeria* bacterium comprising a recombinant nucleic acid molecule, wherein the recombinant nucleic acid molecule comprises:
(a) a first polynucleotide encoding a secretory signal peptide that is a signal peptide native to *Listeria*; and
(b) a second polynucleotide sequence encoding a polypeptide heterologous to the signal peptide, wherein the second polynucleotide sequence is in the same translational reading frame as the first polynucleotide sequence,
wherein the recombinant nucleic acid molecule encodes a fusion protein comprising the signal peptide and the polypeptide;
wherein said method comprises
(i) providing said recombinant nucleic acid molecule and codon-optimizing the first polynucleotide to replace at least one codon of the native coding sequence of the polynucleotide with a codon that is more frequently used by Listeria, and
(ii) expressing said recombinant nucleic acid molecule in the Listeria bacterium.

21. The method of claim 20 wherein the bacterium is as defined in any one of claims 1-15.

## Patentansprüche

1. Rekombinantes Listeria-Bakterium, umfassend ein rekombinantes Nucleinsäuremolekül, worin das rekombinante Nucleinsäuremolekül umfasst:
(a) ein erstes Polynucleotid, das für ein sekretorisches Signalpeptid kodiert, das ein für Listeria natives Signalpeptid ist; worin das erste Polynucleotid zur Expression in dem Listeria-Bakterium Codon-optimiert ist, sodass die Codons, die für die Signalpeptidsequenz kodieren, die Codons aus Tabelle 3 sind:
| Aminosäure | Optimales Listeria-Codon |
|---|---|
| Alanin | GCA |
| Arginin | CGT |
| Asparagin | AAT |
| Aspartat | GAT |
| Cystein | TGT |
| Glutamin | CAA |
| Glutamat | GAA |
| Glycin | GGT |
| Histidin | CAT |
| Isoleucin | ATT |
| Leucin | TTA |
| Lvsin | AAA |
| Methionin | ATG |
| Phenylalanin | TTT |
| Prolin | CCA |
| Serin | AGT |
| Threonin | ACA |
| Tryptophan | TGG |
| Tyrosin | TAT |
| Valin | GTT |
und
(b) eine zweite Polynucleotidsequenz, die für ein zu dem Signalpeptid heterologes Polypeptid kodiert, worin die zweite Polynucleotidsequenz in demselben Translationsleserahmen wie die erste Polynucleotidsequenz liegt,
worin das rekombinante Nucleinsäuremolekül für ein Fusionsprotein kodiert, das das Signalpeptid und das Polypeptid umfasst.

2. Rekombinantes Listeria-Bakterium nach Anspruch 1, das eine Expressionskassette umfasst, die das rekombinante Nucleinsäuremolekül umfasst, worin die Expressionskassette ferner einen Promotor umfasst, der mit sowohl dem ersten als auch dem zweiten Polynucleotid des rekombinanten Nucleinsäuremoleküls operabel verbunden ist.

3. Rekombinantes Listeria-Bakterium nach Anspruch 2, worin die Expressionskassette eine polycistronische Expressionskassette ist.

4. Rekombinantes Listeria-Bakterium nach Anspruch 2, worin der Promotor ein prfA-abhängiger Promotor ist.

5. Rekombinantes Listeria-Bakterium nach Anspruch 1, worin das Signalpeptid ein LLO-Signalpeptid aus Listeria monocytogenes ist oder ein p60-Signalpeptid aus Listeria monocytogenes ist.

6. Rekombinantes Listeria-Bakterium nach Anspruch 1, worin sowohl das erste als auch das zweite Polynucleotid zur Expression in dem Listeria-Bakterium codonoptimiert sind.

7. Rekombinantes Listeria-Bakterium nach Anspruch 1, worin das Bakterium Listeria monocytogenes ist.

8. Rekombinantes Listeria-Bakterium nach Anspruch 1, worin das Signalpeptid ein Signalpeptid ist, das einen secA1-Sekretionspfad verwendet.

9. Rekombinantes Listeria-Bakterium nach Anspruch 1, worin das Polypeptid, für das das zweite Polynucleotid kodiert, ein Antigen umfasst, das aus der aus einem tumorassoziierten Antigen, einem von einem tumorassoziierten Antigen abgeleiteten Polypeptid, einem Infektionserkrankungsantigen und einem von einem Infektionserkrankungsantigen abgeleiteten Polypeptid bestehenden Gruppe ausgewählt ist.

10. Rekombinantes Listeria-Bakterium nach Anspruch 1, worin das Polypeptid, für das das zweite Polynucleotid kodiert, ein Antigen umfasst, das aus der aus K-Ras, H-Ras, N-Ras, 12-K-Ras, Mesothelin, PSCA, NY-ESO-1, WT-1, Survivin, gp100, PAP, Proteinase 3, SPAS-1, SP-17, PAGE-4, TARP und CEA bestehenden Gruppe ausgewählt ist, oder ein Polypeptid umfasst, das von einem Antigen abgeleitet ist, das aus der aus K-Ras, H-Ras, N-Ras, 12-K-Ras, Mesothelin, PSCA, NY-ESO-1, WT-1, Survivin, gp100, PAP, Proteinase 3, SPAS-1, SP-17, PAGE-4, TARP und CEA bestehenden Gruppe ausgewählt ist.

11. Rekombinantes Listeria-Bakterium nach Anspruch 10, worin das Polypeptid, für das das zweite Polynucleotid kodiert, Mesothelin oder ein antigenes Fragment oder eine antigene Variante davon umfasst.

12. Rekombinantes Listeria-Bakterium nach Anspruch 11, worin das Polypeptid, für das das zweite Polynucleotid kodiert, menschliches Mesothelin umfasst, bei dem sein Signalpeptid und GPI-Anker deletiert wurde.

13. Rekombinantes Listeria-Bakterium nach Anspruch 1, das zur Zelle-zu-Zelle-Ausbreitung, zum Eintritt in nicht phagozytische Zellen oder Proliferation attenuiert ist.

14. Rekombinantes Listeria-Bakterium nach Anspruch 1, das in Bezug auf ActA, Internalin B oder sowohl ActA als auch Internalin B defizient ist.

15. Rekombinantes Listeria-Bakterium nach Anspruch 1, worin die Nucleinsäure des rekombinanten Bakteriums durch eine Reaktion mit einem auf Nucleinsäure abzielenden Alkylator oder einem Psoralen modifiziert wurde, sodass eine Proliferation des Bakteriums attenuiert ist.

16. Immunogene Zusammensetzung oder Vakzine, umfassend das rekombinante Listeria-Bakterium nach Anspruch 1, 10, 14 oder 15.

17. Rekombinantes Listeria-Bakterium nach Anspruch 1, 10, 14 oder 15 zur Verwendung in einem Verfahren zum Behandeln eines menschlichen Wirts oder eines Säugetier-Wirts.

18. Rekombinantes Listeria-Bakterium zur Verwendung nach Anspruch 17, worin die Behandlung eine von einem Krebs oder von einer Infektionserkrankung ist.

19. Rekombinantes Listeria-Bakterium zur Verwendung nach Anspruch 17, worin die Behandlung in einem Wirt eine Immunantwort auf ein Antigen induziert und worin das Polypeptid, für das das zweite Polynucleotid kodiert, das Antigen umfasst.

20. Verfahren zum Herstellen eines rekombinanten Listeria-Bakteriums, das ein rekombinantes Nucleinsäuremolekül umfasst, worin das rekombinante Nucleinsäuremolekül umfasst:
(a) ein erstes Polynucleotid, das für ein sekretorisches Signalpeptid kodiert, das ein Signalpeptid ist, das für Listeria nativ ist; und
(b) eine zweite Polynucleotidsequenz, die für ein Polypeptid kodiert, das heterolog zu dem Signalpeptid ist, worin die zweite Polynucleotidsequenz in demselben Translationsleserahmen liegt wie die erste Polynucleotidsequenz,
worin das rekombinante Nucleinsäuremolekül für ein Fusionsprotein kodiert, das das Signalpeptid und das Polypeptid umfasst;
worin das Verfahren umfasst
(i) Bereitstellen des rekombinanten Nucleinsäuremoleküls und Codon-Op-timieren des ersten Polynucleotids, um zumindest ein Codon der nativen Kodierungssequenz des Polynucleotids mit einem Codon zu ersetzen, das von Listeria häufiger verwendet wird, und
(ii) Exprimieren des rekombinanten Nucleinsäuremoleküls in dem Listeria-Bakterium.

21. Verfahren nach Anspruch 20, worin das Bakterium ist wie in einem der Ansprüche 1-15 definiert.

## Revendications

1. Bactérie *Listeria* recombinante comprenant une molécule d'acide nucléique recombinante, où la molécule d'acide nucléique recombinante comprend :
(a) un premier polynucléotide codant pour un peptide signal de sécrétion qui est un peptide signal natif de *Listeria ;* où le premier polynucléotide est optimisé en codons pour une expression dans la bactérie *Listeria* de sorte que les codons codant pour ladite séquence de peptide signal soient les codons du Tableau 3 :
| Acide aminé | Codon de *Listeria* optimal |
|---|---|
| Alanine | GCA |
| Arginine | CGT |
| Asparagine | AAT |
| Aspartate | GAT |
| Cystéine | TGT |
| Glutamine | CAA |
| Glutamate | GAA |
| Glycine | GGT |
| Histidine | CAT |
| Isoleucine | ATT |
| Leucine | TTA |
| Lysine | AAA |
| Méthionine | ATG |
| Phénylalanine | TTT |
| Proline | CCA |
| Sérine | AGT |
| Thréonine | ACA |
| Tryptophane | TGG |
| Tyrosine | TAT |
| Valine | GTT |
et
(b) une seconde séquence polynucléotidique codant pour un polypeptide hétérologue par rapport au peptide signal, où la seconde séquence polynucléotidique est dans le même cadre de lecture traductionnel que la première séquence polynucléotidique,
où la molécule d'acide nucléique recombinante code pour une protéine de fusion comprenant le peptide signal et le polypeptide.

2. Bactérie *Listeria* recombinante selon la revendication 1, qui comprend une cassette d'expression comprenant la molécule d'acide nucléique recombinante, où la cassette d'expression comprend en outre un promoteur en liaison fonctionnelle avec à la fois le premier et le second polynucléotide de la molécule d'acide nucléique recombinante.

3. Bactérie *Listeria* recombinante selon la revendication 2, où la cassette d'expression est une cassette d'expression polycistronique.

4. Bactérie *Listeria* recombinante selon la revendication 2, où le promoteur est un promoteur dépendant de prfA.

5. Bactérie *Listeria* recombinante selon la revendication 1, où le peptide signal est un peptide signal LLO de *Listeria monocytogenes* ou est un peptide signal p60 de *Listeria monocytogenes.*

6. Bactérie *Listeria* recombinante selon la revendication 1, où à la fois le premier et le second polynucléotide sont optimisés en codons pour une expression dans la bactérie *Listeria.*

7. Bactérie *Listeria* recombinante selon la revendication 1, où la bactérie est *Listeria monocytogenes.*

8. Bactérie *Listeria* recombinante selon la revendication 1, où le peptide signal est un peptide signal qui utilise une voie de sécrétion secA1.

9. Bactérie *Listeria* recombinante selon la revendication 1, où le polypeptide codé par le second polynucléotide comprend est un antigène sélectionné dans le groupe consistant en un antigène associé aux tumeurs, un polypeptide dérivé d'un antigène associé aux tumeurs, un antigène de maladie infectieuse, et un polypeptide dérivé d'un antigène de maladie infectieuse.

10. Bactérie *Listeria* recombinante selon la revendication 1, où le polypeptide codé par le second polynucléotide comprend un antigène sélectionné dans le groupe consistant en K-Ras, H-Ras, N-Ras, 12-K-Ras, la mésothéline, le PSCA, NY-ESO-1, WT-1, la survivine, gp100, PAP, la protéinase 3, SPAS-1, SP-17, PAGE-4, TARP, et l'ACE, ou comprend un polypeptide dérivé d'un antigène sélectionné dans le groupe consistant en K-Ras, H-Ras, N-Ras, 12-K-Ras, la mésothéline, le PSCA, NY-ESO-1, WT-1, la survivine, gp100, PAP, la protéinase 3, SPAS-1, SP-17, PAGE-4, TARP, et l'ACE.

11. Bactérie *Listeria* recombinante selon la revendication 10, où le polypeptide codé par le second polynucléotide comprend la mésothéline, ou un fragment antigénique ou variant antigénique de celle-ci.

12. Bactérie *Listeria* recombinante selon la revendication 11, où le polypeptide codé par le second polynucléotide comprend une mésothéline humaine dont le peptide signal et l'ancre GPI ont été supprimés.

13. Bactérie *Listeria* recombinante selon la revendication 1, qui est atténuée pour la propagation intercellulaire, la pénétration dans les cellules non phagocytaires, ou la prolifération.

14. Bactérie *Listeria* recombinante selon la revendication 1, qui est déficiente par rapport à ActA, l'internaline B, ou à la fois ActA et l'internaline B.

15. Bactérie *Listeria* recombinante selon la revendication 1, où l'acide nucléique de la bactérie recombinante a été modifié par une réaction avec un agent alkylant ciblant l'acide nucléique ou un psoralène de sorte que la prolifération de la bactérie soit atténuée.

16. Composition immunogène ou vaccin comprenant la bactérie *Listeria* recombinante selon la revendication 1, 10, 14, ou 15.

17. Bactérie *Listeria* recombinante selon la revendication 1, 10, 14, ou 15 destinée à être utilisée dans un procédé de traitement d'un hôte humain ou mammifère.

18. Bactérie *Listeria* recombinante destinée à être utilisée selon la revendication 17, où le traitement est celui d'un cancer ou d'une maladie infectieuse.

19. Bactérie *Listeria* recombinante destinée à être utilisée selon la revendication 17, où le traitement induit une réponse immunitaire chez un hôte contre un antigène, et où le polypeptide codé par le second polynucléotide comprend l'antigène.

20. Procédé de fabrication d'une bactérie *Listeria* recombinante comprenant une molécule d'acide nucléique recombinante, où la molécule d'acide nucléique recombinante comprend :
(a) un premier polynucléotide codant pour un peptide signal de sécrétion qui est un peptide signal natif de *Listeria* ; et
(b) une seconde séquence polynucléotidique codant pour un polypeptide hétérologue par rapport au peptide signal, où la seconde séquence polynucléotidique est dans le même cadre de lecture traductionnel que la première séquence polynucléotidique,
où la molécule d'acide nucléique recombinante code pour une protéine de fusion comprenant le peptide signal et le polypeptide ;
où ledit procédé comprend
(i) la mise à disposition de ladite molécule d'acide nucléique recombinante et l'optimisation en codons du premier polynucléotide afin de remplacer au moins un codon de la séquence codante native du polynucléotide par un codon qui est plus fréquemment utilisé par Listeria, et
(ii) l'expression de ladite molécule d'acide nucléique recombinante dans la bactérie Listeria.

21. Procédé selon la revendication 20, dans lequel la bactérie est telle que définie dans l'une quelconque des revendications 1 à 15.
